# EUROPEAN PATENT APPLICATION

(11) **EP 4 656 635 A1**
(43) Date of publication of application: **03.12.2025**
(21) Application number: 24747280.6
(22) Date of filing: 23.01.2024
(51) Int. Cl.: C07D 401/06, A61K 31/501, A61K 31/502, A61K 31/551, A61K 31/5025, A61P 1/00, A61P 1/04, A61P 1/16, A61P 3/10, A61P 9/04, A61P 9/10, A61P 9/14, A61P 11/00, A61P 11/06, A61P 13/10, A61P 13/12, A61P 17/00, A61P 17/06, A61P 19/02, A61P 21/00, A61P 25/00

(54) **SUBSTITUTED BENZENE COMPOUND**

(30) Priority: 24.01.2023 JP 2023008731
(71) Applicant: Daiichi Sankyo Company, Limited, Tokyo 103-8426 (JP)
(72) Inventor: KATAYAMA, Katsushi, Tokyo 103-8426 (JP); NAKAZAWA, Yusuke, Tokyo 103-8426 (JP); TSUCHIYA, Yuto, Tokyo 103-8426 (JP); KAWAI, Junya, Tokyo 103-8426 (JP); MATSUI, Satoshi, Tokyo 103-8426 (JP); FUJIMOTO, Teppei, Tokyo 103-8426 (JP); MURAKAMI, Keigo, Tokyo 103-8426 (JP); INOUE, Masahiro, Tokyo 103-8426 (JP); ISHIBASHI, Koutaro, Tokyo 103-8426 (JP)
(74) Representative: Marks & Clerk LLP
(86) International application number: PCT/JP2024/001755
(87) International publication number: WO 2024/157953

(57) **Abstract**

The present invention aims to provide a compound or a pharmaceutically acceptable salt thereof that has an inhibitory effect on NLRP3 inflammasome activation and is useful for the prevention and/or treatment of autoinflammatory diseases, autoimmune diseases, and/or inflammatory diseases, as a medicine. The solution of the present invention is a compound of the general formula (1) or a pharmaceutically acceptable salt thereof: 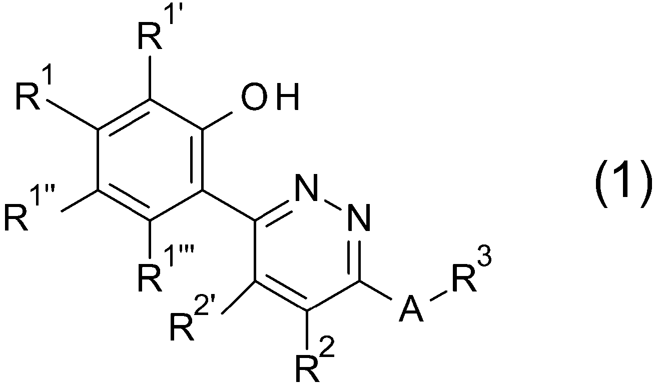 wherein the symbols in the formula are defined as follows: A is a carbon atom or the like, R¹ is a halogen atom or the like,
R¹' is a hydrogen atom or the like, R¹" is a hydrogen atom or the like, R¹‴ is a hydrogen atom or the like, R² is a hydrogen atom or the like, R²' is a hydrogen atom or the like, and R³ is a C1-C3 alkyl group or the like.

## Description

### [Technical Field]

The present invention relates to novel substituted benzene compounds or pharmaceutically acceptable salts thereof that have inhibitory effect on NLRP3 inflammasome activation, pharmaceutical compositions containing them as active ingredients, and methods for their production.

### [Background Art]

NLRP3 (NLR family pyrin domain containing 3) belongs to the NLR family (nucleotide-binding domain and leucine rich repeat containing family) and is a protein that contains a pyrin domain (PYD), a nucleotide-binding site (NACHT) domain, and a leucine-rich repeat (LRR) domain. NLRP3 functions as an intracellular sensor that responds to various inflammatory danger signals, and interacts with apoptosis-associated speck-like protein containing a CARD (ASC), procaspase-1 or the like to forms the NLRP3 inflammasome complex. Activation of the NLRP3 inflammasome induces activation of the inflammatory cytokines IL-1β and IL-18 and programmed cell death (pyroptosis).

Activation of the NLRP3 inflammasome and increased production of IL-1β and IL-18 by the NLRP3 inflammasome have been reported in a wide range of autoinflammatory, autoimmune and inflammatory diseases.

For example, it has been suggested that there is an association with the following diseases:
autoinflammatory diseases such as cryopyrin-associated periodic syndrome (CAPS),
autoimmune diseases such as systemic lupus erythematosus (SLE), rheumatoid arthritis, psoriasis, systemic scleroderma (SSc), vasculitis syndrome, systemic amyloidosis and the like, and
inflammatory diseases such as
liver-related diseases (e.g., alcoholic liver disease (ALD), alcoholic steatohepatitis (ASH), non-alcoholic steatohepatitis (NASH), viral hepatitis, non-alcoholic fatty liver disease (NAFLD), etc.),
lung-related diseases (e.g., chronic obstructive pulmonary diseases (COPD), asthma, acute lung injury (ALI), interstitial lung disease (ILD), sarcoidosis, etc.),
renal and urinary-related diseases (e.g., diabetic nephropathy, lupus nephritis, primary hyperoxaluria, interstitial cystitis, etc.),
bowel-related diseases (e.g., inflammatory bowel disease (IBD), etc.),
joint-related diseases (e.g., gout, pseudogout, osteoarthritis, etc.),
cardiovascular and metabolic-related diseases (e.g., atherosclerosis, diabetes type 1/type 2, heart failure, etc.),
infection-related diseases (e.g., SARS-CoV-2 infection, EBV infection, viral encephalitis, etc.),
eye-related diseases (e.g., glaucoma, optic neuritis, retinitis pigmentosa, age-related macular degeneration, etc.),
cancer-related diseases (e.g., myeloproliferative neoplasms, leukemia, myelodysplastic syndrome, myelofibrosis, lung cancer, colorectal cancer, tumor lysis syndrome, cytokine release syndrome, etc.),
neuro-related diseases (e.g., multiple sclerosis (including relapsing remitting multiple sclerosis, secondary progressive multiple sclerosis and primary progressive multiple sclerosis as subtypes of multiple sclerosis), neurodegenerative diseases (Alzheimer's disease, Huntington's disease, Parkinson's disease, amyotrophic lateral sclerosis, multiple system atrophy, frontotemporal dementia), etc.),
psychiatric disorders (e.g., epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, etc.), and
cerebrovascular diseases (e.g., ischemic stroke, hemorrhagic stroke, traumatic brain injury, etc.).

Therefore, inhibitors of the NLRP3 inflammasome pathway are needed as new therapeutic agents for these inflammasome-related diseases, and several NLRP3 inflammasome inhibitors have been discovered so far.

For example, the low molecular weight compound MCC-950 has strong NLRP3 inflammasome inhibitory activity and excellent pharmacokinetics, and has been shown to have pharmacological effects such as suppression of IL-1β production and improvement of pathology in various pathological model animals (Non-Patent Literature 1, Non-Patent Literature 2, Non-Patent Literature 3).

Thus, since the NLRP3 inflammasome is activated in various autoinflammatory, autoimmune, and/or inflammatory diseases, and NLRP3 inhibitory compounds have shown efficacy in various disease model animals, it has been suggested that NLRP3 inhibitory compounds may be therapeutic agents for NLRP3 inflammasome-related diseases. In fact, clinical trials are currently being conducted on several NLRP3 inhibitory compounds, including MCC-950 analogs.

Patent Literature 1 describes the following compound. It is described that the compound has an inhibitory effect on NLRP3 inflammasome activation and is effective in treating inflammatory diseases, and neurodegenerative diseases, and the like.

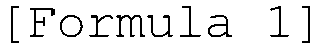

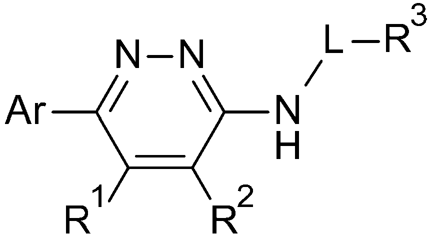

Patent Literature 2 describes the following compound. It is described that the compound has an inhibitory effect on NLRP3 inflammasome activation and is effective in treating NLRP3-related diseases, and the like.

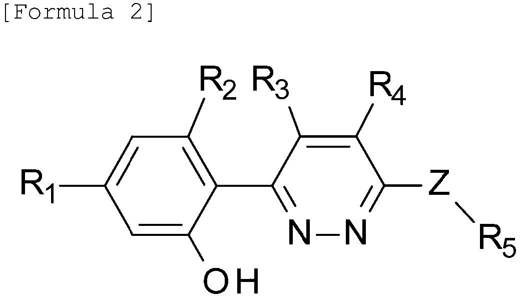

Patent Literature 3 describes the following compound. It is described that the compound has an inhibitory effect on NLRP3 inflammasome activation and is effective in treating NLRP3-related diseases, and the like.

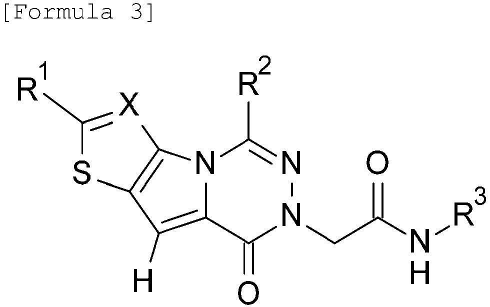

Patent Literature 4 describes the following compound. It is described that the compound has an inhibitory effect on NLRP3 inflammasome activation and is effective in treating NLRP3-related diseases, and the like.

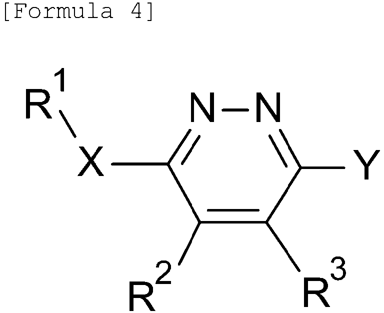

Patent Literature 5 describes the following compound. It is described that the compound has an inhibitory effect on NLRP3 inflammasome activation and is effective in treating NLRP3-related diseases, and the like.

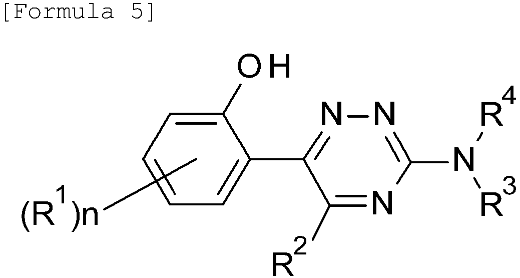

### [Citation List]

### [Patent Literature]

[Patent Literature 1]
   WO 2021/193897
[Patent Literature 2]
   WO 2020/234715
[Patent Literature 3]
   WO 2020/021447
[Patent Literature 4]
   US11,319,319
[Patent Literature 5]
   WO 2022/230912

### [Non-Patent Literature]

[Non-Patent Literature 1]
   Coll et al., Nat. Med. 2015, 21, 248-255
[Non-Patent Literature 2]
   Perera et al., Sci. Rep. 2018, 8, 8618
[Non-Patent Literature 3]
   Zeng et al., Sci. Rep. 2021, 11, 19305

### [Summary of Invention]

### [Technical Problem]

The present invention aims to provide a novel compound or a pharmaceutically acceptable salt thereof that has an excellent inhibitory effect on NLRP3 inflammasome activation and has also excellent pharmacokinetics, particularly excellent central transferability, and a medicament using the same.

### [Solution to Problem]

The present inventors have conducted intensive research to solve the above-mentioned problems and have completed the invention described below.

A preferred compound or a pharmaceutically acceptable salt thereof is described as [1].

More preferred are [2] and below, and particularly preferred are [8], [9], [10], [11] and the like.

[1] A compound of the general formula (1) or a pharmaceutically acceptable salt thereof: 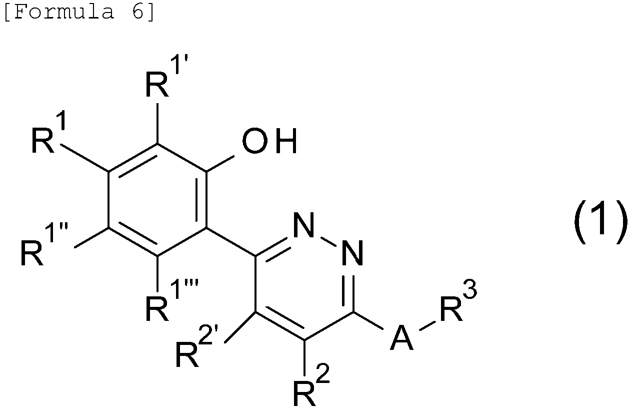 wherein
   A is a single bond, a nitrogen atom, or a carbon atom,
   R¹ is a halogen atom, a cyano group, a C1-C3 alkyl group optionally substituted with 1 to 3 halogen atoms, or a C1-C3 alkoxy group optionally substituted with 1 to 3 fluorine atoms, R^{1'} is a hydrogen atom, a halogen atom, or a C1-C3 alkyl group, or
   R¹ and R^{1'} are bonded to each other to form
   a C1-C4 alkylene group optionally substituted with 1 to 3 substituents selected from substituent group a, or -NH-(CH₂)n-(n is an integer of 1, 2 or 3) which is optionally substituted with 1 to 3 substituents selected from substituent group a, thereby constituting a ring structure,
   R^{1"} is a hydrogen atom, or a halogen atom,
   R^{1‴} is a hydrogen atom, a halogen atom, a cyano group, or - NH-(CH₂)n- (n is an integer of 1, 2 or 3),
   when A is a nitrogen atom,
   R² and A are bonded to each other to form any partial structure selected from the group shown below: 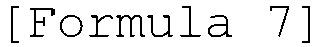 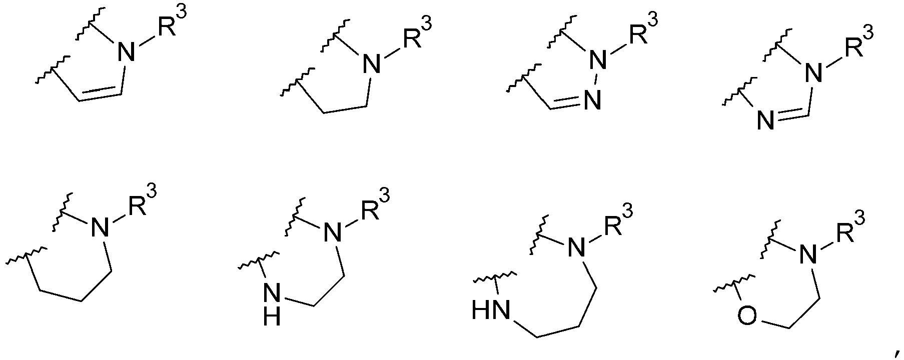
   wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group a,
   when A is a single bond, or a carbon atom,
   R² is a hydrogen atom, or a C1-C3 alkyl group optionally substituted with 1 to 3 substituents selected from substituent group a, or
   R² and A are bonded to each other to form any partial structure selected from the group shown below: 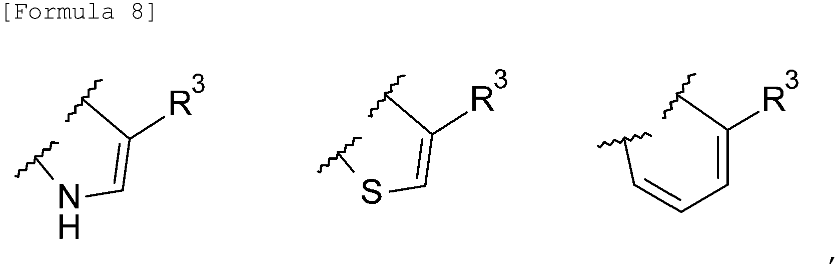
   wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group a,
   R^{2'} is a hydrogen atom, or a C1-C3 alkyl group, or
   R^{2'} and R² are bonded to each other to form -NH-(CH₂)n- (n is an integer of 1, 2 or 3), thereby constituting a ring structure,
   R³ is
   a C1-C3 alkyl group optionally substituted with 1 to 3 substituents selected from substituent group b,
   a C2-C3 alkenyl group optionally substituted with 1 to 3 substituents selected from substituent group b,
   a C3-C6 cycloalkyl group optionally substituted with 1 to 3 substituents selected from substituent group b,
   a C5-C8 bicycloalkyl group optionally substituted with 1 to 3 substituents selected from substituent group b,
   a C5-C8 azabicycloalkyl group optionally substituted with 1 to 3 substituents selected from substituent group b,
   a C5-C8 oxabicycloalkyl group optionally substituted with 1 to 3 substituents selected from substituent group b,
   a 3-6-membered nitrogen-containing saturated heterocyclic group optionally substituted with 1 to 3 substituents selected from substituent group b, or
   a 5-6-membered nitrogen-containing aromatic heterocyclic group optionally substituted with 1 to 3 substituents selected from substituent group b,
   the substituent group a consists of
   a halogen atom, a cyano group, a C2-C3 alkenyl group, a C3-C6 cycloalkyl group, a C1-C3 alkyl group optionally substituted with 1 to 3 fluorine atoms, a C1-C3 alkoxy group optionally substituted with 1 to 3 fluorine atoms, a C1-C4 alkylene group, a C2-C4 alkanediyl group (including a 1,3-butadiene-1,4-diyl group), a C1-C3 oxyalkylene group, and a cyclopropylacethylene group, and
   the substituent group b consists of
   a hydroxyl group, a halogen atom, a hydroxy C1-C4 alkyl group, a C3-C6 cycloalkyl group, a C3-C6 cycloalkylmethyl group, a C1-C3 alkoxy group, a C1-C3 alkyl group optionally substituted with 1 to 3 fluorine atoms, and a 5-membered oxygen-containing saturated heterocyclic group.
[2] A compound of the general formula (1a) or a pharmaceutically acceptable salt thereof: 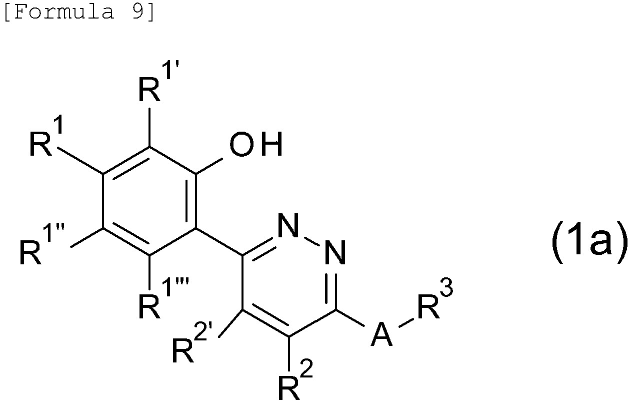 wherein
   A is a nitrogen atom,
   R¹ is a halogen atom, a cyano group, a C1-C3 alkyl group optionally substituted with 1 to 3 fluorine atoms, or a C1-C3 alkoxy group optionally substituted with 1 to 3 fluorine atoms, R^{1'} is a hydrogen atom, a halogen atom, or a C1-C3 alkyl group, or
   R¹ and R^{1'} are bonded to each other to form a C1-C4 alkylene group optionally substituted with 1 to 3 substituents selected from substituent group a', thereby constituting a ring structure,
   R^{1"} is a hydrogen atom, or a halogen atom,
   R^{1‴} is a hydrogen atom, a halogen atom, a cyano group, or a methyl group,
   R² and A are bonded to each other to form any partial structure selected from the group shown below: 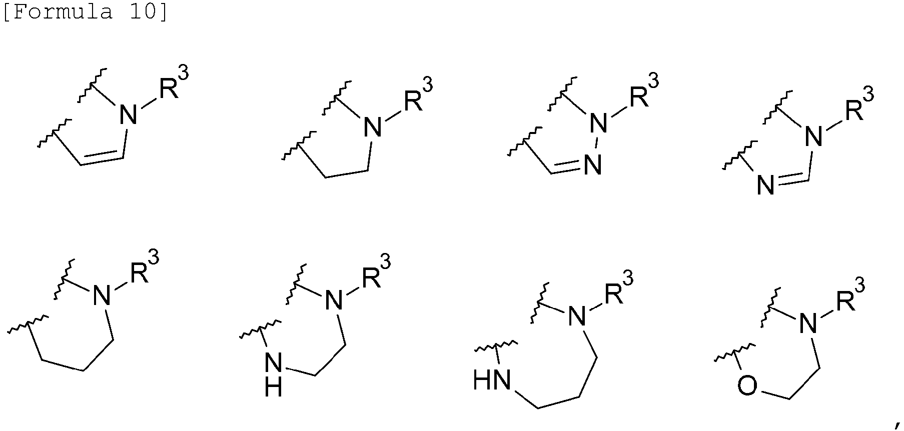
   wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group a',
   R^{2'} is a hydrogen atom, or a C1-C3 alkyl group, or
   R^{2'} and R² are bonded to each other to form -NH-(CH₂)n- (n is an integer of 1, 2 or 3), thereby constituting a ring structure,
   R³ is
   a C1-C3 alkyl group optionally substituted with 1 to 3 substituents selected from substituent group b',
   a C3-C6 cycloalkyl group optionally substituted with 1 to 3 substituents selected from substituent group b',
   a C5-C8 bicycloalkyl group optionally substituted with 1 to 3 substituents selected from substituent group b',
   a C5-C8 azabicycloalkyl group optionally substituted with 1 to 3 substituents selected from substituent group b',
   a C5-C8 oxabicycloalkyl group optionally substituted with 1 to 3 substituents selected from substituent group b',
   a 3-6-membered nitrogen-containing saturated heterocyclic group optionally substituted with 1 to 3 substituents selected from substituent group b', or
   a 5-6-membered nitrogen-containing aromatic heterocyclic group optionally substituted with 1 to 3 substituents selected from substituent group b',
   the substituent group a' consists of
   a halogen atom, a cyano group, a C3-C6 cycloalkyl group, a C1-C3 alkyl group optionally substituted with 1 to 3 fluorine atoms, a C1-C3 alkoxy group optionally substituted with 1 to 3 fluorine atoms, a C1-C4 alkylene group, a C2-C4 alkanediyl group (including a 1,3-butadiene-1,4-diyl group), a C1-C3 oxyalkylene group, and a cyclopropylacethylene group, and the substituent group b' consists of
   a hydroxyl group, a halogen atom, a C1-C3 alkyl group, a hydroxy C1-C3 alkyl group, a C3-C6 cycloalkyl group, a C1-C3 alkoxy group, and a 5-membered oxygen-containing saturated heterocyclic group.
[3] A compound of the general formula (1b) or a pharmaceutically acceptable salt thereof: 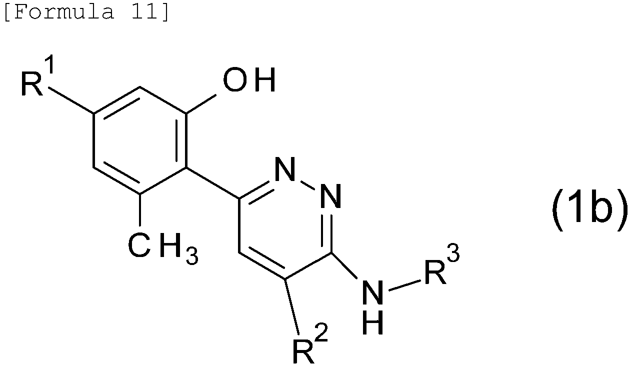 wherein
   R¹ is a halogen atom, a C1-C3 alkyl group optionally substituted with 1 to 3 fluorine atoms, or a C1-C3 alkoxy group optionally substituted with 1 to 3 fluorine atoms,
   R² and the nitrogen atom are bonded to each other to form any partial structure selected from the group shown below: 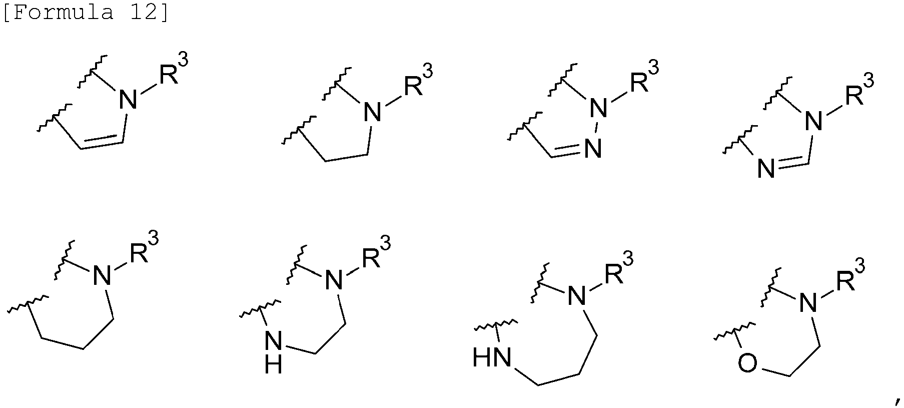
   wherein the left end of the partial structure is a bond to R², and the right end of the partial structure is a bond to the nitrogen atom, and for convenience, the partial structure is shown as being substituted with R³, and optionally, the partial structure may be substituted with 1 to 3 substituents selected from substituent group a'',
   R³ is
   a C3-C6 cycloalkyl group optionally substituted with 1 to 3 substituents selected from substituent group b'',
   a C6-C7 bicycloalkyl group optionally substituted with 1 to 3 substituents selected from substituent group b'',
   a 3-6-membered nitrogen-containing saturated heterocyclic group optionally substituted with 1 to 3 substituents selected from substituent group b'', or
   a 5-6-membered nitrogen-containing aromatic heterocyclic group optionally substituted with 1 to 3 substituents selected from substituent group b",
   the substituent group a'' consists of
   a halogen atom, a cyano group, a C1-C3 alkyl group, a C1-C3 alkoxy group, a C1-C3 alkyl group optionally substituted with 1 to 3 fluorine atoms, a C1-C3 alkoxy group optionally substituted with 1 to 3 fluorine atoms, a C1-C4 alkylene group, and a C1-C3 oxyalkylene group, and
   the substituent group b'' consists of
   a hydroxyl group, a halogen atom, and a C1-C3 alkyl group.
[4] The compound according to [3], or a pharmaceutically acceptable salt thereof, wherein R¹ is a trifluoromethyl group, a chlorine atom, a difluoromethoxy group, or a trifluoromethoxy group.
[5] The compound according to [3] or [4], or a pharmaceutically acceptable salt thereof, wherein R² and the nitrogen atom are bonded to each other to form any partial structure selected from the group shown below: 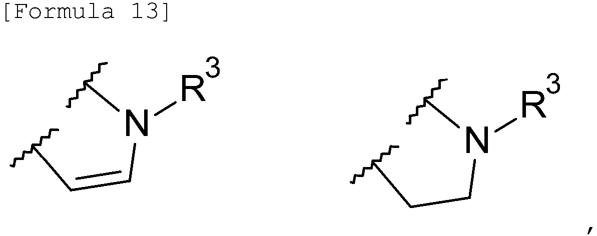 wherein the left end of the partial structure is a bond to R², and the right end of the partial structure is a bond to the nitrogen atom, and for convenience, the partial structure is shown as being substituted with R³, and optionally, the partial structure may be substituted with 1 to 3 substituents selected from substituent group a''.
[6] The compound according to any of [3] to [5], or a pharmaceutically acceptable salt thereof, wherein the partial structure may be substituted with 1 to 3 substituents selected from the group consisting of a cyano group, a fluorine atom, a methyl group, a methoxy group, a difluoromethoxy group, a propylene group, a 1-oxyethylene group, and a 2-oxyethylene group.
[7] The compound according to any of [3] to [6], or a pharmaceutically acceptable salt thereof, wherein R³ is any substituent selected from the following substituent group: 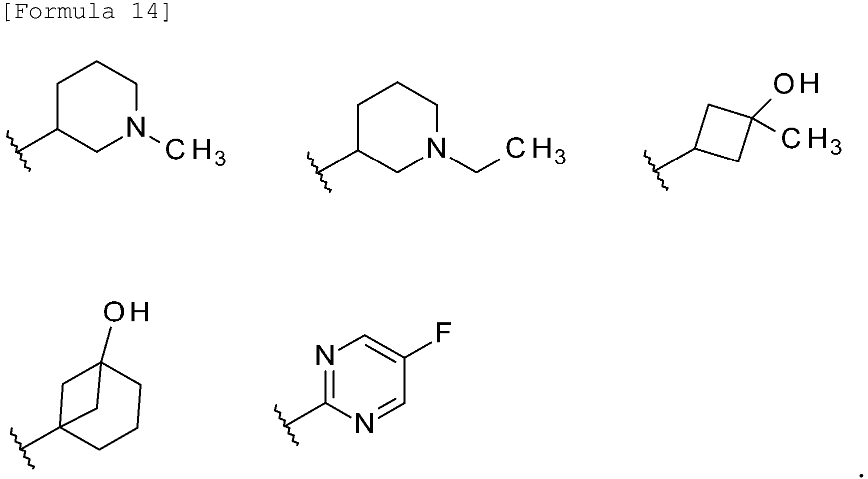
[8] Any compound selected from the following group, or a pharmaceutically acceptable salt thereof:
   3-methyl-2-{7-[(3R)-1-methylpiperidin-3-yl]-6,7-dihydro-5H-pyrrolo[2,3-c]pyridazin-3-yl}-5-(trifluoromethyl)phenol,
   3-methyl-2-{7-[(3R)-1-methylpiperidin-3-yl]-7H-pyrrolo[2,3-c]pyridazin-3-yl}-5-(trifluoromethyl)phenol,
   2-{7-[(1s,3s)-3-hydroxy-3-methylcyclobutyl]-7H-pyrrolo[2,3-c]pyridazin-3-yl}-3-methyl-5-(trifluoromethyl)phenol,
   2-{7-[(1s,3s)-3-hydroxy-3-methylcyclobutyl]-5-methyl-7H-pyrrolo[2,3-c]pyridazin-3-yl}-3-methyl-5-(trifluoromethyl)phenol,
   2-{5-fluoro-7-[(1s,3s)-3-hydroxy-3-methylcyclobutyl]-7H-pyrrolo[2,3-c]pyridazin-3-yl}-3-methyl-5-(trifluoromethyl)phenol,
   5-chloro-2-{7-[(1s,3s)-3-hydroxy-3-methylcyclobutyl]-5-methyl-7H-pyrrolo[2,3-c]pyridazin-3-yl}-3-methylphenol,
   2-{7-[(1s,3s)-3-hydroxy-3-methylcyclobutyl]-5-methoxy-7H-pyrrolo[2,3-c]pyridazin-3-yl}-3-methyl-5-(trifluoromethyl)phenol,
   2-{7-[(3R)-1-ethylpiperidin-3-yl]-6,7-dihydro-5H-pyrrolo[2,3-c]pyridazin-3-yl}-3-methyl-5-(trifluoromethyl)phenol,
   2-{8-[(1s,3s)-3-hydroxy-3-methylcyclobutyl]-5,6,7,8-tetrahydrocyclopenta[4,5]pyrrolo[2,3-c]pyridazin-3-yl}-3-methyl-5-(trifluoromethyl)phenol,
   2-{5-(difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol,
   5-(difluoromethoxy)-2-{7-[(3R)-1-ethylpiperidin-3-yl]-6,7-dihydro-5H-pyrrolo[2,3-c]pyridazin-3-yl}-3-methylphenol,
   2-{7-[(3R)-1-ethylpiperidin-3-yl]-6,7-dihydro-5H-pyrrolo[2,3-c]pyridazin-3-yl}-3-methyl-5-(trifluoromethoxy)phenol,
   5-{3-[2-hydroxy-6-methyl-4-(trifluoromethyl)phenyl]-5-methoxy-7H-pyrrolo[2,3-c]pyridazin-7-yl}bicyclo[3.1.1]heptan-1-ol,
   5-chloro-2-{5-fluoro-7-[(1s,3s)-3-hydroxy-3-methylcyclobutyl]-7H-pyrrolo[2,3-c]pyridazin-3-yl}-3-methylphenol,
   5-chloro-2-{7-[(1s,3s)-3-hydroxy-3-methylcyclobutyl]-5-methoxy-7H-pyrrolo[2,3-c]pyridazin-3-yl}-3-methylphenol,
   5-chloro-2-{5-(difluoromethoxy)-7-[(1s,3s)-3-hydroxy-3-methylcyclobutyl]-7H-pyrrolo[2,3-c]pyridazin-3-yl}-3-methylphenol,
   2-{8-[(1s,3s)-3-hydroxy-3-methylcyclobutyl]-7,8-dihydro-6H-furo[2',3':4,5]pyrrolo[2,3-c]pyridazin-3-yl}-3-methyl-5-(trifluoromethyl)phenol,
   7-[(1s,3s)-3-hydroxy-3-methylcyclobutyl]-3-[2-hydroxy-6-methyl-4-(trifluoromethyl)phenyl]-7H-pyrrolo[2,3-c]pyridazine-5-carbonitrile,
   2-[7-(5-fluoropyrimidin-2-yl)-7H-pyrrolo[2,3-c]pyridazin-3-yl]-3-methyl-5-(trifluoromethyl)phenol,
   2-[(4bR,7aR)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol,
   2-[(4bS,7aS)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, and
   2-{8-[(1s,3s)-3-hydroxy-3-methylcyclobutyl]-7,8-dihydro-5H-furo[3',4':4,5]pyrrolo[2,3-c]pyridazin-3-yl}-3-methyl-5-(trifluoromethyl)phenol.
[9]
   2-{5-Fluoro-7-[(1s,3s)-3-hydroxy-3-methylcyclobutyl]-7H-pyrrolo[2,3-c]pyridazin-3-yl}-3-methyl-5-(trifluoromethyl)phenol,
   2-{5-(difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol,
   2-{8-[(1s,3s)-3-hydroxy-3-methylcyclobutyl]-7,8-dihydro-6H-furo[2',3':4,5]pyrrolo[2,3-c]pyridazin-3-yl}-3-methyl-5-(trifluoromethyl)phenol,
   2-[(4bR,7aR)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol,
   2-[(4bS,7aS)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, or
   2-{8-[(1s,3s)-3-hydroxy-3-methylcyclobutyl]-7,8-dihydro-5H-furo[3',4':4,5]pyrrolo[2,3-c]pyridazin-3-yl}-3-methyl-5-(trifluoromethyl)phenol, or a pharmaceutically acceptable salt thereof.
[10] 2-{5-Fluoro-7-[(1s,3s)-3-hydroxy-3-methylcyclobutyl]-7H-pyrrolo[2,3-c]pyridazin-3-yl}-3-methyl-5-(trifluoromethyl)phenol, or a pharmaceutically acceptable salt thereof.
[11] 2-{5-(Difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol, or a pharmaceutically acceptable salt thereof.
[12] 2-{8-[(1s,3s)-3-Hydroxy-3-methylcyclobutyl]-7,8-dihydro-6H-furo[2',3':4,5]pyrrolo[2,3-c]pyridazin-3-yl}-3-methyl-5-(trifluoromethyl)phenol, or a pharmaceutically acceptable salt thereof.
[13] 2-[(4bR,7aR)-8-(5-Fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, or a pharmaceutically acceptable salt thereof.
[14] 2-[(4bS,7aS)-8-(5-Fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, or a pharmaceutically acceptable salt thereof.
[15] 2-{8-[(1s,3s)-3-Hydroxy-3-methylcyclobutyl]-7,8-dihydro-5H-furo[3',4':4,5]pyrrolo[2,3-c]pyridazin-3-yl}-3-methyl-5-(trifluoromethyl)phenol, or a pharmaceutically acceptable salt thereof.
[16] A compound of the general formula (1c) or a pharmaceutically acceptable salt thereof: 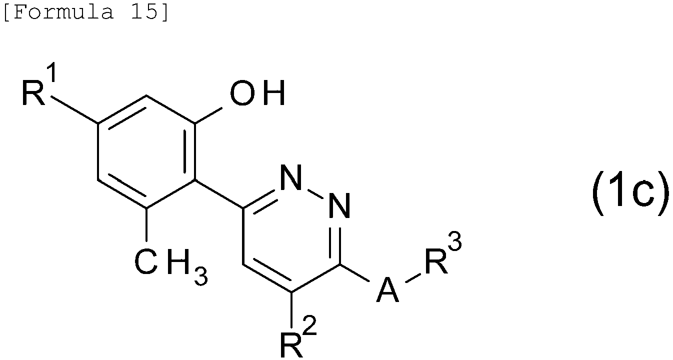 wherein
   A is a single bond, or a carbon atom,
   R¹ is a halogen atom, a C1-C3 alkyl group optionally substituted with 1 to 3 fluorine atoms, or a C1-C3 alkoxy group optionally substituted with 1 to 3 fluorine atoms,
   R² is a hydrogen atom, or a C1-C3 alkyl group optionally substituted with 1 to 3 substituents selected from substituent group c, or
   R² and A are bonded to each other to form any partial structure selected from the group shown below: 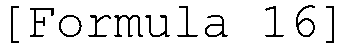 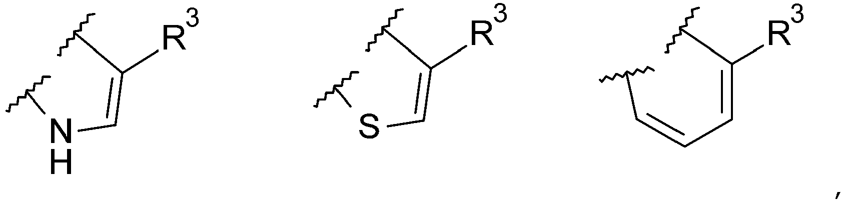
   wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group c,
   R³ is
   a C3-C6 cycloalkyl group optionally substituted with 1 to 3 substituents selected from substituent group d,
   a C6-C7 bicycloalkyl group optionally substituted with 1 to 3 substituents selected from substituent group d, or
   a 3-6-membered nitrogen-containing saturated heterocyclic group optionally substituted with 1 to 3 substituents selected from substituent group d,
   the substituent group c consists of
   a halogen atom, a C1-C3 alkyl group optionally substituted with 1 to 3 fluorine atoms, a C1-C3 alkoxy group optionally substituted with 1 to 3 fluorine atoms, a C1-C4 alkylene group, and a C1-C3 oxyalkylene group, and
   the substituent group d consists of
   a hydroxyl group, and a C1-C3 alkyl group.
[17] The compound according to [16], or a pharmaceutically acceptable salt thereof, wherein R¹ is a trifluoromethyl group, a chlorine atom, a difluoromethoxy group, or a trifluoromethoxy group.
[18] The compound according to [16], or a pharmaceutically acceptable salt thereof, wherein R¹ is a trifluoromethyl group.
[19] The compound according to any of [16] to [18], or a pharmaceutically acceptable salt thereof, wherein R² is a hydrogen atom.
[20] The compound according to any of [16] to [18], or a pharmaceutically acceptable salt thereof, wherein R² and A are bonded to each other to form any partial structure selected from the group shown below: 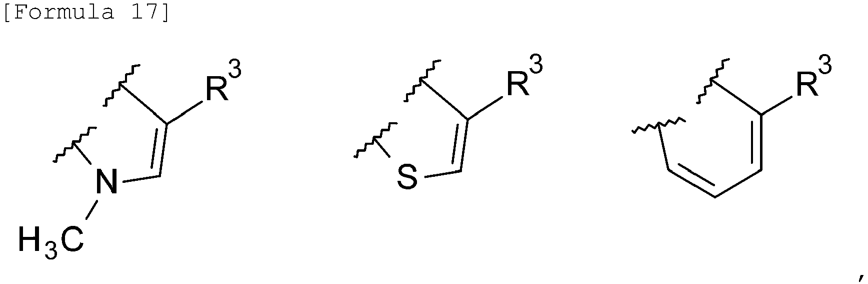 wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group c.
[21] The compound according to any of [16] to [20], or a pharmaceutically acceptable salt thereof, wherein R³ is any substituent selected from the following substituents: 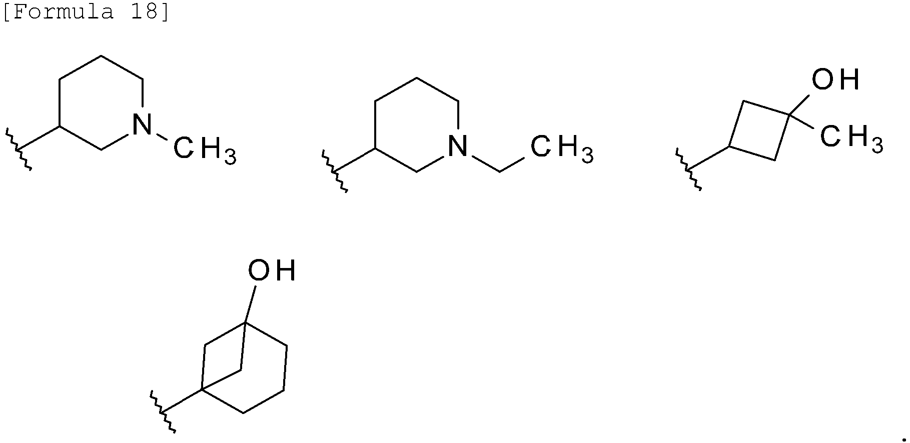
[22] Any compound selected from the following group, or a pharmaceutically acceptable salt thereof:
   3-methyl-2-{6-[(1-methylpiperidin-3-yl)methyl]pyridazin-3-yl}-5-(trifluoromethyl)phenol,
   3-methyl-2-[5-methyl-7-(1-methylpiperidin-3-yl)-5H-pyrrolo[3,2-c]pyridazin-3-yl]-5-(trifluoromethyl)phenol,
   3-methyl-2-[7-(1-methylpiperidin-3-yl)thieno[3,2-c]pyridazin-3-yl]-5-(trifluoromethyl)phenol,
   3-methyl-2-{8-[(3R)-1-methylpiperidin-3-yl]cinnolin-3-yl}-5-(trifluoromethyl)phenol, and
   2-{8-[(3R)-1-ethylpiperidin-3-yl]cinnolin-3-yl}-3-methyl-5-(trifluoromethyl)phenol.
[23] A pharmaceutical composition comprising the compound according to any of [1] to [22], or a pharmaceutically acceptable salt thereof, as an active ingredient.
[24] The pharmaceutical composition according to [23], for inhibiting NLRP3 inflammasome activation.
[25] The pharmaceutical composition according to [23], for suppressing inflammatory cytokine production mediated by NLRP3 inflammasome activation.
[26] The pharmaceutical composition according to [23], for suppressing inflammation reaction mediated by NLRP3 inflammasome activation.
[27] The pharmaceutical composition according to [23], for treating an autoinflammatory disease, an autoimmune disease, and/or an inflammatory disease.
[28] The pharmaceutical composition according to [27], wherein the autoinflammatory disease, autoimmune disease, and/or inflammatory disease is a disease selected from the diseases listed below:
   cryopyrin-associated periodic syndrome (CAPS), systemic lupus erythematosus (SLE), rheumatoid arthritis, psoriasis, systemic scleroderma (SSc), vasculitis syndrome, systemic amyloidosis, alcoholic liver disease (ALD), alcoholic steatohepatitis (ASH), non-alcoholic steatohepatitis (NASH), viral hepatitis, non-alcoholic fatty liver disease (NAFLD), chronic obstructive pulmonary diseases (COPD), asthma, acute lung injury (ALI), interstitial lung disease (ILD), sarcoidosis, diabetic nephropathy, lupus nephritis, primary hyperoxaluria, interstitial cystitis, inflammatory bowel disease (IBD), gout, pseudogout, osteoarthritis, atherosclerosis, diabetes type 1/type 2, heart failure, SARS-CoV-2 infection, EBV infection, viral encephalitis, glaucoma, optic neuritis, retinitis pigmentosa, age-related macular degeneration, myeloproliferative neoplasms, leukemia, myelodysplastic syndrome, myelofibrosis, lung cancer, colorectal cancer, tumor lysis syndrome, cytokine release syndrome, multiple sclerosis (including relapsing remitting multiple sclerosis, secondary progressive multiple sclerosis and primary progressive multiple sclerosis as subtypes of multiple sclerosis), Alzheimer's disease, Huntington's disease, Parkinson's disease, amyotrophic lateral sclerosis, multiple system atrophy, frontotemporal dementia, epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, ischemic stroke, hemorrhagic stroke, and traumatic brain injury.
[29] The pharmaceutical composition according to [23], for treating multiple sclerosis, Alzheimer's disease, Huntington's disease, Parkinson's disease, amyotrophic lateral sclerosis, multiple system atrophy, frontotemporal dementia, epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, ischemic stroke, hemorrhagic stroke, or traumatic brain injury.
[30] The pharmaceutical composition according to [23], for treating multiple sclerosis, Alzheimer's disease, Parkinson's disease, multiple system atrophy, major depressive disorder, schizophrenia, or autism spectrum disorder.
[31] The pharmaceutical composition according to [23], for treating multiple sclerosis.
[32] The pharmaceutical composition according to [23], for treating Alzheimer's disease.
[33] The pharmaceutical composition according to [23], for treating Parkinson's disease.
[34] The pharmaceutical composition according to [23], for treating multiple system atrophy.
[35] The pharmaceutical composition according to [23], for treating major depressive disorder.
[36] The pharmaceutical composition according to [23], for treating schizophrenia.
[37] The pharmaceutical composition according to [23], for treating autism spectrum disorder.
[38] Use of the compound according to any of [1] to [22], or a pharmaceutically acceptable salt thereof, for the manufacture of a pharmaceutical composition.
[39] The use according to [23], wherein the pharmaceutical composition is a pharmaceutical composition for treating an autoinflammatory disease, an autoimmune disease, and/or an inflammatory disease.
[40] A formulation intended for administration to a mammal, for treating an autoinflammatory disease, an autoimmune disease, and/or an inflammatory disease, comprising an effective amount of the compound according to any of [1] to [22], or a pharmaceutically acceptable salt thereof.
[41] A method for treating an autoinflammatory disease, an autoimmune disease, and/or an inflammatory disease with the compound according to any of [1] to [22], or a pharmaceutically acceptable salt thereof.

### [Advantageous Effects of Invention]

The present invention can provide a novel compound or a pharmaceutically acceptable salt thereof that has an excellent inhibitory effect on NLRP3 inflammasome activation and has also unexpectedly excellent pharmacokinetics, particularly excellent central transferability.

### [Description of Embodiments]

The definitions of the terms and symbols used in the present specification are explained below. Unless otherwise defined, all technical and scientific terms used in the present specification have the same meaning as commonly understood by those skilled in the art to which the present invention belongs.

A "halogen atom" means a fluorine atom, a chlorine atom, a bromine atom, or an iodine atom.

A "carbon atom" is a carbon atom having four bonds, to which some substituent is usually attached. In the present specification, a hydrogen atom is assumed to be attached to a bond unless it is explicitly stated that a substituent is attached to the bond.

A "nitrogen atom" is a nitrogen atom having three bonds, to which some substituent is usually attached. In the present specification, a hydrogen atom is assumed to be attached to a bond unless it is explicitly stated that a substituent is attached to the bond.

A "C1-C3 alkyl group" means a linear or branched alkyl group having 1 to 3 carbon atoms. Examples of the C1-C3 alkyl group include a methyl group, an ethyl group, an n-propyl group, an isopropyl group and the like.

A "hydroxy C1-C4 alkoxy group" means a group in which a hydroxyl group is bonded to a "C1-C4 alkyl group". Examples of the hydroxy C1-C4 alkoxy group include a hydroxy-methyl group, a 2-hydroxy-ethyl group, a 1-hydroxy-ethyl group, a 3-hydroxy-n-propyl group, a 2-hydroxy-isopropyl group, a 2-hydroxy-2,2-dimethyl-ethyl group and the like.

A "C1-C3 alkoxy group" means a group in which the above-mentioned "C1-C3 alkyl group" is bonded to an oxygen atom. Examples of the C1-C3 alkoxy group include a methoxy group, an ethoxy group, an n-propoxy group, an isopropoxy group and the like.

A "C1-C4 alkylene group" means a linear or branched alkylene group having 1 to 4 carbon atoms. Examples of the C1-C4 alkylene group include a methylene group, a 1,2-ethylene group, a 1,3-propylene group, a 1,2-isopropylene group, a 1,4-butylene group, a 1,3-isobutylene group and the like.

"-NH-(CH₂)n- (n is an integer of 1, 2 or 3)" means a group in which a "C1-C3 alkylene group" is bonded to an amino group. Examples of the -NH-(CH₂)n- (n is an integer of 1, 2 or 3) include an amino-methylene group, a 1-amino-1,2-ethylene group, a 1-amino-1,3-propylene group, a 1-amino-1,2-isopropylene group, a 1-amino-1,4butylene group, a 1-amino-1,3-isobutylene group and the like.

A "C1-C3 oxyalkylene group" means a group in which a "C1-C3 alkylene group" is bonded to an oxygen atom. Examples of the C1-C3 oxyalkylene group include an oxy-methylene group, a 1-oxy-1,2-ethylene group, a 2-oxy-1,3-ethylene group, a 1-oxy-1,3-propylene group, a 1-oxy-1,2-isopropylene group, a 1-oxy-1,4butylene group, a 1-oxy-1,3-isobutylene group and the like.

A "C2-C4 alkanediyl group (including a 1,3-butadiene-1,4-diyl group)" means an alkanediyl group having 2 to 4 carbon atoms. Examples of the C2-C4 alkanediyl group include a 1,2-vinylene group, a 1,3-butadiene-1,4-diyl group and the like.

A "C3-C6 cycloalkyl group" means a 3- to 6-membered monocyclic saturated hydrocarbon ring group, and examples thereof include a cyclopropyl group, a cyclobutyl group, a cyclopentyl group, and a cyclohexyl group.

A "C3-C6 cycloalkyl methyl group" means a group in which the above-mentioned "C3-C6 cycloalkyl group" is bonded to a methyl group, and examples thereof include a cyclopropylmethyl group, a cyclobutylmethyl group, a cyclopentylmethyl group, and a cyclohexylmethyl group.

A "C5-C8 bicycloalkyl group" means a bicycloalkyl group having 5 to 8 carbon atoms, and examples thereof include the following groups:

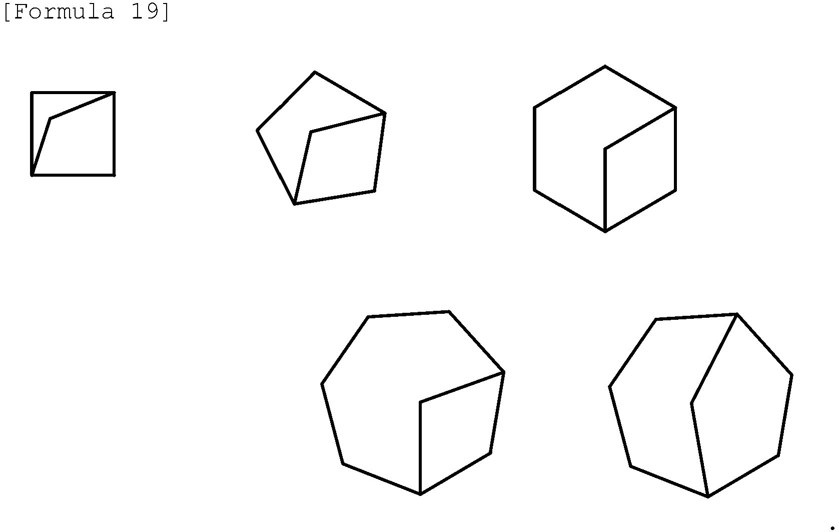

A "C5-C8 azabicycloalkyl group" means a group in which one of the carbon atoms of the above-mentioned "C5-C8 bicycloalkyl group" is replaced with a nitrogen atom, and examples thereof include the following groups:

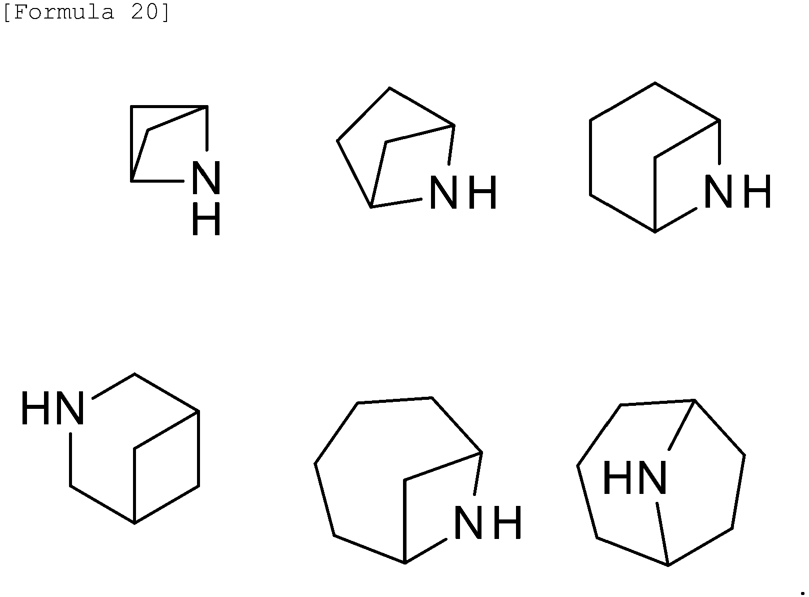

A "C5-C8 oxabicycloalkyl group" means a group in which one of the carbon atoms of the above-mentioned "C5-C8 bicycloalkyl group" is replaced with an oxygen atom, and examples thereof include the following groups:

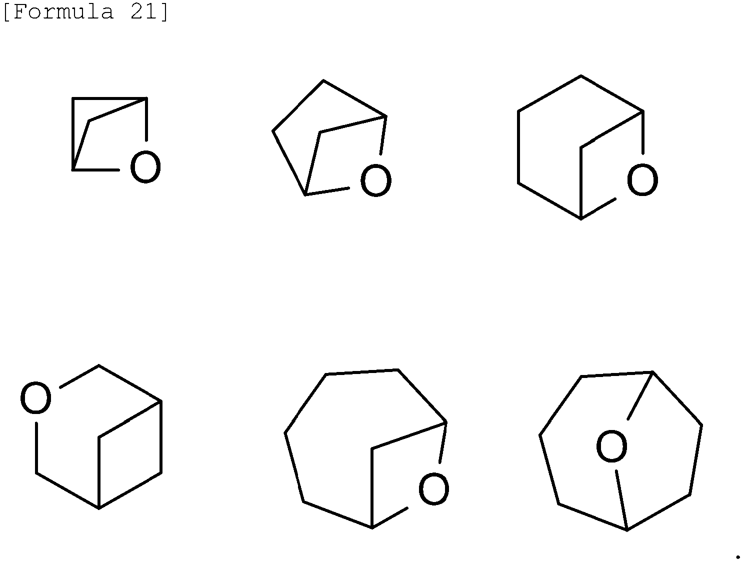

A "3-6-membered nitrogen-containing saturated heterocyclic group" means a 3- to 6-membered nitrogen-containing saturated heterocyclic group, and examples thereof include an aziridine group, an azetidine group, a pyrrolidine group, a pyrazolidine group, a piperidine group, a piperazine group and the like.

A "5-6-membered nitrogen-containing aromatic heterocyclic group" means a 5-membered or 6-membered monocyclic nitrogen-containing aromatic heterocyclic group, and examples thereof include a pyrrole group, an imidazole group, a triazole group, a pyridine group, a pyridazine group, a pyrimidine group, a pyrazine group and the like.

A "5-membered oxygen-containing saturated heterocyclic group" means a 5-membered monocyclic oxygen-containing saturated heterocyclic group, and examples thereof include a tetrahydrofuran and the like.

The expression "optionally substituted" means unsubstituted or substituted with a specific number of specific substituents at any substitutable position (any hydrogen atom is replaced with a substituent).

A "pharmaceutically acceptable salt" means a salt that can be used as a medicament, and includes both pharmaceutically acceptable acid addition salts and pharmaceutically acceptable base addition salts.

"Treatment" and its derivatives mean remission, alleviation or delay of exacerbation of clinical symptoms of diseases, illnesses, disorders and the like (hereinafter referred to as "diseases and the like") in a patient who develops the diseases and the like.

"Prevention" and its derivatives mean inhibiting, suppressing, controlling, slowing or stopping the onset of clinical symptoms of the diseases and the like in human who may develop the diseases and the like, but have not yet developed, or are concerned about recurrence of the diseases and the like after treatment.

When the compound of the present invention has a basic group such as a nitrogen-containing heterocyclic group in the molecule, it can generally form a pharmaceutically acceptable acid addition salt. Examples of such acid addition salt include salts with inorganic acid, such as hydrohalides (e.g., hydrofluoride, hydrochloride, hydrobromide, hydroiodide etc.); nitrate, perchlorate, sulfurate, phosphate and the like; lower alkanesulfonates such as methanesulfonate, trifluoromethanesulfonate, ethanesulfonate and the like; arylsulfonates such as benzenesulfonate, p-toluenesulfonate and the like; salts with organic acid, such as acetate, malate, fumarate, succinate, citrate, tartarate, oxalate, maleic acid, mucic acid, adipate and the like; salts with amino acid, ornithinate, glutamate, aspartate and the like, and the like. Preferred are hydrohalides, arylsulfonates, and salts with organic acid.

The acid addition salt of the compound of the present invention includes acid addition salts that can be formed by combining the acid to be added to the compound of the present invention with the compound of the present invention in any ratio. For example, hydrochlorides include salts that can be formed as monohydrochloride, dihydrochloride, trihydrochloride, etc., fumarates include salts that can be formed as monofumarate, 1/2 fumarate, etc., and succinates include salts that can be formed as monosuccinate, 2/3 succinate, 1/3 succinate, etc.

When the compound of the present invention has a carboxy group in the molecule, it can generally form a pharmaceutically acceptable base addition salt. Examples of such base addition salt include inorganic salts such as alkali metal salts (e.g., sodium salt, potassium salt, lithium salt etc.); alkaline-earth metal salts (e.g., calcium salt, magnesium salt etc.); ammonium salt, and the like; salts with organic amine, such as dibenzylamine salt, morpholine salt, phenylglycine alkyl ester salt, ethylene diamine salt, N-methylglucamine salt, diethylamine salt, triethylamine salt, cyclohexylamine salt, dicyclohexylamine salt, N,N'-dibenzyl ethylene diamine salt, diethanolamine salt, N-benzyl-N-(2-phenylethoxy)amine salt, piperazine salt, tetramethylammonium salt, tris(hydroxymethyl)aminomethane salt and the like, and the like.

When the compound of the present invention has an asymmetric carbon atom in the molecule, it may exist as a plurality of stereoisomers (i.e., diastereoisomers, optical isomers) due to the asymmetric carbon atom, and the present invention includes any one of these stereoisomers and mixtures containing the plurality of stereoisomers in any ratio. In addition, isomers due to conformation or tautomerism may occur, and such isomers or mixtures thereof are also included in the compound of the present invention.

In the names of the compounds of the present invention, if the compound has a carbon atom that is an asymmetric center in its structure, its absolute configuration is indicated by R and S (along with its position number).

In addition, when optical isomers are separated, if the configuration of the carbon atom that is an asymmetric center in the compound's structure has not been determined, it is indicated by R* or S*.

Furthermore, by using R* and S* simultaneously, the relative configuration may be indicated even when the absolute configuration has not been determined.

Specific examples of descriptions in the present specification for cases where the absolute configuration has not been determined are shown below. For example, in Example 19, a single enantiomer is isolated, but its absolute configuration has not been determined. In the present specification, such a compound is named as "3-methyl-2-{7-[(1R,2R,5S or 1S,2S,5R)-8-methyl-8-azabicyclo[3.2.1]octan-2-yl]-6,7-dihydro-5H-pyrrolo[2,3-c]pyridazin-3-yl}-5-(trifluoromethyl)phenol".

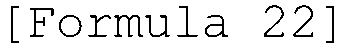

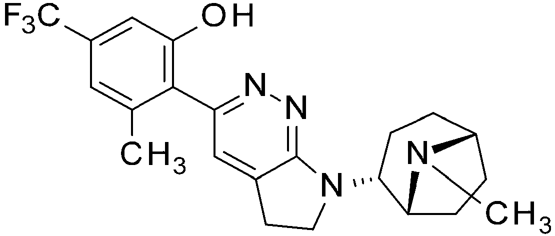

That is, the moiety whose absolute configuration is undetermined is denoted as (1R,2R,5S or 1S,2S,5R)- to indicate that the absolute configuration is either 1R,2R,5S or 1S,2S,5R, and that it is a single enantiomer. In the above structural formula, the stereochemistry of one enantiomer is shown for convenience, but which absolute configuration it has is actually undetermined.

Furthermore, the nomenclature relating to the stereochemistry used in the present specification is described below.

Many of the compounds of the present invention have a 3-hydroxy-3-methylcyclobutyl structure as a partial structure, as shown below.

In the present specification, these compounds are described as
(1s,3s)-3-hydroxy-3-methylcyclobutyl or
cis-3-hydroxy-3-methylcyclobutyl
in the case of the partial structure below.

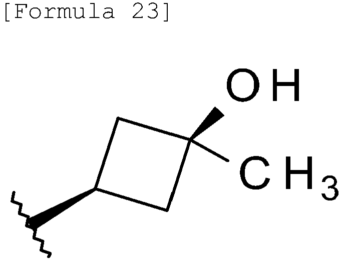

For example, in Example 3, the compound having the following structure is described as 2-{7-[(1s,3s)-3-hydroxy-3-methylcyclobutyl]-7H-pyrrolo[2,3-c]pyridazin-3-yl}-3-methyl-5-(trifluoromethyl)phenol, or 2-[7-(cis-3-hydroxy-3-methylcyclobutyl)-7H-pyrrolo[2,3-c]pyridazin-3-yl]-3-methyl-5-(trifluoromethyl)phenol, both of which indicate the same compound.

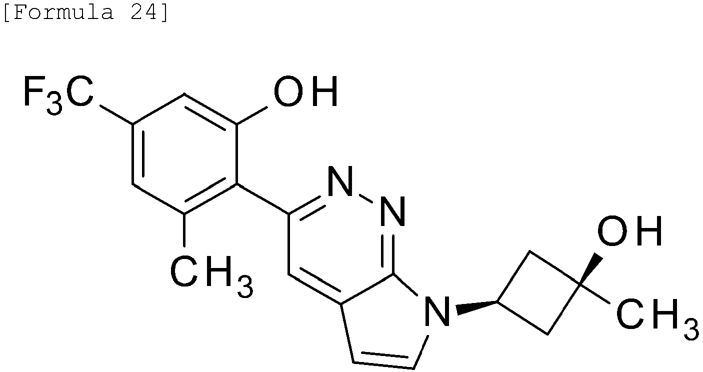

Needless to say, the "yields" described in the examples of the compounds of the present invention may include impurities to the extent that is commonly considered in the field of organic synthesis.

The diastereomers described in the examples of the compounds of the present invention may be in equal proportions, may contain more of a particular diastereomer, or may be a single diastereomer.

A "single diastereomer" may be a mixture of diastereomers in which the content of a particular diastereomer is 75% or more, 80% or more, or 90% or more.

For the enantiomers described in the examples of the compounds of the present invention, the enantiomeric excess (ee) may not be specifically stated.

In such cases, the ee is not particularly limited, and may be, for example, 0% ee or more (including racemic mixture), 20% ee or more, 40% ee or more, 60% ee or more, 80% ee or more, or 90% ee or more.

A "single enantiomer" may be a mixture of enantiomers in which the ee is, for example, 95% ee or more, 96% ee or more, 98% ee or more, or 99% ee or more.

The compounds of the present invention may be compounds labeled or substituted with isotopes (e.g., 2H, 3H, 13C, 14C, 15N, 18F, 32P, 35S, 125I, etc.), and the compounds labeled or substituted with isotopes are useful as therapeutic or preventive agents, research reagents (e.g., assay reagents), and diagnostic agents (e.g., in vivo imaging diagnostic agents). The compounds of the present invention containing radioactive or non-radioactive isotopes in all proportions are included in the scope of the present invention.

The compounds of the present invention or pharmaceutically acceptable salts thereof may be crystalline, and may be in a single crystalline form or a mixture of several crystalline forms.

The compounds of the present invention may also exist as a non-solvate or a solvate. The solvate is not particularly limited as long as it is pharmaceutically acceptable, and preferred are hydrates, ethanolates, and the like.

The compounds of the present invention may also be prodrugs.

A prodrug of the compound of the present invention refer to a compound which is converted to the compound by a reaction due to an enzyme, a gastric acid, etc., in the living body. A prodrug of the compound may have a structure in which it is readily hydrolyzed or metabolized after administration to humans.

Examples of the prodrug of the compound include, when the compound has an amino group, compounds in which the amino group is acylated, alkylated, or phosphorylated (e.g., compounds in which the amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, or tert-butylated, etc.); when the compound has a hydroxy group, compounds in which the hydroxy group is acylated, alkylated, phosphorylated, or borated (e.g., compounds in which the hydroxy group is acetylated, palmitoylation, propanoylated, pivaloylated, succinylated, fumarylated, alanylated, or dimethylaminomethylcarbonylated, etc.); compounds in which the carboxy group is esterificated, or amidatied (e.g., compounds in which the carboxy group is ethyl esterificated, phenyl esterificated, carboxymethyl esterificated, dimethylaminomethyl esterificated, pivaloyloxymethyl esterificated, 1-{(ethoxycarbonyl)oxy}ethyl esterificated, phthalidyl esterificated, (5-methyl-2-oxo-1,3-dioxolen-4-yl)methyl esterificated, 1-{[(cyclohexyloxy)carbonyl]oxy}ethyl esterificated, or methylamidated, etc.) and the like.

The prodrug of the compound of the present invention can be produced from the compound by a known method. The prodrug of the compound includes prodrugs that convert to the compound under physiological conditions as described in Development of Pharmaceutical Products, vol. 7, Molecule Design, 163-198, Hirokawa Shoten (1990). Furthermore, the prodrug of the compound may be any of a hydrate and a non-hydrate.

The compound of the present invention may also be applied as a partial structure of a degradation inducer for target proteins, collectively known as TPD (Targeted Protein Degradation), by binding to a ligand of E3 ubiquitin ligase via a linker structure at any site in its chemical structure. The TPD to which the compound of the present invention is applied can be prepared by any known method, for example, the methods described in the following patent literatures, WO 2015/160845, WO 2016/197032, WO 2019/199816, WO 2017/011371, WO 2016/105518, US 10849980 B, US 10464925 B, WO 2013106643, US 10730862 B, WO 2022/081927, WO 2022/081928, WO 2017/197051, WO 2019/060742, WO 2019/060693, WO 2019/099868, WO 2018/237026, etc.

### (Production Method of the compound of the present invention)

Representative methods for producing the compound of the present invention or a pharmaceutically acceptable salt thereof are described below.

The compound of the present invention can be produced by various production methods, and the production methods shown below and Reference Examples and Examples described below are only examples, and the present invention should not be interpreted as being limited to these.

Each raw material compound may form a salt as long as it does not inhibit the reaction, and examples of such salts include the same as the pharmaceutically acceptable salts of the compound described above.

When no specific production method is described, the raw material compounds may be readily available commercially, or can be produced according to a method known per se or a method analogous thereto. In addition, the production intermediates generated in the following production methods may be isolated and purified by a method such as column chromatography (including normal phase and reverse phase) using silica gel or alumina, recrystallization, reprecipitation, distillation, and the like, or may be used directly in the next reaction without isolation and purification.

In the present specification, all patent literatures, non-patent literatures, or references expressly cited herein may all be cited herein as a part of the present specification.

The compound, a pharmaceutically acceptable salt thereof, and a production intermediate therefor can be produced by utilizing the characteristics based on the kind of the basic skeleton or substituent, and applying various known production methods. Examples of the known methods include the methods described in "ORGANIC FUNCTIONAL GROUP PREPARATIONS", 2nd edition, ACADEMIC PRESS, INC., 1989, "Comprehensive Organic Transformations", 2nd edition, VCH Publishers Inc., 1999, and the like.

In such cases, depending on the type of functional group present in the compound, it may be effective in terms of manufacturing technology to protect the functional group with an appropriate protecting group at the starting material or intermediate stage, or to replace the functional group with a group that can be easily converted to the functional group concerned.

Examples of the functional group include amino group, hydroxy group, formyl group, carbonyl group, carboxy group, and the like, and examples of the protecting group thereof include the protecting groups described in P. G. Wuts, "Protective Groups in Organic Synthesis", 5th edition, Wiley, 2014.

The protecting group or the group that can be easily converted into the functional group may be appropriately selected depending on the reaction conditions of the production method for producing the compound.

According to such a method, a desired compound can be obtained by introducing the group and performing a reaction, and then removing the protecting group as necessary or converting same into a desired group.

A prodrug of the compound can be produced by, similar to the above-mentioned protecting groups, introducing a particular group in the stage of a starting material or intermediate, or by a reaction using the obtained compound. The reaction for producing a prodrug can be performed by those of ordinary skill in the art, by applying a known method such as general esterification, amidation, dehydration, hydrogenation and the like.

Furthermore, conversion of functional groups on heterocycles in the production intermediates used in each step of the following methods can be performed by known methods or methods analogous thereto, or the methods described in the below-mentioned examples or methods analogous thereto.

The compound of the present invention can be produced, for example, by the following methods.

In the reactions of each step in the methods described below, the reaction temperature varies depending on the solvent, starting material, reagents, etc., and the reaction time varies depending on the solvent, starting material, reagents, reaction temperature, etc. In addition, the amounts of the solvent, starting material, reagent, etc. to be used can be appropriately determined depending on the progress of the reaction.

Furthermore, conversion of functional groups on rings and use of protecting groups in the production intermediates used in each step of the following methods can be performed by known methods or methods analogous thereto, or the methods described in the below-mentioned examples or methods analogous thereto.

In the following description, the abbreviations shown here may be used.
DCE: 1,2-dichloroethane
DCM: dichloromethane
DDQ: 2,3-dichloro-5,6-dicyano-p-benzoquinone
DEE: diethyl ether
DIPEA: N,N-diisopropylethylamine
DMF: N,N-dimethylformamide
DMSO: dimethyl sulfoxide
NMP: N-methyl-2-pyrrolidinone
Pd₂(dba)₃: tris(dibenzylideneacetone)dipalladium(0)
Pd(dppf)Cl₂: [1,1'-
bis(diphenylphosphino)ferrocene]dichloropalladium(II) dichloromethane additive
Pd(PPh₃)₄: tetrakis(triphenylphosphine)palladium(0)
RuPhos: 2-dicyclohexylphosphino-2',6'-diisopropoxybiphenyl
TBAF: tetrabutylammonium fluoride
tBuXPhos: 2-di-tert-butylphosphino-2',4',6'-triisopropylbiphenyl
tBuXPhos Pd G1: [2-(di-tert-butylphosphino)-2',4',6'-triisopropyl-1,1'-biphenyl][2-(2-aminoethyl)phenyl)]palladium(II) chloride
TEA: triethylamine
TFA: trifluoroacetic acid
Tf₂O: trifluoromethanesulfonic anhydride
THF: tetrahydrofuran
XPhos: 2-dicyclohexylphosphino-2',4',6'-triisopropylbiphenyl
XPhos Pd G2: chloro(2-dicyclohexylphosphino-2',4',6'-triisopropyl-1,1'-biphenyl)[2-(2'-amino-1,1'-biphenyl)]palladium(II)
XPhos Pd G3: (2-dicyclohexylphosphino-2',4',6'-triisopropyl-1,1'-biphenyl)[2-(2'-amino-1,1'-biphenyl)]palladium (II) methanesulfonate

### (Method A)

Method A shows a method for producing compound (A-VII), (A-IX) or (A-XI) of the present invention. 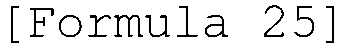 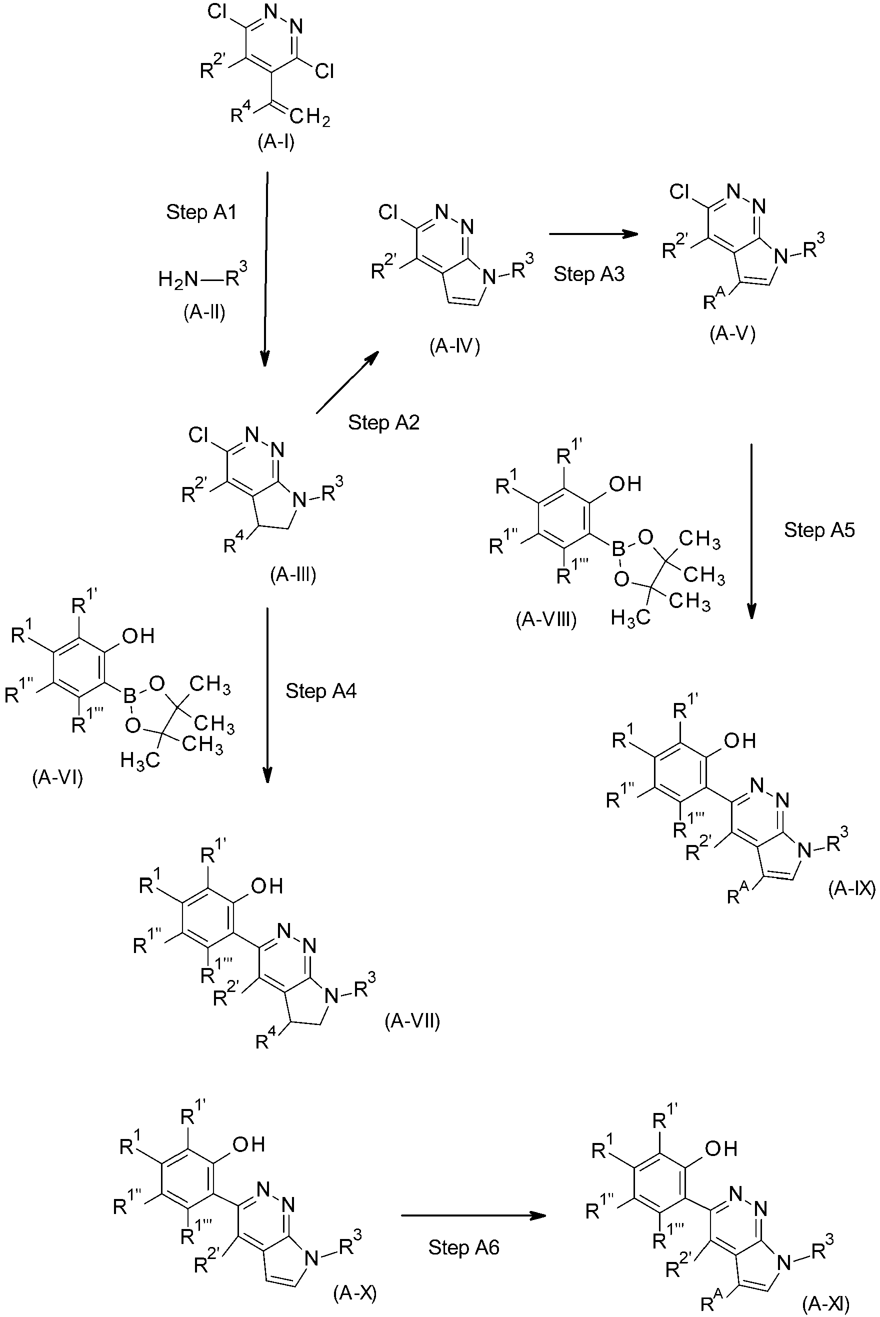 wherein R⁴ is a hydrogen atom, or a substituent selected from the above-mentioned substituent group a, R^{A} is a hydrogen atom, or a substituent selected from the above-mentioned substituent group a, and the other symbols used are as defined above.

### (Step A1)

This step is a step of producing compound (A-III) by reacting compound (A-I) with compound (A-II) in the presence of a base, in a solvent.

Examples of the solvent to be used include ethers such as DEE, THF, 1,4-dioxane and the like, alcohols such as methanol, ethanol, 1-propanol, 2-propanol, 1-butanol, 1-decanol and the like, water, acetonitrile, NMP, DMF, DMSO, and mixtures thereof. Preferred are NMP, and 1,4-dioxane.

Examples of the base to be used include organic bases such as TEA, DIPEA and the like, and inorganic bases such as potassium carbonate, sodium carbonate, cesium carbonate and the like. Preferred is DIPEA.

The reaction temperature is usually 0°C to 200°C, preferably 100°C to 150°C.

The reaction time is usually 5 min to 48 hr, preferably 30 min to 5 hr.

This reaction may also be carried out under microwave irradiation.

### (Step A2)

This step is a step of producing compound (A-IV) by reacting compound (A-III) wherein R⁴=H with an oxidant in a solvent.

Examples of the solvent to be used include halogenated hydrocarbons such as DCM, DCE, chloroform and the like, nitriles such as acetonitrile and the like, and ethers such as DEE, THF, 1,4-dioxane and the like. Preferred is DCE.

Examples of the oxidant to be used include manganese dioxide, DDQ, and oxygen. Preferred are manganese dioxide, and DDQ.

The reaction temperature is usually 0°C to 200°C, preferably 80°C.

The reaction time is usually 5 min to 48 hr, preferably 30 min to 5 hr.

### (Step A3)

This step is a step of producing compound (A-V) by introducing substituent R^{A} into compound (A-IV) by a reaction according to the method described in any of (Step A3-1) to (Step A3-3).

### (Step A3-1)

This step is a step of producing compound (A-V) by subjecting compound (A-IV) to the following steps:
(1) a step of introducing an acetoxy group,
(2) a step of converting the acetoxy group into a hydroxyl group, and
(3) a step of alkylating the hydroxyl group.

Each step is described in detail below.

### (1) a step of introducing an acetoxy group

This step is a step of producing a compound in which an acetoxy group is introduced by reacting compound (A-IV) with an oxidant in a solvent.

Examples of the solvent to be used include ethers such as DEE, THF, 1,4-dioxane and the like, nitriles such as acetonitrile and the like, organic acids such as acetic acid, TFA and the like, amides such as DMF, N,N-dimethylacetamide and the like, and sulfoxides such as DMSO and the like. Preferred are 1,4-dioxane, and acetic acid.

Examples of the catalyst to be used include palladium(II) acetate, copper(II) acetate, and manganese (III) acetate. Preferred is palladium(II) acetate.

Examples of the oxidant to be used include diacetoxyiodobenzene, and [bis(trifluoroacetoxy)iodo]benzene. Preferred is diacetoxyiodobenzene.

The reaction temperature is usually 0°C to 200°C, preferably 100°C.

The reaction time is usually 5 min to 48 hr, preferably 10 min to 3 hr.

### (2) a step of converting the acetoxy group into a hydroxyl group

Examples of the solvent to be used include alcohols such as methanol, ethanol and the like. Preferred is methanol.

Examples of the base to be used include alkali metal carbonates such as sodium carbonate, potassium carbonate and the like, alkali metal hydrogencarbonates such as potassium hydrogencarbonate and the like, and alkali metal hydroxides such as sodium hydroxide, potassium hydroxide and the like. Preferred is potassium carbonate.

The reaction temperature is usually 0°C to 100°C, preferably room temperature.

The reaction time is usually 5 min to 48 hr, preferably 30 min to 3 hr.

### (3) a step of alkylating the hydroxyl group

Examples of the solvent to be used include ethers such as THF, 1,4-dioxane and the like, amides such as DMF, N,N-dimethylacetamide and the like, nitriles such as acetonitrile and the like, sulfoxides such as DMSO and the like, water, and mixtures thereof. Preferred are DMF, acetonitrile and water.

Examples of the base to be used include alkali metal carbonates such as sodium carbonate, potassium carbonate and the like, alkali metal hydrogencarbonates such as potassium hydrogencarbonate and the like, and alkali metal hydroxides such as sodium hydroxide, potassium hydroxide and the like. Preferred are potassium carbonate, and potassium hydroxide.

Examples of the alkalizing agent to be used include iodomethane, iodoethane, diethyl (bromodifluoromethyl)phosphonate, sodium bromodifluoroacetate and the like.

The reaction temperature is usually 0°C to 200°C, preferably room temperature.

The reaction time is usually 5 min to 48 hr, preferably 30 min to 24 hr.

### (Step A3-2)

This step is a step of producing compound (A-V) by subjecting compound (A-IV) to the following steps:
(1) a step of introducing an iodo group, and
(2) a step of converting the iodo group into a cyano group.

Each step is described in detail below.

### (1) a step of introducing an iodo group

This step is a step of producing a compound in which an iodo group is introduced by reacting compound (A-IV) in a solvent.

Examples of the solvent to be used include ethers such as DEE, THF, 1,4-dioxane and the like, halogenated hydrocarbons such as DCM, DCE, chloroform and the like, nitriles such as acetonitrile and the like, organic acids such as acetic acid, TFA and the like, amides such as DMF, N,N-dimethylacetamide and the like, and sulfoxides such as DMSO and the like. Preferred is DCE.

Examples of the iodinating agent to be used include N-iodosuccinimide, and iodine. Preferred is N-iodosuccinimide.

The reaction temperature is usually 0°C to 200°C, preferably 60°C to 80°C.

The reaction time is usually 5 min to 48 hr, preferably 1 hr to 5 hr.

### (2) a step of converting the iodo group into a cyano group

Examples of the solvent to be used include ethers such as DEE, THF, 1,4-dioxane and the like, halogenated hydrocarbons such as DCM, DCE, chloroform and the like, nitriles such as acetonitrile and the like, organic acids such as acetic acid, TFA and the like, amides such as DMF, N,N-dimethylacetamide and the like, sulfoxides such as DMSO and the like, and mixed solvents thereof. Preferred are DMF, 1,4-dioxane, and water.

Examples of the catalyst to be used include Pd(PPh₃)₄, Pd₂(dba)₃, and tBuXPhos Pd G1. Preferred are Pd₂(dba)₃, Pd(PPh₃)₄, and tBuXPhos Pd G1.

Preferred examples of the cyanating agent to be used include zinc cyanide, and potassium hexacyanoferrate(II) trihydrate.

Preferred examples of the additive to be used include tBuXPhos, and XPhos.

The reaction temperature is usually 0°C to 200°C, preferably 100°C.

The reaction time is usually 5 min to 48 hr, preferably 30 min to 5 hr.

### (Step A3-3)

This step is a step of producing compound (A-V) by reacting compound (A-IV) in a solvent to introduce fluorine into compound (A-IV).

Examples of the solvent to be used include ethers such as DEE, THF, 1,4-dioxane and the like, nitriles such as acetonitrile and the like, organic acids such as acetic acid, TFA and the like, amides such as DMF, N,N-dimethylacetamide and the like, sulfoxides such as DMSO and the like, and water. Preferred is acetonitrile.

Examples of the fluorinating agent to be used include Selectfluor (registered trademark), N-fluorobenzenesulfonimide, and 1-fluoropyridinium trifluoromethanesulfonate. Preferred is Selectfluor (registered trademark).

The reaction temperature is usually 0°C to 200°C, preferably room temperature.

The reaction time is usually 5 min to 48 hr, preferably 1 hr to 5 hr.

### (Step A4)

This step is a step of producing compound (A-VII) by reacting compound (A-III) with compound (A-VI) in the presence of a catalyst and a base, in a solvent.

Examples of the solvent to be used include ethers such as DEE, THF, 1,4-dioxane and the like, aromatic hydrocarbons such as benzene, toluene, xylene and the like, water, acetonitrile, NMP, DMF, DMSO, and mixed solvents thereof. Preferred is a mixed solvent of 1,4-dioxane and water.

Examples of the catalyst to be used include Pd(PPh₃)₄, Pd(dppf)Cl₂, XPhos Pd G2, and XPhos Pd G3. Preferred is XPhos Pd G2.

Examples of the base to be used include sodium carbonate, potassium carbonate, cesium carbonate, tripotassium phosphate, sodium hydroxide, and sodium tert-butoxide. Preferred is potassium carbonate.

The reaction temperature is usually room temperature to 200°C, preferably 90°C to 120°C.

The reaction time is usually 5 min to 48 hr, preferably 30 min to 5 hr.

This reaction may also be carried out under microwave irradiation.

### (Step A5)

This step is a step of producing compound (A-IX) by reacting compound (A-V) with compound (A-VIII) in the presence of a catalyst and a base, in a solvent, and is carried out according to (Step A4).

### (Step A6)

This step is a step of producing compound (A-XI) by introducing substituent R^{A} into compound (A-X) by a reaction according to the method described in any of (Step A6-1) or (Step A6-2).

### (Step A6-1)

This step is a step of producing compound (A-XI) by subjecting compound (A-X) to the following step:
(2a) a step of introducing a bromo group, and
(2b) a step of converting the bromo group into a cyano group, or
(2c) a step of converting the bromo group into an alkyl group or an alkenyl group.

Each step is described in detail below.

### (1a) a step of introducing a bromo group

Examples of the solvent to be used include ethers such as DEE, THF, 1,4-dioxane and the like, halogenated hydrocarbons such as DCM, DCE, chloroform and the like, nitriles such as acetonitrile and the like, organic acids such as acetic acid, TFA and the like, amides such as DMF, N,N-dimethylacetamide and the like, and sulfoxides such as DMSO and the like. Preferred is DCE.

Examples of the brominating agent to be used include N-bromosuccinimide, and bromine. Preferred is N-bromosuccinimide.

The reaction temperature is usually 0°C to 200°C, preferably room temperature.

The reaction time is usually 5 min to 48 hr, preferably 10 min to 5 hr.

### (2a) a step of converting the bromo group into a cyano group

This step is a step of producing compound (A-XI) by reacting the compound obtained in (1) in a solvent to introduce a cyano group into the compound, and is carried out according to (2) of (Step A3-2).

### (2c) a step of converting the bromo group into an alkyl group or an alkenyl group

This step is a step of producing compound (A-XI) by reacting the compound obtained in (1) with various boronic acids or boronic acid esters in a solvent to introduce an alkyl group or an alkenyl group into the compound, and is carried out according to (Step A4).

### (Step A6-2)

This step is a step of producing compound (A-XI) by subjecting compound (A-X) to the following step:
(1) a step of introducing an iodo group, and
(2a) a step of converting the iodo group into a cyano group, or
(2b) a step of converting the iodo group into a trifluoromethyl group.

Each step is described in detail below.

### (1) a step of introducing an iodo group

This step is a step of producing a compound in which an iodo group is introduced by reacting compound (A-X) in a solvent, and is carried out according to (1) of (Step A3-2).

### (2a) a step of converting the iodo group into a cyano group

This step is a step of producing compound (A-XI) by reacting the compound obtained in (1) in a solvent to introduce a cyano group into the compound, and is carried out according to (2) of (Step A3-2).

### (2b) a step of converting the iodo group into a trifluoromethyl group

This step is a step of producing compound (A-XI) by reacting the compound obtained in (1) in a solvent to introduce a trifluoromethyl group into the compound.

Examples of the solvent to be used include ethers such as DEE, THF, 1,4-dioxane and the like, halogenated hydrocarbons such as DCM, DCE, chloroform and the like, nitriles such as acetonitrile and the like, organic acids such as acetic acid, TFA and the like, amides such as DMF, N,N-dimethylacetamide and the like, and sulfoxides such as DMSO and the like. Preferred is DMF.

Examples of the catalyst to be used include Pd(PPh₃)₄, and Pd₂(dba)₃. Preferred is Pd₂(dba)₃.

Examples of the additive to be used include copper(I) iodide, and copper(II) acetate. Preferred is copper(I) iodide.

Examples of the trifluoromethylating agent to be used include methyl difluoro(fluorosulfonyl)acetate, trimethyl(trifluoromethyl)silane, and 1-trifluoromethyl-1,2-benziodoxol-3(1H)-one. Preferred is methyl difluoro(fluorosulfonyl)acetate.

The reaction temperature is usually 0°C to 200°C, preferably 100°C.

The reaction time is usually 5 min to 48 hr, preferably 1 hr to 5 hr.

### (Method B)

Method B shows a method for producing compound (B-IX) of the present invention. 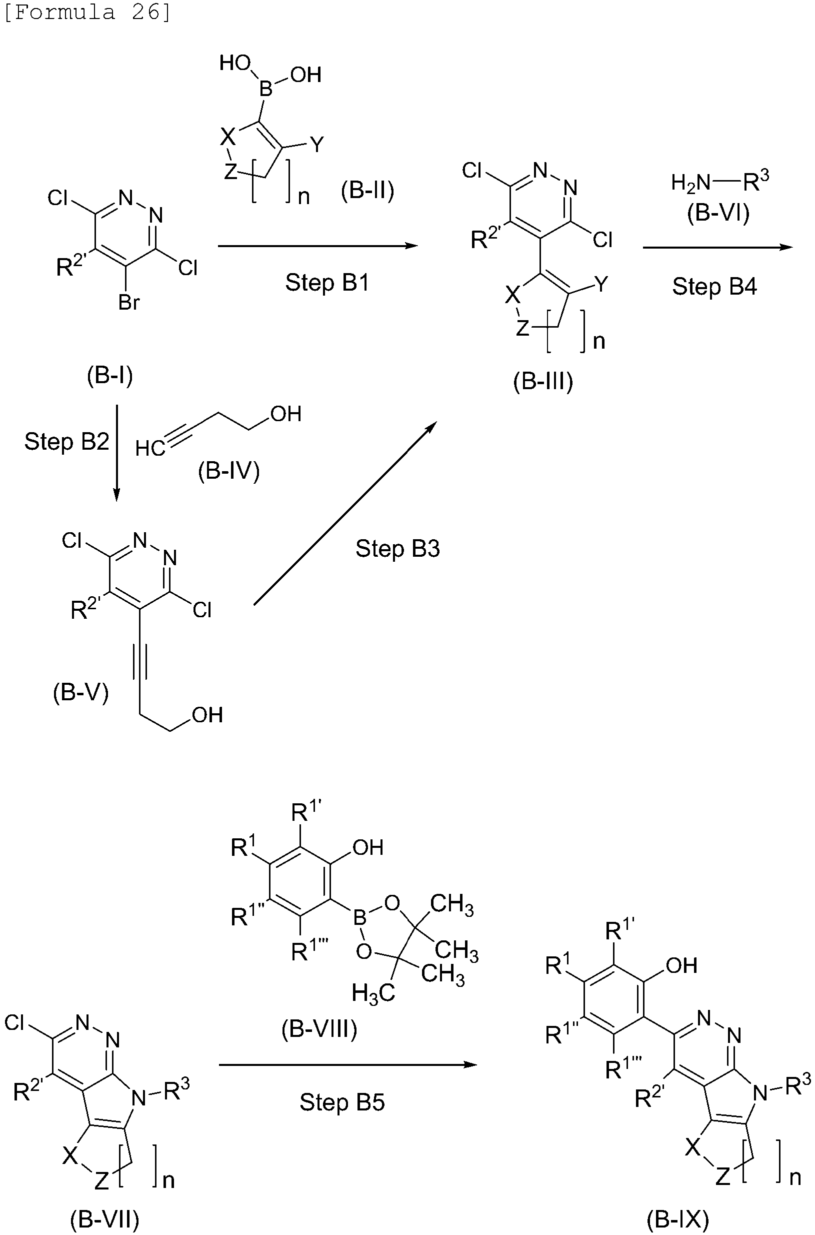 wherein X is -CH₂-, or an oxygen atom, Y is a bromine atom, or an iodine atom, Z is -CH₂-, or an oxygen atom, n is an integer of 1 or 2, and the other symbols used are as defined above.

### (Step B1)

This step is a step of producing compound (B-III) by reacting compound (B-I) with compound (B-II) in the presence of a catalyst and a base, in a solvent.

Examples of the solvent to be used include ethers such as DEE, THF, 1,4-dioxane and the like, aromatic hydrocarbons such as benzene, toluene, xylene and the like, water, acetonitrile, NMP, DMF, DMSO, and mixed solvents thereof. Preferred is a mixed solvent of 1,4-dioxane and water.

Examples of the catalyst to be used include Pd(PPh₃)₄, Pd(dppf)Cl₂, XPhos Pd G2, and XPhos Pd G3. Preferred is Pd(dppf)Cl₂.

Examples of the base to be used include sodium carbonate, potassium carbonate, cesium carbonate, tripotassium phosphate, sodium hydroxide, and sodium tert-butoxide. Preferred is potassium carbonate.

The reaction temperature is usually room temperature to 200°C, preferably 90°C to 120°C.

The reaction time is usually 5 min to 48 hr, preferably 30 min to 5 hr.

### (Step B2)

This step is a step of producing compound (B-V) by reacting compound (B-I) with compound (B-IV) in the presence of a catalyst and a base, in a solvent.

Examples of the solvent to be used include ethers such as DEE, THF, 1,4-dioxane and the like, aromatic hydrocarbons such as benzene, toluene, xylene and the like, halogenated hydrocarbons such as DCM, DCE, chloroform and the like, water, acetonitrile, NMP, DMF, and DMSO. Preferred is DMF.

Examples of the catalyst to be used include Pd(PPh₃)₄, Pd(dppf)Cl₂, and bis(triphenylphosphine)palladium(II) dichloride. Preferred is bis(triphenylphosphine)palladium(II) dichloride.

Examples of the base to be used include organic bases such as TEA, DIPEA and the like, and inorganic bases such as sodium carbonate, potassium carbonate, cesium carbonate and the like. Preferred is TEA.

Preferred examples of the additive to be used include copper (I) iodide.

The reaction temperature is usually 0°C to 200°C, preferably room temperature.

The reaction time is usually 5 min to 48 hr, preferably 30 min to 3 hr.

### (Step B3)

This step is a step of producing compound (B-III) by subjecting compound (B-V) to the following steps:
(1) a step of constructing dihydrofuran by cyclization,
(2) a step of introducing iodine and an acetoxy group, and
(3) a step of synthesizing a vinyl iodo compound.

Each step is described in detail below.

### (1) a step of constructing dihydrofuran by cyclization

Examples of the solvent to be used include ethers such as DEE, THF, 1,4-dioxane and the like, halogenated hydrocarbons such as DCM, DCE, chloroform and the like, nitriles such as acetonitrile and the like, amides such as DMF, N,N-dimethylacetamide and the like, and sulfoxides such as DMSO and the like. Preferred is DCE.

Preferred examples of the catalyst to be used include silver trifluoromethanesulfonate, and (triphenylphosphine)gold(I) chloride.

The reaction temperature is usually -20°C to 100°C, preferably 80°C.

The reaction time is usually 5 min to 48 hr, preferably 30 min to 5 hr.

### (2) a step of introducing iodine and an acetoxy group

Examples of the solvent to be used include ethers such as DEE, THF, 1,4-dioxane and the like, halogenated hydrocarbons such as DCM, DCE, chloroform and the like, nitriles such as acetonitrile and the like, organic acids such as acetic acid, TFA and the like, amides such as DMF, N,N-dimethylacetamide and the like, and sulfoxides such as DMSO and the like. Preferred is acetic acid.

Examples of the iodinating agent to be used include N-iodosuccinimide, and iodine. Preferred is N-iodosuccinimide.

The reaction temperature is usually 0°C to 200°C, preferably room temperature.

The reaction time is usually 5 min to 24 hr, preferably 10 min to 3 hr.

### (3) a step of synthesizing a vinyl iodo compound

Examples of the solvent to be used include ethers such as DEE, THF, 1,4-dioxane and the like, halogenated hydrocarbons such as DCM, DCE, chloroform and the like, nitriles such as acetonitrile and the like, amides such as DMF, N,N-dimethylacetamide and the like, and sulfoxides such as DMSO and the like. Preferred is DCM.

Preferred examples of the acid to be used include trimethylsilyl trifluoromethanesulfonate.

The reaction temperature is usually 0°C to 200°C, preferably room temperature.

The reaction time is usually 1 min to 1 hr, preferably 3 min to 30 min.

### (Step B4)

This step is a step of producing compound (B-VII) by reacting compound (B-III) with compound (B-VI) in the presence of a catalyst, an additive and a base, in a solvent.

Examples of the solvent to be used include ethers such as DEE, THF, 1,4-dioxane and the like, aromatic hydrocarbons such as benzene, toluene, xylene and the like, acetonitrile, NMP, DMF, DMSO, and mixed solvents thereof. Preferred are 1,4-dioxane, toluene, and NMP.

Examples of the catalyst to be used include Pd(PPh₃)₄, Pd₂(dba)₃, and copper(I) iodide. Preferred are Pd₂(dba)₃, and copper (I) iodide.

Examples of the additive to be used include RuPhos, 4,5-bis(diphenylphosphino)-9,9-dimethylxanthene, 2-(dicyclohexylphosphino)-3,6-dimethoxy-2',4',6'-triisopropyl-1,1'-biphenyl, and L-proline. Preferred are RuPhos, and L-proline.

Examples of the base to be used include sodium carbonate, potassium carbonate, cesium carbonate, tripotassium phosphate, sodium hydroxide, and sodium tert-butoxide. Preferred is sodium tert-butoxide.

The reaction temperature is usually room temperature to 200°C, preferably 90°C to 120°C.

The reaction time is usually 5 min to 48 hr, preferably 30 min to 5 hr.

### (Step B5)

This step is a step of producing compound (B-IX) by reacting compound (B-VII) with compound (B-VIII) in the presence of a catalyst and a base, in a solvent, and is carried out according to (Step A4).

### (Method C)

Method C shows a method for producing compound (C-VI) of the present invention. 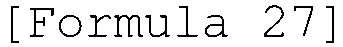 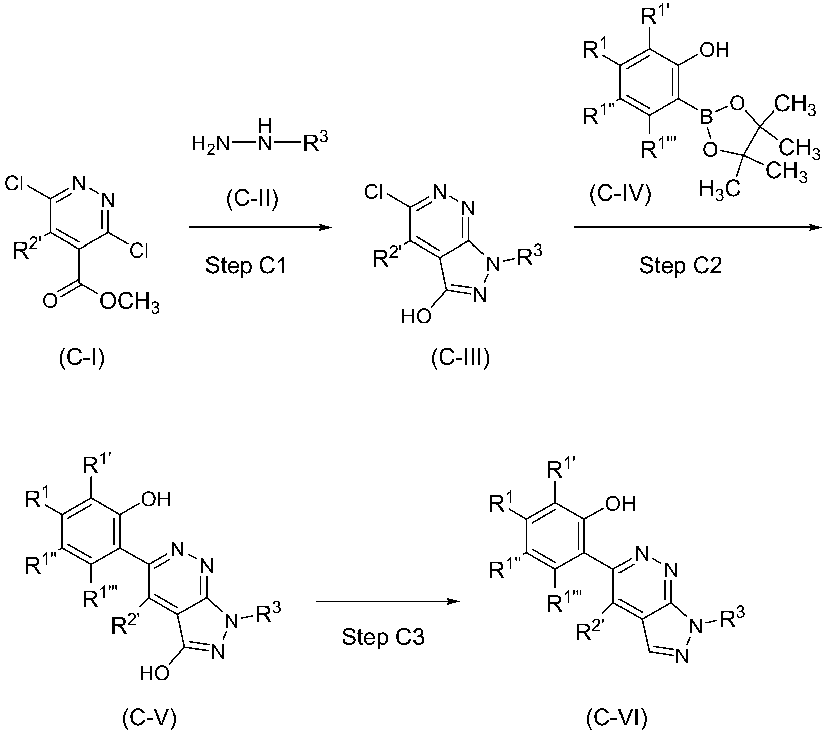 wherein the symbols used are as defined above.

### (Step C1)

This step is a step of producing compound (C-III) by reacting compound (C-I) with compound (C-II) in the presence of a base, in a solvent.

Examples of the solvent to be used include ethers such as DEE, THF, 1,4-dioxane and the like, aromatic hydrocarbons such as benzene, toluene, xylene and the like, alcohols such as methanol, ethanol, 1-propanol, 2-propanol, 1-butanol and the like, acetonitrile, NMP, DMF, DMSO, and mixed solvents thereof. Preferred is methanol.

Examples of the base to be used include organic bases such as TEA, DIPEA and the like, and inorganic bases such as potassium carbonate, sodium carbonate, cesium carbonate and the like. Preferred is DIPEA.

The reaction temperature is usually room temperature to 200°C, preferably room temperature to 70°C.

The reaction time is usually 5 min to 48 hr, preferably 1 hr to 24 hr.

### (Step C2)

This step is a step of producing compound (C-V) by reacting compound (C-III) with compound (C-IV) in the presence of a catalyst and a base, in a solvent, and is carried out according to (Step A4).

### (Step C3)

This step is a step of producing compound (C-VI) by subjecting compound (C-V) to the following steps:
(1) a step of trifluoromethanesulfonylating the hydroxyl group, and
(2) a step of reducing the trifluoromethanesulfonate.

Each step is described in detail below.

### (1) a step of trifluoromethanesulfonylating the hydroxyl group

Examples of the solvent to be used include ethers such as DEE, THF, 1,4-dioxane and the like, halogenated hydrocarbons such as DCM, DCE, chloroform and the like, nitriles such as acetonitrile and the like, organic acids such as acetic acid, TFA and the like, amides such as DMF, N,N-dimethylacetamide and the like, and sulfoxides such as DMSO and the like. Preferred is DCM.

Examples of the trifluoromethanesulfonylating agent to be used include Tf₂O, N-phenylbis(trifluoromethanesulfonimide), and N,N-bis(trifluoromethylsulfonyl)-5-chloropyridin-2-amine. Preferred is N-phenylbis(trifluoromethanesulfonimide).

The reaction temperature is usually -20°C to 100°C, preferably room temperature.

The reaction time is usually 5 min to 48 hr, preferably 30 min to 5 hr.

### (2) a step of reducing the trifluoromethanesulfonate

Examples of the solvent to be used include ethers such as DEE, THF, 1,4-dioxane and the like, aromatic hydrocarbons such as benzene, toluene, xylene and the like, acetonitrile, NMP, DMF, DMSO, and mixed solvents thereof. Preferred is THF.

Examples of the catalyst to be used include Pd(PPh₃)₄, Pd(dppf)Cl₂, XPhos Pd G2, and XPhos Pd G3. Preferred is Pd(dppf)Cl₂.

Examples of the reducing agent to be used include ammonium formate, and dimethylphenylsilane. Preferred is ammonium formate.

Examples of the base to be used include organic bases such as TEA, DIPEA and the like, and inorganic bases such as potassium carbonate, sodium carbonate, cesium carbonate and the like. Preferred is TEA.

The reaction temperature is usually room temperature to 200°C, preferably 90°C to 120°C.

The reaction time is usually 5 min to 48 hr, preferably 1 hr to 10 hr.

### (Method D)

Method D shows a method for producing compound (D-VI) of the present invention. 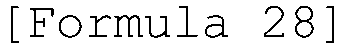 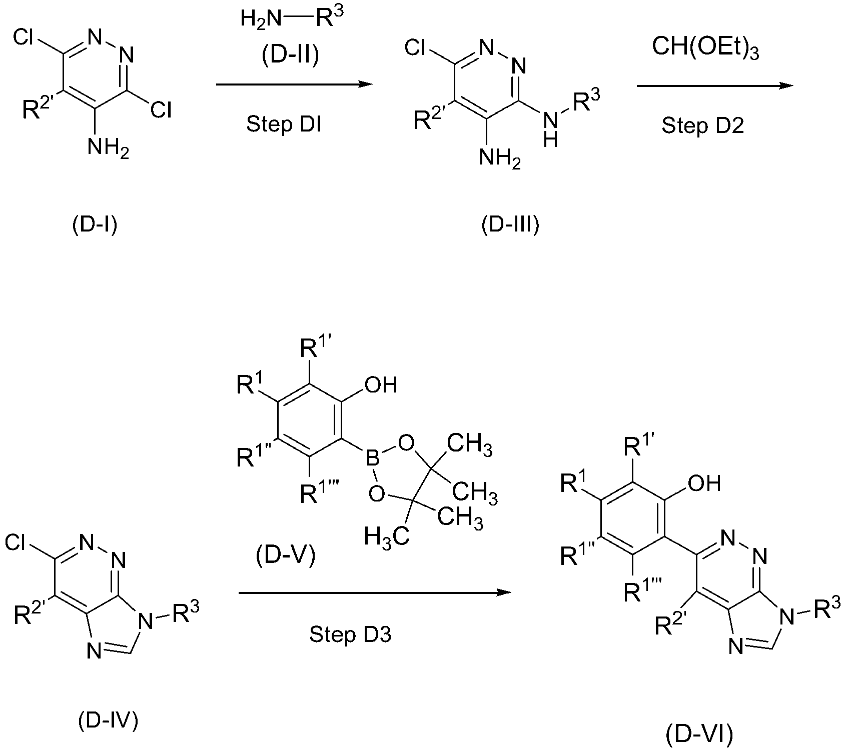 wherein the symbols used are as defined above.

### (Step D1)

This step is a step of producing compound (D-III) by reacting compound (D-I) with compound (D-II) in the presence of a base, in a solvent, and is carried out according to (Step A1).

### (Step D2)

This step is a step of producing compound (D-IV) by reacting compound (D-III) in the presence of an acid, for example, by reacting compound (D-III) with triethyl orthoformate.

Examples of the acid to be used include organic acids such as formic acid, acetic acid, TFA, p-toluenesulfonic acid and the like. Preferred is formic acid.

The reaction temperature is usually -20°C to 100°C, preferably 100°C.

The reaction time is usually 5 min to 48 hr, preferably 3 hr to 24 hr.

### (Step D3)

This step is a step of producing compound (D-VI) by reacting compound (D-IV) with compound (D-V) in the presence of a catalyst and a base, in a solvent, and is carried out according to (Step A4).

### (Method E)

Method E shows a method for producing compound (E-VI) of the present invention. 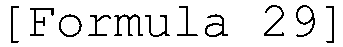 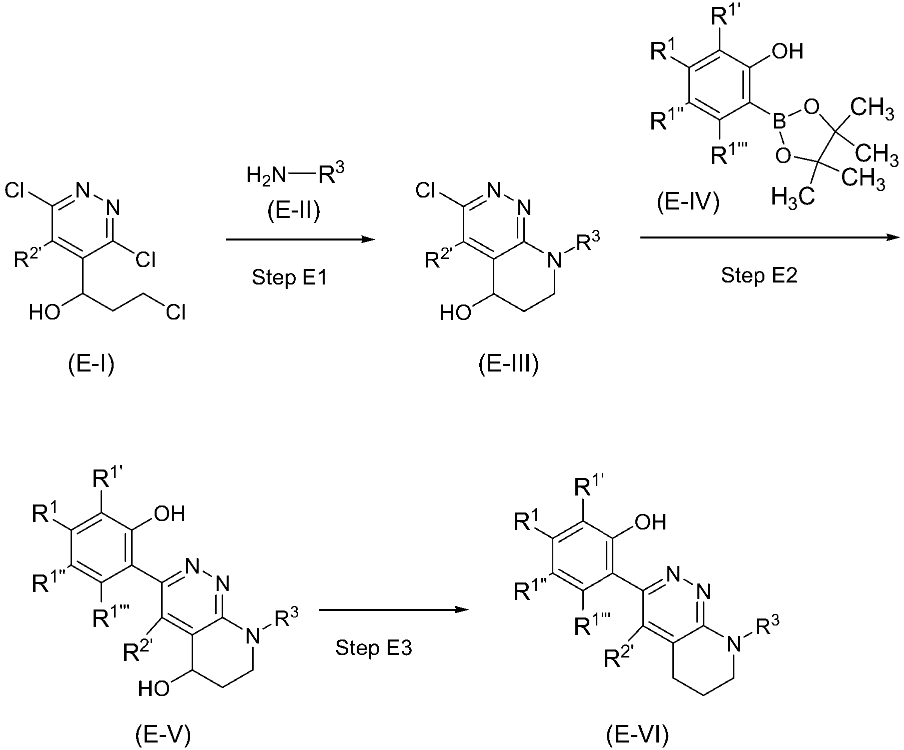 wherein the symbols used are as defined above.

### (Step E1)

This step is a step of producing compound (E-III) by reacting compound (E-I) with compound (E-II) in the presence of a base, in a solvent, and is carried out according to (Step A1).

### (Step E2)

This step is a step of producing compound (E-V) by reacting compound (E-III) with compound (E-IV) in the presence of a catalyst and a base, in a solvent, and is carried out according to (Step A4).

### (Step E3)

This step is a step of producing compound (E-VI) by subjecting compound (E-V) to the following steps:
(1) a step of thiocarbonating the hydroxyl group, and
(2) a step of reducing the thiocarbonate compound.

Each step is described in detail below.

### (1) a step of thiocarbonating the hydroxyl group

Examples of the solvent to be used include ethers such as DEE, THF, 1,4-dioxane and the like, halogenated hydrocarbons such as DCM, DCE, chloroform and the like, aromatic hydrocarbons such as benzene, toluene, xylene and the like, acetonitrile, NMP, DMF, and DMSO. Preferred is DCM.

Examples of the base to be used include organic bases such as TEA, DIPEA, 4-dimethylaminopyridine and the like, and inorganic bases such as potassium carbonate, sodium carbonate, cesium carbonate and the like. Preferred is 4-dimethylaminopyridine.

Preferred examples of the thiocarbonylation reagent to be used include phenyl chlorothionoformate.

The reaction temperature is usually 0°C to 200°C, preferably room temperature.

The reaction time is usually 5 min to 96 hr, preferably 5 hr to 72 hr.

### (2) a step of reducing the thiocarbonate compound

Examples of the solvent to be used include ethers such as DEE, THF, 1,4-dioxane and the like, halogenated hydrocarbons such as DCM, DCE, chloroform and the like, aromatic hydrocarbons such as benzene, toluene, xylene and the like, acetonitrile, NMP, DMF, and DMSO. Preferred is toluene.

Examples of the radical initiator to be used include azobisisobutyronitrile, benzoyl peroxide, triethylborane, and diethylzinc. Preferred is azobisisobutyronitrile.

Examples of the reducing agent to be used include tributyltin hydride, and tris(trimethylsilyl)silane. Preferred is tributyltin hydride.

The reaction temperature is usually 0°C to 200°C, preferably 110°C.

The reaction time is usually 5 min to 72 hr, preferably 1 hr to 5 hr.

### (Method F)

Method F shows a method for producing compound (F-V) of the present invention. 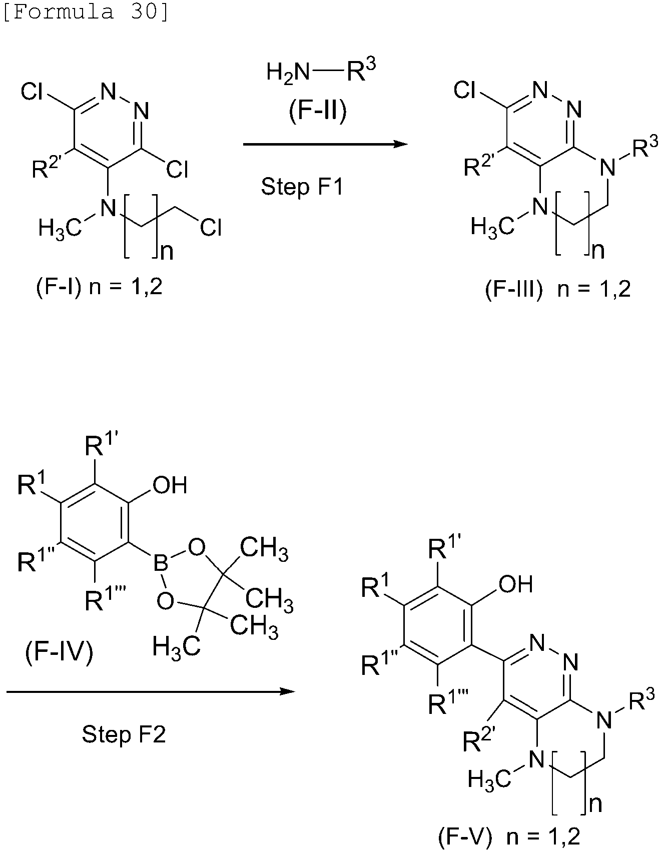 wherein n is an integer of 1 or 2, and the other symbols used are as defined above.

### (Step F1)

This step is a step of producing compound (F-III) by reacting compound (F-I) with compound (F-II) in the presence of a base, in a solvent, and is carried out according to (Step A1).

### (Step F2)

This step is a step of producing compound (F-V) by reacting compound (F-III) with compound (F-IV) in the presence of a catalyst and a base, in a solvent, and is carried out according to (Step A4).

### (Method G)

Method G shows a method for producing compound (G-V) of the present invention. 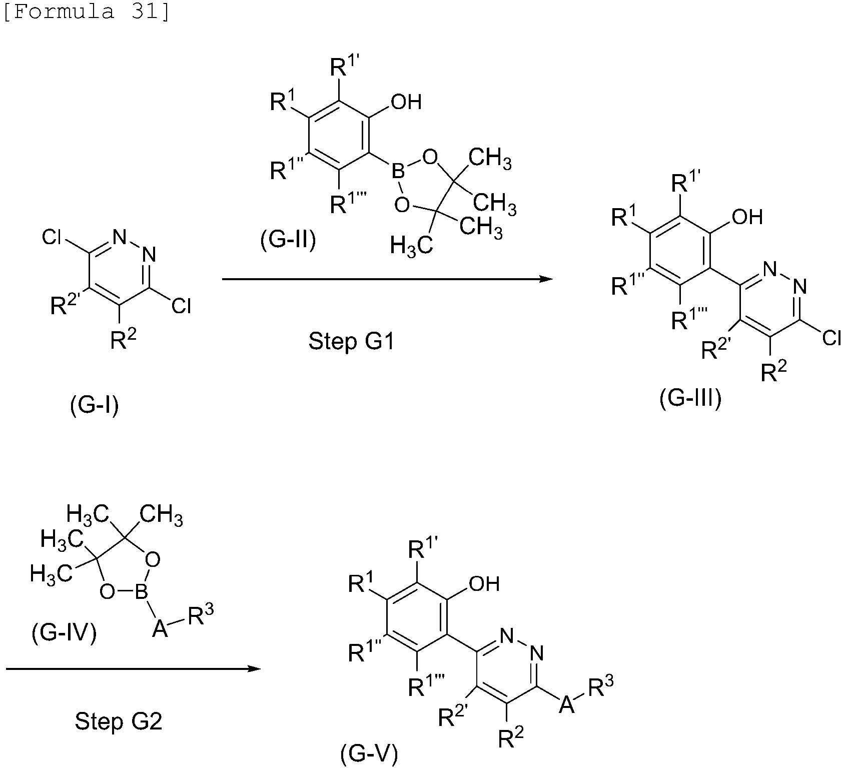 wherein the symbols used are as defined above, except that A is not a nitrogen atom.

### (Step G1)

This step is a step of producing compound (G-III) by reacting compound (G-I) with compound (G-II) in the presence of a catalyst and a base, in a solvent, and is carried out according to (Step A4).

### (Step G2)

This step is a step of producing compound (G-V) by reacting compound (G-III) with compound (G-IV) in the presence of a catalyst and a base, in a solvent, and is carried out according to (Step A4).

### (Method H)

Method H shows a method for producing compound (H-VI) of the present invention. 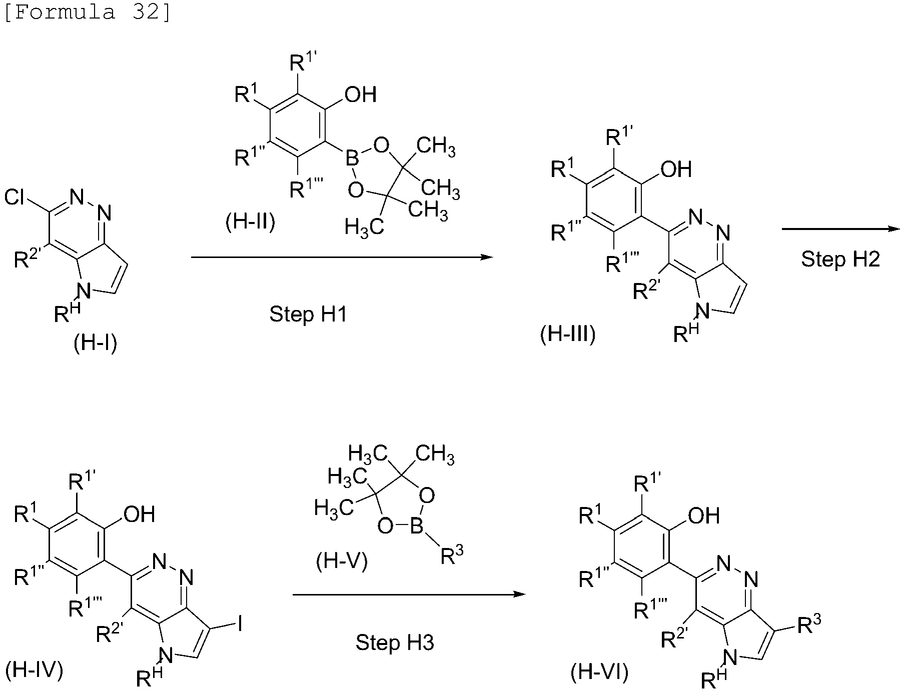 wherein R^{H} is a hydrogen atom, or a substituent selected from the above-mentioned substituent group a, and the other symbols used are as defined above.

### (Step H1)

This step is a step of producing compound (H-III) by reacting compound (H-I) with compound (H-II) in the presence of a catalyst and a base, in a solvent, and is carried out according to (Step A4).

### (Step H2)

This step is a step of producing compound (H-IV) by reacting compound (H-III) in the presence of an iodinating agent, in a solvent.

Examples of the solvent to be used include ethers such as DEE, THF, 1,4-dioxane and the like, nitriles such as acetonitrile and the like, organic acids such as acetic acid, TFA and the like, amides such as DMF, N,N-dimethylacetamide and the like, and sulfoxides such as DMSO and the like. Preferred is DMF.

Examples of the iodinating agent to be used include N-iodosuccinimide, and iodine. Preferred is N-iodosuccinimide.

The reaction temperature is usually 0°C to 200°C, preferably room temperature.

The reaction time is usually 5 min to 48 hr, preferably 30 min to 5 hr.

### (Step H3)

This step is a step of producing compound (H-VI) by reacting compound (H-IV) with compound (H-V) in the presence of a catalyst and a base, in a solvent, and is carried out according to (Step A4).

### (Method I)

Method I shows a method for producing compound (I-VI) of the present invention. 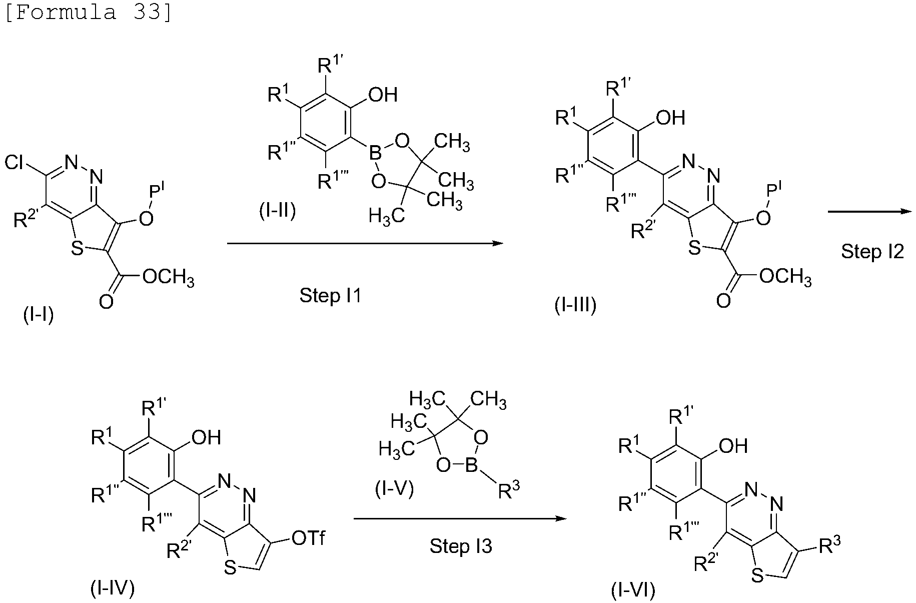 wherein P^{I} is a group for protecting a hydroxyl group, and the other symbols used are as defined above.

### (Step I1)

This step is a step of producing compound (I-III) by reacting compound (I-I) with compound (I-II) in the presence of a catalyst and a base, in a solvent, and is carried out according to (Step A4).

### (Step I2)

This step is a step of producing compound (I-IV) by subjecting compound (I-III) to the following steps:
(1) a step of removing the hydroxyl-protecting group, followed by ester hydrolysis and decarboxylation reaction, and
(2) a step of trifluoromethanesulfonylating the hydroxyl group.

Each step is described in detail below.

### (1) a step of removing the hydroxyl-protecting group, followed by ester hydrolysis and decarboxylation reaction

Examples of the hydroxyl-protecting group commonly used include silyl groups such as a tert-butyldimethylsilyl group, a triisopropylsilyl group, a tert-butyldiphenylsilyl group and the like. Preferred is a triisopropyl group.

Examples of the solvent to be used include alcohols such as methanol, ethanol, 1-propanol, 2-propanol, 1-butanol and the like, water, acetonitrile, NMP, DMF, DMSO, and mixed solvents thereof. Preferred is methanol.

Examples of the base to be used include alkali metal carbonates such as sodium carbonate, potassium carbonate and the like, alkali metal hydrogencarbonates such as potassium hydrogencarbonate and the like, and alkali metal hydroxides such as sodium hydroxide, potassium hydroxide and the like. Preferred is sodium hydroxide.

The reaction temperature is usually 0°C to 200°C, preferably 100°C.

The reaction time is usually 5 min to 48 hr, preferably 3 hr to 24 hr.

### (2) a step of trifluoromethanesulfonylating the hydroxyl group

Examples of the solvent to be used include ethers such as DEE, THF, 1,4-dioxane and the like, halogenated hydrocarbons such as DCM, DCE, chloroform and the like, nitriles such as acetonitrile and the like, organic acids such as acetic acid, TFA and the like, amides such as DMF, N,N-dimethylacetamide and the like, and sulfoxides such as DMSO and the like. Preferred is DCM.

Examples of the trifluoromethanesulfonylating agent to be used include Tf₂O, N-phenylbis(trifluoromethanesulfonimide), and N,N-bis(trifluoromethylsulfonyl)-5-chloropyridin-2-amine. Preferred is Tf₂O.

The reaction temperature is usually -20°C to 100°C, preferably 0°C to room temperature.

The reaction time is usually 5 min to 48 hr, preferably 30 min to 5 hr.

### (Step I3)

This step is a step of producing compound (I-VI) by reacting compound (I-IV) with compound (I-V) in the presence of a catalyst and a base, in a solvent, and is carried out according to (Step A4).

### (Method J)

Method J shows a method for producing compound (J-VI) of the present invention. 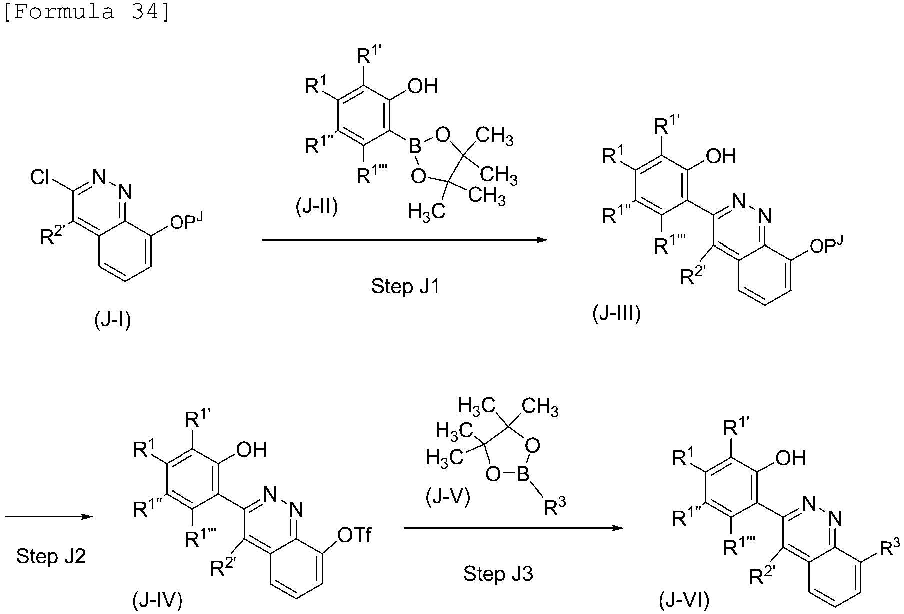 wherein P^{J} is a group for protecting a hydroxyl group, and the other symbols used are as defined above.

### (Step J1)

This step is a step of producing compound (J-III) by reacting compound (J-I) with compound (J-II) in the presence of a catalyst and a base, in a solvent, and is carried out according to (Step A4).

### (Step J2)

This step is a step of producing compound (J-IV) by subjecting compound (J-III) to the following steps:
(1) a step of removing the hydroxyl-protecting group, and
(2) a step of trifluoromethanesulfonylating the hydroxyl group.

Each step is described in detail below.

### (1) a step of removing the hydroxyl-protecting group

Examples of the hydroxyl-protecting group commonly used include silyl groups such as a tert-butyldimethylsilyl group, a triisopropylsilyl group, a tert-butyldiphenylsilyl group and the like. Preferred is a triisopropylsilyl group.

Examples of the solvent to be used include ethers such as DEE, THF, 1,4-dioxane and the like, alcohols such as methanol, ethanol, 1-propanol, 2-propanol, 1-butanol and the like, water, acetonitrile, NMP, DMF, DMSO, and mixtures thereof. Preferred is THF.

Examples of the reagent to be used include TBAF, pyridine hydrogen fluoride, and triethylamine trihydrofluoride. Preferred is TBAF.

The reaction temperature is usually 0°C to 200°C, preferably room temperature.

The reaction time is usually 5 min to 48 hr, preferably 10 min to 3 hr.

### (2) a step of trifluoromethanesulfonylating the hydroxyl group

The step of trifluoromethanesulfonylating the hydroxyl group is carried out according to (2) of (Step I2).

### (Step J3)

This step is a step of producing compound (J-VI) by reacting compound (J-IV) with compound (J-V) in the presence of a catalyst and a base, in a solvent, and is carried out according to (Step A4).

The compound or a pharmaceutically acceptable salt thereof obtained by the above-mentioned production method can be isolated and purified by a conventional separation means such as recrystallization, distillation, chromatography, and the like.

When the compound of the present invention or a pharmaceutically acceptable salt thereof exists as an optical isomer based on an asymmetric carbon, it can be separated into individual optical isomers by a conventional optical resolution method (e.g., fractional crystallization, optical resolution using a chiral column). In addition, optical isomers can be synthesized using optically pure starting materials. Furthermore, optical isomers can be synthesized by stereoselectively carrying out each reaction using an asymmetric auxiliary group or an asymmetric catalyst.

The compound or a pharmaceutically acceptable salt thereof obtained by the above-mentioned production method can be isolated and purified by a conventional separation means such as recrystallization, distillation, chromatography, and the like.

When the compound of the present invention or a pharmaceutically acceptable salt thereof exists as an optical isomer based on an asymmetric carbon, it can be separated into individual optical isomers by a conventional optical resolution method (e.g., fractional crystallization, optical resolution using a chiral column). In addition, optical isomers can be synthesized using optically pure starting materials. Furthermore, optical isomers can be synthesized by stereoselectively carrying out each reaction using an asymmetric auxiliary group or an asymmetric catalyst.

### (Pharmaceutical composition of the present invention)

The pharmaceutical composition of the present invention contains a compound or a pharmaceutically acceptable salt thereof as an active ingredient, and is a pharmaceutical composition for the prevention and/or treatment of diseases, for example, autoinflammatory diseases such as cryopyrin-associated periodic syndrome (CAPS); autoimmune diseases such as systemic lupus erythematosus (SLE), rheumatoid arthritis, psoriasis, systemic scleroderma (SSc), vasculitis syndrome, systemic amyloidosis and the like; and inflammatory diseases such as liver-related diseases (e.g., alcoholic liver disease (ALD), alcoholic steatohepatitis (ASH), non-alcoholic steatohepatitis (NASH), viral hepatitis, non-alcoholic fatty liver disease (NAFLD), etc.), lung-related diseases (e.g., chronic obstructive pulmonary diseases (COPD), asthma, acute lung injury (ALI), interstitial lung disease (ILD), sarcoidosis, etc.), renal and urinary-related diseases (e.g., diabetic nephropathy, lupus nephritis, primary hyperoxaluria, interstitial cystitis, etc.), bowel-related diseases (e.g., inflammatory bowel disease (IBD), etc.), joint-related diseases (e.g., gout, pseudogout, osteoarthritis, etc.), cardiovascular and metabolic-related diseases (e.g., atherosclerosis, diabetes type 1/type 2, heart failure, etc.), infection-related diseases (e.g., SARS-CoV-2 infection, EBV infection, viral encephalitis, etc.), eye-related diseases (e.g., glaucoma, optic neuritis, retinitis pigmentosa, age-related macular degeneration, etc.), cancer-related diseases (e.g., myeloproliferative neoplasms, leukemia, myelodysplastic syndrome, myelofibrosis, lung cancer, colorectal cancer, tumor lysis syndrome, cytokine release syndrome, etc.),
neuro-related diseases (e.g., multiple sclerosis (including relapsing remitting multiple sclerosis, secondary progressive multiple sclerosis and primary progressive multiple sclerosis as subtypes of multiple sclerosis), neurodegenerative diseases (Alzheimer's disease, Huntington's disease, Parkinson's disease, amyotrophic lateral sclerosis, multiple system atrophy, frontotemporal dementia), etc.), psychiatric disorders (e.g., epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, etc.), and cerebrovascular diseases (e.g., ischemic stroke, hemorrhagic stroke, traumatic brain injury, etc.).

The pharmaceutical composition of the present invention may be either a medicament consisting of only the compound or a pharmaceutically acceptable salt thereof, or a pharmaceutical composition containing the compound or a pharmaceutically acceptable salt thereof and a pharmaceutically acceptable carrier, and the like. The pharmaceutical composition of the present invention can be administered to a subject (e.g., a human) in a pharmaceutically effective amount.

Examples of the pharmaceutically acceptable carrier include excipients (e.g., starch, lactose, sugar, calcium carbonate, phosphoric acid calcium, etc.), binders (e.g., starch, gum arabic, carboxymethyl cellulose, hydroxypropyl cellulose, crystalline cellulose, etc.), lubricants (e.g., magnesium stearate, talc, etc.), disintegrants (e.g., carboxymethyl cellulose, talc, etc.), solvents (e.g., water for injection, physiological saline, Ringer's solution, alcohol, propylene glycol, polyethylene glycol, sesame oil, corn oil, olive oil, cottonseed oil, etc.), solubilizing agents (e.g., polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, etc.), suspending agents (e.g., surfactants such as stearyltriethanolamine, sodium lauryl sulfate, laurylaminopropionic acid, lecithin, benzalkonium chloride, benzethonium chloride, glyceryl monostearate and the like; hydrophilic polymers such as polyvinyl alcohol, polyvinyl pyrrolidone, sodium carboxymethyl cellulose, methyl cellulose, hydroxymethyl cellulose, hydroxyethyl cellulose, hydroxypropyl cellulose and the like; polysorbates, polyoxyethylene hydrogenated castor oil, etc.), isotonicity agents (e.g., sodium chloride, glycerin, D-mannitol, D-sorbitol, glucose, etc.), buffers (e.g., buffer solutions such as phosphorate, acetate, carbonate, citrate and the like, etc.), soothing agents (e.g., benzyl alcohol, etc.), preservatives (e.g., parahydroxybenzoate esters, chlorobutanol, benzyl alcohol, phenethyl alcohol, dehydroacetic acid, sorbic acid, etc.), antioxidants (e.g., sulfite, ascorbate, etc.), colorants (e.g., water-soluble food tar colors (e.g., food colors such as Food Color Red Nos. 2 and 3, Food Color Yellow Nos. 4 and 5, Food Color Blue Nos. 1 and 2, etc.), water-insoluble lake dyes (e.g., aluminum salts of the aforementioned water-soluble food tar colors), natural colors (e.g., β-carotene, chlorophyll, red iron oxide), etc.), sweetening agents (e.g., saccharine sodium, dipotassium glycyrrhizinate, stevia, etc.), taste masking agents (e.g., fennel oil, cinnamon oil, ethyl vanillin, orange oil, etc.), fragrances, and the like.

By mixing the above ingredients and then subjecting the mixture to a method known per se, the pharmaceutical composition of the present invention can be formulated into tablets, pills, fine granules, granules, capsules, dry syrup, elixirs and other preparations for oral administration; or injections (e.g., subcutaneous injections, intravenous injections, intramuscular injections, intraperitoneal injections, drip infusions, etc.), external preparations (e.g., transdermal preparations, ointments, lotions, patches), suppositories (e.g., rectal suppositories, vaginal suppositories), pellets, nasal preparations, pulmonary preparations (inhalants), eye drops, implants, microcapsules, liposome preparations and other preparations for parenteral administration. Tablets or pills may be coated with sugar coating or gastric or enteric coating agents as necessary. Preparations for parenteral administration may be sterilized, for example, by filtration through a bacteria-retaining filter, mixing with a bactericide, or irradiation. Also, a composition obtained by dissolving or suspending a sterile solid composition in sterile water or a solvent for injection before use can also be used as a preparation for parenteral administration.

The content of the compound of the present invention or a pharmaceutically acceptable salt thereof in the pharmaceutical composition of the present invention varies depending on the form of the formulation, and is usually in the range of about 0.001 to 100% by weight, preferably about 0.01 to 50% by weight, and more preferably about 0.01 to 20% by weight, based on the total formulation.

The dosage and frequency of administration of the compound of the present invention or a pharmaceutically acceptable salt thereof are appropriately determined for each individual case, taking into consideration the symptom, age or sex of the subject, etc. The dosage is usually 0.001 mg/kg to 100 mg/kg per dose for adults in the case of oral administration, and usually 0.0001 mg/kg to 10 mg/kg per dose for adults in the case of intravenous administration. The frequency of administration is usually from once to six times a day or from once a day to once every seven days.

The compound of the present invention or a pharmaceutically acceptable salt thereof is useful for the prevention and/or treatment of, for example, autoinflammatory diseases such as cryopyrin-associated periodic syndrome (CAPS); autoimmune diseases such as systemic lupus erythematosus (SLE), rheumatoid arthritis, psoriasis, systemic scleroderma (SSc), vasculitis syndrome, systemic amyloidosis and the like; and inflammatory diseases such as liver-related diseases (e.g., alcoholic liver disease (ALD), alcoholic steatohepatitis (ASH), non-alcoholic steatohepatitis (NASH), viral hepatitis, non-alcoholic fatty liver disease (NAFLD), etc.), lung-related diseases (e.g., chronic obstructive pulmonary diseases (COPD), asthma, acute lung injury (ALI), interstitial lung disease (ILD), sarcoidosis, etc.), renal and urinary-related diseases (e.g., diabetic nephropathy, lupus nephritis, primary hyperoxaluria, interstitial cystitis, etc.), bowel-related diseases (e.g., inflammatory bowel disease (IBD), etc.), joint-related diseases (e.g., gout, pseudogout, osteoarthritis, etc.), cardiovascular and metabolic-related diseases (e.g., atherosclerosis, diabetes type 1/type 2, heart failure, etc.), infection-related diseases (e.g., SARS-CoV-2 infection, EBV infection, viral encephalitis, etc.), eye-related diseases (e.g., glaucoma, optic neuritis, retinitis pigmentosa, age-related macular degeneration, etc.), cancer-related diseases (e.g., myeloproliferative neoplasms, leukemia, myelodysplastic syndrome, myelofibrosis, lung cancer, colorectal cancer, tumor lysis syndrome, cytokine release syndrome, etc.),
neuro-related diseases (e.g., multiple sclerosis (including relapsing remitting multiple sclerosis, secondary progressive multiple sclerosis and primary progressive multiple sclerosis as subtypes of multiple sclerosis), and neurodegenerative diseases (Alzheimer's disease, Huntington's disease, Parkinson's disease, amyotrophic lateral sclerosis, multiple system atrophy, frontotemporal dementia)), psychiatric disorders (e.g., epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, etc.), and cerebrovascular diseases (e.g., ischemic stroke, hemorrhagic stroke, traumatic brain injury, etc.).

The compound of the present invention or a pharmaceutically acceptable salt thereof can be used in combination with other drugs (concomitant drugs) as long as the efficacy of the compound is not impaired. The concomitant drugs are not particularly limited, and for example, one or more known drugs conventionally used in the treatment of autoinflammatory diseases such as cryopyrin-associated periodic syndrome (CAPS); autoimmune diseases such as systemic lupus erythematosus (SLE), rheumatoid arthritis, psoriasis, systemic scleroderma (SSc), vasculitis syndrome, systemic amyloidosis and the like; and inflammatory diseases such as liver-related diseases (e.g., alcoholic liver disease (ALD), alcoholic steatohepatitis (ASH), non-alcoholic steatohepatitis (NASH), viral hepatitis, non-alcoholic fatty liver disease (NAFLD), etc.), lung-related diseases (e.g., chronic obstructive pulmonary diseases (COPD), asthma, acute lung injury (ALI), interstitial lung disease (ILD), sarcoidosis, etc.), renal and urinary-related diseases (e.g., diabetic nephropathy, lupus nephritis, primary hyperoxaluria, interstitial cystitis, etc.), bowel-related diseases (e.g., inflammatory bowel disease (IBD), etc.), joint-related diseases (e.g., gout, pseudogout, osteoarthritis, etc.), cardiovascular and metabolic-related diseases (e.g., atherosclerosis, diabetes type 1/type 2, heart failure, etc.), infection-related diseases (e.g., SARS-CoV-2 infection, EBV infection, viral encephalitis, etc.), eye-related diseases (e.g., glaucoma, optic neuritis, retinitis pigmentosa, age-related macular degeneration, etc.), cancer-related diseases (e.g., myeloproliferative neoplasms, leukemia, myelodysplastic syndrome, myelofibrosis, lung cancer, colorectal cancer, tumor lysis syndrome, cytokine release syndrome, etc.),
neuro-related diseases (e.g., multiple sclerosis (including relapsing remitting multiple sclerosis, secondary progressive multiple sclerosis and primary progressive multiple sclerosis as subtypes of multiple sclerosis), and neurodegenerative diseases (Alzheimer's disease, Huntington's disease, Parkinson's disease, amyotrophic lateral sclerosis, multiple system atrophy, frontotemporal dementia)), psychiatric disorders (e.g., epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, etc.), and cerebrovascular diseases (e.g., ischemic stroke, hemorrhagic stroke, traumatic brain injury, etc.), as exemplified above, can be suitably used.

Examples of other drugs (concomitant drugs) suitable for use in combination with the compound of the present invention or a pharmaceutically acceptable salt thereof include other therapeutic drugs useful in combination therapy, such as IL-1β inhibitors (e.g., Anakinra); type I IFN receptor antagonists (e.g., Anifrolumab); TLR7/8 inhibitors (e.g., Enpatoran); JAK inhibitors (e.g., Tofacitinib); BTK inhibitors (e.g., Fenebrutinib); S1P receptor modulators (e.g., Fingolimod); anti-CD20 antibodies (e.g., Ocrelizumab); cholesterol-lowering drugs (e.g., Pravastatin); anti-inflammatory drugs (e.g., Prednisolone); non-steroidal anti-inflammatory drugs (NSAIDs) (e.g., Loxonin); acetylsalicylic acid drugs (ASA) (e.g., Aspirin), and the like.

When using a concomitant drug, the administration time is not limited, and they may be administered at the same time or different times to the subject. In the time-difference administration, the medicament of the present invention may be administered first and the concomitant drug may be administered later, or the concomitant drug may be administered first and the medicament of the present invention may be administered later. The administration method of each may be the same or different. In addition, the compound of the present invention or a pharmaceutically acceptable salt thereof and the concomitant drug may be administered in combination as a single preparation (combined preparation). Examples of pharmaceutically acceptable carriers that may be used in the preparation of the combined preparation include the same ones as those used in the pharmaceutical composition of the present invention described above.

The dosage of the concomitant drug can be appropriately selected based on the dosage usually used in clinical practice. The mixing ratio of the compound of the present invention or a pharmaceutically acceptable salt thereof to the concomitant drug can be appropriately selected depending on the subject of administration (the subject's age, body weight, general health condition, sex, degree of disease, etc.), the administration route, the type of disease, the type of concomitant drug, etc.

The mass ratio of the compound or a pharmaceutically acceptable salt thereof to the concomitant drug is not particularly limited.

Furthermore, the concomitant drugs that complement and/or enhance the therapeutic effect of the compound or a pharmaceutically acceptable salt thereof include not only those that have been found so far, but also those that will be found in the future, based on the above-mentioned mechanism.

The pharmaceutical composition of the present invention may be provided in the form of a kit together with instructions on the administration method and the like. The drug contained in the kit is supplied in a container made of a material that effectively maintains the activity of the components of the pharmaceutical composition for a long period of time, does not adsorb to the inside of the container, and does not deteriorate the components. For example, a sealed glass ampoule may contain a buffer or the like sealed in the presence of a neutral and inert gas such as nitrogen gas.

The kit may also include an instruction manual for use. The instruction manual for use of the kit may be provided to the user in a form printed on paper or stored on an electromagnetically readable medium such as a CD-ROM or DVD-ROM.

### Test Example 1: In vitro IL-1β production test

Human monocyte-derived THP-1 cells were cultured for one day in 10% bovine serum-containing RPMI-1640 medium (Thermo #A10491-01) supplemented with 200 nM phorbol 12-myristate 13-acetate (PMA, Sigma #P8139) to induce differentiation.

NLRP3 inflammasome was induced by replacing the culture medium with bovine serum-free RPMI-1640 medium supplemented with 0.015 mM Nigericin (Sigma #SML1779) and the test compound of known concentrations.

After 2 hours, the culture medium supernatant was collected and the IL-1β protein concentration was measured using the AlphaLISA (registered trademark) assay (PerkinElmer #AL220C).

The IL-1β concentration without the addition of the test compound and with Nigericin stimulation was set as 0% inhibitory activity, and the IL-1β concentration without Nigericin stimulation was set as 100% inhibitory activity, and the inhibition rate (% inhibition) of the test compound at each added concentration was calculated.

The % inhibition value was subjected to sigmoid fitting using a logistic regression model to calculate the 50% inhibitory concentration (IC₅₀ value).

The pharmacological activity data of the test compounds described in the Examples are shown in Table 1. These results indicate that the compounds of the present invention suppress NLRP3 inflammasome-dependent IL-1β production.

In the table, Ex indicates the Example number.

**[Table 1]**

| Ex | IC₅₀ (nM) | Ex | IC₅₀ (nM) | Ex | IC₅₀ (nM) | Ex | IC₅₀ (nM) |
|---|---|---|---|---|---|---|---|
| 1 | 5.27 | 26 | 6.18 | 51 | 33.62 | 76 | 10.08 |
| 2 | 5.26 | 27 | 25.56 | 52 | 39.09 | 77 | 14.00 |
| 3 | 52.48 | 28 | 12.00 | 53 | 275.31 | 78 | 35.04 |
| 4 | 19.76 | 29 | 12.38 | 54 | 84.31 | 79 | 7.49 |
| 5 | 31.32 | 30 | 14.37 | 55 | 23.79 | 80 | 22.36 |
| 6 | 26.02 | 31 | 412.75 | 56 | 14.40 | 81 | 54.72 |
| 7 | 67.61 | 32 | 30.59 | 57 | 57.24 | 82 | 7.83 |
| 8 | 15.42 | 33 | 10.00 | 58 | 55.82 | 83 | 9.16 |
| 9 | 13.19 | 34 | 9.07 | 59 | 95.81 | 84 | 12.83 |
| 10 | 23.72 | 35 | 25.6 | 60 | 74.75 | 85 | 65.33 |
| 11 | 7.32 | 36 | 42.07 | 61 | 25.34 | 86 | 141.47 |
| 12 | 25.89 | 37 | 25.77 | 62 | 70.78 | 87 | 40.09 |
| 13 | 37.63 | 38 | 108.13 | 63 | 42.29 | 88 | 43.06 |
| 14 | 7.87 | 39 | 400.96 | 64 | 9.60 | 89 | 138.18 |
| 15 | 4.86 | 40 | 161.89 | 65 | 47.01 | 90 | 56.46 |
| 16 | 8.60 | 41 | 77.46 | 66 | 16.36 | 91 | 124.85 |
| 17 | 9.47 | 42 | 74.02 | 67 | 4.93 | 92 | 59.16 |
| 18 | 6.65 | 43 | 22.14 | 68 | 6.01 | 93 | 30.05 |
| 19 | 9.99 | 44 | 74.96 | 69 | 47.76 | 94 | 79.59 |
| 20 | 8.19 | 45 | 8.81 | 70 | 31.22 | 95 | 19.69 |
| 21 | 19.79 | 46 | 91.41 | 71 | 8.18 | 96 | 11.49 |
| 22 | 10.51 | 47 | 80.82 | 72 | 53.09 | 97 | 8.33 |
| 23 | 11.81 | 48 | 100.98 | 73 | 13.83 | 98 | 13.79 |
| 24 | 34.81 | 49 | 78.04 | 74 | 26.07 | 99 | 9.53 |
| 25 | 29.19 | 50 | 235.66 | 75 | 98.41 | | |

### Test Example 2: Pharmacokinetics Test in Mice

Two hours after oral administration of the test compound (10 mg/kg) to male BALB/c mice, blood was collected from the posterior vena cava under isoflurane anesthesia, and plasma was obtained by centrifugation. In addition, the brain was collected after euthanasia by exsanguination, and distilled water was added to the obtained brain sample in an amount four times the wet weight of the brain to prepare a 20% homogenate. The compound concentrations in the obtained plasma and brain homogenate were measured by LC/MS/MS. The central transfer parameter Kp,brain was calculated by standardizing the concentration in the brain homogenate with the concentration in plasma. In the table, Ex indicates the example number. In the table, Ex indicates the Example number.

**[Table 2]**

| Ex | Kp, brain |
|---|---|
| 3 | 1.10 |
| 4 | 0.65 |
| 5 | 1.68 |
| 10 | 0.70 |
| 21 | 0.83 |
| 27 | 0.92 |

The results of this test show that the compound of the present invention exhibits excellent pharmacokinetics and central transferability, and is therefore expected to have excellent efficacy as a medicament.

### [Examples]

The present invention is described in detail by the following examples, but these examples are merely illustrative and do not limit the present invention, and may be changed without departing from the scope of the present invention.

In the following examples, "%" indicates mol/mol% for yield, volume % for solvents used in chromatography, and weight % for others.

For nuclear magnetic resonance spectra (hereinafter ¹H-NMR, resonance frequency 400 MHz or 500 MHz), chemical shift value is expressed as a δ value (ppm) using tetramethylsilane as a standard substance or the chemical shift value of the used deuterated solvent as a reference value.

Other abbreviations used in the text have the following meanings.
s: singlet
d: doublet
dd: doublet of doublets
t: triplet
q: quartet
m: multiplet
br: broad
J: coupling constant
Hz: hertz
CDCl₃: deuterochloroform
DMSO-d₆: deuterodimethyl sulfoxide
CD₃OD: deuteromethanol
¹H-NMR: proton nuclear magnetic resonance
HPLC: high-performance liquid chromatography
APCI: atmospheric pressure chemical ionization
ESI: electrospray ionization

In addition, the following abbreviations may be used in the description of the examples.
ASCA-2: A metal-supported catalyst mainly consisting of palladium that is very effective in hydrogenolysis reactions. It is particularly highly reactive in debenzylation reactions. Manufactured by N.E. Chemcat Corporation.
DCE: 1,2-dichloroethane
DCM: dichloromethane
DEE: diethyl ether
DDQ: 2,3-dichloro-5,6-dicyano-p-benzoquinone
DIPEA: N,N-diisopropylethylamine
DMF: N,N-dimethylformamide
DMSO: dimethyl sulfoxide
iPrOBPin: 2-isopropoxy-4,4,5,5-tetramethyl-1,3,2-dioxaborolane
NaBH(OAc)₃: sodium triacetoxyborohydride
NMP: N-methyl-2-pyrrolidinone
Pd₂(dba)₃: tris(dibenzylideneacetone)dipalladium(0)
Pd(dppf)Cl₂: [1,1'-bis(diphenylphosphino)ferrocene]dichloropalladium(II) dichloromethane additive
Pd(PPh₃)₄: tetrakis(triphenylphosphine)palladium(0)
RuPhos: 2-dicyclohexylphosphino-2',6'-diisopropoxybiphenyl Selectfluor (registered trademark): 1-chloromethyl-4-fluoro-1,4- diazoniabicyclo[2.2.2]octane bis(tetrafluoroborate)
TBAF: tetrabutylammonium fluoride
tBuXPhos: 2-di-tert-butylphosphino-2',4',6'-triisopropylbiphenyl
tBuXPhos Pd G1: [2-(di-tert-butylphosphino)-2',4',6'-triisopropyl-1,1'-biphenyl][2-(2-aminoethyl)phenyl) ]palladium(II) chloride
Tf₂O: trifluoromethanesulfonic anhydride
TEA: triethylamine
THF: tetrahydrofuran
TFA: trifluoroacetic acid
XPhos: 2-dicyclohexylphosphino-2',4',6'-triisopropylbiphenyl
XPhos Pd G2: chloro(2-dicyclohexylphosphino-2',4',6'-triisopropyl-1,1'-biphenyl)[2-(2'-amino-1,1'-biphenyl)]palladium(II)
XPhos Pd G3: (2-dicyclohexylphosphino-2',4',6'-triisopropyl-1,1'-biphenyl) [2-(2'-amino-1,1'-biphenyl)]palladium(II)

The reagents, solvents, devices, etc. used in the following examples are commercially available unless otherwise specified. Furthermore, the raw material compounds used are known compounds and commercially available, or compounds synthesized and identified according to a method known per se or a method analogous thereto, unless otherwise specified.

### (Example 1)

### 3-methyl-2-{7-[(3R)-1-methylpiperidin-3-yl]-6,7-dihydro-5H-pyrrolo[2,3-c]pyridazin-3-yl}-5-(trifluoromethyl)phenol

### (1A) 2-iodo-1-(methoxymethoxy)-3-methyl-5-(trifluoromethyl)benzene

2-Iodo-3-methyl-5-trifluoromethylphenol (US20200361898) (32 g, 110 mmol) and TEA (37 mL, 210 mmol) were dissolved in DCE (210 mL), chloromethyl methyl ether (10 mL, 140 mmol) was added at 0°C, and the mixture was stirred in the nitrogen-substituted system at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-90/10(V/V)] to give the title compound (21 g, yield: 57%) as an oil.

### (1B) 2-[2-(methoxymethoxy)-6-methyl-4-(trifluoromethyl)phenyl]-4,4,5,5-tetramethyl-1,3,2-dioxaborolane

2-Iodo-1-(methoxymethoxy)-3-methyl-5-(trifluoromethyl)benzene (17.1 g, 49 mmol) of Example 1A was dissolved in THF (250 mL), n-butyllithium (1.6 M n-hexane solution) (40 mL, 64 mmol) was added at -78°C, and the mixture was stirred in the nitrogen-substituted system at -78°C for 30 min. iPrOBPin (15 mL, 74 mmol) was added at -78°C, and the mixture was stirred at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-60/40(V/V)] to give the title compound (13.5 g, yield: 79%) as a solid.

### (1C) 3,6-dichloro-4-ethenylpyridazine

4-Bromo-3,6-dichloropyridazine (2.04 g, 9.0 mmol), potassium vinyltrifluoroborate (1.44 g, 11 mmol) and potassium carbonate (2.47 g, 18 mmol) were dissolved in a mixed solvent of 1,4-dioxane (45 mL) and water (10 mL), Pd(dppf)Cl₂ (731 mg, 0.90 mmol) was added, and the mixture was stirred in the nitrogen-substituted system at 100°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-70/30(V/V)] to give the title compound (1.34 g, yield: 86%) as a solid.

### (1D) tert-butyl (3R)-3-(3-chloro-5,6-dihydro-7H-pyrrolo[2,3-c]pyridazin-7-yl)piperidine-1-carboxylate

3,6-Dichloro-4-ethenylpyridazine (1.45 g, 8.3 mmol) of Example 1C, and DIPEA (2.16 mL, 12 mmol) were dissolved in NMP (16 mL), tert-butyl (3R)-3-aminopiperidine-1-carboxylate (1.99 g, 9.9 mmol) was added, and the mixture was stirred under microwave irradiation at 150°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-0/100(V/V)] to give the title compound (971 mg, yield: 35%) as an amorphous form.

### (1E) tert-butyl (3R)-3-{3-[2-(methoxymethoxy)-6-methyl-4-(trifluoromethyl)phenyl]-5,6-dihydro-7H-pyrrolo[2,3-c]pyridazin-7-yl}piperidine-1-carboxylate

tert-Butyl (3R)-3-(3-chloro-5,6-dihydro-7H-pyrrolo[2,3-c]pyridazin-7-yl)piperidine-1-carboxylate (969 mg, 2.9 mmol) of Example 1D, 2-[2-(methoxymethoxy)-6-methyl-4-(trifluoromethyl)phenyl]-4,4,5,5-tetramethyl-1,3,2-dioxaborolane (1.19 g, 3.4 mmol) of Example 1B, and potassium carbonate (790 mg, 5.7 mmol) were dissolved in a mixed solvent of 1,4-dioxane (4.0 mL) and water (1.0 mL), XPhos Pd G2 (225 mg, 0.29 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =70/30-0/100(V/V)] to give the title compound (870 mg, yield: 58%) as an amorphous form.

### (1F) 3-methyl-2-{7-[(3R)-piperidin-3-yl]-6,7-dihydro-5H-pyrrolo[2,3-c]pyridazin-3-yl}-5-(trifluoromethyl)phenol dihydrochloride

To tert-butyl (3R)-3-{3-[2-(methoxymethoxy)-6-methyl-4-(trifluoromethyl)phenyl]-5,6-dihydro-7H-pyrrolo[2,3-c]pyridazin-7-yl}piperidine-1-carboxylate (865 mg, 1.7 mmol) of Example 1E was added 4 M hydrogen chloride-1,4-dioxane (16 mL) at 0°C, and the mixture was stirred at room temperature for 1 hr. The reaction solution was concentrated under reduced pressure to give a crude product (689 mg, yield: 92%) of the title compound as a solid.

### (1G) 3-methyl-2-{7-[(3R)-1-methylpiperidin-3-yl]-6,7-dihydro-5H-pyrrolo[2,3-c]pyridazin-3-yl}-5-(trifluoromethyl)phenol

3-Methyl-2-{7-[(3R)-piperidin-3-yl]-6,7-dihydro-5H-pyrrolo[2,3-c]pyridazin-3-yl}-5-(trifluoromethyl)phenol dihydrochloride (689 mg, 1.5 mmol) of Example 1F, and DIPEA (0.53 mL, 3.1 mmol) were dissolved in DCM (15 mL), then 37% formaldehyde (0.12 mL, 1.7 mmol), acetic acid (0.175 mL, 3.1 mmol) and NaBH(OAc)₃ (1.30 g, 6.1 mmol) were added at 0°C, and the mixture was stirred at room temperature for 1 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-40/60(V/V)]. To the obtained solid were added ethyl acetate and n-hexane, and the mixture was subjected to ultrasonic treatment. The solid was collected by filtration to give the title compound (479 mg, yield: 80%) as a solid.

### (Example 2)

### 3-methyl-2-{7-[(3R)-1-methylpiperidin-3-yl]-7H-pyrrolo[2,3-c]pyridazin-3-yl}-5-(trifluoromethyl)phenol

### (2A) tert-butyl (3R)-3-(3-chloro-7H-pyrrolo[2,3-c]pyridazin-7-yl)piperidine-1-carboxylate

tert-Butyl (3R)-3-(3-chloro-5,6-dihydro-7H-pyrrolo[2,3-c]pyridazin-7-yl)piperidine-1-carboxylate (830 mg, 2.4 mmol) of Example 1D was dissolved in toluene (12 mL), DDQ (667 mg, 2.9 mmol) was added, and the mixture was stirred in the nitrogen-substituted system at 80°C for 1.5 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =60/40-33/67(V/V)] to give the title compound (483 mg, yield: 59%) as an amorphous form.

### (2B) 3-chloro-7-[(3R)-piperidin-3-yl]-7H-pyrrolo[2,3-c]pyridazine monohydrochloride

To tert-butyl (3R)-3-(3-chloro-7H-pyrrolo[2,3-c]pyridazin-7-yl)piperidine-1-carboxylate (284 mg, 0.84 mmol) of Example 2A was added 4 M hydrogen chloride-1,4-dioxane (4.0 mL), and the mixture was stirred at room temperature for 1 hr. The reaction solution was concentrated under reduced pressure to give a crude product (230 mg, yield: quantitative) of the title compound as a solid.

### (2C) 3-chloro-7-[(3R)-1-methylpiperidin-3-yl]-7H-pyrrolo[2,3-c]pyridazine

3-Chloro-7-[(3R)-piperidin-3-yl]-7H-pyrrolo[2,3-c]pyridazine monohydrochloride (230 mg, 0.84 mmol) of Example 2B, and DIPEA (0.293 mL, 1.7 mmol) were dissolved in DCM (4.0 mL), then 37% formaldehyde (0.094 mL, 1.3 mmol), acetic acid (0.101 mL, 1.7 mmol) and NaBH(OAc)₃ (713 mg, 3.4 mmol) were added at 0°C, and the mixture was stirred in the nitrogen-substituted system at room temperature for 30 min. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =50/50-34/66(V/V)] to give the title compound (190 mg, yield: 90%) as an oil.

### (2D) 3-[2-methoxy-6-methyl-4-(trifluoromethyl)phenyl]-7-[(3R)-1-methylpiperidin-3-yl]-7H-pyrrolo[2,3-c]pyridazine

3-Chloro-7-[(3R)-1-methylpiperidin-3-yl]-7H-pyrrolo[2,3-c]pyridazine (315 mg, 1.3 mmol) of Example 2C, 2-(2-methoxy-6-methyl-4-(trifluoromethylphenyl)-4,4,5,5-tetramethyl-1,3,2-dioxaborolane (US20200361898) (595 mg, 1.9 mmol), and potassium carbonate (347 mg, 2.5 mmol) were dissolved in a mixed solvent of 1,4-dioxane (6.0 mL) and water (1.5 mL), XPhos Pd G2 (98 mg, 0.13 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =40/60-20/80(V/V)] to give the title compound (497 mg, yield: 98%) as an amorphous form.

### (2E) 3-methyl-2-{7-[(3R)-1-methylpiperidin-3-yl]-7H-pyrrolo[2,3-c]pyridazin-3-yl}-5-(trifluoromethyl)phenol

3-[2-Methoxy-6-methyl-4-(trifluoromethyl)phenyl]-7-[(3R)-1-methylpiperidin-3-yl]-7H-pyrrolo[2,3-c]pyridazine (497 mg, 1.2 mmol) of Example 2D was dissolved in DCM (12 mL), boron tribromide (1.0 M DCM solution) (6.0 mL, 6.0 mmol) was added at 0°C, and the mixture was stirred in the nitrogen-substituted system at the same temperature for 1 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=98/2-95/5(V/V)]. To the obtained solid were added ethyl acetate and n-hexane, and the mixture was subjected to ultrasonic treatment. The solid was collected by filtration to give the title compound (345 mg, yield: 72%) as a solid.

### (Example 3)

### 2-{7-[(1s,3s)-3-hydroxy-3-methylcyclobutyl]-7H-pyrrolo[2,3-c]pyridazin-3-yl}-3-methyl-5-(trifluoromethyl)phenol (also referred to as 2-[7-(cis-3-hydroxy-3-methylcyclobutyl)-7H-pyrrolo[2,3-c]pyridazin-3-yl]-3-methyl-5-(trifluoromethyl)phenol)

### (3A) (1s,3s)-3-(3-chloro-5,6-dihydro-7H-pyrrolo[2,3-c]pyridazin-7-yl)-1-methylcyclobutanol

3,6-Dichloro-4-ethenylpyridazine (2.0 g, 11 mmol) of Example 1C, and DIPEA (4.4 mL, 25 mmol) were dissolved in NMP (19 mL), (1s,3s)-3-amino-1-methyl-cyclobutanol hydrochloride (1.7 g, 13 mmol) was added, and the mixture was stirred under microwave irradiation at 150°C for 1 hr. The reaction solution was concentrated under reduced pressure, and the obtained residue was purified by silica gel column chromatography [eluent: ethyl acetate/methanol=98/2-90/10(V/V)] to give the title compound (1.87 g, yield: 68%) as an oil.

### (3B) (1s,3s)-3-(3-chloro-7H-pyrrolo[2,3-c]pyridazin-7-yl)-1-methylcyclobutanol

(1s,3s)-3-(3-Chloro-5,6-dihydro-7H-pyrrolo[2,3-c]pyridazin-7-yl)-1-methylcyclobutanol (1.87 g, 7.8 mmol) of Example 3A was dissolved in DCE (25 mL), manganese dioxide (6.78 g, 78 mmol) was added, and the mixture was stirred in the nitrogen-substituted system at 80°C for 1 hr. The reaction solution was allowed to room temperature, and filtered through Celite, and the filtrate was concentrated under reduced pressure. The obtained residue was purified by silica gel column chromatography [eluent: n-hexane/ethyl acetate =30/70-0/100(V/V)] to give the title compound (855 mg, yield: 46%) as an amorphous form.

### (3C) (1s,3s)-3-(3-[2-(methoxymethoxy)-6-methyl-4-(trifluoromethyl)phenyl]-7H-pyrrolo[2,3-c]pyridazin-7-yl}-1-methylcyclobutanol

(1s,3s)-3-(3-Chloro-7H-pyrrolo[2,3-c]pyridazin-7-yl)-1-methylcyclobutanol (166 mg, 0.74 mmol) of Example 3B, 2-[2-(methoxymethoxy)-6-methyl-4-(trifluoromethyl)phenyl]-4,4,5,5-tetramethyl-1,3,2-dioxaborolane (308 mg, 0.89 mmol) of Example 1B, and potassium carbonate (204 mg, 1.5 mmol) were dissolved in a mixed solvent of 1,4-dioxane (14 mL) and water (6.0 mL), XPhos Pd G2 (58 mg, 0.074 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=100/0-90/10(V/V)] to give the title compound (301 mg, yield: 96%) as an amorphous form.

### (3D) 2-{7-[(1s,3s)-3-hydroxy-3-methylcyclobutyl]-7H-pyrrolo[2,3-c]pyridazin-3-yl}-3-methyl-5-(trifluoromethyl)phenol

To (1s,3s)-3-{3-[2-(methoxymethoxy)-6-methyl-4-(trifluoromethyl)phenyl]-7H-pyrrolo[2,3-c]pyridazin-7-yl}-1-methylcyclobutanol (387 mg, 0.91 mmol) of Example 3C was added 4 M hydrogen chloride-1,4-dioxane (4.6 mL) at 0°C, and the mixture was stirred at room temperature for 1 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=100/0-80/20(V/V)] to give the title compound (305 mg, yield: 88%) as a solid.

### (Example 4)

### 2-{7-[(1s,3s)-3-hydroxy-3-methylcyclobutyl]-5-methyl-7H-pyrrolo[2,3-c]pyridazin-3-yl}-3-methyl-5-(trifluoromethyl)phenol (also referred to as 2-[7-(cis-3-hydroxy-3-methylcyclobutyl)-5-methyl-7H-pyrrolo[2,3-c]pyridazin-3-yl]-3-methyl-5-(trifluoromethyl)phenol)

### (4A) 3,6-dichloro-4-(prop-1-en-2-yl)pyridazine

4-Bromo-3,6-dichloropyridazine (4.94 g, 22 mmol), potassium isopropenyltrifluoroborate (3.85 g, 26 mmol) and potassium carbonate (5.99 g, 43 mmol) were dissolved in a mixed solvent of 1,4-dioxane (108 mL) and water (40 mL), Pd(dppf)Cl₂ (1.77 g, 2.2 mmol) was added, and the mixture was stirred in the nitrogen-substituted system at 100°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-70/30(V/V)] to give the title compound (3.62 g, yield: 88%) as an oil.

### (4B) (1s,3s)-3-(3-chloro-5-methyl-5,6-dihydro-7H-pyrrolo[2,3-c]pyridazin-7-yl)-1-methylcyclobutanol

3,6-Dichloro-4-(prop-1-en-2-yl)pyridazine (244 mg, 1.3 mmol) of Example 4A, and DIPEA (0.67 mL, 3.9 mmol) were dissolved in NMP (4.3 mL), (1s,3s)-3-amino-1-methylcyclobutanol hydrochloride (213 mg, 1.5 mmol) was added, and the mixture was stirred under microwave irradiation at 150°C for 2 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =60/40-0/100(V/V)] to give the title compound (103 mg, yield: 31%) as an amorphous form.

### (4C) (1s,3s)-3-(3-chloro-5-methyl-7H-pyrrolo[2,3-c]pyridazin-7-yl)-1-methylcyclobutanol

(1s,3s)-3-(3-Chloro-5-methyl-5,6-dihydro-7H-pyrrolo[2,3-c]pyridazin-7-yl)-1-methylcyclobutanol (102 mg, 0.40 mmol) of Example 4B was dissolved in DCE (4.0 mL), manganese dioxide (350 mg, 4.0 mmol) was added, and the mixture was stirred in the nitrogen-substituted system at 80°C for 1 hr. The reaction solution was allowed to room temperature, and filtered through Celite, and the filtrate was concentrated under reduced pressure. The obtained residue was purified by silica gel column chromatography [eluent: n-hexane/ethyl acetate =70/30-0/100(V/V)] to give the title compound (65 mg, yield: 64%) as a solid.

### (4D) (1s,3s)-3-(3-[2-(methoxymethoxy)-6-methyl-4-(trifluoromethyl)phenyl]-5-methyl-7H-pyrrolo[2,3-c]pyridazin-7-yl}-1-methylcyclobutanol

(1s,3s)-3-(3-Chloro-5-methyl-7H-pyrrolo[2,3-c]pyridazin-7-yl)-1-methylcyclobutanol (25 mg, 0.099 mmol) of Example 4C, 2-[2-(methoxymethoxy)-6-methyl-4-(trifluoromethyl)phenyl]-4,4,5,5-tetramethyl-1,3,2-dioxaborolane (41 mg, 0.12 mmol) of Example 1B, and potassium carbonate (27 mg, 0.20 mmol) were dissolved in a mixed solvent of 1,4-dioxane (1.0 mL) and water (0.5 mL), XPhos Pd G2 (7.8 mg, 0.0099 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =60/40-0/100(V/V)] to give the title compound (34 mg, yield: 79%) as an amorphous form.

### (4E) 2-{7-[(1s,3s)-3-hydroxy-3-methylcyclobutyl]-5-methyl-7H-pyrrolo[2,3-c]pyridazin-3-yl}-3-methyl-5-(trifluoromethyl)phenol

To (1s,3s)-3-{3-[2-(methoxymethoxy)-6-methyl-4-(trifluoromethyl)phenyl]-5-methyl-7H-pyrrolo[2,3-c]pyridazin-7-yl}-1-methylcyclobutanol (34 mg, 0.078 mmol) of Example 4D was added 4 M hydrogen chloride-1,4-dioxane (1.0 mL), and the mixture was stirred at room temperature for 1 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=100/0-80/20(V/V)] to give the title compound (27 mg, yield: 88%) as a solid.

### (Example 5)

### 2-{5-fluoro-7-[(1s,3s)-3-hydroxy-3-methylcyclobutyl]-7H-pyrrolo[2,3-c]pyridazin-3-yl}-3-methyl-5-(trifluoromethyl)phenol (also referred to as 2-[5-fluoro-7-(cis-3-hydroxy-3-methylcyclobutyl)-7H-pyrrolo[2,3-c]pyridazin-3-yl]-3-methyl-5-(trifluoromethyl)phenol)

### (5A) 3-chloro-7-{(1s,3s)-3-[(4-methoxybenzyl)oxy]-3-methylcyclobutyl}-7H-pyrrolo[2,3-c]pyridazine

(1s,3s)-3-(3-Chloro-7H-pyrrolo[2,3-c]pyridazin-7-yl)-1-methylcyclobutanol (1.5 g, 6.3 mmol) of Example 3B was dissolved in DMF (21 mL), then 55% sodium hydride (550 mg, 13 mmol) and paramethoxybenzyl chloride (2.6 mL, 19 mmol) were added at 0°C, and the mixture was stirred in the nitrogen-substituted system at room temperature for 6 hr. To the reaction solution was added water at 0°C, and the mixture was subjected to extraction with ethyl acetate. The organic layer was washed with water and saturated brine, and dried over anhydrous sodium sulfate. After filtration, the filtrate was concentrated under reduced pressure, and the obtained residue was purified by silica gel column chromatography [eluent: n-hexane/ethyl acetate =75/25-60/40(V/V)] and NH silica gel column chromatography [eluent: n-hexane/ethyl acetate =75/25-65/35(V/V)] to give the title compound (2.18 g, yield: 97%) as an oil.

### (5B) 3-chloro-5-fluoro-7-f(1s,3s)-3-[(4-methoxybenzyl)oxy]-3-methylcyclobutyl}-7H-pyrrolo[2,3-c]pyridazine

3-Chloro-7-{ (1s,3s)-3-[(4-methoxybenzyl)oxy]-3-methylcyclobutyl}-7H-pyrrolo[2,3-c]pyridazine (2.18 g, 6.1 mmol) of Example 5A was dissolved in acetonitrile (30 mL), Selectfluor (registered trademark) (2.59 g, 7.3 mmol) was added at 0°C, and the mixture was stirred in the nitrogen-substituted system at room temperature for 4 hr. To the reaction solution were added methanol and water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-65/35(V/V)] to give the title compound (759 mg, yield: 33%) as an oil.

### (5C) 5-fluoro-7-{(1s,3s)-3-[(4-methoxybenzyl)oxy]-3-methylcyclobutyl}-3-[2-(methoxymethoxy)-6-methyl-4-(trifluoromethyl)phenyl]-7H-pyrrolo[2,3-c]pyridazine

3-Chloro-5-fluoro-7-{(1s,3s)-3-[(4-methoxybenzyl)oxy]-3-methylcyclobutyl}-7H-pyrrolo[2,3-c]pyridazine (80 mg, 0.21 mmol) of Example 5B, 2-[2-(methoxymethoxy)-6-methyl-4-(trifluoromethyl)phenyl]-4,4,5,5-tetramethyl-1,3,2-dioxaborolane (88 mg, 0.26 mmol) of Example 1B, and potassium carbonate (58 mg, 0.43 mmol) were dissolved in a mixed solvent of 1,4-dioxane (1.0 mL) and water (0.3 mL), XPhos Pd G2 (33 mg, 0.043 mmol) was added, and the mixture was stirred under microwave irradiation in the nitrogen-substituted system at 120°C for 1 hr. The reaction solution was allowed to room temperature, and the reaction solution was purified by NH silica gel column chromatography [eluent: n-hexane/ethyl acetate =75/25-65/35(V/V)] to give the title compound (83 mg, yield: 70%) as an amorphous form.

### (5D) 2-[5-fluoro-7-[(1s,3s)-3-hydroxy-3-methylcyclobutyl]-7H-pyrrolo[2,3-c]pyridazin-3-yl]-3-methyl-5-(trifluoromethyl)phenol

5-Fluoro-7-{ (1s,3s)-3-[(4-methoxybenzyl)oxy]-3-methylcyclobutyl}-3-[2-(methoxymethoxy)-6-methyl-4-(trifluoromethyl)phenyl]-7H-pyrrolo[2,3-c]pyridazine (83 mg, 0.15 mmol) of Example 5C was dissolved in DCM (0.7 mL), TFA (0.7 mL) was added, and the mixture was stirred at room temperature for 2 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=97/3-90/10(V/V)]. To the obtained solid were added ethyl acetate and n-hexane, and the mixture was subjected to ultrasonic treatment. The solid was collected by filtration to give the title compound (42 mg, yield: 72%) as a solid.

### (Example 6)

### 2-{5-chloro-7-[(1s,3s)-3-hydroxy-3-methylcyclobutyl]-7H-pyrrolo[2,3-c]pyridazin-3-yl}-3-methyl-5-(trifluoromethyl)phenol (also referred to as 2-[5-chloro-7-(cis-3-hydroxy-3-methylcyclobutyl)-7H-pyrrolo[2,3-c]pyridazin-3-yl]-3-methyl-5-(trifluoromethyl)phenol)

### (6A) (1s,3s)-3-{5-chloro-3-[2-(methoxymethoxy)-6-methyl-4-(trifluoromethyl)phenyl]-7H-pyrrolo[2,3-c]pyridazin-7-yl}-1-methylcyclobutanol

(1s,3s)-3-(3-[2-(Methoxymethoxy)-6-methyl-4-(trifluoromethyl)phenyl]-7H-pyrrolo[2,3-c]pyridazin-7-yl}-1-methylcyclobutanol (54 mg, 0.13 mmol) of Example 3C was dissolved in 1,4-dioxane (1.5 mL), 1-chloro-1,2-benziodoxol-3(1H)-one (75 mg, 0.27 mmol) was added, and the mixture was stirred in the nitrogen-substituted system at 120°C for 40 min. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =40/60-20/80(V/V)] to give the title compound (35 mg, yield: 60%) as an oil.

### (6B) 2-{5-chloro-7-[(1s,3s)-3-hydroxy-3-methylcyclobutyl]-7H-pyrrolo[2,3-c]pyridazin-3-yl}-3-methyl-5-(trifluoromethyl)phenol

(1s,3s)-3-{5-Chloro-3-[2-(methoxymethoxy)-6-methyl-4-(trifluoromethyl)phenyl]-7H-pyrrolo[2,3-c]pyridazin-7-yl}-1-methylcyclobutanol (35 mg, 0.77 mmol) of Example 6A was dissolved in DCM (0.4 mL), TFA (0.8 mL) was added, and the mixture was stirred at room temperature for 2 hr. Saturated aqueous sodium bicarbonate solution was added at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by preparative thin layer chromatography [eluent: DCM/methanol=90/10(V/V)] to give the title compound (15 mg, yield: 47%) as a solid.

### (Example 7)

### 2-{7-[(1s,3s)-3-(hydroxymethyl)-3-methylcyclobutyl]-7H-pyrrolo[2,3-c]pyridazin-3-yl}-3-methyl-5-(trifluoromethyl)phenol (also referred to as 2-[7-[cis-3-(hydroxymethyl)-3-methylcyclobutyl]-7H-pyrrolo[2,3-c]pyridazin-3-yl}-3-methyl-5-(trifluoromethyl)phenol)

### (7A) [(1s,3s)-3-(3-chloro-5,6-dihydro-7H-pyrrolo[2,3-c]pyridazin-7-yl)-1-methylcyclobutyl]methanol

(3-Amino-1-methyl-cyclobutyl)methanol hydrochloride (101.4 mg, 0.67 mmol), and 3,6-dichloro-4-ethenylpyridazine (117 mg, 0.67 mmol) of Example 1C were suspended in NMP (1.3 mL), DIPEA (0.343 mL, 2.0 mmol) was added, and the mixture was stirred in the nitrogen-substituted system at 130°C for 1 hr. The reaction solution was allowed to room temperature, water was added, and the mixture was subjected to extraction with ethyl acetate. Then, to the aqueous layer was added saturated aqueous sodium bicarbonate solution, and the mixture was subjected to extraction with ethyl acetate. The combined organic layers were washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (58 mg, yield: 34%) as an oil.

### (7B) 7-[(1s,3s)-3-({[tert-butyl(dimethyl)silyl]oxy}methyl)-3-methylcyclobutyl]-3-chloro-6,7-dihydro-5H-pyrrolo[2,3-c]pyridazine

[(1s,3s)-3-(3-Chloro-5,6-dihydro-7H-pyrrolo[2,3-c]pyridazin-7-yl)-1-methylcyclobutyl]methanol (58 mg, 0.23 mmol) of Example 7A was dissolved in DMF (1.5 mL), then imidazole (47 mg, 0.69 mmol) and tert-butyldimethylsilyl chloride (69 mg, 0.46 mmol) were added, and the mixture was stirred at room temperature for 30 min. To the reaction solution was added saturated aqueous sodium bicarbonate solution, and the mixture was subjected to extraction with ethyl acetate. The organic layer was washed with water and saturated brine, and dried over anhydrous sodium sulfate. After filtration, the filtrate was concentrated under reduced pressure to give a crude product (90 mg, yield: quantitative) of the title compound as an oil.

### (7C) 7-[(1s,3s)-3-({[tert-butyl(dimethyl)silyl]oxy}methyl)-3-methylcyclobutyl]-3-chloro-7H-pyrrolo[2,3-c]pyridazine

7-[(1s,3s)-3-({[tert-Butyl (dimethyl)silyl]oxy}methyl)-3-methylcyclobutyl]-3-chloro-6,7-dihydro-5H-pyrrolo[2,3-c]pyridazine (90 mg, 0.24 mmol) of Example 7B was dissolved in DCE (2.0 mL), manganese dioxide (689 mg, 8.0 mmol) was added, and the mixture was stirred at 80°C for 3 hr. The reaction solution was allowed to room temperature, and filtered through Celite, and the filtrate was concentrated under reduced pressure, and the residue was purified by silica gel column chromatography [eluent: n-hexane/ethyl acetate =90/10-67/33(V/V)] to give the title compound (51 mg, yield: 57%) as an oil.

### (7D) 7-[(1s,3s)-3-(([tert-butyl(dimethyl)silyl]oxylmethyl)-3-methylcyclobutyl]-3-[2-(methoxymethoxy)-6-methyl-4-(trifluoromethyl)phenyl]-7H-pyrrolo[2,3-c]pyridazine

7-[(1s,3s)-3-({[tert-Butyl(dimethyl)silyl]oxy}methyl)-3-methylcyclobutyl]-3-chloro-7H-pyrrolo[2,3-c]pyridazine (51 mg, 0.14 mmol) of Example 7C, 2-[2-(methoxymethoxy)-6-methyl-4-(trifluoromethyl)phenyl]-4,4,5,5-tetramethyl-1,3,2-dioxaborolane (85 mg, 0.25 mmol) of Example 1B, and potassium carbonate (39 mg, 0.28 mmol) were dissolved in a mixed solvent of 1,4-dioxane (1.5 mL) and water (0.375 mL), XPhos Pd G2 (22 mg, 0.028 mmol) was added, and the mixture was stirred under microwave irradiation at 120°C for 2 hr. The reaction solution was allowed to room temperature, and diluted with DCM, and the organic layer was separated through a phase separator (Biotage). The solvent was evaporated under reduced pressure, and the obtained residue was purified by silica gel column chromatography [eluent: n-hexane/ethyl acetate =100/0-80/20(V/V)] to give the title compound (29 mg, yield: 38%) as an oil.

### (7E) 2-{7-[(1s,3s)-3-(hydroxymethyl)-3-methylcyclobutyl]-7H-pyrrolo[2,3-c]pyridazin-3-yl}-3-methyl-5-(trifluoromethyl)phenol

To 7-[(1s,3s)-3-({[tert-butyl(dimethyl)silyl]oxy}methyl)-3-methylcyclobutyl]-3-[2-(methoxymethoxy)-6-methyl-4-(trifluoromethyl)phenyl]-7H-pyrrolo[2,3-c]pyridazine (29 mg, 0.053 mmol) of Example 7D was added 4 M hydrogen chloride-1,4-dioxane (1 mL), and the mixture was stirred at room temperature for 40 min. The reaction solution was concentrated under reduced pressure, and to the obtained residue were added DCM and saturated aqueous sodium bicarbonate solution. The mixture was stirred, and the organic layer was separated through a phase separator (Biotage). The solvent was evaporated under reduced pressure, and the obtained residue was purified by NH silica gel column chromatography [eluent: DCM/methanol=100/0-90/10(V/V)] and NH silica gel column chromatography [eluent: DCM/methanol=99/1-90/10(V/V)]. To the obtained residue were added ethyl acetate/n-hexane, and the mixture was subjected to ultrasonic treatment. The solid was collected by filtration to give the title compound (8.3 mg, yield: 40%) as a solid.

### (Example 8)

### 5-{3-[2-hydroxy-6-methyl-4-(trifluoromethyl)phenyl]-5-methyl-7H-pyrrolo[2,3-c]pyridazin-7-yl}bicyclo[3.1.1]heptan-1-ol

### (8A) 5-(3-chloro-5-methyl-5,6-dihydro-7H-pyrrolo[2,3-c]pyridazin-7-yl)bicyclo[3.1.1]heptan-1-ol

3,6-Dichloro-4-(prop-1-en-2-yl)pyridazine (250 mg, 1.3 mmol) of Example 4A, and DIPEA (0.460 mL, 2.6 mmol) were dissolved in NMP (3.5 mL), 5-aminobicyclo[3.1.1]heptanol (185 mg, 1.5 mmol) was added, and the mixture was stirred under microwave irradiation at 150°C for 5 hr. The reaction solution was concentrated under reduced pressure, and the obtained residue was purified by silica gel column chromatography [eluent: ethyl acetate/methanol=98/2-95/5(V/V)] to give the title compound (122 mg, yield: 33%) as an amorphous form.

### (8B) 5-(3-chloro-5-methyl-7H-pyrrolo[2,3-c]pyridazin-7-yl)bicyclo[3.1.1]heptan-1-ol

5-(3-Chloro-5-methyl-5,6-dihydro-7H-pyrrolo[2,3-c]pyridazin-7-yl)bicyclo[3.1.1]heptan-1-ol (122 mg, 0.44 mmol) of Example 8A was dissolved in DCE (2.0 mL), manganese dioxide (760 mg, 4.4 mmol) was added, and the mixture was stirred in the nitrogen-substituted system at 80°C for 80 min. The reaction solution was allowed to room temperature, and filtered through Celite, and the filtrate was concentrated under reduced pressure. The obtained residue was purified by silica gel column chromatography [eluent: n-hexane/ethyl acetate =40/60-20/80(V/V)] to give the title compound (43 mg, yield: 35%) as a solid.

### (8C) 5-{3-[2-(methoxymethoxy)-6-methyl-4-(trifluoromethyl)phenyl]-5-methyl-7H-pyrrolo[2,3-c]pyridazin-7-yl}bicyclo[3.1.1]heptan-1-ol

5-(3-Chloro-5-methyl-7H-pyrrolo[2,3-c]pyridazin-7-yl)bicyclo[3.1.1]heptan-1-ol (43 mg, 0.15 mmol) of Example 8B, 2-[2-(methoxymethoxy)-6-methyl-4-(trifluoromethyl)phenyl]-4,4,5,5-tetramethyl-1,3,2-dioxaborolane (64 mg, 0.19 mmol) of Example 1B, and potassium carbonate (42 mg, 0.31 mmol) were dissolved in a mixed solvent of 1,4-dioxane (0.8 mL) and water (0.2 mL), XPhos Pd G2 (24 mg, 0.031 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =40/60-10/90(V/V)] to give the title compound (64 mg, yield: 90%) as an oil.

### (8D) 5-{3-[2-hydroxy-6-methyl-4-(trifluoromethyl)phenyl]-5-methyl-7H-pyrrolo[2,3-c]pyridazin-7-yl}bicyclo[3.1.1]heptan-1-ol

5-{3-[2-(Methoxymethoxy)-6-methyl-4-(trifluoromethyl)phenyl]-5-methyl-7H-pyrrolo[2,3-c]pyridazin-7-yl}bicyclo[3.1.1]heptan-1-ol (64 mg, 0.14 mmol) of Example 8C was dissolved in DCM (0.7 mL), TFA (1.4 mL) was added, and the mixture was stirred at room temperature for 1.5 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=97/3-90/10(V/V)]. To the obtained solid were added ethyl acetate and n-hexane, and the mixture was subjected to ultrasonic treatment. The solid was collected by filtration to give the title compound (28 mg, yield: 48%) as a solid.

### (Example 9)

### 5-chloro-2-{7-[(1s,3s)-3-hydroxy-3-methylcyclobutyl]-5-methyl-7H-pyrrolo[2,3-c]pyridazin-3-yl}-3-methylphenol (also referred to as 5-chloro-2-[7-(cis-3-hydroxy-3-methylcyclobutyl)-5-methyl-7H-pyrrolo[2,3-c]pyridazin-3-yl]-3-methylphenol)

### (9A) 5-chloro-2-iodo-3-methylphenol

3-Chloro-5-methylphenol (1.16 g, 8.1 mmol) was dissolved in toluene (25 mL), 55% sodium hydride (0.717 g, 16 mmol) was added at 0°C, and the mixture was stirred for 30 min. To the reaction solution was added iodine (2.08 g, 8.2 mmol), and the mixture was stirred at 0°C for 5 hr. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =97/3-76/24(V/V)] to give the title compound (1.01 g, yield: 46%) as a solid.

### (9B) 5-chloro-2-iodo-1-(methoxymethoxy)-3-methylbenzene

5-Chloro-2-iodo-3-methylphenol (1.01 g, 3.8 mmol) of Example 9A was dissolved in acetone (15 mL), then potassium carbonate (1.11 g, 8.0 mmol) and chloromethyl methyl ether (0.43 mL, 5.7 mmol) were added, and the mixture was stirred at room temperature for 2 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =99/1-90/10(V/V)] to give the title compound (1.09 g, yield: 93%) as an oil.

### (9C) 2-[4-chloro-2-(methoxymethoxy)-6-methylphenyl]-4,4,5,5-tetramethyl-1,3,2-dioxaborolane

5-Chloro-2-iodo-1-(methoxymethoxy)-3-methylbenzene (1.09 g, 3.5 mmol) of Example 9B was dissolved in THF (12 mL), n-butyllithium (1.6 M n-hexane solution) (3.3 mL, 5.2 mmol) was added in the nitrogen-substituted system at -78°C, and the mixture was stirred for 30 min. To the reaction solution was added iPrOBPin (1.41 mL, 7.0 mmol) at -78°C, and the mixture was stirred at room temperature for 2 hr. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =99/1-90/10(V/V)] to give the title compound (0.740 g, yield: 68%) as a solid.

### (9D) (1s,3s)-3-{3-[4-chloro-2-(methoxymethoxy)-6-methylphenyl]-5-methyl-7H-pyrrolo[2,3-c]pyridazin-7-yl}-1-methylcyclobutanol

(1s,3s)-3-(3-Chloro-5-methyl-7H-pyrrolo[2,3-c]pyridazin-7-yl)-1-methylcyclobutanol (0.150 g, 0.60 mmol) of Example 4C, 2-[4-chloro-2-(methoxymethoxy)-6-methylphenyl]-4,4,5,5-tetramethyl-1,3,2-dioxaborolane (0.234 g, 0.75 mmol) of Example 9C, and potassium carbonate (0.173 g, 1.3 mmol) were dissolved in a mixed solvent of 1,4-dioxane (2.4 mL) and water (0.6 mL), XPhos Pd G2 (0.051 g, 0.065 mmol) was added, and the mixture was stirred in the nitrogen-substituted system at 120°C for 3 hr. The reaction solution was concentrated under reduced pressure, and the obtained residue was purified by silica gel column chromatography [eluent: n-hexane/ethyl acetate =50/50-0/100(V/V)] to give the title compound (0.068 g, yield: 28%) as an oil.

### (9E) 5-chloro-2-{7-[(1s,3s)-3-hydroxy-3-methylcyclobutyl]-5-methyl-7H-pyrrolo[2,3-c]pyridazin-3-yl}-3-methylphenol

To (1s,3s)-3-{3-[4-chloro-2-(methoxymethoxy)-6-methylphenyl]-5-methyl-7H-pyrrolo[2,3-c]pyridazin-7-yl}-1-methylcyclobutanol (0.068 g, 0.17 mmol) of Example 9D was added 4 M hydrogen chloride-1,4-dioxane (2 mL), and the mixture was stirred at room temperature for 4 hr. The reaction solution was concentrated under reduced pressure, and to the residue was added saturated aqueous sodium bicarbonate solution. The mixture was subjected to extraction with DCM, and the organic layer was dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (0.045 g, yield: 74%) as a solid.

### (Example 10)

### 2-{7-[(1s,3s)-3-hydroxy-3-methylcyclobutyl]-5-methoxy-7H-pyrrolo[2,3-c]pyridazin-3-yl}-3-methyl-5-(trifluoromethyl)phenol (also referred to as 2-[7-(cis-3-hydroxy-3-methylcyclobutyl)-5-methoxy-7H-pyrrolo[2,3-c]pyridazin-3-yl]-3-methyl-5-(trifluoromethyl)phenol)

### (10A) 7-[(1s,3s)-3-(benzyloxy)-3-methylcyclobutyl]-3-chloro-7H-pyrrolo[2,3-c]pyridazine

(1s,3s)-3-(3-Chloro-7H-pyrrolo[2,3-c]pyridazin-7-yl)-1-methylcyclobutanol (4.0 g, 17 mmol) of Example 3B was dissolved in DMF (42 mL), then 55% sodium hydride (1.0 g, 24 mmol) and benzyl bromide (2.6 mL, 22 mmol) were added at 0°C, and the mixture was stirred in the nitrogen-substituted system at room temperature 2.5 hr. Water was added at 0°C, and the mixture was subjected to extraction with ethyl acetate. The organic layer was washed with saturated brine, and dried over anhydrous sodium sulfate. After filtration, the filtrate was concentrated under reduced pressure, and the residue was purified by silica gel column chromatography [eluent: n-hexane/ethyl acetate =80/20-65/35(V/V)] to give the title compound (5.47 g, yield: 99%) as an oil.

### (10B) 7-[(1s,3s)-3-(benzyloxy)-3-methylcyclobutyl]-3-chloro-7H-pyrrolo[2,3-c]pyridazin-5-yl acetate

7-[(1s,3s)-3-(Benzyloxy)-3-methylcyclobutyl]-3-chloro-7H-pyrrolo[2,3-c]pyridazine (5.47 g, 17 mmol) of Example 10A was dissolved in acetic acid (33 mL), then palladium(II) acetate (750 mg, 3.3 mmol) and diacetoxyiodobenzene (8.0 g, 25 mmol) were added, and the mixture was stirred in the nitrogen-substituted system at 100°C 30 min. The reaction solution was concentrated under reduced pressure, to the obtained residue was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the residue was purified by silica gel column chromatography [eluent: n-hexane/ethyl acetate =80/20-65/35(V/V)] to give the title compound (2.1 g, yield: 33%) as an oil.

### (10C) 7-[(1s,3s)-3-(benzyloxy)-3-methylcyclobutyl]-3-chloro-7H-pyrrolo[2,3-c]pyridazin-5-ol

7-[(1s,3s)-3-(Benzyloxy)-3-methylcyclobutyl]-3-chloro-7H-pyrrolo[2,3-c]pyridazin-5-yl acetate (2.1 g, 5.4 mmol) of Example 10B was dissolved in methanol (27 mL), potassium carbonate (1.9 g, 14 mmol) was added at 0°C, and the mixture was stirred at room temperature for 30 min. To the reaction solution was added saturated ammonium chloride aqueous solution at 0°C, and the mixture was subjected to extraction with ethyl acetate. The organic layer was washed with saturated brine, and dried over anhydrous sodium sulfate. After filtration, the filtrate was concentrated under reduced pressure, to the obtained residue was added ethyl acetate, and the solid was collected by filtration to give the title compound (1.4 g, yield: 75%) as a solid.

### (10D) 7-[(1s,3s)-3-(benzyloxy)-3-methylcyclobutyl]-3-chloro-5-methoxy-7H-pyrrolo[2,3-c]pyridazine

7-[(1s,3s)-3-(Benzyloxy)-3-methylcyclobutyl]-3-chloro-7H-pyrrolo[2,3-c]pyridazin-5-ol (400 mg, 1.2 mmol) of Example 10C was dissolved in DMF (6.0 mL), then potassium carbonate (257 mg, 1.9 mmol) and iodomethane (0.094 mL, 1.5 mmol) were added in the nitrogen-substituted system at 0°C, and the mixture was stirred at room temperature for 3.5 hr. To the reaction solution was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =70/30-60/40(V/V)] to give the title compound (317 mg, yield: 76%) as an oil.

### (10E) 7-[(1s,3s)-3-(benzyloxy)-3-methylcyclobutyl]-5-methoxy-3-[2-(methoxymethoxy)-6-methyl-4-(trifluoromethyl)phenyl]-7H-pyrrolo[2,3-c]pyridazine

7-[(1s,3s)-3-(Benzyloxy)-3-methylcyclobutyl]-3-chloro-5-methoxy-7H-pyrrolo[2,3-c]pyridazine (220 mg, 0.61 mmol) of Example 10D, 2-[2-(methoxymethoxy)-6-methyl-4-(trifluoromethyl)phenyl]-4,4,5,5-tetramethyl-1,3,2-dioxaborolane (255 mg, 0.74 mmol) of Example 1B, and potassium carbonate (170 mg, 1.2 mmol) were dissolved in a mixed solvent of 1,4-dioxane (3.0 mL) and water (0.8 mL), XPhos Pd G2 (96 mg, 0.12 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =75/25-65/35(V/V)] to give the title compound (265 mg, yield: 80%) as a solid.

### (10F) (1s,3s)-3-{5-methoxy-3-[2-(methoxymethoxy)-6-methyl-4-(trifluoromethyl)phenyl]-7H-pyrrolo[2,3-c]pyridazin-7-yl}-1-methylcyclobutanol

7-[(1s,3s)-3-(Benzyloxy)-3-methylcyclobutyl]-5-methoxy-3-[2-(methoxymethoxy)-6-methyl-4-(trifluoromethyl)phenyl]-7H-pyrrolo[2,3-c]pyridazine (265 mg, 0.49 mmol) of Example 10E was dissolved in methanol (5.0 mL), ASCA-2 (100 mg) was added, and the mixture was stirred in the hydrogen-substituted system at 65°C for 6 hr. The reaction solution was filtered through Celite, the filtrate was concentrated under reduced pressure, and the obtained residue was purified by silica gel column chromatography [eluent: ethyl acetate/methanol=100/0-97/3(V/V)] to give the title compound (136 mg, yield: 62%) as a solid.

### (10G) 2-{7-[(1s,3s)-3-hydroxy-3-methylcyclobutyl]-5-methoxy-7H-pyrrolo[2,3-c]pyridazin-3-yl}-3-methyl-5-(trifluoromethyl)phenol

(1s,3s)-3-(5-Methoxy-3-[2-(methoxymethoxy)-6-methyl-4-(trifluoromethyl)phenyl]-7H-pyrrolo[2,3-c]pyridazin-7-yl}-1-methylcyclobutanol (136 mg, 0.30 mmol) of Example 10F was dissolved in DCM (1.5 mL), TFA (1.5 mL) was added, and the mixture was stirred at room temperature for 3 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=97/3-86/14(V/V)]. To the obtained solid were added ethyl acetate and n-hexane, and the mixture was subjected to ultrasonic treatment. The solid was collected by filtration to give the title compound (80 mg, yield: 65%) as a solid.

### (Example 11)

### 2-{7-[(3R)-1-ethylpiperidin-3-yl]-6,7-dihydro-5H-pyrrolo[2,3-c]pyridazin-3-yl}-3-methyl-5-(trifluoromethyl)phenol

3-Methyl-2-f7-[(3R)-piperidin-3-yl]-6,7-dihydro-5H-pyrrolo[2,3-c]pyridazin-3-yl}-5-(trifluoromethyl)phenol dihydrochloride (108 mg, 0.23 mmol) of Example 1F, and DIPEA (0.083 mL, 0.48 mmol) were dissolved in DCM (2.4 mL), then acetaldehyde (0.036 mL, 0.72 mmol), acetic acid (0.027 mL, 0.48 mmol) and NaBH(OAc)₃ (203 mg, 0.95 mmol) were added at 0°C, and the mixture was stirred at room temperature for 1 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=100/0-80/20(V/V)] to give the title compound (87 mg, yield: 89%) as a solid.

### (Example 12)

### 2-{8-[ (1s,3s)-3-hydroxy-3-methylcyclobutyl]-5,6,7,8-tetrahydrocyclopenta[4,5]pyrrolo[2,3-c]pyridazin-3-yl}-3-methyl-5-(trifluoromethyl)phenol (also referred to as 2-[8-(cis-3-hydroxy-3-methylcyclobutyl)-5,6,7,8-tetrahydrocyclopenta[4,5]pyrrolo[2,3-c]pyridazin-3-yl]-3-methyl-5-(trifluoromethyl)phenol)

### (12A) 4-(2-bromocyclopent-1-en-1-yl)-3,6-dichloropyridazine

4-Bromo-3,6-dichloropyridazine (3.20 g, 14 mmol), 2-bromo-1-cyclopentenylboronic acid (2.73 g, 14 mmol) and potassium carbonate (3.88 g, 28 mmol) were dissolved in a mixed solvent of 1,4-dioxane (70 mL) and water (15 mL), Pd(dppf)Cl₂ (0.51 g, 0.70 mmol) was added, and the mixture was stirred in the nitrogen-substituted system at 90°C for 1 hr. The reaction solution was allowed to room temperature, saturated aqueous sodium bicarbonate solution was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =97/3-76/24(V/V)] to give the title compound (3.42 g, yield: 83%) as an oil.

### (12B) (1s,3s)-3-{ [tert-butyl(dimethyl)silyl]oxy}-3-methylcyclobutanamine

(1s,3s)-3-Hydroxy-3-methylcyclobutylamine hydrochloride (2.10 g, 15 mmol) was suspended in DCM (30 mL), then imidazole (3.75 g, 55 mmol) and tert-butyldimethylsilyl chloride (3.37 g, 22 mmol) were added, and the mixture was stirred at room temperature for 23 hr. The reaction solution was concentrated under reduced pressure, to the residue was added water, and the mixture was subjected to extraction with ethyl acetate. The organic layer was washed with 1 M sodium hydroxide aqueous solution, water and saturated brine, and dried over anhydrous sodium sulfate. After filtration, the filtrate was concentrated under reduced pressure, and the obtained residue was purified by silica gel column chromatography [eluent: DCM/methanol=100/0-90/10(V/V)] to give the title compound (2.50 g, yield: 76%) as an oil.

### (12C) 8-((1s,3s)-3-{[tert-butyl(dimethyl)silyl]oxy}-3-methylcyclobutyl)-3-chloro-5,6,7,8-tetrahydrocyclopenta[4,5]pyrrolo[2,3-c]pyridazine

4-(2-Bromocyclopent-1-en-1-yl)-3,6-dichloropyridazine (3.21 g, 11 mmol) of Example 12A, and (1s,3s)-3-{[tert-butyl(dimethyl)silyl]oxy}-3-methylcyclobutanamine (2.35 g, 11 mmol) of Example 12B were dissolved in 1,4-dioxane (55 mL), then Pd₂(dba)₃ (2.00 g, 2.2 mmol), RuPhos (1.00 g, 2.1 mmol) and sodium tert-butoxide (3.15 g, 33 mmol) were added, and the mixture was stirred in the nitrogen-substituted system at 110°C for 2 hr. The reaction solution was filtered through Celite, the filtrate was concentrated under reduced pressure, and the obtained residue was purified by silica gel column chromatography [eluent: n-hexane/ethyl acetate =100/0-75/25(V/V)] and NH silica gel column chromatography [eluent: n-hexane/ethyl acetate =100/0-75/25(V/V)] to give the title compound (0.587 g, yield: 14%) as a solid.

### (12D) 8-((1s,3s)-3-{[tert-butyl(dimethyl)silyl]oxy}-3-methylcyclobutyl)-3-[2-(methoxymethoxy)-6-methyl-4-(trifluoromethyl)phenyl]-5,6,7,8-tetrahydrocyclopenta[4,5]pyrrolo[2,3-c]pyridazine

8-((1s,3s)-3-{[tert-Butyl (dimethyl)silyl]oxy}-3-methylcyclobutyl)-3-chloro-5,6,7,8-tetrahydrocyclopenta[4,5]pyrrolo[2,3-c]pyridazine (0.154 g, 0.39 mmol) of Example 12C, 2-[2-(methoxymethoxy)-6-methyl-4-(trifluoromethyl)phenyl]-4,4,5,5-tetramethyl-1,3,2-dioxaborolane (0.180 g, 0.52 mmol) of Example 1B, and potassium carbonate (0.110 g, 0.80 mmol) were dissolved in a mixed solvent of 1,4-dioxane (1.6 mL) and water (0.4 mL), XPhos Pd G2 (0.053 g, 0.067 mmol) was added, and the mixture was stirred under microwave irradiation in the nitrogen-substituted system at 120°C for 1 hr. The reaction solution was filtered, the filtrate was concentrated under reduced pressure, and the obtained residue was purified by silica gel column chromatography [eluent: n-hexane/ethyl acetate =100/0-75/25(V/V)] to give the title compound (0.112 g, yield: 50%) as a solid.

### (12E) 2-{8-[(1s,3s)-3-hydroxy-3-methylcyclobutyl]-5,6,7,8-tetrahydrocyclopenta[4,5]pyrrolo[2,3-c]pyridazin-3-yl}-3-methyl-5-(trifluoromethyl)phenol

To 8-((1s,3s)-3-{[tert-butyl(dimethyl)silyl]oxy}-3-methylcyclobutyl)-3-[2-(methoxymethoxy)-6-methyl-4-(trifluoromethyl)phenyl]-5,6,7,8-tetrahydrocyclopenta[4,5]pyrrolo[2,3-c]pyridazine (0.112 g, 0.20 mmol) of Example 12D was added 4 M hydrogen chloride-1,4-dioxane (2 mL), and the mixture was stirred at room temperature for 1 hr. The reaction solution was concentrated under reduced pressure, to the obtained residue was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (0.066 g, yield: 81%) as a solid.

### (Example 13)

### 5-(difluoromethoxy)-2-{7-[(1s,3s)-3-hydroxy-3-methylcyclobutyl]-5-methyl-7H-pyrrolo[2,3-c]pyridazin-3-yl}-3-methylphenol (also referred to as 5-(difluoromethoxy)-2-[7-(cis-3-hydroxy-3-methylcyclobutyl)-5-methyl-7H-pyrrolo[2,3-c]pyridazin-3-yl]-3-methylphenol)

### (13A) 3-(benzyloxy)-5-methylphenol

5-Methylresorcinol (7.0 g, 56 mmol) was dissolved in DMF (60 mL), 55% sodium hydride (4.5 g, 100 mmol) was added at 0°C, and the mixture was stirred for 30 min. To the reaction solution was added a solution of benzyl bromide (6.0 mL, 51 mmol) in DMF (20 mL), and the mixture was stirred at room temperature for 16 hr. To the reaction solution was added saturated ammonium chloride aqueous solution at 0°C, and the mixture was subjected to extraction with ethyl acetate. The organic layer was washed successively with water and saturated brine, and dried over anhydrous sodium sulfate. After filtration, the filtrate was concentrated under reduced pressure, and the obtained residue was purified by silica gel column chromatography [eluent: n-hexane/ethyl acetate =97/3-76/24(V/V)] to give the title compound (3.7 g, yield: 34%) as an oil.

### (13B) 1-(benzyloxy)-3-(difluoromethoxy)-5-methylbenzene

3-(Benzyloxy)-5-methylphenol (3.68 g, 17 mmol) of Example 13A was dissolved in a mixed solvent of acetonitrile (40 mL) and water (40 mL), then potassium hydroxide (12.4 g, 221 mmol) and diethyl (bromodifluoromethyl)phosphonate (9.2 mL, 52 mmol) were added at 0°C, and the mixture was stirred for 30 min. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =99/1-90/10(V/V)] to give the title compound (3.34 g, yield: 74%) as an oil.

### (13C) 3-(difluoromethoxy)-5-methylphenol

1-(Benzyloxy)-3-(difluoromethoxy)-5-methylbenzene (3.34 g, 13 mmol) of Example 13B was dissolved in ethanol (40 mL), ASCA-2 (0.218 g) was added, and the mixture was stirred in the hydrogen-substituted system at 60°C for 2 hr. The reaction solution was filtered through Celite, the filtrate was concentrated under reduced pressure, and the obtained residue was purified by silica gel column chromatography [eluent: n-hexane/ethyl acetate =97/3-76/24(V/V)] to give the title compound (2.65 g, yield: quantitative) as an oil.

### (13D) 5-(difluoromethoxy)-2-iodo-3-methylphenol

3-(Difluoromethoxy)-5-methylphenol (2.65 g, 15 mmol) of Example 13C was dissolved in toluene (45 mL), 55% sodium hydride (1.10 g, 25 mmol) was added at 0°C, and the mixture was stirred for 30 min. To the reaction solution was added iodine (3.85 g, 15 mmol), and the mixture was stirred at 0°C for 2 hr. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =97/3-85/15(V/V)] to give the title compound (2.34 g, yield: 51%) as an oil.

### (13E) 5-(difluoromethoxy)-2-iodo-1-(methoxymethoxy)-3-methylbenzene

5-(Difluoromethoxy)-2-iodo-3-methylphenol (2.34 g, 7.8 mmol) of Example 13D was dissolved in DCM (25 mL), then DIPEA (2.7 mL, 16 mmol) and chloromethyl methyl ether (0.88 mL, 12 mmol) were added, and the mixture was stirred at room temperature for 14 hr. To the reaction solution was added saturated ammonium chloride aqueous solution, and the mixture was subjected to extraction with DCM. The organic layer was dried over anhydrous sodium sulfate. After filtration, the filtrate was concentrated under reduced pressure, and the obtained residue was purified by silica gel column chromatography [eluent: n-hexane/ethyl acetate =99/1-90/10(V/V)] to give the title compound (2.24 g, yield: 84%) as an oil.

### (13F) 2-[4-(difluoromethoxy)-2-(methoxymethoxy)-6-methylphenyl]-4,4,5,5-tetramethyl-1,3,2-dioxaborolane

5-(Difluoromethoxy)-2-iodo-1-(methoxymethoxy)-3-methylbenzene (1.92 g, 5.6 mmol) of Example 13E, and iPrOBPin (2.3 mL, 11 mmol) were dissolved in THF (20 mL), n-butyllithium (1.6 M n-hexane solution) (5.3 mL, 8.4 mmol) was added in the nitrogen-substituted system at -78°C, and the mixture was stirred at room temperature for 2 hr. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =97/3-76/24(V/V)] to give the title compound (1.61 g, yield: 84%) as an oil.

### (13G) (1s,3s)-3-{3-[4-(difluoromethoxy)-2-(methoxymethoxy)-6-methylphenyl]-5-methyl-7H-pyrrolo[2,3-c]pyridazin-7-yl}-1-methylcyclobutanol

(1s,3s)-3-(3-Chloro-5-methyl-7H-pyrrolo[2,3-c]pyridazin-7-yl)-1-methylcyclobutanol (0.160 g, 0.64 mmol) of Example 4C, 2-[4-(difluoromethoxy)-2-(methoxymethoxy)-6-methylphenyl]-4,4,5,5-tetramethyl-1,3,2-dioxaborolane (0.283 g, 0.82 mmol) of Example 13F, and potassium carbonate (0.183 g, 1.3 mmol) were dissolved in a mixed solvent of 1,4-dioxane (2.5 mL) and water (0.6 mL), XPhos Pd G2 (0.100 g, 0.13 mmol) was added, and the mixture was stirred under microwave irradiation in the nitrogen-substituted system at 120°C for 1 hr. The reaction solution was concentrated under reduced pressure, and the obtained residue was purified by silica gel column chromatography [eluent: n-hexane/ethyl acetate =50/50-0/100(V/V)] to give the title compound (0.169 g, yield: 61%) as an oil.

### (13H) 5-(difluoromethoxy)-2-{7-[(1s,3s)-3-hydroxy-3-methylcyclobutyl]-5-methyl-7H-pyrrolo[2,3-c]pyridazin-3-yl}-3-methylphenol

To (1s,3s)-3-{3-[4-(difluoromethoxy)-2-(methoxymethoxy)-6-methylphenyl]-5-methyl-7H-pyrrolo[2,3-c]pyridazin-7-yl}-1-methylcyclobutanol (0.166 g, 0.38 mmol) of Example 13G was added 4 M hydrogen chloride-1,4-dioxane (4 mL), and the mixture was stirred at room temperature for 4 hr. The reaction solution was concentrated under reduced pressure, to the residue was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (0.115 g, yield: 77%) as a solid.

### (Example 14)

### 3-methyl-2-{7-[(3R)-1-propylpiperidin-3-yl]-6,7-dihydro-5H-pyrrolo[2,3-c]pyridazin-3-yl}-5-(trifluoromethyl)phenol

### (14A) 7-[(3R)-1-benzylpiperidin-3-yl]-3-chloro-6, 7-dihydro-5H-pyrrolo[2,3-c]pyridazine

3,6-Dichloro-4-ethenylpyridazine (1.00 g, 5.7 mmol) of Example 1C, and DIPEA (2.0 mL, 11 mmol) were dissolved in NMP (28 mL), (R)-3-amino-1-benzylpiperidine (1.30 g, 6.8 mmol) was added, and the mixture was stirred under microwave irradiation at 150°C for 30 min. The reaction solution was concentrated under reduced pressure, and the obtained residue was purified by NH silica gel column chromatography [eluent: n-hexane/ethyl acetate =90/10-50/50(V/V)] to give the title compound (1.11 g, yield: 59%) as an amorphous form.

### (14B) 7-[(3R)-1-benzylpiperidin-3-yl]-3-[2-(methoxymethoxy)-6-methyl-4-(trifluoromethyl)phenyl]-6,7-dihydro-5H-pyrrolo[2,3-c]pyridazine

7-[(3R)-1-Benzylpiperidin-3-yl]-3-chloro-6,7-dihydro-5H-pyrrolo[2,3-c]pyridazine (0.715 g, 2.2 mmol) of Example 14A, 2-[2-(methoxymethoxy)-6-methyl-4-(trifluoromethyl)phenyl]-4,4,5,5-tetramethyl-1,3,2-dioxaborolane (0.913 g, 2.6 mmol) of Example 1B, and potassium carbonate (0.601 g, 4.4 mmol) were dissolved in a mixed solvent of 1,4-dioxane (8 mL) and water (2 mL), XPhos Pd G2 (0.342 g, 0.44 mmol) was added, and the mixture was stirred under microwave irradiation in the nitrogen-substituted system at 120°C for 3 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =90/10-50/50(V/V)] to give the title compound (0.508 g, yield: 46%) as an amorphous form.

### (14C) 3-[2-(methoxymethoxy)-6-methyl-4-(trifluoromethyl)phenyl]-7-[(3R)-piperidin-3-yl]-6,7-dihydro-5H-pyrrolo[2,3-c]pyridazine

7-[(3R)-1-Benzylpiperidin-3-yl]-3-[2-(methoxymethoxy)-6-methyl-4-(trifluoromethyl)phenyl]-6,7-dihydro-5H-pyrrolo[2,3-c]pyridazine (0.630 g, 1.2 mmol) of Example 14B was dissolved in ethanol (12 mL), ASCA-2 (0.323 g) was added, and the mixture was stirred in the hydrogen-substituted system at room temperature for 2 hr, and then heated under reflux for 1 hr. The reaction solution was filtered through Celite, and the filtrate was concentrated under reduced pressure to give a crude product (0.479 g, yield: 92%) of the title compound as a solid.

### (14D) 3-[2-(methoxymethoxy)-6-methyl-4-(trifluoromethyl)phenyl]-7-[(3R)-1-propylpiperidin-3-yl]-6,7-dihydro-5H-pyrrolo[2,3-c]pyridazine

3-[2-(Methoxymethoxy)-6-methyl-4-(trifluoromethyl)phenyl]-7-[(3R)-piperidin-3-yl]-6,7-dihydro-5H-pyrrolo[2,3-c]pyridazine (0.067 g, 0.16 mmol) of Example 14C was dissolved in DCM (2 mL), then propionaldehyde (0.034 mL, 0.47 mmol) and NaBH(OAc)₃ (0.13 g, 0.61 mmol) were added, and the mixture was stirred at room temperature for 3 hr. To the reaction solution was added saturated aqueous sodium bicarbonate solution, the mixture was subjected to extraction with DCM, and the organic layer was dried over anhydrous sodium sulfate. After filtration, the filtrate was concentrated under reduced pressure, and the obtained residue was purified by silica gel column chromatography [eluent: DCM/methanol=100/0-70/30(V/V)] to give the title compound (0.044 g, yield: 60%) as an amorphous form.

### (14E) 3-methyl-2-{7-[(3R)-1-propylpiperidin-3-yl]-6,7-dihydro-5H-pyrrolo[2,3-c]pyridazin-3-yl}-5-(trifluoromethyl)phenol

To 3-[2-(methoxymethoxy)-6-methyl-4-(trifluoromethyl)phenyl]-7-[(3R)-1-propylpiperidin-3-yl]-6,7-dihydro-5H-pyrrolo[2,3-c]pyridazine (0.044 g, 0.095 mmol) of Example 14D was added 4 M hydrogen chloride-1,4-dioxane (1 mL), and the mixture was stirred at room temperature for 3 hr. The reaction solution was concentrated under reduced pressure, and to the residue was added saturated aqueous sodium bicarbonate solution. The mixture was subjected to extraction with DCM, and the organic layer was dried over anhydrous sodium sulfate. After filtration, the filtrate was concentrated under reduced pressure, and the obtained residue was purified by silica gel column chromatography [eluent: DCM/methanol=100/0-70/30(V/V)] and NH silica gel column chromatography [eluent: ethyl acetate/methanol=100/0-90/10(V/V)] to give the title compound (28 mg, yield: 70%) as a solid.

### (Example 15)

### 3-methyl-2-{7-[(3R)-1-(propan-2-yl)piperidin-3-yl]-6,7-dihydro-5H-pyrrolo[2,3-c]pyridazin-3-yl}-5-(trifluoromethyl)phenol

### (15A) 3-[2-(methoxymethoxy)-6-methyl-4-(trifluoromethyl)phenyl]-7-[(3R)-1-(propan-2-yl)piperidin-3-yl]-6,7-dihydro-5H-pyrrolo[2,3-c]pyridazine

3-[2-(Methoxymethoxy)-6-methyl-4-(trifluoromethyl)phenyl]-7-[(3R)-piperidin-3-yl]-6,7-dihydro-5H-pyrrolo[2,3-c]pyridazine (0.067 g, 0.16 mmol) of Example 14C was dissolved in DCM (2 mL), then acetone (0.56 mL, 7.7 mmol), acetic acid (0.018 mL, 0.31 mmol) and NaBH(OAc)₃ (0.13 g, 0.61 mmol) were added, and the mixture was stirred at room temperature for 5 hr. To the reaction solution was added saturated aqueous sodium bicarbonate solution, the mixture was subjected to extraction with DCM, and the organic layer was dried over anhydrous sodium sulfate. After filtration, the filtrate was concentrated under reduced pressure, and the obtained residue was purified by NH silica gel column chromatography [eluent: n-hexane/ethyl acetate =50/50-0/100(V/V)] to give the title compound (0.059 g, yield: 80%) as an amorphous form.

### (15B) 3-methyl-2-{7-[(3R)-1-(propan-2-yl)piperidin-3-yl]-6,7-dihydro-5H-pyrrolo[2,3-c]pyridazin-3-yl}-5-(trifluoromethyl)phenol

To 3-[2-(methoxymethoxy)-6-methyl-4-(trifluoromethyl)phenyl]-7-[(3R)-1-(propan-2-yl)piperidin-3-yl]-6,7-dihydro-5H-pyrrolo[2,3-c]pyridazine (0.059 g, 0.13 mmol) of Example 15A was added 4 M hydrogen chloride-1,4-dioxane (1 mL), and the mixture was stirred at room temperature for 3 hr. The reaction solution was concentrated under reduced pressure, and to the residue was added saturated aqueous sodium bicarbonate solution. The mixture was subjected to extraction with DCM, and the organic layer was dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (43 mg, yield: 81%) as a solid.

### (Example 16)

### 7-(5-hydroxybicyclo[3.1.1]heptan-1-yl)-3-[2-hydroxy-6-methyl-4-(trifluoromethyl)phenyl]-7H-pyrrolo[2,3-c]pyridazine-5-carbonitrile

### (16A) 5-(3-chloro-5,6-dihydro-7H-pyrrolo[2,3-c]pyridazin-7-yl)bicyclo[3.1.1]heptan-1-ol

3,6-Dichloro-4-ethenylpyridazine (1.0 g, 5.7 mmol) of Example 1C, and DIPEA (3.0 mL, 17 mmol) were dissolved in NMP (14 mL), 5-aminobicyclo[3.1.1]heptanol (800 mg, 6.3 mmol) was added, and the mixture was stirred under microwave irradiation at 150°C for 1 hr. The reaction solution was concentrated under reduced pressure, and the obtained residue was purified by silica gel column chromatography [eluent: ethyl acetate/methanol=98/2-95/5(V/V)] to give the title compound (690 mg, yield: 45%) as a solid.

### (16B) 5-(3-chloro-7H-pyrrolo[2,3-c]pyridazin-7-yl)bicyclo[3.1.1]heptan-1-ol

5-(3-Chloro-5,6-dihydro-7H-pyrrolo[2,3-c]pyridazin-7-yl)bicyclo[3.1.1]heptan-1-ol (687 mg, 2.6 mmol) of Example 16A was dissolved in DCE (13 mL), manganese dioxide (7.0 g, 78 mmol) was added, and the mixture was stirred in the nitrogen-substituted system at 80°C for 30 min. The reaction solution was filtered, the filtrate was concentrated under reduced pressure, and the obtained residue was purified by silica gel column chromatography [eluent: n-hexane/ethyl acetate =25/75-0/100(V/V)] to give the title compound (326 mg, yield: 48%) as a solid.

### (16C) 5-{3-[2-(methoxymethoxy)-6-methyl-4-(trifluoromethyl)phenyl]-7H-pyrrolo[2,3-c]pyridazin-7-yl}bicyclo[3.1.1]heptan-1-ol

5-(3-Chloro-7H-pyrrolo[2,3-c]pyridazin-7-yl)bicyclo[3.1.1]heptan-1-ol (260 mg, 0.99 mmol) of Example 16B, 2-[2-(methoxymethoxy)-6-methyl-4-(trifluoromethyl)phenyl]-4,4,5,5-tetramethyl-1,3,2-dioxaborolane (375 mg, 1.1 mmol) of Example 1B, and potassium carbonate (272 mg, 2.0 mmol) were dissolved in a mixed solvent of 1,4-dioxane (3.0 mL) and water (1.0 mL), XPhos Pd G2 (155 mg, 0.20 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =25/75-0/100(V/V)] to give the title compound (340 mg, yield: 77%) as a solid.

### (16D) 7-{5-[(4-methoxybenzyl)oxy]bicyclo[3.1.1]heptan-1-yl}-3-[2-(methoxymethoxy)-6-methyl-4-(trifluoromethyl)phenyl]-7H-pyrrolo[2,3-c]pyridazine

5-{3-[2-(Methoxymethoxy)-6-methyl-4-(trifluoromethyl)phenyl]-7H-pyrrolo[2,3-c]pyridazin-7-yl}bicyclo[3.1.1]heptan-1-ol (127 mg, 0.28 mmol) of Example 16C was dissolved in DMF (1.5 mL), then 55% sodium hydride (24 mg, 0.57 mmol) and paramethoxybenzyl chloride (0.116 mL, 0.85 mmol) were added at 0°C, and the mixture was stirred in the nitrogen-substituted system at room temperature for 70 min. Water was added at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-50/50(V/V)] to give the title compound (135 mg, yield: 84%) as an oil.

### (16E) 5-iodo-7-{5-[(4-methoxybenzyl)oxy]bicyclo[3.1.1]heptan-1-yl}-3-[2-(methoxymethoxy)-6-methyl-4-(trifluoromethyl)phenyl]-7H-pyrrolo[2,3-c]pyridazine

7-{5-[(4-Methoxybenzyl)oxy]bicyclo[3.1.1]heptan-1-yl}-3-[2-(methoxymethoxy)-6-methyl-4-(trifluoromethyl)phenyl]-7H-pyrrolo[2,3-c]pyridazine (135 mg, 0.24 mmol) of Example 16D was dissolved in DCE (1.0 mL), N-iodosuccinimide (64 mg, 0.29 mmol) was added, and the mixture was stirred in the nitrogen-substituted system at 60°C for 3 hr. To the reaction solution was added water at room temperature, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =75/25-65/35(V/V)] to give the title compound (82 mg, yield: 50%) as an amorphous form.

### (16F) 7-{5-[(4-methoxybenzyl)oxy]bicyclo[3.1.1]heptan-1-yl}-3-[2-(methoxymethoxy)-6-methyl-4-(trifluoromethyl)phenyl]-7H-pyrrolo[2,3-c]pyridazine-5-carbonitrile

5-Iodo-7-{5-[(4-methoxybenzyl)oxy]bicyclo[3.1.1]heptan-1-yl}-3-[2-(methoxymethoxy)-6-methyl-4-(trifluoromethyl)phenyl]-7H-pyrrolo[2,3-c]pyridazine (54 mg, 0.078 mmol) of Example 16E was dissolved in DMF (0.8 mL), then 1,1'-bis(diphenylphosphino)ferrocene (17 mg, 0.031 mmol), zinc cyanide (18 mg, 0.15 mmol) and Pd₂(dba)₃ (14 mg, 0.016 mmol) were added, and the mixture was stirred in the nitrogen-substituted system at 100°C for 1.5 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-55/45(V/V)] to give the title compound (11 mg, yield: 24%) as an oil.

### (16G) 7-(5-hydroxybicyclo[3.1.1]heptan-1-yl)-3-[2-hydroxy-6-methyl-4-(trifluoromethyl)phenyl]-7H-pyrrolo[2,3-c]pyridazine-5-carbonitrile

7-{5-[ (4-Methoxybenzyl)oxy]bicyclo[3.1.1]heptan-1-yl}-3-[2-(methoxymethoxy)-6-methyl-4-(trifluoromethyl)phenyl]-7H-pyrrolo[2,3-c]pyridazine-5-carbonitrile (14 mg, 0.024 mmol) of Example 16F was dissolved in DCM (0.3 mL), TFA (0.3 mL) was added, and the mixture was stirred at room temperature for 2 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=96/4-90/10(V/V)] to give the title compound (5 mg, yield: 49%) as a solid.

### (Example 17)

### 2-{7-[(1S*,2S*,5R*)-8-ethyl-8-azabicyclo[3.2.1]octan-2-y1]-6,7-dihydro-5H-pyrrolo[2,3-c]pyridazin-3-yl}-3-methyl-5-(trifluoromethyl)phenol

### (17A) 8-tert-butyl 2-methyl (1S*,2S*,5S*)-8-azabicyclo[3.2.1]octane-2,8-dicarboxylate

8-tert-Butyl 2-methyl 8-aza-bicyclo[3.2.1]oct-2-ene-2,8-dicarboxylate (J. Am. Chem. Soc. 2007, 129, 10312-10313.) (2.44 g, 9.1 mmol) was dissolved in ethanol (100 mL), 10% palladium on carbon (491 mg) was added, and the mixture was stirred in the hydrogen-substituted system at room temperature for 1 hr. The reaction solution was filtered, the filtrate was concentrated under reduced pressure, and the obtained residue was purified twice by silica gel column chromatography [eluent: DCM/ethyl acetate =98/2-92/8(V/V)] to give the title compound eluting earlier (0.99 g, yield: 40%) as a racemate (an oil).

### (17B) (1S*,2S*,5S*)-8-(tert-butoxycarbonyl)-8-azabicyclo[3.2.1]octane-2-carboxylic acid

8-tert-Butyl 2-methyl (1S*,2S*,5S*)-8-azabicyclo[3.2.1]octane-2,8-dicarboxylate (0.99 g, 3.7 mmol) of Example 17A was dissolved in methanol (20 mL), 5 M sodium hydroxide aqueous solution (7 mL) was added, and the mixture was stirred at room temperature for 80 min. To the reaction solution was added 6 M hydrochloric acid at 0°C, and the mixture was subjected to extraction with DCM. The organic layer was dried over anhydrous sodium sulfate. After filtration, the filtrate was concentrated under reduced pressure to give a crude product (0.88 g, yield: 94%) of the title compound as a racemate (a solid).

### (17C) tert-butyl (1S*, 2S*, 5S*) -2-{ [ (benzyloxy) carbonyl] amino}-8-azabicyclo[3.2.1]octane-8-carboxylate

(1S*, 2S*, 5S*) -8- (tert-Butoxycarbonyl) -8-azabicyclo[3.2.1]octane-2-carboxylic acid (0.84 g, 3.3 mmol) of Example 17B was suspended in toluene (33 mL), then TEA (0.912 mL, 6.6 mmol) and diphenylphosphorylazide (0.851 mL, 4.0 mmol) were added, and the mixture was stirred in the nitrogen-substituted system at 100°C for 1 hr. To the reaction solution was added benzyl alcohol (2.72 mL, 26 mmol), and the mixture was stirred at 100°C for 2 hr. The reaction solution was allowed to room temperature, and purified by silica gel column chromatography [eluent: n-hexane/ethyl acetate =90/10-70/30(V/V)] to give the title compound (1.08 g, yield: 91%) as a racemate (a solid).

### (17D) tert-butyl (1S*,2S*,5S*)-2-amino-8-azabicyclo[3.2.1]octane-8-carboxylate

tert-Butyl (1S*,2S*,5S*)-2-{[(benzyloxy) carbonyl]amino}-8-azabicyclo[3.2.1]octane-8-carboxylate (1.08 g, 3.0 mmol) of Example 17C was dissolved in ethanol (50 mL), 10% palladium on carbon (212 mg) was added, and the mixture was stirred in the hydrogen-substituted system at room temperature for 1 hr. The reaction solution was filtered, and the filtrate was concentrated under reduced pressure to give a crude product (716 mg, yield: quantitative) of the title compound as a racemate (an oil).

### (17E) tert-butyl (1S*,2S*,5S*)-2-(3-chloro-5,6-dihydro-7H-pyrrolo[2,3-c]pyridazin-7-yl)-8-azabicyclo[3.2.1]octane-8-carboxylate

tert-Butyl (1S*,2S*,5S*)-2-amino-8-azabicyclo[3.2.1]octane-8-carboxylate (457 mg, 2.0 mmol) of Example 17D was dissolved in NMP (4 mL), then DIPEA (0.691 mL, 4.0 mmol) and 3,6-dichloro-4-ethenylpyridazine (424 mg, 2.4 mmol) of Example 1C were added, and the mixture was stirred in the nitrogen-substituted system at 150°C for 30 min. The reaction solution was allowed to room temperature, and concentrated under reduced pressure, and the obtained residue was purified by silica gel column chromatography [eluent: n-hexane/ethyl acetate =70/30-0/100(V/V)] to give the title compound (139 mg, yield: 19%) as a racemate (an oil).

### (17F) tert-butyl (1S*,2S*,5S*)-2-{3-[2-(methoxymethoxy)-6-methyl-4-(trifluoromethyl)phenyl]-5,6-dihydro-7H-pyrrolo[2,3-c]pyridazin-7-yl}-8-azabicyclo[3.2.1]octane-8-carboxylate

tert-Butyl (1S*,2S*,5S*)-2-(3-chloro-5,6-dihydro-7H-pyrrolo[2,3-c]pyridazin-7-yl)-8-azabicyclo[3.2.1]octane-8-carboxylate (61 mg, 0.17 mmol) of Example 17E, 2-[2-(methoxymethoxy)-6-methyl-4-(trifluoromethyl)phenyl]-4,4,5,5-tetramethyl-1,3,2-dioxaborolane (82 mg, 0.23 mmol) of Example 1B, and potassium carbonate (45 mg, 0.33 mmol) were dissolved in a mixed solvent of 1,4-dioxane (1.6 mL) and water (0.4 mL), XPhos Pd G2 (26 mg, 0.033 mmol) was added, and the mixture was stirred in the nitrogen-substituted system at 110°C for 1 hr. The reaction solution was allowed to room temperature, and DCM and water were added. The mixture was stirred, and the organic layer was separated through a phase separator (Biotage). The solvent was evaporated under reduced pressure, and the obtained residue was purified by silica gel column chromatography [eluent: n-hexane/ethyl acetate =75/25-0/100(V/V)] to give the title compound (72 mg, yield: 79%) as a racemate (a solid).

### (17G) 2-{7-[(1S*,2S*,5R*)-8-azabicyclo[3.2.1]octan-2-yl]-6,7-dihydro-5H-pyrrolo[2,3-c]pyridazin-3-yl}-3-methyl-5-(trifluoromethyl)phenol

To tert-butyl (1S*,2S*,5S*)-2-{3-[2-(methoxymethoxy)-6-methyl-4-(trifluoromethyl)phenyl]-5,6-dihydro-7H-pyrrolo[2,3-c]pyridazin-7-yl}-8-azabicyclo[3.2.1]octane-8-carboxylate (72 mg, 0.13 mmol) of Example 17F were added 4 M hydrogen chloride-1,4-dioxane solution (1.5 mL) and methanol (0.15 mL), and the mixture was stirred at room temperature for 1 hr. The reaction solution was diluted with methanol, and concentrated under reduced pressure. To the residue was added saturated aqueous sodium bicarbonate solution, the mixture was subjected to extraction with a mixed solvent of DCM/methanol, and the organic layer was dried over anhydrous sodium sulfate. After filtration, the filtrate was concentrated under reduced pressure to give the title compound (50 mg, yield: 94%) as a racemate (a solid) .

### (17H) 2-{7-[(1S*,2S*,5R*)-8-ethyl-8-azabicyclo[3.2.1]octan-2-yl]-6,7-dihydro-5H-pyrrolo[2,3-c]pyridazin-3-yl}-3-methyl-5-(trifluoromethyl)phenol

2-{7-[(1S*,2S*,5R*)-8-Azabicyclo[3.2.1]octan-2-yl]-6,7-dihydro-5H-pyrrolo[2,3-c]pyridazin-3-yl}-3-methyl-5-(trifluoromethyl)phenol (9 mg, 0.022 mmol) of Example 17G was dissolved in a mixed solvent of methanol (0.33 mL) and DCM (1 mL), then acetaldehyde (5 drops) and NaBH(OAc)₃ (36 mg, 0.18 mmol) were added, and the mixture was stirred at room temperature for 1 hr. The reaction solution was diluted with DCM/methanol, saturated aqueous sodium bicarbonate solution was added, and the mixture was stirred. The organic layer was separated through a phase separator (Biotage), the solvent was evaporated under reduced pressure, and the obtained residue was purified by NH silica gel column chromatography [eluent: DCM/methanol=100/0-90/10(V/V)]. To the obtained residue were added n-hexane/diisopropyl ether, and the mixture was subjected to ultrasonic treatment. The solid was collected by filtration to give the title compound (6.3 mg, yield: 65%) as a racemate (a solid).

### (Example 18)

### 2-{7-[(3R)-1-cyclopropylpiperidin-3-yl]-6,7-dihydro-5H-pyrrolo[2,3-c]pyridazin-3-yl}-3-methyl-5-(trifluoromethyl)phenol

### (18A) 7-[(3R)-1-cyclopropylpiperidin-3-yl]-3-[2-(methoxymethoxy)-6-methyl-4-(trifluoromethyl)phenyl]-6,7-dihydro-5H-pyrrolo[2,3-c]pyridazine

3-[2-(Methoxymethoxy)-6-methyl-4-(trifluoromethyl)phenyl]-7-[(3R)-piperidin-3-yl]-6,7-dihydro-5H-pyrrolo[2,3-c]pyridazine (0.038 g, 0.090 mmol) of Example 14C was dissolved in methanol (1 mL), then (1-ethoxycyclopropoxy)trimethylsilane (0.11 mL, 0.55 mmol), acetic acid (0.051 mL, 0.89 mmol) and sodium cyanoborohydride (0.030 g, 0.48 mmol) were added, and the mixture was stirred at 60°C for 5 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =90/10-0/100(V/V)] to give the title compound (0.012 g, yield: 29%) as an oil.

### (18B) 2-{7-[(3R)-1-cyclopropylpiperidin-3-yl]-6,7-dihydro-5H-pyrrolo[2,3-c]pyridazin-3-yl}-3-methyl-5-(trifluoromethyl)phenol

7-[(3R)-1-Cyclopropylpiperidin-3-yl]-3-[2-(methoxymethoxy)-6-methyl-4-(trifluoromethyl)phenyl]-6,7-dihydro-5H-pyrrolo[2,3-c]pyridazine (12 mg, 0.026 mmol) of Example 18A was dissolved in DCM (0.5 mL), TFA (0.5 mL) was added, and the mixture was stirred at room temperature for 3 hr. The reaction solution was concentrated under reduced pressure, and to the residue was added saturated aqueous sodium bicarbonate solution. The mixture was subjected to extraction with DCM, and the organic layer was dried over anhydrous sodium sulfate. After filtration, the filtrate was concentrated under reduced pressure, and the obtained residue was purified by NH silica gel column chromatography [eluent: ethyl acetate/methanol=100/0-95/5(V/V)] to give the title compound (5.4 mg, yield: 50%) as a solid.

### (Example 19)

### 3-methyl-2-{7-[(1R,2R,5S or 1S,2S,5R)-8-methyl-8-azabicyclo[3.2.1]octan-2-yl]-6,7-dihydro-5H-pyrrolo[2,3-c]pyridazin-3-yl}-5-(trifluoromethyl)phenol

### (19A) 3-methyl-2-{7-[(1S*,2S*,5R*)-8-methyl-8-azabicyclo[3.2.1]octan-2-yl]-6,7-dihydro-5H-pyrrolo[2,3-c]pyridazin-3-yl}-5-(trifluoromethyl)phenol

2-{7-[(1S*,2S*,5R*)-8-Azabicyclo[3.2.1]octan-2-y1]-6,7-dihydro-5H-pyrrolo[2,3-c]pyridazin-3-yl}-3-methyl-5-(trifluoromethyl)phenol (40 mg, 0.099 mmol) of Example 17G was dissolved in a mixed solvent of methanol (0.67 mL) and DCM (2 mL), then 37% formaldehyde (0.022 mL, 0.30 mmol) and NaBH(OAc)₃ (149 mg, 0.70 mmol) were added in the nitrogen-substituted system, and the mixture was stirred at room temperature for 1 hr and 20 min. The reaction solution was diluted with DCM/methanol, saturated aqueous sodium bicarbonate solution was added, and the mixture was stirred. The organic layer was separated through a phase separator (Biotage), the solvent was evaporated under reduced pressure, and the obtained residue was purified by NH silica gel column chromatography [eluent: DCM/methanol=100/0-90/10(V/V)] to give the title compound (39 mg, yield: 94%) as a racemate (a solid).

### (19B) 3-methyl-2-{7-[(1R,2R,5S or 1S,2S,5R)-8-methyl-8-azabicyclo[3.2.1]octan-2-yl]-6,7-dihydro-5H-pyrrolo[2,3-c]pyridazin-3-yl}-5-(trifluoromethyl)phenol

3-Methyl-2-{7-[(1S*,2S*,5R*) -8-methyl-8-azabicyclo[3.2.1]octan-2-yl]-6,7-dihydro-5H-pyrrolo[2,3-c]pyridazin-3-yl}-5-(trifluoromethyl)phenol (28 mg, 0.067 mmol) of Example 19A was subjected to chiral HPLC [column: CHIRALPAK IG (20 mm I.D.× 250 mm), mobile phase: n-hexane/2-propanol=60/40(V/V), temperature: 40°C] to give the title compound eluting later (14.8 mg, yield: 53%) as a single enantiomer (a solid).

### (Example 20)

### 2-{7-[(3R)-1-ethylpiperidin-3-yl]-5-methyl-7H-pyrrolo[2,3-c]pyridazin-3-yl}-3-methyl-5-(trifluoromethyl)phenol

### (20A) tert-butyl (3R)-3-(3-chloro-5-methyl-5,6-dihydro-7H-pyrrolo[2,3-c]pyridazin-7-yl)piperidine-1-carboxylate

3,6-Dichloro-4-(prop-1-en-2-yl)pyridazine (8.07 g, 43 mmol) of Example 4A, and DIPEA (15 mL, 86 mmol) were dissolved in NMP (45 mL), tert-butyl (3R)-3-aminopiperidine-1-carboxylate (10.5 g, 52 mmol) was added, and the mixture was stirred under microwave irradiation at 150°C for 3 hr. The reaction solution was concentrated under reduced pressure, and the obtained residue was purified by silica gel column chromatography [eluent: n-hexane/ethyl acetate =80/20-40/60(V/V)] to give the title compound (2.55 g, yield: 17%) as an amorphous form.

### (20B) tert-butyl (3R)-3-(3-chloro-5-methyl-7H-pyrrolo[2,3-c]pyridazin-7-yl)piperidine-1-carboxylate

tert-Butyl (3R)-3-(3-chloro-5-methyl-5,6-dihydro-7H-pyrrolo[2,3-c]pyridazin-7-yl)piperidine-1-carboxylate (2.55 g, 7.2 mmol) of Example 20A was dissolved in DCE (36 mL), DDQ (1.97 g, 8.7 mmol) was added, and the mixture was stirred at 80°C for 1 hr. To the reaction solution was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =80/20-50/50(V/V)] to give the title compound (1.74 g, yield: 69%) as an amorphous form.

### (20C) tert-butyl (3R)-3-{3-[2-(methoxymethoxy)-6-methyl-4-(trifluoromethyl)phenyl]-5-methyl-7H-pyrrolo[2,3-c]pyridazin-7-yl}piperidine-1-carboxylate

tert-Butyl (3R)-3-(3-chloro-5-methyl-7H-pyrrolo[2,3-c]pyridazin-7-yl)piperidine-1-carboxylate (0.157 g, 0.45 mmol) of Example 20B, 2-[2-(methoxymethoxy)-6-methyl-4-(trifluoromethyl)phenyl]-4,4,5,5-tetramethyl-1,3,2-dioxaborolane (0.188 g, 0.54 mmol) of Example 1B, and potassium carbonate (0.120 g, 0.87 mmol) were dissolved in a mixed solvent of 1,4-dioxane (2 mL) and water (0.5 mL), XPhos Pd G2 (0.070 g, 0.089 mmol) was added, and the mixture was stirred under microwave irradiation in the nitrogen-substituted system at 120°C for 1 hr. The reaction solution was concentrated under reduced pressure, and the obtained residue was purified by silica gel column chromatography [eluent: n-hexane/ethyl acetate =90/10-50/50(V/V)] to give the title compound (0.247 g, yield: quantitative) as an amorphous form.

### (20D) 2-{7-[(3R)-1-ethylpiperidin-3-yl]-5-methyl-7H-pyrrolo[2,3-c]pyridazin-3-yl}-3-methyl-5-(trifluoromethyl)phenol

To tert-butyl (3R)-3-{3-[2-(methoxymethoxy)-6-methyl-4-(trifluoromethyl)phenyl]-5-methyl-7H-pyrrolo[2,3-c]pyridazin-7-yl}piperidine-1-carboxylate (0.247 g, 0.46 mmol) of Example 20C was added 4 M hydrogen chloride-1,4-dioxane (3.5 mL), and the mixture was stirred at room temperature for 3 hr. The reaction solution was concentrated under reduced pressure, and to the residue was added saturated aqueous sodium bicarbonate solution. The mixture was subjected to extraction with DCM, and the organic layer was dried over anhydrous sodium sulfate. After filtration, the filtrate was concentrated under reduced pressure, and the obtained residue was dissolved in DCM (3 mL). Methanol (0.3 mL), acetaldehyde (0.20 mL, 8.0 mmol) and NaBH(OAc)₃ (0.364 g, 1.7 mmol) were added, and the mixture was stirred at room temperature for 1 hr. To the reaction solution was added saturated aqueous sodium bicarbonate solution, the mixture was subjected to extraction with DCM, and the organic layer was dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (0.158 g, yield: 82%) as an amorphous form.

### (Example 21)

### 2-{5-(difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol (also referred to as 2-[5-(difluoromethoxy)-7-(cis-3-hydroxy-3-methylcyclobutyl)-7H-pyrrolo[2,3-c]pyridazin-3-yl]-3-methyl-5-(trifluoromethyl)phenol)

### (21A) 3-chloro-7-[(1s,3s)-3-hydroxy-3-methylcyclobutyl]-7H-pyrrolo[2,3-c]pyridazin-5-yl acetate

(1s,3s)-3-(3-Chloro-7H-pyrrolo[2,3-c]pyridazin-7-yl)-1-methylcyclobutanol (2.0 g, 8.4 mmol) of Example 3B was dissolved in acetic acid (17 mL), then palladium(II) acetate (570 mg, 2.5 mmol) and diacetoxyiodobenzene (4.3 g, 14 mmol) were added, and the mixture was stirred in the nitrogen-substituted system at 100°C for 1 hr. The reaction solution was concentrated under reduced pressure, to the obtained residue was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =60/40-20/80(V/V)] to give the title compound (422 mg, yield: 17%) as an oil.

### (21B) 7-((1s,3s)-3-{[tert-butyl(dimethyl)silyl]oxy}-3-methylcyclobutyl)-3-chloro-7H-pyrrolo[2,3-c]pyridazin-5-yl acetate

3-Chloro-7-[(1s,3s)-3-hydroxy-3-methylcyclobutyl]-7H-pyrrolo[2,3-c]pyridazin-5-yl acetate (1.66 g, 5.6 mmol) of Example 21A was dissolved in DMF (18 mL), then imidazole (1.15 g, 17 mmol) and tert-butyldimethylsilyl chloride (1.69 g, 11 mmol) were added, and the mixture was stirred in the nitrogen-substituted system at room temperature 2.5 hr. To the reaction solution was added ammonium chloride aqueous solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-10/90(V/V)] to give the title compound (1.28 g, yield: 56%) as an oil.

### (21C) 7-((1s,3s)-3-{[tert-butyl(dimethyl)silyl]oxy}-3-methylcyclobutyl)-3-chloro-7H-pyrrolo[2,3-c]pyridazin-5-ol

7-((1s,3s)-3-{[tert-Butyl (dimethyl)silyl]oxy}-3-methylcyclobutyl)-3-chloro-7H-pyrrolo[2,3-c]pyridazin-5-yl acetate (1.28 g, 3.1 mmol) of Example 21B was dissolved in methanol (15 mL), potassium carbonate (1.08 g, 7.8 mmol) was added at 0°C, and the mixture was stirred at room temperature for 40 min. To the reaction solution was added saturated ammonium chloride aqueous solution at 0°C, and the mixture was subjected to extraction with ethyl acetate. The organic layer was washed with saturated brine, and dried over anhydrous sodium sulfate. After filtration, the filtrate was concentrated under reduced pressure, ethyl acetate was added, and the mixture was subjected to ultrasonic treatment. The solid was collected by filtration to give the title compound (1.04 g, yield: 90%) as a solid.

### (21D) 7-((1s,3s)-3-{[tert-butyl(dimethyl)silyl]oxy}-3-methylcyclobutyl)-3-chloro-5-(difluoromethoxy)-7H-pyrrolo[2,3-c]pyridazine

7-((1s,3s)-3-{[tert-Butyl(dimethyl)silyl]oxy}-3-methylcyclobutyl)-3-chloro-7H-pyrrolo[2,3-c]pyridazin-5-ol (150 mg, 0.41 mmol) of Example 21C was dissolved in a mixed solvent of acetonitrile (6.0 mL) and water (3.0 mL), then diethyl (bromodifluoromethyl)phosphonate (0.217 mL, 1.2 mmol) and potassium hydroxide (231 mg, 4.0 mmol) were added, and the mixture was stirred in the nitrogen-substituted system at room temperature for 5.5 hr. To the reaction solution was added ammonium chloride aqueous solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-60/40(V/V)] to give the title compound (35 mg, yield: 21%) as an oil.

### (21E) 7-((1s,3s)-3-{[tert-butyl(dimethyl)silyl]oxy}-3-methylcyclobutyl)-5-(difluoromethoxy)-3-[2-(methoxymethoxy)-6-methyl-4-(trifluoromethyl)phenyl]-7H-pyrrolo[2,3-c]pyridazine

7-((1s,3s)-3-{[tert-Butyl (dimethyl)silyl]oxy}-3-methylcyclobutyl)-3-chloro-5-(difluoromethoxy)-7H-pyrrolo[2,3-c]pyridazine (20 mg, 0.048 mmol) of Example 21D, 2-[2-(methoxymethoxy)-6-methyl-4-(trifluoromethyl)phenyl]-4,4,5,5-tetramethyl-1,3,2-dioxaborolane (24 mg, 0.067 mmol) of Example 1B, and potassium carbonate (13 mg, 0.096 mmol) were dissolved in a mixed solvent of 1,4-dioxane (0.6 mL) and water (0.15 mL), XPhos Pd G2 (7 mg, 0.0096 mmol) was added, and the mixture was stirred in the nitrogen-substituted system at 110°C for 1 hr. The reaction solution was allowed to room temperature, and purified by silica gel column chromatography [eluent: n-hexane/ethyl acetate =95/5-80/20(V/V)] to give the title compound (22.8 mg, yield: 79%) as a solid.

### (21F) 2-{5-(difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol

7-((1s,3s)-3-{[tert-Butyl (dimethyl)silyl]oxy}-3-methylcyclobutyl)-5-(difluoromethoxy)-3-[2-(methoxymethoxy)-6-methyl-4-(trifluoromethyl)phenyl]-7H-pyrrolo[2,3-c]pyridazine (21.5 mg, 0.036 mmol) of Example 21E was dissolved in DCM (0.5 mL), TFA (0.5 mL) was added, and the mixture was stirred at room temperature for 2 hr. To the reaction solution was added saturated aqueous sodium bicarbonate solution at 0°C, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: DCM/methanol=98/2-88/12(V/V)] to give the title compound (8.65 mg, yield: 55%) as a solid.

### (Example 22)

### 5-(difluoromethoxy)-2-{7-[(3R)-1-ethylpiperidin-3-yl]-6,7-dihydro-5H-pyrrolo[2,3-c]pyridazin-3-yl}-3-methylphenol

### (22A) tert-butyl (3R)-3-{3-[4-(difluoromethoxy)-2-(methoxymethoxy)-6-methylphenyl]-5,6-dihydro-7H-pyrrolo[2,3-c]pyridazin-7-yl}piperidine-1-carboxylate

tert-Butyl (3R)-3-(3-chloro-5,6-dihydro-7H-pyrrolo[2,3-c]pyridazin-7-yl)piperidine-1-carboxylate (0.400 g, 1.2 mmol) of Example 1D, 2-[4-(difluoromethoxy)-2-(methoxymethoxy)-6-methylphenyl]-4,4,5,5-tetramethyl-1,3,2-dioxaborolane (0.488 g, 1.4 mmol) of Example 13F, and potassium carbonate (0.326 g, 2.4 mmol) were dissolved in a mixed solvent of 1,4-dioxane (4 mL) and water (1 mL), XPhos Pd G2 (0.186 g, 0.24 mmol) was added, and the mixture was stirred under microwave irradiation in the nitrogen-substituted system at 120°C for 1 hr. The reaction solution was concentrated under reduced pressure, and the obtained residue was purified by NH silica gel column chromatography [eluent: n-hexane/ethyl acetate =90/10-25/75(V/V)] to give the title compound (0.634 g, yield: quantitative) as an oil.

### (22B) 5-(difluoromethoxy)-2-{7-[(3R)-1-ethylpiperidin-3-yl]-6,7-dihydro-5H-pyrrolo[2,3-c]pyridazin-3-yl}-3-methylphenol

To tert-butyl (3R)-3-{3-[4-(difluoromethoxy)-2-(methoxymethoxy)-6-methylphenyl]-5,6-dihydro-7H-pyrrolo[2,3-c]pyridazin-7-yl}piperidine-1-carboxylate (0.634 g, 1.2 mmol) of Example 22A was added 4 M hydrogen chloride-1,4-dioxane (12 mL), and the mixture was stirred at room temperature for 1 hr. The reaction solution was concentrated under reduced pressure, and to the residue was added saturated aqueous sodium bicarbonate solution. The mixture was subjected to extraction with DCM, and the organic layer was dried over anhydrous sodium sulfate. After filtration, the filtrate was concentrated under reduced pressure, and the obtained residue was dissolved in DCM (12 mL). Methanol (1.2 mL), NaBH(OAc)₃ (1.08 g, 5.1 mmol) and acetaldehyde (1.2 mL, 24 mmol) were added, and the mixture was stirred at room temperature for 3.5 hr. To the reaction solution was added saturated aqueous sodium bicarbonate solution, the mixture was subjected to extraction with DCM, and the organic layer was dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (0.310 g, yield: 63%) as a solid.

### (Example 23)

### 2-{7-[(3R)-1-ethylpiperidin-3-yl]-6,7-dihydro-5H-pyrrolo[2,3-c]pyridazin-3-yl}-3-methyl-5-(trifluoromethoxy)phenol

### (23A) 3-methyl-5-(trifluoromethoxy)phenol

3-Chloro-5-trifluoromethoxyphenol (3.3 g, 16 mmol), trimethylboroxin (50% THF solution) (7.8 mL, 28 mmol) and cesium carbonate (13 g, 39 mmol) were dissolved in a mixed solvent of 1,4-dioxane (78 mL) and water (20 mL), XPhos Pd G2 (1.2 g, 1.6 mmol) was added, and the mixture was stirred in the nitrogen-substituted system at 120°C for 5 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-60/40(V/V)] to give the title compound (3.4 g, yield: quantitative) as an oil.

### (23B) 2-iodo-3-methyl-5-(trifluoromethoxy)phenol

3-Methyl-5-(trifluoromethoxy)phenol (3.4 g, 18 mmol) of Example 23A was dissolved in toluene (60 mL), 55% sodium hydride (1.5 g, 35 mmol) was added at 0°C, and the mixture was stirred for 30 min. To the reaction solution was added iodine (4.5 g, 18 mmol), and the mixture was stirred at 0°C for 2 hr. To the reaction solution was added saturated ammonium chloride aqueous solution, and the mixture was subjected to extraction with ethyl acetate. The organic layer was washed with water and saturated brine, and dried over anhydrous sodium sulfate. After filtration, the filtrate was concentrated under reduced pressure to give the title compound (5.6 g, yield: quantitative) as an oil.

### (23C) 2-iodo-1- (methoxymethoxy)-3-methyl-5-(trifluoromethoxy)benzene

2-Iodo-3-methyl-5-(trifluoromethoxy)phenol (5.6 g, 18 mmol) of Example 23B, and TEA (6.1 mL, 35 mmol) were dissolved in DCM (88 mL), chloromethyl methyl ether (1.7 mL, 23 mmol) was added at 0°C, and the mixture was stirred in the nitrogen-substituted system at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-85/15(V/V)] to give the title compound (3.1 g, yield: 49%) as an oil.

### (23D) 2-[2-(methoxymethoxy)-6-methyl-4-(trifluoromethoxy)phenyl]-4,4,5,5-tetramethyl-1,3,2-dioxaborolane

2-Iodo-1-(methoxymethoxy)-3-methyl-5-(trifluoromethoxy)benzene (3.06 g, 8.5 mmol) of Example 23C was dissolved in THF (42 mL), n-butyllithium (1.6 M n-hexane solution) (6.9 mL, 11 mmol) was added at -78°C, and the mixture was stirred in the nitrogen-substituted system at -78°C for 30 min. iPrOBPin (3.42 mL, 17 mmol) was added at -78°C, and the mixture was stirred at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-65/35(V/V)] to give the title compound (1.87 g, yield: 61%) as an oil.

### (23E) tert-butyl (3R)-3-{3-[2-(methoxymethoxy)-6-methyl-4-(trifluoromethoxy)phenyl]-5,6-dihydro-7H-pyrrolo[2,3-c]pyridazin-7-yl}piperidine-1-carboxylate

tert-Butyl (3R)-3-(3-chloro-5,6-dihydro-7H-pyrrolo[2,3-c]pyridazin-7-yl)piperidine-1-carboxylate (0.224 g, 0.66 mmol) of Example 1D, 2-[2-(methoxymethoxy)-6-methyl-4-(trifluoromethoxy)phenyl]-4,4,5,5-tetramethyl-1,3,2-dioxaborolane (0.286 g, 0.79 mmol) of Example 23D, and potassium carbonate (0.183 g, 1.3 mmol) were dissolved in a mixed solvent of 1,4-dioxane (2.8 mL) and water (0.7 mL), XPhos Pd G2 (0.104 g, 0.13 mmol) was added, and the mixture was stirred under microwave irradiation in the nitrogen-substituted system at 120°C for 1 hr. The reaction solution was concentrated under reduced pressure, and the obtained residue was purified by silica gel column chromatography [eluent: n-hexane/ethyl acetate =75/25-25/75(V/V)] to give the title compound (0.126 g, yield: 35%) as an amorphous form.

### (23F) 2-{7-[(3R)-1-ethylpiperidin-3-yl]-6,7-dihydro-5H-pyrrolo[2,3-c]pyridazin-3-yl}-3-methyl-5-(trifluoromethoxy)phenol

To tert-butyl (3R)-3-{3-[2-(methoxymethoxy)-6-methyl-4-(trifluoromethoxy)phenyl]-5,6-dihydro-7H-pyrrolo[2,3-c]pyridazin-7-yl}piperidine-1-carboxylate (0.126 g, 0.23 mmol) of Example 23E was added 4 M hydrogen chloride-1,4-dioxane (3 mL), and the mixture was stirred at room temperature for 1 hr. The reaction solution was concentrated under reduced pressure, and to the residue was added saturated aqueous sodium bicarbonate solution. The mixture was subjected to extraction with DCM, and the organic layer was dried over anhydrous sodium sulfate. After filtration, the filtrate was concentrated under reduced pressure, and the obtained residue was dissolved in DCM (3 mL). Methanol (0.3 mL), acetaldehyde (0.50 mL, 10 mmol) and NaBH(OAc)₃ (0.253 g, 1.2 mmol) were added, and the mixture was stirred at room temperature for 30 min. To the reaction solution was added saturated aqueous sodium bicarbonate solution, the mixture was subjected to extraction with DCM, and the organic layer was dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (0.055 g, yield: 56%) as a solid.

### (Example 24)

### 5-{3-[2-hydroxy-6-methyl-4-(trifluoromethyl)phenyl]-5-methoxy-7H-pyrrolo[2,3-c]pyridazin-7-yl}bicyclo[3.1.1]heptan-1-ol

### (24A) 7-[5-(benzyloxy)bicyclo[3.1.1]heptan-1-yl]-3-chloro-7H-pyrrolo[2,3-c]pyridazine

5-(3-Chloro-7H-pyrrolo[2,3-c]pyridazin-7-yl)bicyclo[3.1.1]heptan-1-ol (477 mg, 1.8 mmol) of Example 16B was dissolved in DMF (6.0 mL), then 55% sodium hydride (173 mg, 3.9 mmol) and benzyl bromide (0.537 mL, 4.5 mmol) were added at 0°C, and the mixture was stirred in the nitrogen-substituted system at room temperature for 30 min. To the reaction solution was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-70/30(V/V)] to give the title compound (419 mg, yield: 65%) as an oil.

### (24B) 7-[5-(benzyloxy)bicyclo[3.1.1]heptan-1-yl]-3-chloro-7H-pyrrolo[2,3-c]pyridazin-5-yl acetate

7-[5-(Benzyloxy)bicyclo[3.1.1]heptan-1-yl]-3-chloro-7H-pyrrolo[2,3-c]pyridazine (452 mg, 1.3 mmol) of Example 24A was dissolved in acetic acid (3.0 mL), then palladium(II) acetate (57 mg, 0.26 mmol) and diacetoxyiodobenzene (617 mg, 1.9 mmol) were added, and the mixture was stirred in the nitrogen-substituted system at 100°C for 50 min. The reaction solution was concentrated under reduced pressure, to the obtained residue was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-70/30(V/V)] to give the title compound (171 mg, yield: 33%) as an oil.

### (24C) 7-[5-(benzyloxy)bicyclo[3.1.1]heptan-1-yl]-3-chloro-7H-pyrrolo[2,3-c]pyridazin-5-ol

7-[5-(Benzyloxy)bicyclo[3.1.1]heptan-1-yl]-3-chloro-7H-pyrrolo[2,3-c]pyridazin-5-yl acetate (171 mg, 0.42 mmol) of Example 24B was dissolved in methanol (2.0 mL), potassium carbonate (143 mg, 1.0 mmol) was added at 0°C, and the mixture was stirred in the nitrogen-substituted system at room temperature for 30 min. To the reaction solution was added saturated ammonium chloride aqueous solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =60/40-50/50(V/V)] to give the title compound (90 mg, yield: 59%) as an amorphous form.

### (24D) 7-[5-(benzyloxy)bicyclo[3.1.1]heptan-1-yl]-3-chloro-5-methoxy-7H-pyrrolo[2,3-c]pyridazine

7-[5-(Benzyloxy)bicyclo[3.1.1]heptan-1-yl]-3-chloro-7H-pyrrolo[2,3-c]pyridazin-5-ol (90 mg, 0.24 mmol) of Example 24C was dissolved in DMF (1.2 mL), then potassium carbonate (53 mg, 0.39 mmol) and iodomethane (0.020 mL, 0.32 mmol) were added at 0°C, and the mixture was stirred at room temperature for 2 hr. To the reaction solution was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-65/35(V/V)] to give the title compound (71 mg, yield: 76%) as an amorphous form.

### (24E) 7-[5-(benzyloxy)bicyclo[3.1.1]heptan-1-yl]-5-methoxy-3-[2-(methoxymethoxy)-6-methyl-4-(trifluoromethyl)phenyl]-7H-pyrrolo[2,3-c]pyridazine

7-[5-(Benzyloxy)bicyclo[3.1.1]heptan-1-yl]-3-chloro-5-methoxy-7H-pyrrolo[2,3-c]pyridazine (71 mg, 0.18 mmol) of Example 24D, 2-[2-(methoxymethoxy)-6-methyl-4-(trifluoromethyl)phenyl]-4,4,5,5-tetramethyl-1,3,2-dioxaborolane (76 mg, 0.22 mmol) of Example 1B, and potassium carbonate (51 mg, 0.37 mmol) were dissolved in a mixed solvent of 1,4-dioxane (1.0 mL) and water (0.3 mL), XPhos Pd G2 (29 mg, 0.037 mmol) was added, and the mixture was stirred under microwave irradiation in the nitrogen-substituted system at 120°C for 1 hr. The reaction solution was allowed to room temperature, and purified by NH silica gel column chromatography [eluent: n-hexane/ethyl acetate =75/25-60/40(V/V)] to give the title compound (73 mg, yield: 70%) as an amorphous form.

### (24F) 5-{5-methoxy-3-[2-(methoxymethoxy)-6-methyl-4-(trifluoromethyl)phenyl]-7H-pyrrolo[2,3-c]pyridazin-7-yl}bicyclo[3.1.1]heptan-1-ol

7-[5-(Benzyloxy)bicyclo[3.1.1]heptan-1-yl]-5-methoxy-3-[2-(methoxymethoxy)-6-methyl-4-(trifluoromethyl)phenyl]-7H-pyrrolo[2,3-c]pyridazine (73 mg, 0.13 mmol) of Example 24E was dissolved in methanol (1.5 mL), ASCA-2 (30 mg) was added, and the mixture was stirred in the hydrogen-substituted system at 65°C for 2.5 hr. The reaction solution was filtered through Celite, the filtrate was concentrated under reduced pressure, and the obtained residue was purified by silica gel column chromatography [eluent: n-hexane/ethyl acetate =25/75-0/100(V/V)] to give the title compound (33 mg, yield: 54%) as a solid.

### (24G) 5-{3-[2-hydroxy-6-methyl-4-(trifluoromethyl)phenyl]-5-methoxy-7H-pyrrolo[2,3-c]pyridazin-7-yl}bicyclo[3.1.1]heptan-1-ol

5-{5-Methoxy-3-[2-(methoxymethoxy)-6-methyl-4-(trifluoromethyl)phenyl]-7H-pyrrolo[2,3-c]pyridazin-7-yl}bicyclo[3.1.1]heptan-1-ol (33 mg, 0.069 mmol) of Example 24F was dissolved in DCM (0.3 mL), TFA (0.3 mL) was added, and the mixture was stirred at room temperature for 2.5 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=97/3-90/10(V/V)] to give the title compound (19 mg, yield: 63%) as a solid.

### (Example 25)

### 2-{5-ethoxy-7-[(1s,3s)-3-hydroxy-3-methylcyclobutyl]-7H-pyrrolo[2,3-c]pyridazin-3-yl}-3-methyl-5-(trifluoromethyl)phenol (also referred to as 2-[5-ethoxy-7-(cis-3-hydroxy-3-methylcyclobutyl)-7H-pyrrolo[2,3-c]pyridazin-3-yl]-3-methyl-5-(trifluoromethyl)phenol)

### (25A) 7-[(1s,3s)-3-(benzyloxy)-3-methylcyclobutyl]-3-chloro-5-ethoxy-7H-pyrrolo[2,3-c]pyridazine

7-[(1s,3s)-3-(Benzyloxy)-3-methylcyclobutyl]-3-chloro-7H-pyrrolo[2,3-c]pyridazin-5-ol (160 mg, 0.47 mmol) of Example 10C was dissolved in DMF (2.5 mL), then potassium carbonate (102 mg, 0.74 mmol) and iodoethane (0.048 mL, 0.60 mmol) were added at 0°C, and the mixture was stirred in the nitrogen-substituted system at room temperature for 4 hr. To the reaction solution was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-60/40(V/V)] to give the title compound (83 mg, yield: 48%) as an oil.

### (25B) 7-[(1s,3s)-3-(benzyloxy)-3-methylcyclobutyl]-5-ethoxy-3-[2-(methoxymethoxy)-6-methyl-4-(trifluoromethyl)phenyl]-7H-pyrrolo[2,3-c]pyridazine

7-[(1s,3s)-3-(Benzyloxy)-3-methylcyclobutyl]-3-chloro-5-ethoxy-7H-pyrrolo[2,3-c]pyridazine (83 mg, 0.22 mmol) of Example 25A, 2-[2-(methoxymethoxy)-6-methyl-4-(trifluoromethyl)phenyl]-4,4,5,5-tetramethyl-1,3,2-dioxaborolane (92 mg, 0.27 mmol) of Example 1B, and potassium carbonate (61 mg, 0.45 mmol) were dissolved in a mixed solvent of 1,4-dioxane (1.2 mL) and water (0.3 mL), XPhos Pd G2 (35 mg, 0.045 mmol) was added, and the mixture was stirred under microwave irradiation in the nitrogen-substituted system at 120°C for 50 min. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =65/35-55/45(V/V)] and silica gel column chromatography [eluent: n-hexane/ethyl acetate =80/20-50/50(V/V)] to give the title compound (88 mg, yield: 71%) as an amorphous form.

### (25C) (1s,3s)-3-{5-ethoxy-3-[2-(methoxymethoxy)-6-methyl-4-(trifluoromethyl)phenyl]-7H-pyrrolo[2,3-c]pyridazin-7-yl}-1-methylcyclobutanol

7-[(1s,3s)-3-(Benzyloxy)-3-methylcyclobutyl]-5-ethoxy-3-[2-(methoxymethoxy)-6-methyl-4-(trifluoromethyl)phenyl]-7H-pyrrolo[2,3-c]pyridazine (88 mg, 0.16 mmol) of Example 25B was dissolved in methanol (1.5 mL), ASCA-2 (40 mg) was added, and the mixture was stirred in the hydrogen-substituted system at 60°C for 2.5 hr. The reaction solution was filtered through Celite, the filtrate was concentrated under reduced pressure, and the obtained residue was purified by silica gel column chromatography [eluent: n-hexane/ethyl acetate =25/75-0/100(V/V)] to give the title compound (36 mg, yield: 48%) as a solid.

### (25D) 2-{5-ethoxy-7-[(1s,3s)-3-hydroxy-3-methylcyclobutyl]-7H-pyrrolo[2,3-c]pyridazin-3-yl}-3-methyl-5-(trifluoromethyl)phenol

(1s,3s)-3-(5-Ethoxy-3-[2-(methoxymethoxy)-6-methyl-4-(trifluoromethyl)phenyl]-7H-pyrrolo[2,3-c]pyridazin-7-yl}-1-methylcyclobutanol (36 mg, 0.077 mmol) of Example 25C was dissolved in DCM (0.4 mL), TFA (0.4 mL) was added, and the mixture was stirred at room temperature for 2 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=97/3-90/10(V/V)] to give the title compound (24 mg, yield: 74%) as a solid.

### (Example 26)

### 5-chloro-2-{7-[(3R)-1-ethylpiperidin-3-yl]-6,7-dihydro-5H-pyrrolo[2,3-c]pyridazin-3-yl}-3-methylphenol

### (26A) tert-butyl (3R)-3-{3-[4-chloro-2-(methoxymethoxy)-6-methylphenyl]-5,6-dihydro-7H-pyrrolo[2,3-c]pyridazin-7-yl}piperidine-1-carboxylate

tert-Butyl (3R)-3-(3-chloro-5,6-dihydro-7H-pyrrolo[2,3-c]pyridazin-7-yl)piperidine-1-carboxylate (355 mg, 1.1 mmol) of Example 1D, 2-[4-chloro-2-(methoxymethoxy)-6-methylphenyl]-4,4,5,5-tetramethyl-1,3,2-dioxaborolane (396 mg, 1.3 mmol) of Example 9C, and potassium carbonate (308 mg, 2.1 mmol) were dissolved in a mixed solvent of 1,4-dioxane (10.4 mL) and water (2.6 mL), XPhos Pd G2 (248 mg, 0.32 mmol) was added, and the mixture was stirred in the nitrogen-substituted system at 120°C for 5 hr. The reaction solution was allowed to room temperature, water and ethyl acetate were added, and the mixture was filtered through Celite. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =65/35-5/95(V/V)] to give the title compound (55 mg, yield: 11%) as an amorphous form.

### (26B) 5-chloro-3-methyl-2-{7-[(3R)-piperidin-3-yl]-6,7-dihydro-5H-pyrrolo[2,3-c]pyridazin-3-yl}phenol

tert-Butyl (3R)-3-{3-[4-chloro-2-(methoxymethoxy)-6-methylphenyl]-5,6-dihydro-7H-pyrrolo[2,3-c]pyridazin-7-yl}piperidine-1-carboxylate (54.0 mg, 0.11 mmol) of Example 26A was dissolved in 1,4-dioxane (1.1 mL), 4 M hydrogen chloride-1,4-dioxane (1.1 mL) was added at 0°C, and the mixture was stirred at room temperature for 3 hr. The reaction solution was concentrated under reduced pressure, and to the residue was added saturated aqueous sodium bicarbonate solution. The mixture was subjected to extraction with ethyl acetate, and the organic layer was dried over anhydrous sodium sulfate. After filtration, the filtrate was concentrated under reduced pressure, and the obtained residue was purified by NH silica gel column chromatography [eluent: DCM/methanol=99/1-85/15(V/V)] to give the title compound (25 mg, yield: 65%) as a solid.

### (26C) 5-chloro-2-{7-[(3R)-1-ethylpiperidin-3-yl]-6,7-dihydro-5H-pyrrolo[2,3-c]pyridazin-3-yl}-3-methylphenol

5-Chloro-3-methyl-2-{7-[(3R)-piperidin-3-yl]-6,7-dihydro-5H-pyrrolo[2,3-c]pyridazin-3-yl}phenol (24.5 mg, 0.071 mmol) of Example 26B was dissolved in DCM (1.0 mL), then methanol (0.6 mL), acetaldehyde (0.021 mL, 0.42 mmol) and NaBH(OAc)₃ (195.2 mg, 0.92 mmol) were added, and the mixture was stirred at room temperature for 2 days. To the reaction solution was added saturated aqueous sodium bicarbonate solution, the mixture was subjected to extraction with ethyl acetate, and the organic layer was dried over anhydrous sodium sulfate. After filtration, the filtrate was concentrated under reduced pressure, and the obtained residue was purified by NH silica gel column chromatography [eluent: ethyl acetate/methanol=100/0-92/8(V/V)] to give the title compound (17 mg, yield: 65%) as a solid.

### (Example 27)

### 5-chloro-2-{5-fluoro-7-[(1s,3s)-3-hydroxy-3-methylcyclobutyl]-7H-pyrrolo[2,3-c]pyridazin-3-yl}-3-methylphenol (also referred to as 5-chloro-2-[5-fluoro-7-(cis-3-hydroxy-3-methylcyclobutyl)-7H-pyrrolo[2,3-c]pyridazin-3-yl]-3-methylphenol)

### (27A) 3-[4-chloro-2-(methoxymethoxy)-6-methylphenyl]-5-fluoro-7-{(1s,3s)-3-[(4-methoxybenzyl)oxy]-3-methylcyclobutyl}-7H-pyrrolo[2,3-c]pyridazine

3-Chloro-5-fluoro-7-{(1s,3s)-3-[(4-methoxybenzyl)oxy]-3-methylcyclobutyl}-7H-pyrrolo[2,3-c]pyridazine (200 mg, 0.53 mmol) of Example 5B, 2-[4-chloro-2-(methoxymethoxy)-6-methylphenyl]-4,4,5,5-tetramethyl-1,3,2-dioxaborolane (200 mg, 0.64 mmol) of Example 9C, and potassium carbonate (150 mg, 1.1 mmol) were dissolved in a mixed solvent of 1,4-dioxane (3.0 mL) and water (0.7 mL), XPhos Pd G2 (83 mg, 0.11 mmol) was added, and the mixture was stirred in the nitrogen-substituted system at 110°C for 70 min. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =75/25-60/40(V/V)] to give the title compound (122 mg, yield: 44%) as an amorphous form.

### (27B) 5-chloro-2-{5-fluoro-7-[(1s,3s)-3-hydroxy-3-methylcyclobutyl]-7H-pyrrolo[2,3-c]pyridazin-3-yl}-3-methylphenol

3-[4-Chloro-2-(methoxymethoxy)-6-methylphenyl]-5-fluoro-7-{(1s,3s)-3-[(4-methoxybenzyl)oxy]-3-methylcyclobutyl}-7H-pyrrolo[2,3-c]pyridazine (36 mg, 0.077 mmol) of Example 27A was dissolved in DCM (0.4 mL), TFA (0.4 mL) was added, and the mixture was stirred at room temperature for 2 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=97/3-90/10(V/V)] to give the title compound (24 mg, yield: 74%) as a solid.

### (Example 28) 5-chloro-2-{7-[(1s,3s)-3-hydroxy-3-methylcyclobutyl]-5-methoxy-7H-pyrrolo[2,3-c]pyridazin-3-yl}-3-methylphenol (also referred to as 5-chloro-2-[7-(cis-3-hydroxy-3-methylcyclobutyl)-5-methoxy-7H-pyrrolo[2,3-c]pyridazin-3-yl]-3-methylphenol)

### (28A) 7-((1s,3s)-3-{[tert-butyl(dimethyl)silyl]oxy}-3-methylcyclobutyl)-3-chloro-5-methoxy-7H-pyrrolo[2,3-c]pyridazine

7-((1s,3s)-3-{[tert-Butyl(dimethyl)silyl]oxy}-3-methylcyclobutyl)-3-chloro-7H-pyrrolo[2,3-c]pyridazin-5-ol (350 mg, 0.95 mmol) of Example 21C was dissolved in DMF (5.0 mL), then potassium carbonate (210 mg, 1.5 mmol) and iodomethane (0.076 mL, 1.2 mmol) were added at 0°C, and the mixture was stirred at room temperature for 6 hr. To the reaction solution was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =85/15-75/25(V/V)] to give the title compound (147 mg, yield: 40%) as an oil.

### (28B) 7-((1s,3s)-3-{[tert-butyl(dimethyl)silyl]oxy}-3-methylcyclobutyl)-3-[4-chloro-2-(methoxymethoxy)-6-methylphenyl]-5-methoxy-7H-pyrrolo[2,3-c]pyridazine

7-((1s,3s)-3-{[tert-Butyl(dimethyl)silyl]oxy}-3-methylcyclobutyl)-3-chloro-5-methoxy-7H-pyrrolo[2,3-c]pyridazine (147 mg, 0.38 mmol) of Example 28A, 2-[4-chloro-2-(methoxymethoxy)-6-methylphenyl]-4,4,5,5-tetramethyl-1,3,2-dioxaborolane (144 mg, 0.46 mmol) of Example 9C, and potassium carbonate (106 mg, 0.77 mmol) were dissolved in a mixed solvent of 1,4-dioxane (2.0 mL) and water (0.5 mL), XPhos Pd G2 (60 mg, 0.077 mmol) was added, and the mixture was stirred in the nitrogen-substituted system at 110°C for 80 min. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-70/30(V/V)] to give the title compound (46 mg, yield: 22%) as an amorphous form.

### (28C) 5-chloro-2-{7-[(1s,3s)-3-hydroxy-3-methylcyclobutyl]-5-methoxy-7H-pyrrolo[2,3-c]pyridazin-3-yl}-3-methylphenol

7-((1s,3s)-3-{[tert-Butyl(dimethyl)silyl]oxy}-3-methylcyclobutyl)-3-[4-chloro-2-(methoxymethoxy)-6-methylphenyl]-5-methoxy-7H-pyrrolo[2,3-c]pyridazine (46 mg, 0.086 mmol) of Example 28B was dissolved in DCM (0.4 mL), TFA (0.4 mL) was added, and the mixture was stirred at room temperature for 2.5 hr. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=97/3-90/10(V/V)] to give the title compound (20 mg, yield: 62%) as a solid.

### (Example 29)

### 5-chloro-2-{5-(difluoromethoxy)-7-[(1s,3s)-3-hydroxy-3-methylcyclobutyl]-7H-pyrrolo[2,3-c]pyridazin-3-yl}-3-methylphenol (also referred to as 5-chloro-2-[5-(difluoromethoxy)-7-(cis-3-hydroxy-3-methylcyclobutyl)-7H-pyrrolo[2,3-c]pyridazin-3-yl]-3-methylphenol)

### (29A) 7-((1s,3s)-3-{[tert-butyl(dimethyl)silyl]oxy}-3-methylcyclobutyl)-3-[4-chloro-2-(methoxymethoxy)-6-methylphenyl]-5-(difluoromethoxy)-7H-pyrrolo[2,3-c]pyridazine

7-((1s,3s)-3-{[tert-Butyl(dimethyl)silyl]oxy}-3-methylcyclobutyl)-3-chloro-5-(difluoromethoxy)-7H-pyrrolo[2,3-c]pyridazine (77 mg, 0.18 mmol) of Example 21D, 2-[4-chloro-2-(methoxymethoxy)-6-methylphenyl]-4,4,5,5-tetramethyl-1,3,2-dioxaborolane (70 mg, 0.22 mmol) of Example 9C, and potassium carbonate (51 mg, 0.37 mmol) were dissolved in a mixed solvent of 1,4-dioxane (1.23 mL) and water (0.31 mL), XPhos Pd G2 (14 mg, 0.018 mmol) was added, and the mixture was stirred in the nitrogen-substituted system at 110°C for 2.5 hr. The reaction solution was allowed to room temperature, and diluted with DCM and water, and the organic layer was separated through a phase separator (Biotage). The solvent was evaporated under reduced pressure, and the obtained residue was purified by silica gel column chromatography [eluent: n-hexane/ethyl acetate =95/5-75/25(V/V)] to give the title compound (48.3 mg, yield: 46%) as an oil.

### (29B) 5-chloro-2-{5-(difluoromethoxy)-7-[(1s,3s)-3-hydroxy-3-methylcyclobutyl]-7H-pyrrolo[2,3-c]pyridazin-3-yl}-3-methylphenol

7-((1s,3s)-3-{[tert-Butyl(dimethyl)silyl]oxy}-3-methylcyclobutyl)-3-[4-chloro-2-(methoxymethoxy)-6-methylphenyl]-5-(difluoromethoxy)-7H-pyrrolo[2,3-c]pyridazine (47.3 mg, 0.083 mmol) of Example 29A was dissolved in DCM (1 mL), TFA (1 mL) was added, and the mixture was stirred at room temperature for 3 hr. To the reaction solution was added saturated aqueous sodium bicarbonate solution at 0°C, and then DCM/ethanol were added. The mixture was stirred, and the organic layer was separated through a phase separator (Biotage). The solvent was evaporated under reduced pressure, and the obtained residue was purified by silica gel column chromatography [eluent: DCM/methanol=98/2-88/12(V/V)], and triturated with ethyl acetate/diisopropyl ether/n-hexane to give the title compound (13.9 mg, yield: 41%) as a solid.

### (Example 30)

### 3-methyl-2-{6-methyl-7-[(3R)-1-methylpiperidin-3-yl]-6,7-dihydro-5H-pyrrolo[2,3-c]pyridazin-3-yl}-5-(trifluoromethyl)phenol

### (30A) 3,6-dichloro-4-[(1E)-prop-1-en-1-yl]pyridazine

Using 4-bromo-3,6-dichloropyridazine (1.01 g, 4.4 mmol), propen-1-ylboronic acid pinacol ester (894 mg, 5.3 mmol), potassium carbonate (1.47 g, 8.9 mmol) and Pd(dppf)Cl₂ (434 mg, 0.44 mmol), the title compound (430 mg, yield: 51%) was obtained as a solid by a method similar to that in Example 1C.

### (30B) tert-butyl (3R)-3-(3-chloro-6-methyl-5,6-dihydro-7H-pyrrolo[2,3-c]pyridazin-7-yl)piperidine-1-carboxylate

Using 3,6-dichloro-4-[(1E)-prop-1-en-1-yl]pyridazine (250 mg, 1.3 mmol) of Example 30A, and tert-butyl (3R)-3-aminopiperidine-1-carboxylate (320 mg, 1.6 mmol), the title compound (133 mg, yield: 29%) was obtained as a diastereomeric mixture (an oil) by a method similar to that in Example 1D.

### (30C) 3-chloro-6-methyl-7-[(3R)-piperidin-3-yl]-6,7-dihydro-5H-pyrrolo[2,3-c]pyridazine dihydrochloride

Using tert-butyl (3R)-3-(3-chloro-6-methyl-5,6-dihydro-7H-pyrrolo[2,3-c]pyridazin-7-yl)piperidine-1-carboxylate (112 mg, 0.31 mmol) of Example 30B, and 4 M hydrogen chloride-1,4-dioxane (0.70 mL), the title compound (88 mg, yield: 85%) was obtained as a diastereomeric mixture (a solid) by a method similar to that in Example 2B.

### (30D) 3-chloro-6-methyl-7-[(3R)-1-methylpiperidin-3-yl]-6,7-dihydro-5H-pyrrolo[2,3-c]pyridazine

Using 3-chloro-6-methyl-7-[(3R)-piperidin-3-yl]-6,7-dihydro-5H-pyrrolo[2,3-c]pyridazine dihydrochloride (88 mg, 0.27 mmol) of Example 30C, DIPEA (0.094 mL, 0.54 mmol), 37% formaldehyde (0.030 mL, 0.41 mmol), acetic acid (0.030 mL, 0.54 mmol) and NaBH(OAc)₃ (230 mg, 1.1 mmol), the title compound (47 mg, yield: 65%) was obtained as a diastereomeric mixture (an oil) by a method similar to that in Example 1G.

### (30E) 3-[2-methoxy-6-methyl-4-(trifluoromethyl)phenyl]-6-methyl-7-[(3R)-1-methylpiperidin-3-yl]-6,7-dihydro-5H-pyrrolo[2,3-c]pyridazine

Using 2-(2-methoxy-6-methyl-4-(trifluoromethylphenyl)-4,4,5,5-tetramethyl-1,3,2-dioxaborolane (US20200361898) (44 mg, 0.14 mmol), 3-chloro-6-methyl-7-[(3R)-1-methylpiperidin-3-yl]-6,7-dihydro-5H-pyrrolo[2,3-c]pyridazine (45 mg, 0.17 mmol) of Example 30D, potassium carbonate (39 mg, 0.28 mmol) and XPhos Pd G2 (11 mg, 0.014 mmol), the title compound (36 mg, yield: 62%) was obtained as a diastereomeric mixture (an amorphous form) by a method similar to that in Example 1E.

### (30F) 3-methyl-2-{6-methyl-7-[(3R)-1-methylpiperidin-3-yl]-6,7-dihydro-5H-pyrrolo[2,3-c]pyridazin-3-yl}-5-(trifluoromethyl)phenol

Using 3-[2-methoxy-6-methyl-4-(trifluoromethyl)phenyl]-6-methyl-7-[(3R)-1-methylpiperidin-3-yl]-6,7-dihydro-5H-pyrrolo[2,3-c]pyridazine (31 mg, 0.073 mmol) of Example 30E, and boron tribromide (1.0 M DCM solution) (0.22 mL, 0.22 mmol), the title compound (18 mg, yield: 60%) was obtained as a diastereomeric mixture (a solid) by a method similar to that in Example 2E.

### (Example 31)

### 3-methyl-2-[1-(1-methylpiperidin-3-yl)-1H-pyrazolo[3,4-c]pyridazin-5-yl]-5-(trifluoromethyl)phenol

### (31A) tert-butyl 3-{2-[(benzyloxy)carbonyl]hydrazinyl}piperidine-1-carboxylate

1-(tert-Butoxycarbonyl)-3-piperidone (2.98 g, 15 mmol) was dissolved in methanol (38 mL), benzyl carbazate (2.73 g, 17 mmol) was added, and the mixture was stirred at room temperature for 3.5 hr. To the reaction solution was added sodium cyanoborohydride (1.87 g, 30 mmol), and the mixture was stirred at room temperature for 20 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =70/30-50/50(V/V)] to give the title compound (1.95 g, yield: 37%) as a racemate (an amorphous form).

### (31B) tert-butyl 3-hydrazinylpiperidine-1-carboxylateacetate

tert-Butyl 3-{2-[(benzyloxy)carbonyl]hydrazinyl}piperidine-1-carboxylate (1.95 g, 5.6 mmol) of Example 31A was dissolved in ethanol (28 mL), then acetic acid (1.28 mL, 22 mmol) and 5% palladium on carbon (661 mg) were added, and the mixture was stirred in the hydrogen-substituted system at room temperature for 3.5 hr. The reaction solution was filtered through Celite, and the filtrate was concentrated under reduced pressure to give a crude product of the title compound.

### (31C) tert-butyl 3-(5-chloro-3-hydroxy-1H-pyrazolo[3,4-c]pyridazin-1-yl)piperidine-1-carboxylate

Methyl 3,6-dichloropyridazine-4-carboxylate (970 mg, 4.7 mmol) was dissolved in methanol (6 mL), then tert-butyl 3-hydrazinylpiperidine-1-carboxylateacetate (1.54 g, 5.6 mmol) of Example 31B, and DIPEA (2.5 mL, 14 mmol) were added, and the mixture was stirred at 70°C for 12 hr. To the reaction solution was added water at room temperature, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: DCM/ethyl acetate =80/20-60/40(V/V)] to give the title compound (155 mg, yield: 9% in two steps) as a racemate (a solid).

### (31D) tert-butyl 3-{3-hydroxy-5-[2-methoxy-6-methyl-4-(trifluoromethyl)phenyl]-1H-pyrazolo[3,4-c]pyridazin-1-yl}piperidine-1-carboxylate

Using tert-butyl 3-(5-chloro-3-hydroxy-1H-pyrazolo[3,4-c]pyridazin-1-yl)piperidine-1-carboxylate (206 mg, 0.58 mmol) of Example 31C, 2-(2-methoxy-6-methyl-4-(trifluoromethylphenyl)-4,4,5,5-tetramethyl-1,3,2-dioxaborolane (US20200361898) (367 mg, 1.2 mmol), potassium carbonate (160 mg, 1.2 mmol) and XPhos Pd G2 (90 mg, 0.12 mmol), the title compound (193 mg, yield: 65%) was obtained as a racemate (an amorphous form) by a method similar to that in Example 1E.

### (31E) tert-butyl 3-(5-[2-methoxy-6-methyl-4-(trifluoromethyl)phenyl]-3-{[(trifluoromethyl)sulfonyl]oxy}-1H-pyrazolo[3,4-c]pyridazin-1-yl)piperidine-1-carboxylate

tert-Butyl 3-{3-hydroxy-5-[2-methoxy-6-methyl-4-(trifluoromethyl)phenyl]-1H-pyrazolo[3,4-c]pyridazin-1-yl}piperidine-1-carboxylate (193 mg, 0.38 mmol) of Example 31D was dissolved in DCM (4 mL), then TEA (0.106 mL, 0.76 mmol) and N-phenylbis(trifluoromethanesulfonimide) (203 mg, 0.57 mmol) were added, and the mixture was stirred at room temperature for 1.5 hr. The reaction solution was filtered through Celite, the filtrate was concentrated under reduced pressure, and the obtained residue was purified by silica gel column chromatography [eluent: n-hexane/ethyl acetate =80/20-70/30(V/V)] to give the title compound (150 mg, yield: 62%) as a racemate (an oil).

### (31F) tert-butyl 3-{5-[2-methoxy-6-methyl-4-(trifluoromethyl)phenyl]-1H-pyrazolo[3,4-c]pyridazin-1-yl}piperidine-1-carboxylate

tert-Butyl 3-(5-[2-methoxy-6-methyl-4-(trifluoromethyl)phenyl]-3-{[(trifluoromethyl)sulfonyl]oxy}-1H-pyrazolo[3,4-c]pyridazin-1-yl)piperidine-1-carboxylate (150 mg, 0.23 mmol) of Example 31E was dissolved in THF (2 mL), then Pd(dppf)Cl₂ (34 mg, 0.047 mmol), 1,1'-bis(diphenylphosphino)ferrocene (26 mg, 0.047 mmol), ammonium formate (73 mg, 1.2 mmol) and TEA (0.163 mL, 1.2 mmol) were added, and the mixture was stirred in the nitrogen-substituted system at 75°C for 3 hr. The reaction solution was allowed to room temperature, saturated aqueous sodium bicarbonate solution was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-60/40(V/V)] to give the title compound (87 mg, yield: 75%) as a racemate (an oil).

### (31G) 5-[2-methoxy-6-methyl-4-(trifluoromethyl)phenyl]-1-(piperidin-3-yl)-1H-pyrazolo[3,4-c]pyridazine monohydrochloride

Using tert-butyl 3-{5-[2-methoxy-6-methyl-4-(trifluoromethyl)phenyl]-1H-pyrazolo[3,4-c]pyridazin-1-yl}piperidine-1-carboxylate (87 mg, 0.18 mmol) of Example 31F, and 4 M hydrogen chloride-1,4-dioxane (0.9 mL), a crude product (75 mg, yield: quantitative) of the title compound was obtained as a racemate (a solid) by a method similar to that in Example 2B.

### (31H) 5-[2-methoxy-6-methyl-4-(trifluoromethyl)phenyl]-1-(1-methylpiperidin-3-yl)-1H-pyrazolo[3,4-c]pyridazine

Using 5-[2-methoxy-6-methyl-4-(trifluoromethyl)phenyl]-1-(piperidin-3-yl)-1H-pyrazolo[3,4-c]pyridazine monohydrochloride (75 mg, 0.18 mmol) of Example 31G, DIPEA (0.061 mL, 0.35 mmol), 37% formaldehyde (0.019 mL, 0.26 mmol), acetic acid (0.020 mL, 0.35 mmol) and NaBH(OAc)₃ (148 mg, 0.70 mmol), the title compound (62 mg, yield: 87%) was obtained as a racemate (an oil) by a method similar to that in Example 1G.

### (31I) 3-methyl-2-[1-(1-methylpiperidin-3-yl)-1H-pyrazolo[3,4-c]pyridazin-5-yl]-5-(trifluoromethyl)phenol

Using 5-[2-methoxy-6-methyl-4-(trifluoromethyl)phenyl]-1-(1-methylpiperidin-3-yl)-1H-pyrazolo[3,4-c]pyridazine (60 mg, 0.15 mmol) of Example 31H, and boron tribromide (1.0 M DCM solution) (0.75 mL, 0.75 mmol), the title compound (35 mg, yield: 60%) was obtained as a racemate (a solid) by a method similar to that in Example 2E.

### (Example 32)

### 3-methyl-2-{7-[(3R)-1-methylpiperidin-3-yl]-7H-imidazo[4, 5-c]pyridazin-3-yl}-5-(trifluoromethyl)phenol

### (32A) tert-butyl (3R)-3-[(4-amino-6-chloropyridazin-3-yl)amino]piperidine-1-carboxylate

3,6-Dichloropyridazin-4-amine (700 mg, 4.3 mmol), and tert-butyl (3R)-3-aminopiperidine-1-carboxylate (940 mg, 4.7 mmol) were dissolved in 1-decanol (1.4 mL), DIPEA (1.1 mL, 6.4 mmol) was added, and the mixture was stirred at 150°C for 13 hr. The reaction solution was concentrated under reduced pressure, and the obtained residue was purified by silica gel column chromatography [eluent: DCM/methanol=95/5-80/20(V/V)] to give a crude product of the title compound as an oil.

### (32B) tert-butyl (3R)-3-(3-chloro-7H-imidazo[4,5-c]pyridazin-7-yl)piperidine-1-carboxylate

To tert-butyl (3R)-3-[(4-amino-6-chloropyridazin-3-yl)amino]piperidine-1-carboxylate (590 mg, 1.8 mmol) of Example 32A were added triethyl orthoformate (6.0 mL, 36 mmol) and formic acid (0.3 mL, 9.0 mmol), and the mixture was stirred at 100°C for 11 hr. The reaction solution was concentrated under reduced pressure, and the obtained residue was purified by silica gel column chromatography [eluent: n-hexane/ethyl acetate =75/25-0/100(V/V)] to give the title compound (54 mg, yield: 4% in two steps) as an amorphous form.

### (32C) 3-chloro-7-[(3R)-piperidin-3-yl]-7H-imidazo[4,5-c]pyridazine monohydrochloride

Using tert-butyl (3R)-3-(3-chloro-7H-imidazo[4,5-c]pyridazin-7-yl)piperidine-1-carboxylate (54 mg, 0.16 mmol) of Example 32B, and 4 M hydrogen chloride-1,4-dioxane (1.5 mL), a crude product (43 mg, yield: quantitative) of the title compound was obtained as a solid by a method similar to that in Example 2B.

### (32D) 3-chloro-7-[(3R)-1-methylpiperidin-3-yl]-7H-imidazo[4,5-c]pyridazine

Using 3-chloro-7-[(3R)-piperidin-3-yl]-7H-imidazo[4,5-c]pyridazine monohydrochloride (43 mg, 0.16 mmol) of Example 32C, DIPEA (0.081 mL, 0.47 mmol), 37% formaldehyde (0.020 mL, 0.27 mmol), acetic acid (0.017 mL, 0.31 mmol) and NaBH(OAc)₃ (132 mg, 0.63 mmol), the title compound (19 mg, yield: 48%) was obtained as a solid by a method similar to that in Example 1G.

### (32E) 3-[2-methoxy-6-methyl-4-(trifluoromethyl)phenyl]-7-[(3R)-1-methylpiperidin-3-yl]-7H-imidazo[4,5-c]pyridazine

Using 3-chloro-7-[(3R)-1-methylpiperidin-3-yl]-7H-imidazo[4,5-c]pyridazine (19 mg, 0.075 mmol) of Example 32D, 2-(2-methoxy-6-methyl-4-(trifluoromethylphenyl)-4,4,5,5-tetramethyl-1,3,2-dioxaborolane (US20200361898) (40 mg, 0.13 mmol), potassium carbonate (20 mg, 0.15 mmol) and XPhos Pd G2 (8 mg, 0.011 mmol), the title compound (12 mg, yield: 39%) was obtained as an oil by a method similar to that in Example 1E.

### (32F) 3-methyl-2-{7-[(3R)-1-methylpiperidin-3-yl]-7H-imidazo[4,5-c]pyridazin-3-yl}-5-(trifluoromethyl)phenol

Using 3-[2-methoxy-6-methyl-4-(trifluoromethyl)phenyl]-7-[(3R)-1-methylpiperidin-3-yl]-7H-imidazo[4,5-c]pyridazine (12 mg, 0.030 mmol) of Example 32E, and boron tribromide (1.0 M DCM solution) (0.15 mL, 0.15 mmol), the title compound (7.6 mg, yield: 65%) was obtained as a solid by a method similar to that in Example 2E.

### (Example 33)

### 3-methyl-2-{(5S or 5R)-5-methyl-7-[(3R)-1-methylpiperidin-3-yl]-6,7-dihydro-5H-pyrrolo[2,3-c]pyridazin-3-yl}-5-(trifluoromethyl)phenol

### (33A) tert-butyl (3R)-3-[(5S or 5R)-3-chloro-5-methyl-5,6-dihydro-7H-pyrrolo[2,3-c]pyridazin-7-yl]piperidine-1-carboxylate

Using 3,6-dichloro-4-(prop-1-en-2-yl)pyridazine (1.04 g, 5.5 mmol) of Example 4A, tert-butyl (3R)-3-aminopiperidine-1-carboxylate (1.32 g, 6.6 mmol) and DIPEA (1.44 mL, 8.3 mmol), the title compound eluting earlier (92 mg, yield: 5%) was obtained as a single diastereomer (an amorphous form) by a method similar to that in Example 1D and purification by silica gel column chromatography [eluent: n-hexane/ethyl acetate =65/35-45/55(V/V)].

### (33B) (5S or 5R)-3-chloro-5-methyl-7-[(3R)-piperidin-3-yl]-6,7-dihydro-5H-pyrrolo[2,3-c]pyridazine monohydrochloride

Using tert-butyl (3R)-3-[(5S or 5R)-3-chloro-5-methyl-5,6-dihydro-7H-pyrrolo[2,3-c]pyridazin-7-yl]piperidine-1-carboxylate (92 mg, 0.26 mmol) of Example 33A, and 4 M hydrogen chloride-1,4-dioxane (2.5 mL), a crude product (75 mg, yield: quantitative) of the title compound was obtained as a single diastereomer (a solid)by a method similar to that in Example 2B.

### (33C) (5S or 5R)-3-chloro-5-methyl-7-[(3R)-1-methylpiperidin-3-yl]-6,7-dihydro-5H-pyrrolo[2,3-c]pyridazine

Using (5S or 5R)-3-chloro-5-methyl-7-[(3R)-piperidin-3-yl]-6,7-dihydro-5H-pyrrolo[2,3-c]pyridazine monohydrochloride (75 mg, 0.26 mmol) of Example 33B, DIPEA (0.090 mL, 0.52 mmol), 37% formaldehyde (0.039 mL, 0.52 mmol), acetic acid (0.029 mL, 0.52 mmol) and NaBH(OAc)₃ (219 mg, 1.04 mmol), the title compound (42 mg, yield: 61%) was obtained as a single diastereomer (an oil) by a method similar to that in Example 1G.

### (33D) (5S or 5R)-3-[2-methoxy-6-methyl-4-(trifluoromethyl)phenyl]-5-methyl-7-[(3R)-1-methylpiperidin-3-yl]-6,7-dihydro-5H-pyrrolo[2,3-c]pyridazine

Using (5S or 5R)-3-chloro-5-methyl-7-[(3R)-1-methylpiperidin-3-yl]-6,7-dihydro-5H-pyrrolo[2,3-c]pyridazine (35 mg, 0.13 mmol) of Example 33C, 2-(2-methoxy-6-methyl-4-(trifluoromethylphenyl)-4,4,5,5-tetramethyl-1,3,2-dioxaborolane (US20200361898) (70 mg, 0.22 mmol), potassium carbonate (36 mg, 0.26 mmol) and XPhos Pd G2 (15 mg, 0.020 mmol), the title compound (36 mg, yield: 65%) was obtained as a single diastereomer (an oil) by a method similar to that in Example 1E.

### (33E) 3-methyl-2-{(5S or 5R)-5-methyl-7-[(3R)-1-methylpiperidin-3-yl]-6,7-dihydro-5H-pyrrolo[2,3-c]pyridazin-3-yl}-5-(trifluoromethyl)phenol

Using (5S or 5R)-3-[2-methoxy-6-methyl-4-(trifluoromethyl)phenyl]-5-methyl-7-[(3R)-1-methylpiperidin-3-yl]-6,7-dihydro-5H-pyrrolo[2,3-c]pyridazine (36 mg, 0.086 mmol) of Example 33D, and boron tribromide (1.0 M DCM solution) (0.4 mL, 0.40 mmol), the title compound (23 mg, yield: 66%) was obtained as a single diastereomer (a solid) by a method similar to that in Example 2E.

### (Example 34)

### 3-methyl-2-{(5R or 5S)-5-methyl-7-[(3R)-1-methylpiperidin-3-yl]-6,7-dihydro-5H-pyrrolo[2,3-c]pyridazin-3-yl}-5-(trifluoromethyl)phenol

### (34A) tert-butyl (3R)-3-[(5R or 5S)-3-chloro-5-methyl-5,6-dihydro-7H-pyrrolo[2,3-c]pyridazin-7-yl]piperidine-1-carboxylate

Using 3,6-dichloro-4-(prop-1-en-2-yl)pyridazine (1.04 g, 5.5 mmol) of Example 4A, tert-butyl (3R)-3-aminopiperidine-1-carboxylate (1.32 g, 6.6 mmol) and DIPEA (1.44 mL, 8.3 mmol), the title compound eluting later (95 mg, yield: 5%) was obtained as a single diastereomer (an amorphous form) by a method similar to that in Example 1D and purification by silica gel column chromatography [eluent: n-hexane/ethyl acetate =65/35-45/55(V/V)].

### (34B) (5R or 5S)-3-chloro-5-methyl-7-[(3R)-piperidin-3-yl]-6,7-dihydro-5H-pyrrolo[2,3-c]pyridazine monohydrochloride

Using tert-butyl (3R)-3-[(5R or 5S)-3-chloro-5-methyl-5,6-dihydro-7H-pyrrolo[2,3-c]pyridazin-7-yl]piperidine-1-carboxylate (95 mg, 0.27 mmol) of Example 34A, and 4 M hydrogen chloride-1,4-dioxane (2.5 mL), a crude product (77 mg, yield: quantitative) of the title compound was obtained as a single diastereomer (a solid) by a method similar to that in Example 2B.

### (34C) (5R or 5S)-3-chloro-5-methyl-7-[(3R)-1-methylpiperidin-3-yl]-6,7-dihydro-5H-pyrrolo[2,3-c]pyridazine

Using (5R or 5S)-3-chloro-5-methyl-7-[(3R)-piperidin-3-yl]-6,7-dihydro-5H-pyrrolo[2,3-c]pyridazine monohydrochloride (77 mg, 0.27 mmol) of Example 34B, DIPEA (0.092 mL, 0.53 mmol), 37% formaldehyde (0.040 mL, 0.53 mmol), acetic acid (0.030 mL, 0.53 mmol) and NaBH(OAc)₃ (225 mg, 1.1 mmol), the title compound (42 mg, yield: 59%) was obtained as a single diastereomer (an oil) by a method similar to that in Example 1G.

### (34D) (5R or 5S)-3-[2-methoxy-6-methyl-4-(trifluoromethyl)phenyl]-5-methyl-7-[(3R)-1-methylpiperidin-3-yl]-6,7-dihydro-5H-pyrrolo[2,3-c]pyridazine

Using (5R or 5S)-3-chloro-5-methyl-7-[(3R)-1-methylpiperidin-3-yl]-6,7-dihydro-5H-pyrrolo[2,3-c]pyridazine (42 mg, 0.16 mmol) of Example 34C, 2-(2-methoxy-6-methyl-4-(trifluoromethylphenyl)-4,4,5,5-tetramethyl-1,3,2-dioxaborolane (US20200361898) (84 mg, 0.27 mmol), potassium carbonate (43 mg, 0.31 mmol) and XPhos Pd G2 (18 mg, 0.024 mmol), the title compound (60 mg, yield: 90%) was obtained as a single diastereomer (an oil) by a method similar to that in Example 1E.

### (34E) 3-methyl-2-{ (5R or 5S)-5-methyl-7-[(3R)-1-methylpiperidin-3-yl]-6,7-dihydro-5H-pyrrolo[2,3-c]pyridazin-3-yl}-5-(trifluoromethyl)phenol

Using (5R or 5S)-3-[2-methoxy-6-methyl-4-(trifluoromethyl)phenyl]-5-methyl-7-[(3R)-1-methylpiperidin-3-yl]-6,7-dihydro-5H-pyrrolo[2,3-c]pyridazine (60 mg, 0.14 mmol) of Example 34D, and boron tribromide (1.0 M DCM solution) (0.7 mL, 0.70 mmol), the title compound (37 mg, yield: 64%) was obtained as a single diastereomer (a solid) by a method similar to that in Example 2E.

### (Example 35)

### 3-methyl-2-{5-methyl-8-[(3R)-1-methylpiperidin-3-yl]-5,6,7,8-tetrahydropyrazino[2,3-c]pyridazin-3-yl}-5-(trifluoromethyl)phenol

### (35A) N-(2-{[tert-butyl(dimethyl)silyl]oxy}ethyl)-3,6-dichloro-N-methylpyridazin-4-amine

4-Bromo-3,6-dichloropyridazine (500 mg, 2.1 mmol) was dissolved in DMF (4.17 mL), then potassium carbonate (873 mg, 6.3 mmol) and N-[2-(tert-butyldimethylsilyloxyethyl]methylamine (448 mg, 2.3 mmol) were added, and the mixture was stirred in the nitrogen-substituted system at room temperature for 1.5 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =95/5-85/15(V/V)] to give the title compound (656 mg, yield: 94%) as an oil.

### (35B) 2-[(3,6-dichloropyridazin-4-yl)(methyl)amino]ethanol

N-(2-{[tert-Butyl(dimethyl)silyl]oxy}ethyl)-3,6-dichloro-N-methylpyridazin-4-amine (656 mg, 2.0 mmol) of Example 35A was dissolved in THF (4.88 mL), TBAF (1.0 M THF solution) (2.34 mL, 2.3 mmol) was added, and the mixture was stirred in the nitrogen-substituted system at room temperature for 30 min. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =60/40-33/67(V/V)] to give the title compound (279 mg, yield: 64%) as a solid.

### (35C) 2-[(3,6-dichloropyridazin-4-yl)(methyl)amino]ethyl 4-methylbenzenesulfonate

2-[(3,6-Dichloropyridazin-4-yl)(methyl)amino]ethanol (279 mg, 1.3 mmol) of Example 35B was dissolved in DCM (2.51 mL), then TEA (0.26 mL, 1.9 mmol), 4-dimethylaminopyridine (16 mg, 0.13 mmol) and p-toluenesulfonyl chloride (331 mg, 1.6 mmol) were added, and the mixture was stirred in the nitrogen-substituted system at room temperature for 3 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-60/40(V/V)] to give the title compound (357 mg, yield: 76%) as a solid.

### (35D) tert-butyl (3R)-3-(3-chloro-5-methyl-6,7-dihydropyrazino[2,3-c]pyridazin-8(5H)-yl)piperidine-1-carboxylate

2-[(3,6-Dichloropyridazin-4-yl)(methyl)amino]ethyl 4-methylbenzenesulfonate (357 mg, 0.95 mmol) of Example 35C was dissolved in DMSO (4.74 mL), then tert-butyl (3R)-3-aminopiperidine-1-carboxylate (213 mg, 1.0 mmol) and DIPEA (0.328 mL, 1.9 mmol) were added, and the mixture was stirred in the nitrogen-substituted system at 50°C for 15 hr. To the reaction solution was added water, and the mixture was subjected to extraction with ethyl acetate. The organic layer was washed with saturated brine, and dried over anhydrous magnesium sulfate. After filtration, the filtrate was concentrated under reduced pressure, and the obtained residue was purified by silica gel column chromatography [eluent: n-hexane/ethyl acetate =67/33-33/67(V/V)] to give the title compound (116 mg, yield: 33%) as an amorphous form.

### (35E) 3-chloro-5-methyl-8-[(3R)-1-methylpiperidin-3-yl]-5,6,7,8-tetrahydropyrazino[2,3-c]pyridazine

tert-Butyl (3R)-3-(3-chloro-5-methyl-6,7-dihydropyrazino[2,3-c]pyridazin-8(5H)-yl)piperidine-1-carboxylate (116 mg, 0.32 mmol) of Example 35D was dissolved in DCM (2.10 mL), TFA (1.22 mL) was added, and the mixture was stirred at room temperature for 25 min. The reaction solution was concentrated under reduced pressure, and the obtained residue was dissolved in methanol (2.10 mL). DIPEA (1.09 mL, 6.3 mmol), 37% formaldehyde (0.026 mL, 0.35 mmol) and NaBH(OAc)₃ (209 mg, 0.79 mmol) were added, and the mixture was stirred at room temperature for 1 hr. To the reaction solution was added saturated aqueous sodium bicarbonate solution, and the mixture was subjected to extraction with DCM, and the organic layer was dried over anhydrous magnesium sulfate. After filtration, the filtrate was concentrated under reduced pressure, and the obtained residue was purified by NH silica gel column chromatography [eluent: ethyl acetate/methanol=100/0-95/5(V/V)] to give the title compound (65 mg, yield: 73%) as a solid.

### (35F) 3-[2-(ethoxymethoxy)-6-methyl-4-(trifluoromethyl)phenyl]-5-methyl-8-[(3R)-1-methylpiperidin-3-yl]-5,6,7,8-tetrahydropyrazino[2,3-c]pyridazine

Using 3-chloro-5-methyl-8-[(3R)-1-methylpiperidin-3-yl]-5,6,7,8-tetrahydropyrazino[2,3-c]pyridazine (65 mg, 0.23 mmol) of Example 35E, 2-[2-(ethoxymethoxy)-6-methyl-4- 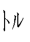 fluoromethylphenyl]-4,4,5,5-tetramethyl-1,3,2-dioxaborolane (US20200361898) (257 mg, 0.71 mmol), XPhos Pd G2 (36 mg, 0.046 mmol) and potassium carbonate (128 mg, 0.92 mmol), the title compound (65 mg, yield: 59%) was obtained as a solid by a method similar to that in Example 9D.

### (35G) 3-methyl-2-{5-methyl-8-[(3R)-1-methylpiperidin-3-yl]-5,6,7,8-tetrahydropyrazino[2,3-c]pyridazin-3-yl}-5-(trifluoromethyl)phenol

3-[2-(Ethoxymethoxy)-6-methyl-4-(trifluoromethyl)phenyl]-5-methyl-8-[(3R)-1-methylpiperidin-3-yl]-5,6,7,8-tetrahydropyrazino[2,3-c]pyridazine (65 mg, 0.14 mmol) of Example 35F was dissolved in DCM (0.904 mL), TFA (0.524 mL) was added, and the mixture was stirred at room temperature for 1 hr. The reaction solution was concentrated under reduced pressure, and the obtained residue was purified by NH silica gel column chromatography [eluent: ethyl acetate/methanol=95/5-80/20(V/V)] to give the title compound (41.7 mg, yield: 73%) as an amorphous form.

### (Example 36)

### 3-methyl-2-{8-[(3R)-1-methylpiperidin-3-yl]-5,6,7,8-tetrahydropyrido[2,3-c]pyridazin-3-yl}-5-(trifluoromethyl)phenol

### (36A) tert-butyl (3R)-3-(3-chloro-5-hydroxy-6,7-dihydropyrido[2,3-c]pyridazin-8(5H)-yl)piperidine-1-carboxylate

Using 3-chloro-1-(3,6-dichloropyridazin-4-yl)propan-1-ol (Org. Lett. 2015, 17, 2362.) (17 mg, 0.071 mmol), DIPEA (0.36 mL, 2.1 mmol) and tert-butyl (3R)-3-aminopiperidine-1-carboxylate (282 mg, 1.4 mmol), the title compound (18 mg, yield: 69%) was obtained as an oil by a method similar to that in Example 1D.

### (36B) tert-butyl (3R)-3-{5-hydroxy-3-[2-(methoxymethoxy)-6-methyl-4-(trifluoromethyl)phenyl]-6,7-dihydropyrido[2,3-c]pyridazin-8(5H)-yl}piperidine-1-carboxylate

Using tert-butyl (3R)-3-(3-chloro-5-hydroxy-6,7-dihydropyrido[2,3-c]pyridazin-8(5H)-yl)piperidine-1-carboxylate (17 mg, 0.046 mmol) of Example 36A, 2-[2-(methoxymethoxy)-6-methyl-4-(trifluoromethyl)phenyl]-4,4,5,5-tetramethyl-1,3,2-dioxaborolane (19 mg, 0.055 mmol) of Example 1B, potassium carbonate (13 mg, 0.092 mmol) and XPhos Pd G2 (3.6 mg, 0.0046 mmol), the title compound (16 mg, yield: 63%) was obtained as an oil by a method similar to that in Example 1E.

### (36C) tert-butyl (3R)-3-{3-[2-(methoxymethoxy)-6-methyl-4-(trifluoromethyl)phenyl]-5-[(phenoxycarbonothioyl)oxy]-6,7-dihydropyrido[2,3-c]pyridazin-8(5H)-yl}piperidine-1-carboxylate

tert-Butyl (3R)-3-{5-hydroxy-3-[2-(methoxymethoxy)-6-methyl-4-(trifluoromethyl)phenyl]-6,7-dihydropyrido[2,3-c]pyridazin-8(5H)-yl}piperidine-1-carboxylate (12 mg, 0.022 mmol) of Example 36B, and 4-dimethylaminopyridine (6.4 mg, 0.052 mmol) were dissolved in DCM (0.21 mL), phenyl chlorothionoformate (0.0035 mL, 0.026 mmol) was added, and the mixture was stirred at room temperature for 3 days. To the reaction solution was added water, the mixture was subjected to extraction with ethyl acetate, and the organic layer was dried over anhydrous sodium sulfate. After filtration, the filtrate was concentrated under reduced pressure to give a crude product (15 mg, yield: quantitative) of the title compound as an oil.

### (36D) tert-butyl (3R)-3-{3-[2-(methoxymethoxy)-6-methyl-4-(trifluoromethyl)phenyl]-6,7-dihydropyrido[2,3-c]pyridazin-8(5H)-yl}piperidine-1-carboxylate

tert-Butyl (3R)-3-{3-[2-(methoxymethoxy)-6-methyl-4-(trifluoromethyl)phenyl]-5-[(phenoxycarbonothioyl)oxy]-6,7-dihydropyrido[2,3-c]pyridazin-8(5H)-yl}piperidine-1-carboxylate (15 mg, 0.022 mmol) of Example 36C was dissolved in toluene (0.22 mL), then tributyltin hydride (0.023 mL, 0.087 mmol) and azobisisobutyronitrile (1.8 mg, 0.011 mmol) were added, and the mixture was stirred in the nitrogen-substituted system at 110°C for 3 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (5.6 mg, yield: 48%) as an oil.

### (36E) 3-methyl-2-{8-[(3R)-piperidin-3-yl]-5,6,7,8-tetrahydropyrido[2,3-c]pyridazin-3-yl}-5-(trifluoromethyl)phenol dihydrochloride

Using tert-butyl (3R)-3-{3-[2-(methoxymethoxy)-6-methyl-4-(trifluoromethyl)phenyl]-6,7-dihydropyrido[2,3-c]pyridazin-8(5H)-yl}piperidine-1-carboxylate (5.6 mg, 0.010 mmol) of Example 36D, and 4 M hydrogen chloride-1,4-dioxane (0.10 mL), the title compound (4.4 mg, yield: 91%) was obtained as an oil by a method similar to that in Example 1F.

### (36F) 3-methyl-2-{8-[(3R)-1-methylpiperidin-3-yl]-5,6,7,8-tetrahydropyrido[2,3-c]pyridazin-3-yl}-5-(trifluoromethyl)phenol

Using 3-methyl-2-{8-[(3R)-piperidin-3-yl]-5,6,7,8-tetrahydropyrido[2,3-c]pyridazin-3-yl}-5-(trifluoromethyl)phenol dihydrochloride (4.4 mg, 0.0096 mmol) of Example 36E, DIPEA (0.0033 mL, 0.019 mmol), 37% formaldehyde (0.0010 mL, 0.014 mmol), acetic acid (0.0011 mL, 0.019 mmol) and NaBH(OAc)₃ (8.0 mg, 0.038 mmol), the title compound (3.2 mg, yield: 83%) was obtained as an oil by a method similar to that in Example 1G.

### (Example 37)

### 3-methyl-2-{5-methyl-9-[(3R)-1-methylpiperidin-3-yl]-6,7,8,9-tetrahydro-5H-pyridazino[3,4-b][1,4]diazepin-3-yl}-5-(trifluoromethyl)phenol

### (37A) 3-[(3,6-dichloropyridazin-4-yl)(methyl)amino]propan-1-ol

4-Bromo-3,6-dichloropyridazine (400 mg, 1.7 mmol) was dissolved in DMF (6 mL), then potassium carbonate (460 mg, 3.3 mmol) and 3-(methylamino)-1-propanol (0.173 mL, 1.8 mmol) were added, and the mixture was stirred at room temperature for 23 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =20/80-0/100(V/V)] to give the title compound (371 mg, yield: 94%) as a solid.

### (37B) 3-[(3,6-dichloropyridazin-4-yl)(methyl)amino]propyl 4-methylbenzenesulfonate

3-[(3,6-Dichloropyridazin-4-yl)(methyl)amino]propan-1-ol (371 mg, 1.6 mmol) of Example 37A was dissolved in DCM (8 mL), then TEA (0.770 mL, 5.5 mmol), p-toluenesulfonyl chloride (508 mg, 2.7 mmol) and dimethylaminopyridine (40 mg, 0.31 mmol) were added, and the mixture was stirred at room temperature for 50 min. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-20/80(V/V)] to give the title compound (383 mg, yield: 98%) as a solid.

### (37C) tert-butyl (3R)-3-(3-chloro-5-methyl-5,6,7,8-tetrahydro-9H-pyridazino[3,4-b][1,4]diazepin-9-yl)piperidine-1-carboxylate

Using 3-[(3,6-dichloropyridazin-4-yl)(methyl)amino]propyl 4-methylbenzenesulfonate (383 mg, 0.98 mmol) of Example 37B, DIPEA (0.376 mL, 2.2 mmol) and tert-butyl (3R)-3-aminopiperidine-1-carboxylate (235 mg, 1.2 mmol), the title compound (97 mg, yield 26%) was obtained as an amorphous form by a method similar to that in Example 1D.

### (37D) 3-chloro-5-methyl-9-[(3R)-piperidin-3-yl]-6,7,8,9-tetrahydro-5H-pyridazino[3,4-b][1,4]diazepine

tert-Butyl (3R)-3-(3-chloro-5-methyl-5,6,7,8-tetrahydro-9H-pyridazino[3,4-b][1,4]diazepin-9-yl)piperidine-1-carboxylate (97 mg, 0.25 mmol) of Example 37C was dissolved in DCM (1 mL), TFA (1.0 mL) was added, and the mixture was stirred at room temperature for 20 min. The reaction solution was concentrated under reduced pressure, and the obtained residue was purified by NH silica gel column chromatography [eluent: ethyl acetate/methanol=98/2, DCM/methanol=80/20(V/V)] to give the title compound (66 mg, yield: 92%) as an amorphous form.

### (37E) 3-chloro-5-methyl-9-[(3R)-1-methylpiperidin-3-yl]-6,7,8,9-tetrahydro-5H-pyridazino[3,4-b][1,4]diazepine

Using 3-chloro-5-methyl-9-[(3R)-piperidin-3-yl]-6,7,8,9-tetrahydro-5H-pyridazino[3,4-b][1,4]diazepine (66 mg, 0.23 mmol) of Example 37D, 37% formaldehyde (0.026 mL, 0.35 mmol) and NaBH(OAc)₃ (148 mg, 0.70 mmol), the title compound (49 mg, yield: 70%) was obtained as a solid by a method similar to that in Example 1G.

### (37F) 3-[2-(methoxymethoxy)-6-methyl-4-(trifluoromethyl)phenyl]-5-methyl-9-[(3R)-1-methylpiperidin-3-yl]-6,7,8,9-tetrahydro-5H-pyridazino[3,4-b][1,4]diazepine

Using 3-chloro-5-methyl-9-[(3R)-1-methylpiperidin-3-yl]-6,7,8,9-tetrahydro-5H-pyridazino[3,4-b][1,4]diazepine (49 mg, 0.17 mmol) of Example 37E, 2-[2-(methoxymethoxy)-6-methyl-4-(trifluoromethyl)phenyl]-4,4,5,5-tetramethyl-1,3,2-dioxaborolane (75 mg, 0.22 mmol) of Example 1B, potassium carbonate (45 mg, 0.33 mmol) and XPhos Pd G2 (13 mg, 0.017 mmol), the title compound (50 mg, yield: 63%) was obtained as an amorphous form by a method similar to that in Example 1E.

### (37G) 3-methyl-2-{5-methyl-9-[(3R)-1-methylpiperidin-3-yl]-6,7,8,9-tetrahydro-5H-pyridazino[3,4-b][1,4]diazepin-3-yl}-5-(trifluoromethyl)phenol

Using 3-[2-(methoxymethoxy)-6-methyl-4-(trifluoromethyl)phenyl]-5-methyl-9-[(3R)-1-methylpiperidin-3-yl]-6,7,8,9-tetrahydro-5H-pyridazino[3,4-b][1,4]diazepine (50 mg, 0.10 mmol) of Example 37F, and TFA (0.5 mL), the title compound (38 mg, yield: 84%) was obtained as a solid by a method similar to that in Example 6B.

### (Example 38)

### 2-{9-[(3R)-1-methylpiperidin-3-yl]-5,6,8,9-tetrahydro-4H,7H-1,2,6a,9-tetraazaphenalen-3-yl}-5-(trifluoromethyl)phenol

### (38A) 1-(2-{[tert-butyl(dimethyl)silyl]oxy}ethyl)piperidin-2-one

2-Piperidone (5.92 g, 60 mmol) was dissolved in THF (200 mL), 63% sodium hydride (2.73 g, 72 mmol) was added at 0°C, and the mixture was stirred at room temperature for 10 min. (2-Bromoethoxy)tert-butyldimethylsilane (15.3 mL, 72 mmol) was added, and the mixture was stirred at 70°C for 14 hr. To the reaction solution was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: DCM/methanol=98/2-95/5(V/V)] to give the title compound (6.15 g, yield: 40%) as an oil.

### (38B) 1-(2-{[tert-butyl(dimethyl)silyl]oxy}ethyl)piperidine-2-thione

1-(2-{[tert-Butyl(dimethyl)silyl]oxy}ethyl)piperidin-2-one (4.34 g, 17 mmol) of Example 38A was dissolved in toluene (34 mL), Lawesson's reagent (7.16 g, 18 mmol) was added, and the mixture was stirred at 80°C for 15 min. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =85/15-76/24(V/V)] to give the title compound (980 mg, yield: 21%) as an oil.

### (38C) (E)-[1-(2-{[tert-butyl(dimethyl)silyl]oxy}ethyl)piperidin-2-ylidene] (methyl)sulfonium iodide

1-(2-{[tert-Butyl(dimethyl)silyl]oxy}ethyl)piperidine-2-thione (980 mg, 3.6 mmol) of Example 38B was dissolved in ethanol (12 mL), iodomethane (0.334 mL, 5.4 mmol) was added, and the mixture was stirred at 80°C for 30 min. The reaction solution was concentrated under reduced pressure, to the obtained residue were added ethyl acetate and n-hexane, and the mixture was subjected to ultrasonic treatment. The solid was collected by filtration to give the title compound (1.0 g, yield: 67%) as a solid.

### (38D) 1-(2-{[tert-butyl(dimethyl)silyl]oxy}ethyl)-5,8-dichloro-1,2,3,4-tetrahydropyrido[2,3-d]pyridazine

(E)-[1-(2-{[tert-Butyl(dimethyl)silyl]oxy}ethyl)piperidin-2-ylidene] (methyl) sulfonium iodide (1.0 g, 2.4 mmol) of Example 38C was suspended in THF (7 mL), potassium tert-butoxide (538 mg, 4.8 mmol) was added at 0°C, and the mixture was stirred at room temperature for 10 min. The reaction solution was filtered through Celite, and the filtrate was concentrated under reduced pressure. The obtained residue was dissolved in toluene (1.5 mL), to a solution of 3,6-dichloro-1,2,4,5-tetrazine (315 mg, 2.1 mmol) in toluene (5 mL) was added at 0°C, and the mixture was stirred at room temperature for 45 min. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =85/15-75/25(V/V)] to give the title compound (160 mg, yield: 21%) as a solid.

### (38E) 2-(5,8-dichloro-3,4-dihydropyrido[2,3-d]pyridazin-1(2H)-yl)ethanol

1-(2-{[tert-Butyl(dimethyl)silyl]oxy}ethyl)-5,8-dichloro-1,2,3,4-tetrahydropyrido[2,3-d]pyridazine (193 mg, 0.53 mmol) of Example 38D was dissolved in THF (2.5 mL), TBAF (1.0 M THF solution) (0.610 mL, 0.61 mmol) was added at 0°C, and the mixture was stirred for 40 min. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =25/75-0/100(V/V)] to give the title compound (90 mg, yield: 68%) as a solid.

### (38F) 2-(5,8-dichloro-3,4-dihydropyrido[2,3-d]pyridazin-1(2H)-yl)ethyl 4-methylbenzenesulfonate

2-(5,8-Dichloro-3,4-dihydropyrido[2,3-d]pyridazin-1(2H)-yl)ethanol (90 mg, 0.36 mmol) of Example 38E was dissolved in DCM (2 mL), then TEA (0.152 mL, 1.1 mmol), p-toluenesulfonyl chloride (138 mg, 0.72 mmol) and dimethylaminopyridine (4 mg, 0.036 mmol) were added, and the mixture was stirred at room temperature for 105 min. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =65/35-40/60(V/V)] to give the title compound (128 mg, yield: 88%) as a solid.

### (38G) 3-chloro-9-[(3R)-1-methylpiperidin-3-yl]-5,6,8,9-tetrahydro-4H,7H-1,2,6a,9-tetraazaphenalene

2-(5,8-Dichloro-3,4-dihydropyrido[2,3-d]pyridazin-1(2H)-yl)ethyl 4-methylbenzenesulfonate (128 mg, 0.32 mmol) of Example 38F was dissolved in NMP (1.6 mL), then DIPEA (0.138 mL, 0.80 mmol) and (R)-3-amino-1-methylpiperidine (47 mg, 0.41 mmol) were added, and the mixture was stirred under microwave irradiation in the nitrogen-substituted system at 100°C 4.5 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=96/4-82/18(V/V)] to give the title compound (21 mg, yield: 21%) as an oil.

### (38H) 2-{9-[(3R)-1-methylpiperidin-3-yl]-5,6,8,9-tetrahydro-4H,7H-1,2,6a,9-tetraazaphenalen-3-yl}-5-(trifluoromethyl)phenol

Using 3-chloro-9-[(3R)-1-methylpiperidin-3-yl]-5,6,8,9-tetrahydro-4H,7H-1,2,6a,9-tetraazaphenalene (21 mg, 0.068 mmol) of Example 38G, 2-hydroxy-4-trifluoromethylphenylboronic acid (18 mg, 0.089 mmol), potassium carbonate (18 mg, 0.14 mmol) and XPhos Pd G2 (10 mg, 0.013 mmol), the title compound (10 mg, yield: 34%) was obtained as a solid by a method similar to that in Example 1E.

### (Example 39)

### 2-{7-[(1s,3s)-3-hydroxy-3-methylcyclobutyl]-4-methyl-6,7-dihydro-5H-pyrrolo[2,3-c]pyridazin-3-yl}-5-(trifluoromethyl)phenol (also referred to as 2-[7-(cis-3-hydroxy-3-methylcyclobutyl)-4-methyl-6,7-dihydro-5H-pyrrolo[2,3-c]pyridazin-3-yl]-5-(trifluoromethyl)phenol)

### (39A) 3,4,6-trichloro-5-methylpyridazine

To 4-bromo-5-methyl-1,2,3,6-tetrahydropyridazine-3,6-dione (CAS registration number: 73295-61-3, 984 mg, 4.8 mmol) was added phosphoryl chloride (7.638 g, 50 mmol), and the mixture was stirred at 100°C for 1.5 hr. The reaction solution was concentrated under reduced pressure, to the obtained residue was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-82/18(V/V)] to give a crude product of the title compound as a solid.

### (39B) 3,6-dichloro-4-ethenyl-5-methylpyridazine

Using 3,4,6-trichloro-5-methylpyridazine (889 mg, 4.5 mmol) of Example 39A, potassium vinyltrifluoroborate (723 mg, 5.4 mmol), potassium carbonate (1.24 g, 9.0 mmol) and Pd(dppf)Cl₂ (367 mg, 0.45 mmol), the title compound (218 mg, yield: 38% in two steps) was obtained as a solid by a method similar to that in Example 1C.

### (39C) (1s,3s)-3-(3-chloro-4-methyl-5,6-dihydro-7H-pyrrolo[2,3-c]pyridazin-7-yl)-1-methylcyclobutanol

Using 3,6-dichloro-4-ethenyl-5-methylpyridazine (218 mg, 1.2 mmol) of Example 39B, DIPEA (0.301 mL, 1.7 mmol) and (1s,3s)-3-amino-1-methyl-cyclobutanol hydrochloride (182 mg, 1.3 mmol), the title compound (124 mg, yield 42%) was obtained as a solid by a method similar to that in Example 1D.

### (39D) 2-{7-[(1s,3s)-3-hydroxy-3-methylcyclobutyl]-4-methyl-6,7-dihydro-5H-pyrrolo[2,3-c]pyridazin-3-yl}-5-(trifluoromethyl)phenol

Using (1s,3s)-3-(3-chloro-4-methyl-5,6-dihydro-7H-pyrrolo[2,3-c]pyridazin-7-yl)-1-methylcyclobutanol (69 mg, 0.27 mmol) of Example 39C, 2-hydroxy-4-trifluoromethylphenylboronic acid (67 mg, 0.33 mmol), potassium carbonate (75 mg, 0.54 mmol) and XPhos Pd G2 (42 mg, 0.054 mmol), the title compound (50 mg, yield: 48%) was obtained as a solid by a method similar to that in Example 1E.

### (Example 40)

### 2-{7-[(1s,3s)-3-hydroxy-3-methylcyclobutyl]-6,7-dihydro-5H-pyrrolo[2,3-c]pyridazin-3-yl}-3-methyl-5-(trifluoromethyl)phenol monohydrochloride (also referred to as 2-[7-(cis-3-hydroxy-3-methylcyclobutyl)-6,7-dihydro-5H-pyrrolo[2,3-c]pyridazin-3-yl]-3-methyl-5-(trifluoromethyl)phenol monohydrochloride)

### (40A) (1s,3s)-3-(3-[2-(methoxymethoxy)-6-methyl-4-(trifluoromethyl)phenyl]-5,6-dihydro-7H-pyrrolo[2,3-c]pyridazin-7-yl}-1-methylcyclobutanol

Using (1s,3s)-3-(3-chloro-5,6-dihydro-7H-pyrrolo[2,3-c]pyridazin-7-yl)-1-methylcyclobutanol (77 mg, 0.32 mmol) of Example 3A, 2-[2-(methoxymethoxy)-6-methyl-4-(trifluoromethyl)phenyl]-4,4,5,5-tetramethyl-1,3,2-dioxaborolane (144 mg, 0.42 mmol) of Example 1B, potassium carbonate (88 mg, 0.64 mmol) and XPhos Pd G2 (25 mg, 0.032 mmol), the title compound (105 mg, yield: 77%) was obtained as an amorphous form by a method similar to that in Example 1E.

### (40B) 2-{7-[(1s,3s)-3-hydroxy-3-methylcyclobutyl]-6,7-dihydro-5H-pyrrolo[2,3-c]pyridazin-3-yl}-3-methyl-5-(trifluoromethyl)phenol monohydrochloride

Using (1s,3s)-3-{3-[2-(methoxymethoxy)-6-methyl-4-(trifluoromethyl)phenyl]-5,6-dihydro-7H-pyrrolo[2,3-c]pyridazin-7-yl}-1-methylcyclobutanol (105 mg, 0.25 mmol) of Example 40A, and TFA (1.2 mL), the title compound (40 mg, yield: 39%) was obtained as a solid by a method similar to that in Example 6B and treatment with 4 M hydrogen chloride-1,4-dioxane.

### (Example 41)

### 5-{7-[(1s,3s)-3-hydroxy-3-methylcyclobutyl]-4-methyl-7H-pyrrolo[2,3-c]pyridazin-3-yl}-2,3-dihydro-1H-inden-4-ol (also referred to as 5-[7-(cis-3-hydroxy-3-methylcyclobutyl)-4-methyl-7H-pyrrolo[2,3-c]pyridazin-3-yl]-2,3-dihydro-1H-inden-4-ol)

### (41A) 4-(methoxymethoxy)-2,3-dihydro-1H-indene

Using 4-indanol (3.00 g, 22 mmol), 55% sodium hydride (1.17 g, 27 mmol) and chloromethyl methyl ether (2.52 mL, 34 mmol), the title compound (3.97 g, yield: 99%) was obtained as an oil by a method similar to that in Example 1A.

### (41B) 2-[4-(methoxymethoxy)-2,3-dihydro-1H-inden-5-yl]-4,4,5,5-tetramethyl-1,3,2-dioxaborolane

4-(Methoxymethoxy)-2,3-dihydro-1H-indene (3.97 g, 22 mmol) of Example 41A was dissolved in DEE (150 mL), n-butyllithium (1.6 M n-hexane solution) (28.4 mL, 45 mmol) was added in the nitrogen-substituted system at 0°C, and the mixture was stirred at room temperature for 2 hr. To the reaction solution was added iPrOBPin (13.5 mL, 67 mmol) at 0°C, and the mixture was stirred at room temperature for 2 hr. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-65/35(V/V)] to give the title compound (2.12 g, yield: 31%) as an oil.

### (41C) (1s,3s)-3-(3-chloro-4-methyl-7H-pyrrolo[2,3-c]pyridazin-7-yl)-1-methylcyclobutanol

Using (1s,3s)-3-(3-chloro-4-methyl-5,6-dihydro-7H-pyrrolo[2,3-c]pyridazin-7-yl)-1-methylcyclobutanol (79 mg, 0.31 mmol) of Example 39C, and manganese dioxide (271 mg, 3.1 mmol), the title compound (42 mg, yield: 54%) was obtained as a solid by a method similar to that in Example 4C.

### (41D) (1s,3s)-3-{3-[4-(methoxymethoxy)-2,3-dihydro-1H-inden-5-yl]-4-methyl-7H-pyrrolo[2,3-c]pyridazin-7-yl}-1-methylcyclobutanol

Using (1s,3s)-3-(3-chloro-4-methyl-7H-pyrrolo[2,3-c]pyridazin-7-yl)-1-methylcyclobutanol (20 mg, 0.079 mmol) of Example 41C, 2-[4-(methoxymethoxy)-2,3-dihydro-1H-inden-5-yl]-4,4,5,5-tetramethyl-1,3,2-dioxaborolane (29 mg, 0.095 mmol) of Example 41B, potassium carbonate (21 mg, 0.16 mmol) and XPhos Pd G2 (6.3 mg, 0.0079 mmol), the title compound (19 mg, yield: 61%) was obtained as an amorphous form by a method similar to that in Example 1E.

### (41E) 5-{7-[(1s,3s)-3-hydroxy-3-methylcyclobutyl]-4-methyl-7H-pyrrolo[2,3-c]pyridazin-3-yl}-2,3-dihydro-1H-inden-4-ol

Using (1s,3s)-3-{3-[4-(methoxymethoxy)-2,3-dihydro-1H-inden-5-yl]-4-methyl-7H-pyrrolo[2,3-c]pyridazin-7-yl}-1-methylcyclobutanol (19 mg, 0.048 mmol) of Example 41D, and 4 M hydrogen chloride-1,4-dioxane (0.48 mL), the title compound (14 mg, yield: 83%) was obtained as a solid by a method similar to that in Example 3D.

### (Example 42)

### 2-{7-[(1S*,2S*,3R*)-3-hydroxy-2,3-dimethylcyclobutyl]-7H-pyrrolo[2,3-c]pyridazin-3-yl}-3-methyl-5-(trifluoromethyl)phenol

### (42A) tert-butyl [(1S*,2S*)-2-methyl-3-oxocyclobutyl]carbamate

tert-Butyl (1Z)-prop-1-en-1-ylcarbamate (J. Org. Chem. 2012, 77, 5894-5906.) (1.61 g, 10 mmol) was dissolved in DEE (34 mL), and zinc-copper couple (13.2 g) was added. Trichloroacetyl chloride (2.3 mL, 21 mmol) was added under ultrasonic irradiation at 0°C, and the mixture was stirred at room temperature for 15 min. To the reaction solution was added a suspension of ammonium chloride (8.2 g, 154 mmol) in methanol (41 mL) at 0°C, and the mixture was stirred at 60°C for 20 min. The reaction solution was filtered through Celite, the filtrate was concentrated under reduced pressure, and the obtained residue was purified by silica gel column chromatography [eluent: n-hexane/ethyl acetate =72/28-60/40(V/V)] to give the title compound (1.4 g, yield: 69%) as a racemate (a solid) .

### (42B) tert-butyl [(1S*,25*,3R*)-3-hydroxy-2,3-dimethylcyclobutyl]carbamate

Cerium chloride (3.13 g, 13 mmol) was suspended in THF (20 mL), 1.2 M methyllithium (11 mL, 13 mmol) was added at - 78°C, and the mixture was stirred at -78°C for 40 min. A solution of tert-butyl [(1S*,2S*)-2-methyl-3-oxocyclobutyl]carbamate (1.15 g, 5.8 mmol) of Example 42A in THF (11 mL) was added, and the mixture was stirred at -78°C for 1 hr. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =60/40-45/55(V/V)] to give the title compound (275 mg, yield: 22%) as a racemate (an amorphous form).

### (42C) (1R*,2S*,3S*)-3-amino-1,2-dimethylcyclobutanol monohydrochloride

Using tert-butyl [(1S*,2S*,3R*)-3-hydroxy-2,3-dimethylcyclobutyl]carbamate (275 mg, 1.3 mmol) of Example 42B, and 4 M hydrogen chloride-1,4-dioxane (6.0 mL), a crude product (193 mg, yield: quantitative) of the title compound was obtained as a racemate (a solid) by a method similar to that in Example 2B.

### (42D) (1R*, 2S*, 3S*) -3- (3-chloro-5, 6-dihydro-7H-pyrrolo [2, 3-c]pyridazin-7-yl)-1,2-dimethylcyclobutanol

Using 3,6-dichloro-4-ethenylpyridazine (80 mg, 0.46 mmol) of Example 1C, (1R*,2S*,3S*)-3-amino-1,2-dimethylcyclobutanol monohydrochloride (83 mg, 0.57 mmol) of Example 42C, and DIPEA (0.199 mL, 1.1 mmol), the title compound (53 mg, yield 45%) was obtained as a racemate (a solid) by a method similar to that in Example 1D.

### (42E) (1R*,2S*,3S*)-3-{3-[2-(methoxymethoxy) -6-methyl-4-(trifluoromethyl)phenyl]-5,6-dihydro-7H-pyrrolo[2,3-c]pyridazin-7-yl}-1,2-dimethylcyclobutanol

Using (1R*,2S*,3S*)-3-(3-chloro-5,6-dihydro-7H-pyrrolo[2,3-c]pyridazin-7-yl)-1,2-dimethylcyclobutanol (52 mg, 0.20 mmol) of Example 42D, 2-[2-(methoxymethoxy)-6-methyl-4-(trifluoromethyl)phenyl]-4,4,5,5-tetramethyl-1,3,2-dioxaborolane (92 mg, 0.27 mmol) of Example 1B, potassium carbonate (57 mg, 0.41 mmol) and XPhos Pd G2 (16 mg, 0.020 mmol), the title compound (62 mg, yield: 69%) was obtained as a racemate (a solid) by a method similar to that in Example 1E.

### (42F) (1R*,2S*,3S*)-3-{3-[2- (methoxymethoxy) -6-methyl-4-(trifluoromethyl)phenyl]-7H-pyrrolo[2,3-c]pyridazin-7-yl}-1,2-dimethylcyclobutanol

Using (1R*,2S*,3S*)-3-{3-[2-(methoxymethoxy)-6-methyl-4-(trifluoromethyl)phenyl]-5,6-dihydro-7H-pyrrolo[2,3-c]pyridazin-7-yl}-1,2-dimethylcyclobutanol (62 mg, 0.14 mmol) of Example 42E, and manganese dioxide (123 mg, 1.4 mmol), the title compound (10 mg, yield: 16%) was obtained as a racemate (a solid) by a method similar to that in Example 4C.

### (42G) 2-{7-[(1S*,2S*,3R*)-3-hydroxy-2,3-dimethylcyclobutyl]-7H-pyrrolo[2,3-c]pyridazin-3-yl}-3-methyl-5-(trifluoromethyl)phenol

Using (1R*,2S*,3S*)-3-{3-[2-(methoxymethoxy)-6-methyl-4-(trifluoromethyl)phenyl]-7H-pyrrolo[2,3-c]pyridazin-7-yl}-1,2-dimethylcyclobutanol (19 mg, 0.043 mmol) of Example 42F, and TFA (0.3 mL), the title compound (9.6 mg, yield: 56%) was obtained as a racemate (a solid) by a method similar to that in Example 3D.

### (Example 43)

### 5-{7-[(1s,3s)-3-hydroxy-3-methylcyclobutyl]-5-methyl-7H-pyrrolo[2,3-c]pyridazin-3-yl}-6-methyl-2,3-dihydro-1H-inden-4-ol (also referred to as 5-[7-(cis-3-hydroxy-3-methylcyclobutyl)-5-methyl-7H-pyrrolo[2,3-c]pyridazin-3-yl]-6-methyl-2,3-dihydro-1H-inden-4-ol)

### (43A) 6-methyl-3-oxo-2,3-dihydro-1H-inden-4-yl dimethylcarbamate

7-Hydroxy-5-methyl-1-indanone (2.00 g, 12 mmol) was dissolved in acetonitrile (100 mL), then potassium carbonate (1.87 g, 14 mmol) and dimethylcarbamoyl chloride (1.25 mL, 14 mmol) were added, and the mixture was stirred at 80°C for 4 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-50/50(V/V)] to give the title compound (2.66 g, yield: 93%) as a solid.

### (43B) 3-hydroxy-6-methyl-2,3-dihydro-1H-inden-4-yl dimethylcarbamate

6-Methyl-3-oxo-2,3-dihydro-1H-inden-4-yl dimethylcarbamate (2.66 g, 11 mmol) of Example 43A was dissolved in methanol (100 mL), sodium borohydride (570 mg, 15 mmol) was added, and the mixture was stirred at 0°C for 30 min. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-40/60(V/V)] to give the title compound (2.88 g, yield: quantitative) as an oil.

### (43C) 6-methyl-1H-inden-4-yl dimethylcarbamate

3-Hydroxy-6-methyl-2,3-dihydro-1H-inden-4-yl dimethylcarbamate (130 mg, 0.55 mmol) of Example 43B was dissolved in DCM (5.0 mL), then pyridine (0.089 mL, 1.1 mmol) and phosphorus tribromide (0.058 mL, 0.61 mmol) were added, and the mixture was stirred at 0°C for 10 min. To the reaction solution was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-70/30(V/V)] to give the title compound (35 mg, yield: 29%) as an oil.

### (43D) 6-methyl-2,3-dihydro-1H-inden-4-yl dimethylcarbamate

6-Methyl-1H-inden-4-yl dimethylcarbamate (716 mg, 3.3 mmol) of Example 43C was dissolved in ethanol (50 mL), 10% palladium on carbon (701 mg) was added, and the mixture was stirred in the hydrogen-substituted system at room temperature for 1 hr. The reaction solution was filtered through Celite, the filtrate was concentrated under reduced pressure, and the obtained residue was purified by silica gel column chromatography [eluent: n-hexane/ethyl acetate =100/0-65/35(V/V)] to give the title compound (593 mg, yield: 82%) as an oil.

### (43E) 5-bromo-6-methyl-2,3-dihydro-1H-inden-4-yl dimethylcarbamate

6-Methyl-2,3-dihydro-1H-inden-4-yl dimethylcarbamate (138 mg, 0.63 mmol) of Example 43D was dissolved in DCE (6.0 mL), then palladium(II) acetate (43 mg, 0.19 mmol), trifluoromethanesulfonic acid (0.045 mL, 0.51 mmol) and N-bromosuccinimide (123 mg, 0.69 mmol) were added, and the mixture was stirred at room temperature for 15 hr. To the reaction solution was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-75/25(V/V)] to give the title compound (97 mg, yield: 52%) as an oil.

### (43F) 5-bromo-6-methyl-2,3-dihydro-1H-inden-4-ol

5-Bromo-6-methyl-2,3-dihydro-1H-inden-4-yl dimethylcarbamate (97 mg, 0.33 mmol) of Example 43E was dissolved in 1-butanol (4.0 mL), sodium hydroxide (65 mg, 1.6 mmol) was added, and the mixture was stirred at 110°C for 1 hr. To the reaction solution was added 1 M hydrochloric acid, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-85/15(V/V)] to give the title compound (51 mg, yield: 69%) as an oil.

### (43G) 5-bromo-4-(methoxymethoxy)-6-methyl-2,3-dihydro-1H-indene

Using 5-bromo-6-methyl-2,3-dihydro-1H-inden-4-ol (51 mg, 0.23 mmol) of Example 43F, 55% sodium hydride (12 mg, 0.28 mmol) and chloromethyl methyl ether (0.025 mL, 0.33 mmol), the title compound (51 mg, yield: 84%) was obtained as an oil by a method similar to that in Example 1A.

### (43H) 2-[4-(methoxymethoxy)-6-methyl-2,3-dihydro-1H-inden-5-yl]-4,4,5,5-tetramethyl-1,3,2-dioxaborolane

Using 5-bromo-4-(methoxymethoxy)-6-methyl-2,3-dihydro-1H-indene (51 mg, 0.19 mmol) of Example 43G, n-butyllithium (1.6 M n-hexane solution) (0.18 mL, 0.28 mmol) and iPrOBPin (0.076 mL, 0.38 mmol), the title compound (47 mg, yield: 79%) was obtained as an oil by a method similar to that in Example 9C.

### (43I) (1s,3s)-3-{3-[4-(methoxymethoxy)-6-methyl-2,3-dihydro-1H-inden-5-yl]-5-methyl-7H-pyrrolo[2,3-c]pyridazin-7-yl}-1-methylcyclobutanol

Using 2-[4-(methoxymethoxy)-6-methyl-2,3-dihydro-1H-inden-5-yl]-4,4,5,5-tetramethyl-1,3,2-dioxaborolane (47 mg, 0.15 mmol) of Example 43H, (1s,3s)-3-(3-chloro-5-methyl-7H-pyrrolo[2,3-c]pyridazin-7-yl)-1-methylcyclobutanol (31 mg, 0.12 mmol) of Example 4C, potassium carbonate (41 mg, 0.30 mmol) and XPhos Pd G2 (23 mg, 0.029 mmol), the title compound (12 mg, yield: 24%) was obtained as an oil by a method similar to that in Example 9D.

### (43J) 5-{7-[(1s,3s)-3-hydroxy-3-methylcyclobutyl]-5-methyl-7H-pyrrolo[2,3-c]pyridazin-3-yl}-6-methyl-2,3-dihydro-1H-inden-4-ol

Using (1s,3s)-3-{3-[4-(methoxymethoxy)-6-methyl-2,3-dihydro-1H-inden-5-yl]-5-methyl-7H-pyrrolo[2,3-c]pyridazin-7-yl}-1-methylcyclobutanol (12 mg, 0.029 mmol) of Example 43I, and 4 M hydrogen chloride-1,4-dioxane (3.0 mL), the title compound (8.0 mg, yield: 75%) was obtained as a solid by a method similar to that in Example 3D.

### (Example 44)

### 2-{5-ethyl-7-[(1s,3s)-3-hydroxy-3-methylcyclobutyl]-7H-pyrrolo[2,3-c]pyridazin-3-yl}-3-methyl-5-(trifluoromethyl)phenol (also referred to as 2-[5-ethyl-7-(cis-3-hydroxy-3-methylcyclobutyl)-7H-pyrrolo[2,3-c]pyridazin-3-yl]-3-methyl-5-(trifluoromethyl)phenol)

### (44A) (1s,3s)-3-{5-bromo-3-[2-(methoxymethoxy)-6-methyl-4-(trifluoromethyl)phenyl]-7H-pyrrolo[2,3-c]pyridazin-7-yl}-1-methylcyclobutanol

(1s,3s)-3-{3-[2-(Methoxymethoxy)-6-methyl-4-(trifluoromethyl)phenyl]-7H-pyrrolo[2,3-c]pyridazin-7-yl}-1-methylcyclobutanol (21 mg, 0.050 mmol) of Example 3C was dissolved in DCE (1 mL), N-bromosuccinimide (10 mg, 0.056 mmol) was added, and the mixture was stirred at room temperature for 20 min. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =40/60-20/80(V/V)] to give the title compound (20 mg, yield: 80%) as a solid.

### (44B) (1s,3s)-3-(5-ethenyl-3-[2-(methoxymethoxy)-6-methyl-4-(trifluoromethyl)phenyl]-7H-pyrrolo[2,3-c]pyridazin-7-yl}-1-methylcyclobutanol

(1s,3s)-3-{5-Bromo-3-[2-(methoxymethoxy)-6-methyl-4-(trifluoromethyl)phenyl]-7H-pyrrolo[2,3-c]pyridazin-7-yl}-1-methylcyclobutanol (20 mg, 0.040 mmol) of Example 44A, 4,4,5,5-tetramethyl-2-vinyl-1,3,2-dioxaborolane (0.008 mL, 0.052 mmol) and potassium carbonate (11 mg, 0.080 mmol) were dissolved in a mixed solvent of 1,4-dioxane (0.8 mL) and water (0.2 mL), Pd(dppf)Cl₂ (5 mg, 0.0080 mmol) was added, and the mixture was stirred in the nitrogen-substituted system at 110°C for 40 min. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =40/60-20/80(V/V)] to give the title compound (14 mg, yield: 78%) as an oil.

### (44C) (1s,3s)-3-{5-ethyl-3-[2-(methoxymethoxy)-6-methyl-4-(trifluoromethyl)phenyl]-7H-pyrrolo[2,3-c]pyridazin-7-yl}-1-methylcyclobutanol

(1s,3s)-3-{5-Ethenyl-3-[2-(methoxymethoxy)-6-methyl-4-(trifluoromethyl)phenyl]-7H-pyrrolo[2,3-c]pyridazin-7-yl}-1-methylcyclobutanol (14 mg, 0.031 mmol) of Example 44B was dissolved in methanol (0.6 mL), 10% palladium on carbon (6 mg) was added, and the mixture was stirred in the hydrogen-substituted system at room temperature for 15 min. The reaction solution was filtered through Celite, and the filtrate was concentrated under reduced pressure to give a crude product of the title compound.

### (44D) 2-{5-ethyl-7-[(1s,3s)-3-hydroxy-3-methylcyclobutyl]-7H-pyrrolo[2,3-c]pyridazin-3-yl}-3-methyl-5-(trifluoromethyl)phenol

Using (1s,3s)-3-{5-ethyl-3-[2-(methoxymethoxy)-6-methyl-4-(trifluoromethyl)phenyl]-7H-pyrrolo[2,3-c]pyridazin-7-yl}-1-methylcyclobutanol (14 mg, 0.031 mmol) of Example 44C, and TFA (0.3 mL), the title compound (5 mg, yield: 40% in two steps) was obtained as a solid by a method similar to that in Example 6B.

### (Example 45)

### 7-[(1s,3s)-3-hydroxy-3-methylcyclobutyl]-3-[2-hydroxy-6-methyl-4-(trifluoromethyl)phenyl]-7H-pyrrolo[2,3-c]pyridazine-5-carbonitrile (also referred to as 7-(cis-3-hydroxy-3-methylcyclobutyl)-3-[2-hydroxy-6-methyl-4-(trifluoromethyl)phenyl]-7H-pyrrolo[2,3-c]pyridazine-5-carbonitrile)

### (45A) 7-[(1s,3s)-3-hydroxy-3-methylcyclobutyl]-3-[2-(methoxymethoxy)-6-methyl-4-(trifluoromethyl)phenyl]-7H-pyrrolo[2,3-c]pyridazine-5-carbonitrile

(1s,3s)-3-{5-Bromo-3-[2-(methoxymethoxy)-6-methyl-4-(trifluoromethyl)phenyl]-7H-pyrrolo[2,3-c]pyridazin-7-yl}-1-methylcyclobutanol (60 mg, 0.12 mmol) of Example 44A, zinc cyanide (21 mg, 0.18 mmol) and Pd(PPh₃)₄ (27 mg, 0.024 mmol) were dissolved in DMF (1 mL), and the mixture was stirred in the nitrogen-substituted system at 100°C for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =30/70-0/100(V/V)] to give the title compound (41 mg, yield: 77%) as a solid.

### (45B) 7-[(1s,3s)-3-hydroxy-3-methylcyclobutyl]-3-[2-hydroxy-6-methyl-4-(trifluoromethyl)phenyl]-7H-pyrrolo[2,3-c]pyridazine-5-carbonitrile

Using 7-[(1s,3s)-3-hydroxy-3-methylcyclobutyl]-3-[2-(methoxymethoxy)-6-methyl-4-(trifluoromethyl)phenyl]-7H-pyrrolo[2,3-c]pyridazine-5-carbonitrile (63 mg, 0.14 mmol) of Example 45A, and TFA (0.7 mL), the title compound (42 mg, yield: 74%) was obtained as a solid by a method similar to that in Example 6B.

### (Example 46)

### 2-[5-cyclopropyl-7-[(1s,3s)-3-hydroxy-3-methylcyclobutyl]-7H-pyrrolo[2,3-c]pyridazin-3-yl]-3-methyl-5-(trifluoromethyl)phenol (also referred to as 2-[5-cyclopropyl-7-(cis-3-hydroxy-3-methylcyclobutyl)-7H-pyrrolo[2,3-c]pyridazin-3-yl]-3-methyl-5-(trifluoromethyl)phenol)

### (46A) (1s,3s)-3-{5-cyclopropyl-3-[2-(methoxymethoxy)-6-methyl-4-(trifluoromethyl)phenyl]-7H-pyrrolo[2,3-c]pyridazin-7-yl}-1-methylcyclobutanol

(1s,3s)-3-{5-Bromo-3-[2-(methoxymethoxy)-6-methyl-4-(trifluoromethyl)phenyl]-7H-pyrrolo[2,3-c]pyridazin-7-yl}-1-methylcyclobutanol (48 mg, 0.096 mmol) of Example 44A was dissolved in a mixed solvent of 1,4-dioxane (2.0 mL) and water (0.5 mL), then potassium cyclopropyltrifluoroborate (17 mg, 0.12 mmol), potassium carbonate (27 mg, 0.19 mmol) and XPhos Pd G2 (15 mg, 0.019 mmol) were added, and the mixture was stirred in the nitrogen-substituted system at 110°C for 3.5 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =40/60-10/90(V/V)] to give the title compound (17 mg, yield: 38%) as an oil.

### (46B) 2-{5-cyclopropyl-7-[(1s,3s)-3-hydroxy-3-methylcyclobutyl]-7H-pyrrolo[2,3-c]pyridazin-3-yl}-3-methyl-5-(trifluoromethyl)phenol

Using (1s,3s)-3-{5-cyclopropyl-3-[2-(methoxymethoxy)-6-methyl-4-(trifluoromethyl)phenyl]-7H-pyrrolo[2,3-c]pyridazin-7-yl}-1-methylcyclobutanol (17 mg, 0.037 mmol) of Example 46A, and TFA (0.4 mL), the title compound (7 mg, yield: 46%) was obtained as a solid by a method similar to that in Example 6B.

### (Example 47)

### 3-chloro-2-{7-[(1s,3s)-3-hydroxy-3-methylcyclobutyl]-5-methyl-7H-pyrrolo[2,3-c]pyridazin-3-yl}-5-(trifluoromethyl)phenol (also referred to as 3-chloro-2-[7-(cis-3-hydroxy-3-methylcyclobutyl)-5-methyl-7H-pyrrolo[2,3-c]pyridazin-3-yl]-5-(trifluoromethyl)phenol)

### (47A) 3-chloro-2-iodo-5-(trifluoromethyl)phenol

Using 3-chloro-5-hydroxybenzotrifluoride (0.506 g, 2.6 mmol), 55% sodium hydride (0.225 g, 5.2 mmol) and iodine (0.653 g, 2.6 mmol), the title compound (0.947 g, yield: quantitative) was obtained as an oil by a method similar to that in Example 9A.

### (47B) 3-chloro-2-iodo-5-(trifluoromethyl)phenyl dimethylcarbamate

3-Chloro-2-iodo-5-(trifluoromethyl)phenol (0.615 g, 1.9 mmol) of Example 47A was dissolved in acetonitrile (10 mL), then potassium carbonate (0.532 g, 3.9 mmol) and N,N-dimethylcarbamoyl chloride (0.264 mL, 2.9 mmol) were added, and the mixture was stirred at 80°C for 13 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =95/5-80/20(V/V)] to give the title compound (0.500 g, yield: 67%) as an oil.

### (47C) 1-chloro-2-iodo-3-(methoxymethoxy)-5-(trifluoromethyl)benzene

3-Chloro-2-iodo-5-(trifluoromethyl)phenyl dimethylcarbamate (0.559 g, 1.42 mmol) of Example 47B was dissolved in 1-butanol (6 mL), sodium hydroxide (0.324 g, 8.1 mmol) was added, and the mixture was stirred at 120°C for 1.5 hr. The reaction solution was allowed to room temperature, 1 M hydrochloric acid was added, and the mixture was subjected to extraction with n-hexane. The organic layer was washed successively with water and saturated brine, and dried over anhydrous sodium sulfate. After filtration, the filtrate was concentrated under reduced pressure, to the obtained residue were added DMF (6 mL), cesium carbonate (0.828 g, 2.5 mmol) and chloromethyl methyl ether (0.143 mL, 1.9 mmol), and the mixture was stirred at room temperature for 14 hr. To the reaction solution was added water, and the mixture was subjected to extraction with n-hexane. The organic layer was washed successively with water and saturated brine, and dried over anhydrous sodium sulfate. After filtration, the filtrate was concentrated under reduced pressure, and the obtained residue was purified by silica gel column chromatography [eluent: n-hexane/ethyl acetate =97/3-90/10(V/V)] to give the title compound (0.430 g, yield: 92%) as an oil.

### (47D) 2-[2-chloro-6-(methoxymethoxy)-4-(trifluoromethyl)phenyl]-4,4,5,5-tetramethyl-1,3,2-dioxaborolane

Using 1-chloro-2-iodo-3-(methoxymethoxy)-5-(trifluoromethyl)benzene (0.430 g, 1.2 mmol) of Example 47C, n-butyllithium (1.6 M n-hexane solution) (0.90 mL, 1.4 mmol) and iPrOBPin (0.48 mL, 2.4 mmol), the title compound (0.279 g, yield: 65%) was obtained as an oil by a method similar to that in Example 9C.

### (47E) (1s,3s)-3-{3-[2-chloro-6-(methoxymethoxy)-4-(trifluoromethyl)phenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (0.063 g, 0.25 mmol) of Example 4C, 2-[2-chloro-6-(methoxymethoxy)-4-(trifluoromethyl)phenyl]-4,4,5,5-tetramethyl-1,3,2-dioxaborolane (0.136 g, 0.37 mmol) of Example 47D, potassium carbonate (0.070 g, 0.51 mmol) and XPhos Pd G2 (0.020 g, 0.025 mmol), the title compound (0.011 g, yield: 10%) was obtained as an amorphous form by a method similar to that in Example 9D.

### (47F) 3-chloro-2-{7-[(1s,3s)-3-hydroxy-3-methylcyclobutyl]-5-methyl-7H-pyrrolo[2,3-c]pyridazin-3-yl}-5-(trifluoromethyl)phenol

Using (1s,3s)-3-{3-[2-chloro-6-(methoxymethoxy)-4-(trifluoromethyl)phenyl]-5-methyl-7H-pyrrolo[2,3-c]pyridazin-7-yl}-1-methylcyclobutanol (0.011 g, 0.024 mmol) of Example 47E, and 4 M hydrogen chloride-1,4-dioxane (0.24 mL), the title compound (6.7 mg, yield: 67%) was obtained as a solid by a method similar to that in Example 3D.

### (Example 48)

### 2-fluoro-6-{7-[(1s,3s)-3-hydroxy-3-methylcyclobutyl]-5-methyl-7H-pyrrolo[2,3-c]pyridazin-3-yl}-5-methyl-3-(trifluoromethyl)phenol (also referred to as 2-fluoro-6-[7-(cis-3-hydroxy-3-methylcyclobutyl)-5-methyl-7H-pyrrolo[2,3-c]pyridazin-3-yl}-5-methyl-3-(trifluoromethyl)phenol)

### (48A) 3-methyl-5-(trifluoromethyl)phenol

3-Bromo-5-(trifluoromethyl)phenol (5.04 g, 21 mmol) was dissolved in a mixed solvent of 1,4-dioxane (40 mL) and water (10 mL), then trimethylboroxin (50% THF solution) (11 mL, 39 mmol), cesium carbonate (17.8 g, 52 mmol) and Pd(dppf)Cl₂ (0.41 g, 0.49 mmol) were added, and the mixture was stirred in the nitrogen-substituted system at 100°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-80/20(V/V)] to give the title compound (3.99 g, yield: 91%) as an oil.

### (48B) 1-(methoxymethoxy)-3-methyl-5-(trifluoromethyl)benzene

Using 3-methyl-5-(trifluoromethyl)phenol (0.508 g, 2.4 mmol) of Example 48A, DIPEA (1.0 mL, 5.7 mmol) and chloromethyl methyl ether (0.33 mL, 4.4 mmol), the title compound (0.441 g, yield: 85%) was obtained as an oil by a method similar to that in Example 1A.

### (48C) 2-fluoro-1-(methoxymethoxy)-5-methyl-3-(trifluoromethyl)benzene

1-(Methoxymethoxy)-3-methyl-5-(trifluoromethyl)benzene (0.220 g, 1.0 mmol) of Example 48B, N,N,N',N'-tetramethylethylene diamine (0.179 mL, 1.2 mmol) and diisopropylamine (0.014 mL, 0.10 mmol) were dissolved in THF (3 mL), n-butyllithium (1.6 M n-hexane solution) (0.76 mL, 1.2 mmol) was added in the nitrogen-substituted system at -78°C, and the mixture was stirred at 0°C for 30 min. To the reaction solution was added N-fluorobenzenesulfonimide (0.408 g, 1.29 mmol) at -78°C, and the mixture was stirred at 0°C for 30 min. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/DEE=97/3-90/10(V/V)] to give the title compound (0.141 g, yield: 59%) as an oil.

### (48D) 2-[3-fluoro-2-(methoxymethoxy)-6-methyl-4-(trifluoromethyl)phenyl]-4,4,5,5-tetramethyl-1,3,2-dioxaborolane

2-Fluoro-1-(methoxymethoxy)-5-methyl-3-(trifluoromethyl)benzene (0.110 g, 0.46 mmol) of Example 48C, and N,N,N',N'-tetramethylethylene diamine (0.083 mL, 0.56 mmol) were dissolved in THF (1.5 mL), n-butyllithium (1.6 M n-hexane solution) (0.35 mL, 0.56 mmol) was added in the nitrogen-substituted system at -78°C, and the mixture was stirred at 0°C for 30 min. To the reaction solution was added iPrOBPin (0.20 mL, 0.99 mmol) at -78°C, and the mixture was stirred at 0°C for 2.5 hr. To the reaction solution was added saturated ammonium chloride aqueous solution, the mixture was subjected to extraction with ethyl acetate, and the organic layer was dried over anhydrous sodium sulfate. After filtration, the filtrate was concentrated under reduced pressure, and the obtained residue was purified by silica gel column chromatography [eluent: n-hexane/ethyl acetate =97/3-76/24(V/V)] to give the title compound (0.144 g, yield: 86%) as an oil.

### (48E) (1s,3s)-3-{3-[3-fluoro-2-(methoxymethoxy)-6-methyl-4-(trifluoromethyl)phenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (0.084 g, 0.33 mmol) of Example 4C, 2-[3-fluoro-2-(methoxymethoxy)-6-methyl-4-(trifluoromethyl)phenyl]-4,4,5,5-tetramethyl-1,3,2-dioxaborolane (0.144 g, 0.40 mmol) of Example 48D, potassium carbonate (0.091 g, 0.66 mmol) and XPhos Pd G2 (0.026 g, 0.033 mmol), the title compound (0.13 g, yield: 86%) was obtained as an amorphous form by a method similar to that in Example 1E.

### (48F) 2-fluoro-6-{7-[(1s,3s)-3-hydroxy-3-methylcyclobutyl]-5-methyl-7H-pyrrolo[2,3-c]pyridazin-3-yl}-5-methyl-3-(trifluoromethyl)phenol

Using (1s,3s)-3-{3-[3-fluoro-2-(methoxymethoxy)-6-methyl-4-(trifluoromethyl)phenyl]-5-methyl-7H-pyrrolo[2,3-c]pyridazin-7-yl}-1-methylcyclobutanol (0.127 g, 0.28 mmol) of Example 48E, and 4 M hydrogen chloride-1,4-dioxane (3 mL), the title compound (0.097 g, yield: 85%) was obtained as a solid by a method similar to that in Example 3D.

### (Example 49)

### (3S,4R)-3-{3-[2-hydroxy-6-methyl-4-(trifluoromethyl)phenyl]-5,6-dihydro-7H-pyrrolo[2,3-c]pyridazin-7-yl}-1-methylpiperidin-4-ol

### (49A) tert-butyl (3S,4R)-3-(3-chloro-5,6-dihydro-7H-pyrrolo[2,3-c]pyridazin-7-yl)-4-hydroxypiperidine-1-carboxylate

Using 3,6-dichloro-4-ethenylpyridazine (208 mg, 1.19 mmol) of Example 1C, DIPEA (0.6 mL, 3.4 mmol) and tert-butyl (3S, 4R)-3-amino-4-hydroxypiperidine-1-carboxylate (297 mg, 1.37 mmol), the title compound (102 mg, yield: 29%) was obtained as an amorphous form by a method similar to that in Example 1D.

### (49B) tert-butyl (3S,4R)-4-hydroxy-3-{3-[2-(methoxymethoxy)-6-methyl-4-(trifluoromethyl)phenyl]-5,6-dihydro-7H-pyrrolo[2,3-c]pyridazin-7-yl}piperidine-1-carboxylate

Using tert-butyl (3S,4R)-3-(3-chloro-5,6-dihydro-7H-pyrrolo[2,3-c]pyridazin-7-yl)-4-hydroxypiperidine-1-carboxylate (100.0 mg, 0.28 mmol) of Example 49A, 2-[2-(methoxymethoxy)-6-methyl-4-(trifluoromethyl)phenyl]-4,4,5,5-tetramethyl-1,3,2-dioxaborolane (180.7 mg, 0.53 mmol) of Example 1B, potassium carbonate (156.7 mg, 1.1 mmol) and XPhos Pd G2 (48.6 mg, 0.061 mmol), the title compound (53 mg, yield: 35%) was obtained as an amorphous form by a method similar to that in Example 9D.

### (49C) (3S,4R)-3-{3-[2-hydroxy-6-methyl-4-(trifluoromethyl)phenyl]-5,6-dihydro-7H-pyrrolo[2,3-c]pyridazin-7-yl}piperidin-4-olmonohydrochloride

Using tert-butyl (3S,4R)-4-hydroxy-3-{3-[2-(methoxymethoxy)-6-methyl-4-(trifluoromethyl)phenyl]-5,6-dihydro-7H-pyrrolo[2,3-c]pyridazin-7-yl}piperidine-1-carboxylate (52.5 mg, 0.098 mmol) of Example 49B, and 4 M hydrogen chloride-1,4-dioxane (1.0 mL), a crude product (42.0 mg, yield: quantitative) of the title compound was obtained as a solid by a method similar to that in Example 1F.

### (49D) (3S,4R)-3-{3-[2-hydroxy-6-methyl-4-(trifluoromethyl)phenyl]-5,6-dihydro-7H-pyrrolo[2,3-c]pyridazin-7-yl}-1-methylpiperidin-4-ol

Using (3S,4R)-3-{3-[2-hydroxy-6-methyl-4-(trifluoromethyl)phenyl]-5,6-dihydro-7H-pyrrolo[2,3-c]pyridazin-7-yl}piperidin-4-olmonohydrochloride (41.5 mg, 0.096 mmol) of Example 49C, DIPEA (0.034 mL, 0.19 mmol), 37% formaldehyde (0.011 mL, 0.14 mmol), acetic acid (0.0115 mL, 0.19 mmol) and NaBH(OAc)₃ (89.5 mg, 0.42 mmol), the title compound (17.3 mg, yield: 44%) was obtained as a solid by a method similar to that in Example 1G.

### (Example 50)

### 4-fluoro-2-{7-[(1s,3s)-3-hydroxy-3-methylcyclobutyl]-5-methyl-7H-pyrrolo[2,3-c]pyridazin-3-yl}-3-methyl-5-(trifluoromethyl)phenol (also referred to as 4-fluoro-2-[7-(cis-3-hydroxy-3-methylcyclobutyl)-5-methyl-7H-pyrrolo[2,3-c]pyridazin-3-yl]-3-methyl-5-(trifluoromethyl)phenol)

### (50A) 4-fluoro-2-iodo-3-methyl-5-(trifluoromethyl)phenol

Using 4-fluoro-3-methyl-5-(trifluoromethyl)phenol (1.09 g, 5.6 mmol), 55% sodium hydride (0.499 g, 11 mmol) and iodine (1.43 g, 5.6 mmol), the title compound (1.48 g, yield: 82%) was obtained as an oil by a method similar to that in Example 9A.

### (50B) 2-fluoro-4-iodo-5-(methoxymethoxy)-3-methyl-1-(trifluoromethyl)benzene

Using 4-fluoro-2-iodo-3-methyl-5-(trifluoromethyl)phenol (1.48 g, 4.6 mmol) of Example 50A, DIPEA (1.6 mL, 9.2 mmol) and chloromethyl methyl ether (0.52 mL, 6.9 mmol), the title compound (0.761 g, yield: 45%) was obtained as an oil by a method similar to that in Example 1A.

### (50C) 2-[3-fluoro-6-(methoxymethoxy)-2-methyl-4-(trifluoromethyl)phenyl]-4,4,5,5-tetramethyl-1,3,2-dioxaborolane

Using 2-fluoro-4-iodo-5-(methoxymethoxy)-3-methyl-1-(trifluoromethyl)benzene (0.761 g, 2.1 mmol) of Example 50B, n-butyllithium (1.6 M n-hexane solution) (1.99 mL, 3.2 mmol) and iPrOBPin (0.85 mL, 4.2 mmol), the title compound (0.595 g, yield: 78%) was obtained as an oil by a method similar to that in Example 9C.

### (50D) (1s,3s)-3-{3-[3-fluoro-6-(methoxymethoxy)-2-methyl-4-(trifluoromethyl)phenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (0.056 g, 0.22 mmol) of Example 4C, 2-[3-fluoro-6-(methoxymethoxy)-2-methyl-4-(trifluoromethyl)phenyl]-4,4,5,5-tetramethyl-1,3,2-dioxaborolane (0.099 g, 0.27 mmol) of Example 50C, potassium carbonate (0.071 g, 0.51 mmol) and XPhos Pd G2 (0.053 g, 0.067 mmol), the title compound (0.034 g, yield: 34%) was obtained as an amorphous form by a method similar to that in Example 1E.

### (50E) 4-fluoro-2-{7-[(1s,3s)-3-hydroxy-3-methylcyclobutyl]-5-methyl-7H-pyrrolo[2,3-c]pyridazin-3-yl}-3-methyl-5-(trifluoromethyl)phenol

Using (1s,3s)-3-{3-[3-fluoro-6-(methoxymethoxy)-2-methyl-4-(trifluoromethyl)phenyl]-5-methyl-7H-pyrrolo[2,3-c]pyridazin-7-yl}-1-methylcyclobutanol (0.034 g, 0.075 mmol) of Example 50D, and 4 M hydrogen chloride-1,4-dioxane (0.75 mL), the title compound (0.025 g, yield: 81%) was obtained as a solid by a method similar to that in Example 3D.

### (Example 51)

### 2-{7-[(1s,3s)-3-hydroxy-3-methylcyclobutyl]-5,6-dimethyl-7H-pyrrolo[2,3-c]pyridazin-3-yl}-3-methyl-5-(trifluoromethyl)phenol (also referred to as 2-[7-(cis-3-hydroxy-3-methylcyclobutyl)-5,6-dimethyl-7H-pyrrolo[2,3-c]pyridazin-3-yl]-3-methyl-5-(trifluoromethyl)phenol)

### (51A) (1s,3s)-3-(3-chloro-6-methyl-5,6-dihydro-7H-pyrrolo[2,3-c]pyridazin-7-yl)-1-methylcyclobutanol

Using 3,6-dichloro-4-[(1E)-prop-1-en-1-yl]pyridazine (539 mg, 2.9 mmol) of Example 30A, (1s,3s)-3-amino-1-methylcyclobutanol hydrochloride (431 mg, 3.1 mmol) and DIPEA (1.5 mL, 8.6 mmol), the title compound (490 mg, yield 68%) was obtained as a solid by a method similar to that in Example 1D.

### (51B) (1s,3s)-3-(3-chloro-6-methyl-7H-pyrrolo[2,3-c]pyridazin-7-yl)-1-methylcyclobutanol

Using (1s,3s)-3-(3-chloro-6-methyl-5,6-dihydro-7H-pyrrolo[2,3-c]pyridazin-7-yl)-1-methylcyclobutanol (490 mg, 1.9 mmol) of Example 51A, and manganese dioxide (5.1 g, 58 mmol), the title compound (271 mg, yield: 56%) was obtained as an amorphous form by a method similar to that in Example 4C.

### (51C) (1s,3s)-3-{3-[2-(methoxymethoxy)-6-methyl-4-(trifluoromethyl)phenyl]-6-methyl-7H-pyrrolo[2,3-c]pyridazin-7-yl}-1-methylcyclobutanol

Using (1s,3s)-3-(3-chloro-6-methyl-7H-pyrrolo[2,3-c]pyridazin-7-yl)-1-methylcyclobutanol (271 mg, 1.1 mmol) of Example 51B, 2-[2-(methoxymethoxy)-6-methyl-4-(trifluoromethyl)phenyl]-4,4,5,5-tetramethyl-1,3,2-dioxaborolane (447 mg, 1.3 mmol) of Example 1B, potassium carbonate (297 mg, 2.2 mmol) and XPhos Pd G2 (170 mg, 0.22 mmol), the title compound (473 mg, yield: quantitative) was obtained as an amorphous form by a method similar to that in Example 1E.

### (51D) (1s,3s)-3-{5-bromo-3-[2-(methoxymethoxy)-6-methyl-4-(trifluoromethyl)phenyl]-6-methyl-7H-pyrrolo[2,3-c]pyridazin-7-yl}-1-methylcyclobutanol

(1s,3s)-3-{3-[2-(Methoxymethoxy)-6-methyl-4-(trifluoromethyl)phenyl]-6-methyl-7H-pyrrolo[2,3-c]pyridazin-7-yl}-1-methylcyclobutanol (100 mg, 0.23 mmol) of Example 51C was dissolved in DCE (3.0 mL), N-bromosuccinimide (42 mg, 0.24 mmol) was added, and the mixture was stirred at room temperature for 45 min. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =40/60-15/85(V/V)] to give the title compound (72 mg, yield: 61%) as an amorphous form.

### (51E) (1s,3s)-3-{3-[2-(methoxymethoxy)-6-methyl-4-(trifluoromethyl)phenyl]-5,6-dimethyl-7H-pyrrolo[2,3-c]pyridazin-7-yl}-1-methylcyclobutanol

(1s,3s)-3-{5-Bromo-3-[2-(methoxymethoxy)-6-methyl-4-(trifluoromethyl)phenyl]-6-methyl-7H-pyrrolo[2,3-c]pyridazin-7-yl}-1-methylcyclobutanol (72 mg, 0.14 mmol) of Example 51D, trimethylboroxin (0.052 mL, 0.18 mmol) and potassium carbonate (38 mg, 0.28 mmol) were dissolved in a mixed solvent of 1,4-dioxane (1.5 mL) and water (0.35 mL), Pd(dppf)Cl₂ (20 mg, 0.028 mmol) was added, and the mixture was stirred in the nitrogen-substituted system at 100°C for 3 hr. The reaction solution was allowed to room temperature, water was added, and the mixture was subjected to extraction with ethyl acetate. The organic layer was washed with saturated brine, and dried over anhydrous sodium sulfate. After filtration, the solvent was evaporated under reduced pressure, and the obtained residue was purified by silica gel column chromatography [eluent: n-hexane/ethyl acetate =50/50-20/80(V/V)] to give the title compound (47 mg, yield: 75%) as an amorphous form.

### (51F) 2-{7-[(1s,3s)-3-hydroxy-3-methylcyclobutyl]-5,6-dimethyl-7H-pyrrolo[2,3-c]pyridazin-3-yl}-3-methyl-5-(trifluoromethyl)phenol

Using (1s,3s)-3-{3-[2-(methoxymethoxy)-6-methyl-4-(trifluoromethyl)phenyl]-5,6-dimethyl-7H-pyrrolo[2,3-c]pyridazin-7-yl}-1-methylcyclobutanol (46 mg, 0.10 mmol) of Example 51E, and TFA (1.0 mL), the title compound (7 mg, yield: 17%) was obtained as a solid by a method similar to that in Example 6B.

### (Example 52)

### 3-hydroxy-4-{7-[(1s,3s)-3-hydroxy-3-methylcyclobutyl]-5-methyl-7H-pyrrolo[2,3-c]pyridazin-3-yl}-5-methylbenzonitrile (also referred to as 3-hydroxy-4-[7-(cis-3-hydroxy-3-methylcyclobutyl)-5-methyl-7H-pyrrolo[2,3-c]pyridazin-3-yl]-5-methylbenzonitrile)

### (52A) 4-{7-[(1s,3s)-3-hydroxy-3-methylcyclobutyl]-5-methyl-7H-pyrrolo[2,3-c]pyridazin-3-yl}-3-(methoxymethoxy)-5-methylbenzonitrile

(1s,3s)-3-{3-[4-Chloro-2-(methoxymethoxy)-6-methylphenyl]-5-methyl-7H-pyrrolo[2,3-c]pyridazin-7-yl}-1-methylcyclobutanol (0.042 g, 0.10 mmol) of Example 9D, potassium hexacyanoferrate(II) trihydrate (0.022 g, 0.052 mmol), tBuXPhos (0.004 g, 0.009 mmol), tBuXPhos Pd G1 (0.007 g, 0.01 mmol) and potassium acetate (0.003 g, 0.03 mmol) were dissolved in a mixed solvent of 1,4-dioxane (0.5 mL) and water (0.5 mL), and the mixture was stirred in the nitrogen-substituted system at 100°C for 5 hr. The reaction solution was allowed to room temperature, saturated brine was added, the mixture was subjected to extraction with ethyl acetate, and the organic layer was dried over anhydrous sodium sulfate. After filtration, the filtrate was concentrated under reduced pressure, and the obtained residue was purified by silica gel column chromatography [eluent: n-hexane/ethyl acetate =50/50-10/90(V/V)] to give the title compound (0.030 g, yield: 73%) as a solid.

### (52B) 3-hydroxy-4-{7-[(1s,3s)-3-hydroxy-3-methylcyclobutyl]-5-methyl-7H-pyrrolo[2,3-c]pyridazin-3-yl}-5-methylbenzonitrile

Using 4-{7-[(1s,3s)-3-hydroxy-3-methylcyclobutyl]-5-methyl-7H-pyrrolo[2,3-c]pyridazin-3-yl}-3-(methoxymethoxy)-5-methylbenzonitrile (0.030 g, 0.076 mmol) of Example 52A, and TFA (0.5 mL), the title compound (19 mg, yield: 71%) was obtained as a solid by a method similar to that in Example 6B.

### (Example 53)

### 3-hydroxy-2-{7-[(1s,3s)-3-hydroxy-3-methylcyclobutyl]-5-methyl-7H-pyrrolo[2,3-c]pyridazin-3-yl}-5-(trifluoromethyl)benzonitrile (also referred to as 3-hydroxy-2-[7-(cis-3-hydroxy-3-methylcyclobutyl)-5-methyl-7H-pyrrolo[2,3-c]pyridazin-3-yl]-5-(trifluoromethyl)benzonitrile)

### (53A) 3-hydroxy-5-(trifluoromethyl)benzonitrile

Using 3-bromo-5-(trifluoromethyl)phenol (1.01 g, 4.2 mmol), potassium hexacyanoferrate(II) trihydrate (0.886 g, 2.1 mmol), tBuXPhos (0.178 g, 0.42 mmol), tBuXPhos Pd G1 (0.283 g, 0.41 mmol) and potassium acetate (0.134 g, 1.4 mmol), the title compound (0.634 g, yield: 81%) was obtained as a solid by a method similar to that in Example 52A.

### (53B) 3-(methoxymethoxy)-5-(trifluoromethyl)benzonitrile

Using 3-hydroxy-5-(trifluoromethyl)benzonitrile (0.307 g, 1.6 mmol) of Example 53A, DIPEA (0.6 mL, 3 mmol) and chloromethyl methyl ether (0.185 mL, 2.5 mmol), the title compound (0.366 g, yield: 97%) was obtained as an oil by a method similar to that in Example 1A.

### (53C) 3-(methoxymethoxy)-2-(4,4,5,5-tetramethyl-1,3,2-dioxaborolan-2-yl)-5-(trifluoromethyl)benzonitrile

2,2,6,6-Tetramethylpiperidine (0.40 mL, 2.4 mmol) was dissolved in THF (4 mL), n-butyllithium (1.6 M n-hexane solution) (1.5 mL, 2.4 mmol) was added in the nitrogen-substituted system at -78°C, and the mixture was stirred at 0°C for 30 min. To the reaction solution was added a solution of 3-(methoxymethoxy)-5-(trifluoromethyl)benzonitrile (0.366 g, 1.6 mmol) of Example 53B in THF (1 mL) at -78°C, and the mixture was stirred for 20 min. iPrOBPin (0.64 mL, 3.2 mmol) was added, and the mixture was stirred at room temperature for 3 hr. To the reaction solution was added saturated ammonium chloride aqueous solution, the mixture was subjected to extraction with ethyl acetate, and the organic layer was dried over anhydrous sodium sulfate. After filtration, the filtrate was concentrated under reduced pressure to give a crude product (0.609 g, yield: quantitative) of the title compound as a solid.

### (53D) 2-{7-[(1s,3s)-3-hydroxy-3-methylcyclobutyl]-5-methyl-7H-pyrrolo[2,3-c]pyridazin-3-yl}-3-(methoxymethoxy)-5-(trifluoromethyl)benzonitrile

Using (1s,3s)-3-(3-chloro-5-methyl-7H-pyrrolo[2,3-c]pyridazin-7-yl)-1-methylcyclobutanol (0.130 g, 0.52 mmol) of Example 4C, 3-(methoxymethoxy)-2-(4,4,5,5-tetramethyl-1,3,2-dioxaborolan-2-yl)-5-(trifluoromethyl)benzonitrile (0.276 g, 0.77 mmol) of Example 53C, potassium carbonate (0.147 g, 1.1 mmol) and XPhos Pd G2 (0.041 g, 0.052 mmol), the title compound (0.083 g, yield: 36%) was obtained as a solid by a method similar to that in Example 1E.

### (53E) 3-hydroxy-2-{7-[(1s,3s)-3-hydroxy-3-methylcyclobutyl]-5-methyl-7H-pyrrolo[2,3-c]pyridazin-3-yl}-5-(trifluoromethyl)benzonitrile

Using 2-{7-[(1s,3s)-3-hydroxy-3-methylcyclobutyl]-5-methyl-7H-pyrrolo[2,3-c]pyridazin-3-yl}-3-(methoxymethoxy)-5-(trifluoromethyl)benzonitrile (0.083 g, 0.19 mmol) of Example 53D, and TFA (2 mL), the title compound (1.3 mg, yield: 2%) was obtained as a solid by a method similar to that in Example 6B.

### (Example 54)

### 2-{7-[(1R,3R)-3-hydroxycyclohexyl]-5-methyl-7H-pyrrolo[2,3-c]pyridazin-3-yl}-3-methyl-5-(trifluoromethyl)phenol

### (54A) (1R,3R)-3-(3-chloro-5-methyl-5,6-dihydro-7H-pyrrolo[2,3-c]pyridazin-7-yl)cyclohexanol

Using 3,6-dichloro-4-(prop-1-en-2-yl)pyridazine (175 mg, 0.91 mmol) of Example 4A, (1R,3R)-3-aminocyclohexan-1-ol hydrochloride (125 mg, 0.82 mmol) and DIPEA (0.423 mL, 2.5 mmol), the title compound (25.6 mg, yield: 12%) was obtained as a diastereomeric mixture (an oil) by a method similar to that in Example 1D.

### (54B) 7-[(1R,3R)-3-{[tert-butyl(dimethyl)silyl]oxy}cyclohexyl]-3-chloro-5-methyl-6,7-dihydro-5H-pyrrolo[2,3-c]pyridazine

A mixture of (1R,3R)-3-(3-chloro-5-methyl-5,6-dihydro-7H-pyrrolo[2,3-c]pyridazin-7-yl)cyclohexanol (25.6 mg, 0.096 mmol) of Example 54A, tert-butyldimethylsilyl chloride (62 mg, 0.38 mmol), imidazole (40 mg, 0.58 mmol) and DMF (1 mL) was stirred at room temperature for 3 days. The reaction solution was diluted with n-hexane/ethyl acetate, and the organic layer was washed successively with saturated aqueous sodium bicarbonate solution, water and saturated brine, and dried over anhydrous sodium sulfate. After filtration, the filtrate was concentrated under reduced pressure, and the obtained residue was purified by silica gel column chromatography [eluent: n-hexane/ethyl acetate =95/5-70/30(V/V)] to give the title compound (23 mg, yield: 63%) as a diastereomeric mixture (an oil).

### (54C) 7-[(1R,3R)-3-{[tert-butyl(dimethyl)silyl]oxy}cyclohexyl]-3-chloro-5-methyl-7H-pyrrolo[2,3-c]pyridazine

Using 7-[(1R,3R)-3-{ [tert-butyl(dimethyl)silyl]oxy}cyclohexyl]-3-chloro-5-methyl-6,7-dihydro-5H-pyrrolo[2,3-c]pyridazine (23 mg, 0.060 mmol) of Example 54B, and manganese dioxide (164 mg, 1.9 mmol), the title compound (19 mg, yield: 83%) was obtained as an enantiomer (an oil) by a method similar to that in Example 4C.

### (54D) 7-[(1R,3R)-3-{[tert-butyl(dimethyl)silyl]oxy}cyclohexyl]-3-[2-(methoxymethoxy)-6-methyl-4-(trifluoromethyl)phenyl]-5-methyl-7H-pyrrolo[2,3-c]pyridazine

Using 7-[(1R,3R)-3-{[tert-butyl(dimethyl)silyl]oxy}cyclohexyl]-3-chloro-5-methyl-7H-pyrrolo[2,3-c]pyridazine (19 mg, 0.050 mmol) of Example 54C, 2-[2-(methoxymethoxy)-6-methyl-4-(trifluoromethyl)phenyl]-4,4,5,5-tetramethyl-1,3,2-dioxaborolane (22.5 mg, 0.065 mmol) of Example 1B, XPhos Pd G2 (4 mg, 0.0050 mmol) and potassium carbonate (14 mg, 0.10 mmol), the title compound (17 mg, yield: 60%) was obtained as an oil by a method similar to that in Example 9D.

### (54E) 2-{7-[(1R,3R)-3-hydroxycyclohexyl]-5-methyl-7H-pyrrolo[2,3-c]pyridazin-3-yl}-3-methyl-5-(trifluoromethyl)phenol

Using 7-[(1R,3R)-3-{[tert-butyl(dimethyl)silyl]oxy}cyclohexyl]-3-[2-(methoxymethoxy)-6-methyl-4-(trifluoromethyl)phenyl]-5-methyl-7H-pyrrolo[2,3-c]pyridazine (17 mg, 0.030 mmol) of Example 54D, and 4 M hydrogen chloride-1,4-dioxane (1 mL), the title compound (11.3 mg, yield: 93%) was obtained as a solid by a method similar to that in Example 12E.

### (Example 55)

### 3-methyl-2-{ (6R or 6S)-6-methyl-7-[(3R)-1-methylpiperidin-3-yl]-6,7-dihydro-5H-pyrrolo[2,3-c]pyridazin-3-yl}-5-(trifluoromethyl)phenol

### (55A) tert-butyl (3R)-3-[(6R or 6S)-3-chloro-6-methyl-5,6-dihydro-7H-pyrrolo[2,3-c]pyridazin-7-yl]piperidine-1-carboxylate

Using 3,6-dichloro-4-[(1E)-prop-1-en-1-yl]pyridazine (246 mg, 1.3 mmol) of Example 30A, DIPEA (0.68 mL, 3.9 mmol) and tert-butyl (3R)-3-aminopiperidine-1-carboxylate (313 mg, 1.6 mmol), the title compound eluting later (84 mg, yield: 18%) was obtained as a single diastereomer (an oil) by a method similar to that in Example 1D and purification by silica gel column chromatography [eluent: n-hexane/ethyl acetate =70/30-10/90(V/V)].

### (55B) tert-butyl (3R)-3-{(6R or 6S)-3-[2-(methoxymethoxy)-6-methyl-4-(trifluoromethyl)phenyl]-6-methyl-5,6-dihydro-7H-pyrrolo[2,3-c]pyridazin-7-yl}piperidine-1-carboxylate

Using tert-butyl (3R)-3-[(6R or 6S)-3-chloro-6-methyl-5,6-dihydro-7H-pyrrolo[2,3-c]pyridazin-7-yl]piperidine-1-carboxylate (79 mg, 0.22 mmol) of Example 55A, 2-[2-(methoxymethoxy)-6-methyl-4-(trifluoromethyl)phenyl]-4,4,5,5-tetramethyl-1,3,2-dioxaborolane (93 mg, 0.27 mmol) of Example 1B, potassium carbonate (62 mg, 0.45 mmol) and XPhos Pd G2 (18 mg, 0.022 mmol), the title compound (59 mg, yield: 49%) was obtained as a single diastereomer (an amorphous form) by a method similar to that in Example 1E.

### (55C) 3-methyl-2-{ (6R or 6S)-6-methyl-7-[(3R)-piperidin-3-yl]-6,7-dihydro-5H-pyrrolo[2,3-c]pyridazin-3-yl}-5-(trifluoromethyl)phenol dihydrochloride

Using tert-butyl (3R)-3-{(6R or 6S)-3-[2-(methoxymethoxy)-6-methyl-4-(trifluoromethyl)phenyl]-6-methyl-5,6-dihydro-7H-pyrrolo[2,3-c]pyridazin-7-yl}piperidine-1-carboxylate (51 mg, 0.095 mmol) of Example 55B, and 4 M hydrogen chloride-1,4-dioxane (1.0 mL), a crude product (40 mg, yield: 90%) of the title compound was obtained as a single diastereomer (a solid) by a method similar to that in Example 1F.

### (55D) 3-methyl-2-{ (6R or 6S)-6-methyl-7-[(3R)-1-methylpiperidin-3-yl]-6,7-dihydro-5H-pyrrolo[2,3-c]pyridazin-3-yl}-5-(trifluoromethyl)phenol

Using 3-methyl-2-{ (6R or 6S)-6-methyl-7-[(3R)-piperidin-3-yl]-6,7-dihydro-5H-pyrrolo[2,3-c]pyridazin-3-yl}-5-(trifluoromethyl)phenol dihydrochloride (40 mg, 0.086 mmol) of Example 55C, DIPEA (0.029 mL, 0.17 mmol), 37% formaldehyde (0.0095 mL, 0.13 mmol), acetic acid (0.010 mL, 0.17 mmol) and NaBH(OAc)₃ (73 mg, 0.34 mmol), the title compound (22 mg, yield: 63%) was obtained as a single diastereomer (a solid) by a method similar to that in Example 1G.

### (Example 56)

### 3-methyl-2-{5-methyl-7-[(3R)-1-methylpiperidin-3-yl]-7H-pyrrolo[2,3-c]pyridazin-3-yl}-5-(trifluoromethyl)phenol

### (56A) tert-butyl (3R)-3-{3-[2-(methoxymethoxy)-6-methyl-4-(trifluoromethyl)phenyl]-7H-pyrrolo[2,3-c]pyridazin-7-yl}piperidine-1-carboxylate

Using tert-butyl (3R)-3-{3-[2-(methoxymethoxy)-6-methyl-4-(trifluoromethyl)phenyl]-5,6-dihydro-7H-pyrrolo[2,3-c]pyridazin-7-yl}piperidine-1-carboxylate (537 mg, 1.0 mmol) of Example 1E, and manganese dioxide (2.68 g, 31 mmol), the title compound (325 mg, yield: 61%) was obtained as a solid by a method similar to that in Example 4C.

### (56B) tert-butyl (3R)-3-{5-bromo-3-[2-(methoxymethoxy)-6-methyl-4-(trifluoromethyl)phenyl]-7H-pyrrolo[2,3-c]pyridazin-7-yl}piperidine-1-carboxylate

tert-Butyl (3R)-3-{3-[2-(methoxymethoxy)-6-methyl-4-(trifluoromethyl)phenyl]-7H-pyrrolo[2,3-c]pyridazin-7-yl}piperidine-1-carboxylate (256 mg, 0.49 mmol) of Example 56A was dissolved in DCE (4.8 mL), N-bromosuccinimide (90.9 mg, 0.51 mmol) was added, and the mixture was stirred at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =82/18-55/45(V/V)] to give the title compound (247 mg, yield: 84%) as an amorphous form.

### (56C) tert-butyl (3R)-3-{3-[2-(methoxymethoxy)-6-methyl-4-(trifluoromethyl)phenyl]-5-methyl-7H-pyrrolo[2,3-c]pyridazin-7-yl}piperidine-1-carboxylate

tert-Butyl (3R)-3-{5-bromo-3-[2-(methoxymethoxy)-6-methyl-4-(trifluoromethyl)phenyl]-7H-pyrrolo[2,3-c]pyridazin-7-yl}piperidine-1-carboxylate (140.2 mg, 0.23 mmol) of Example 56B, methylboronic acid (23.0 mg, 0.38 mmol) and potassium carbonate (67.2 mg, 0.49 mmol) were dissolved in a mixed solvent of 1,4-dioxane (2.4 mL) and water (0.8 mL), XPhos Pd G2 (19.9 mg, 0.025 mmol) was added, and the mixture was stirred in the nitrogen-substituted system at 120°C for 3 hr 20 min. The reaction solution was allowed to room temperature, water and ethyl acetate were added, and the mixture was filtered through Celite, and subjected to liquid separation. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =20/80-0/100(V/V)] to give the title compound (102 mg, yield: 82%) as an amorphous form.

### (56D) 3-methyl-2-{5-methyl-7-[(3R)-piperidin-3-yl]-7H-pyrrolo[2,3-c]pyridazin-3-yl}-5-(trifluoromethyl)phenol

Using tert-butyl (3R)-3-{3-[2-(methoxymethoxy)-6-methyl-4-(trifluoromethyl)phenyl]-5-methyl-7H-pyrrolo[2,3-c]pyridazin-7-yl}piperidine-1-carboxylate (99.7 mg, 0.19 mmol) of Example 56C, and 4 M hydrogen chloride-1,4-dioxane (1.9 mL), the title compound (45.8 mg, yield: 64%) was obtained as a solid by a method similar to that in Example 1F.

### (56E) 3-methyl-2-{5-methyl-7-[(3R)-1-methylpiperidin-3-yl]-7H-pyrrolo[2,3-c]pyridazin-3-yl}-5-(trifluoromethyl)phenol

Using 3-methyl-2-{5-methyl-7-[(3R)-piperidin-3-yl]-7H-pyrrolo[2,3-c]pyridazin-3-yl}-5-(trifluoromethyl)phenol (40.2 mg, 0.10 mmol) of Example 56D, methanol (0.4 mL), 37% formaldehyde (0.015 mL, 0.21 mmol) and NaBH(OAc)₃ (105.5 mg, 0.50 mmol), the title compound (40.4 mg, yield: 97%) was obtained as a solid by a method similar to that in Example 1G.

### (Example 57)

### 3-methyl-2-{7-[(3R)-1-(tetrahydrofuran-3-yl)piperidin-3-yl]-6,7-dihydro-5H-pyrrolo[2,3-c]pyridazin-3-yl}-5-(trifluoromethyl)phenol

### (57A) 3-[2-(methoxymethoxy)-6-methyl-4-(trifluoromethyl)phenyl]-7-[(3R)-1-(tetrahydrofuran-3-yl)piperidin-3-yl]-6,7-dihydro-5H-pyrrolo[2,3-c]pyridazine

Using 3-[2-(methoxymethoxy)-6-methyl-4-(trifluoromethyl)phenyl]-7-[(3R)-piperidin-3-yl]-6,7-dihydro-5H-pyrrolo[2,3-c]pyridazine (0.067 g, 0.16 mmol) of Example 14C, tetrahydrofuran-3-one (0.024 mL, 0.31 mmol), NaBH(OAc)₃ (0.13 g, 0.61 mmol) and acetic acid (0.018 mL, 0.31 mmol), the title compound (0.061 g, yield: 78%) was obtained as a diastereomeric mixture (an amorphous form) by a method similar to that in Example 1G.

### (57B) 3-methyl-2-{7-[(3R)-1-(tetrahydrofuran-3-yl)piperidin-3-yl]-6,7-dihydro-5H-pyrrolo[2,3-c]pyridazin-3-yl}-5-(trifluoromethyl)phenol

Using 3-[2-(methoxymethoxy)-6-methyl-4-(trifluoromethyl)phenyl]-7-[(3R)-1-(tetrahydrofuran-3-yl)piperidin-3-yl]-6,7-dihydro-5H-pyrrolo[2,3-c]pyridazine (0.061 g, 0.12 mmol) of Example 57A, and 4 M hydrogen chloride-1,4-dioxane (1 mL), the title compound (44 mg, yield: 79%) was obtained as a diastereomeric mixture (a solid) by a method similar to that in Example 3D.

### (Example 58)

### 2-{7-[(3R)-1-(2-hydroxy-2-methylpropyl)piperidin-3-yl]-6,7-dihydro-5H-pyrrolo[2,3-c]pyridazin-3-yl}-3-methyl-5-(trifluoromethyl)phenol

### (58A) 1-[(3R)-3-{3-[2-(methoxymethoxy)-6-methyl-4-(trifluoromethyl)phenyl]-5,6-dihydro-7H-pyrrolo[2,3-c]pyridazin-7-yl}piperidin-1-yl]-2-methylpropan-2-ol

3-[2-(Methoxymethoxy)-6-methyl-4-(trifluoromethyl)phenyl]-7-[(3R)-piperidin-3-yl]-6,7-dihydro-5H-pyrrolo[2,3-c]pyridazine (0.067 g, 0.16 mmol) of Example 14C was dissolved in acetonitrile (2 mL), then sodium iodide (0.036 g, 0.24 mmol), potassium carbonate (0.044 g, 0.32 mmol) and 1-chloro-2-methyl-2-propanol (0.048 mL, 0.46 mmol) were added, and the mixture was stirred at 80°C for 3 hr. To the reaction solution was added isobutylene oxide (2.14 mL, 24 mmol), and the mixture was stirred at 80°C for 15.5 hr. The reaction solution was allowed to room temperature, saturated aqueous sodium bicarbonate solution was added, the mixture was subjected to extraction with ethyl acetate, and the organic layer was dried over anhydrous sodium sulfate. After filtration, the filtrate was concentrated under reduced pressure, and the obtained residue was purified by silica gel column chromatography [eluent: DCM/methanol=100/0-70/30(V/V)] to give the title compound (0.057 g, yield: 73%) as an amorphous form.

### (58B) 2-{7-[(3R)-1-(2-hydroxy-2-methylpropyl)piperidin-3-yl]-6,7-dihydro-5H-pyrrolo[2,3-c]pyridazin-3-yl}-3-methyl-5-(trifluoromethyl)phenol

Using 1-[(3R)-3-{3-[2-(methoxymethoxy)-6-methyl-4-(trifluoromethyl)phenyl]-5,6-dihydro-7H-pyrrolo[2,3-c]pyridazin-7-yl}piperidin-1-yl]-2-methylpropan-2-ol (0.057 g, 0.12 mmol) of Example 58A, and 4 M hydrogen chloride-1,4-dioxane (1 mL), the title compound (42 mg, yield: 81%) was obtained as a solid by a method similar to that in Example 3D.

### (Example 59)

### 2-{7-[(1s,3s)-3-hydroxy-3-methylcyclobutyl]-5-(trifluoromethyl)-7H-pyrrolo[2,3-c]pyridazin-3-yl}-3-methyl-5-(trifluoromethyl)phenol (also referred to as 2-[7-(cis-3-hydroxy-3-methylcyclobutyl)-5-(trifluoromethyl)-7H-pyrrolo[2,3-c]pyridazin-3-yl]-3-methyl-5-(trifluoromethyl)phenol)

### (59A) 7-{(1s,3s)-3-[(4-methoxybenzyl)oxy]-3-methylcyclobutyl}-3-[2-(methoxymethoxy)-6-methyl-4-(trifluoromethyl)phenyl]-7H-pyrrolo[2,3-c]pyridazine

(1s,3s)-3-{3-[2-(Methoxymethoxy)-6-methyl-4-(trifluoromethyl)phenyl]-7H-pyrrolo[2,3-c]pyridazin-7-yl}-1-methylcyclobutanol (813 mg, 1.9 mmol) of Example 3C was dissolved in DMF (5 mL), then 55% sodium hydride (168 mg, 3.9 mmol) and paramethoxybenzyl chloride (0.788 mL, 5.8 mmol) were added at 0°C, and the mixture was stirred at room temperature for 1.5 hr. To the reaction solution was added water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =70/30-55/45(V/V)] to give the title compound (1.05 g, yield: quantitative) as an oil.

### (59B) 5-iodo-7-{(1s,3s)-3-[(4-methoxybenzyl)oxy]-3-methylcyclobutyl}-3-[2-(methoxymethoxy)-6-methyl-4-(trifluoromethyl)phenyl]-7H-pyrrolo[2,3-c]pyridazine

7-{(1s,3s)-3-[(4-Methoxybenzyl)oxy]-3-methylcyclobutyl}-3-[2-(methoxymethoxy)-6-methyl-4-(trifluoromethyl)phenyl]-7H-pyrrolo[2,3-c]pyridazine (1.05 g, 1.9 mmol) of Example 59A was dissolved in DCE (10 mL), N-iodosuccinimide (523 mg, 2.3 mmol) was added, and the mixture was stirred at 60°C for 5 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-65/35(V/V)] to give the title compound (924 mg, yield: 71%) as an oil.

### (59C) 7-{(1s,3s)-3-[(4-methoxybenzyl)oxy]-3-methylcyclobutyl}-3-[2-(methoxymethoxy)-6-methyl-4-(trifluoromethyl)phenyl]-5-(trifluoromethyl)-7H-pyrrolo[2,3-c]pyridazine

5-Iodo-7-{(1s,3s)-3-[(4-methoxybenzyl)oxy]-3-methylcyclobutyl}-3-[2-(methoxymethoxy)-6-methyl-4-(trifluoromethyl)phenyl]-7H-pyrrolo[2,3-c]pyridazine (924 mg, 1.4 mmol) of Example 59B was dissolved in DMF (7 mL), then copper(I) iodide (395 mg, 2.1 mmol), methyl difluoro(fluorosulfonyl)acetate (0.437 mL, 3.5 mmol) and Pd₂(dba)₃ (126 mg, 0.14 mmol) were added, and the mixture was stirred at 100°C for 75 min. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-0/100(V/V)] to give the title compound (22 mg, yield: 3%) as an amorphous form.

### (59D) 2-{7-[(1s,3s)-3-hydroxy-3-methylcyclobutyl]-5-(trifluoromethyl)-7H-pyrrolo[2,3-c]pyridazin-3-yl}-3-methyl-5-(trifluoromethyl)phenol

Using 7-{(1s,3s)-3-[(4-methoxybenzyl)oxy]-3-methylcyclobutyl}-3-[2-(methoxymethoxy)-6-methyl-4-(trifluoromethyl)phenyl]-5-(trifluoromethyl)-7H-pyrrolo[2,3-c]pyridazine (22 mg, 0.036 mmol) of Example 59C, and TFA (0.4 mL), the title compound (6 mg, yield: 37%) was obtained as a solid by a method similar to that in Example 5D.

### (Example 60)

### 2-{7-[(3R,5R)-5-fluoro-1-methylpiperidin-3-yl]-6,7-dihydro-5H-pyrrolo[2,3-c]pyridazin-3-yl}-3-methyl-5-(trifluoromethyl)phenol

### (60A) tert-butyl (3R,5R)-3-(3-chloro-5,6-dihydro-7H-pyrrolo[2,3-c]pyridazin-7-yl)-5-fluoropiperidine-1-carboxylate

Using 3,6-dichloro-4-ethenylpyridazine (209 mg, 1.19 mmol) of Example 1C, DIPEA (0.62 mL, 3.6 mmol) and tert-butyl (3R, 5R)-3-amino-5-fluoropiperidine-1-carboxylate (387 mg, 1.79 mmol), the title compound (151 mg, yield: 36%) was obtained as an amorphous form by a method similar to that in Example 1D.

### (60B) tert-butyl (3R,5R)-3-fluoro-5-{3-[2-(methoxymethoxy)-6-methyl-4-(trifluoromethyl)phenyl]-5,6-dihydro-7H-pyrrolo[2,3-c]pyridazin-7-yl}piperidine-1-carboxylate

Using tert-butyl (3R,5R)-3-(3-chloro-5,6-dihydro-7H-pyrrolo[2,3-c]pyridazin-7-yl)-5-fluoropiperidine-1-carboxylate (146.1 mg, 0.41 mmol) of Example 60A, 2-[2-(methoxymethoxy)-6-methyl-4-(trifluoromethyl)phenyl]-4,4,5,5-tetramethyl-1,3,2-dioxaborolane (177.4 mg, 0.51 mmol) of Example 1B, potassium carbonate (128.5 mg, 0.93 mmol) and XPhos Pd G2 (51.4 mg, 0.066 mmol), the title compound (141 mg, yield: 64%) was obtained as an amorphous form by a method similar to that in Example 9D.

### (60C) 2-{7-[(3R,5R)-5-fluoropiperidin-3-yl]-6,7-dihydro-5H-pyrrolo[2,3-c]pyridazin-3-yl}-3-methyl-5-(trifluoromethyl)phenol

Using tert-butyl (3R,5R)-3-fluoro-5-{3-[2-(methoxymethoxy)-6-methyl-4-(trifluoromethyl)phenyl]-5,6-dihydro-7H-pyrrolo[2,3-c]pyridazin-7-yl}piperidine-1-carboxylate (69.5 mg, 0.13 mmol) of Example 60B, and 4 M hydrogen chloride-1,4-dioxane (1.2 mL), the title compound (44.1 mg, yield: 87%) was obtained as a solid by a method similar to that in Example 1F.

### (60D) 2-{7-[(3R,5R)-5-fluoro-1-methylpiperidin-3-yl]-6,7-dihydro-5H-pyrrolo[2,3-c]pyridazin-3-yl}-3-methyl-5-(trifluoromethyl)phenol

Using 2-{7-[(3R,5R)-5-fluoropiperidin-3-yl]-6,7-dihydro-5H-pyrrolo[2,3-c]pyridazin-3-yl}-3-methyl-5-(trifluoromethyl)phenol (44.1 mg, 0.11 mmol) of Example 60C, 37% formaldehyde (0.017 mL, 0.22 mmol) and NaBH(OAc)₃ (116.0 mg, 0.55 mmol), the title compound (45.6 mg, yield: quantitative) was obtained as a solid by a method similar to that in Example 1G.

### (Example 61)

### 2-{7-[(3R)-1-(cyclopropylmethyl)piperidin-3-yl]-6,7-dihydro-5H-pyrrolo[2,3-c]pyridazin-3-yl}-3-methyl-5-(trifluoromethyl)phenol

### (61A) 7-[(3R)-1-(cyclopropylmethyl)piperidin-3-yl]-3-[2-(methoxymethoxy)-6-methyl-4-(trifluoromethyl)phenyl]-6,7-dihydro-5H-pyrrolo[2,3-c]pyridazine

Using 3-[2-(methoxymethoxy)-6-methyl-4-(trifluoromethyl)phenyl]-7-[(3R)-piperidin-3-yl]-6,7-dihydro-5H-pyrrolo[2,3-c]pyridazine (0.075 g, 0.18 mmol) of Example 14C, cyclopropanecarboxyaldehyde (0.027 mL, 0.36 mmol) and NaBH(OAc)₃ (0.11 g, 0.52 mmol), the title compound (0.044 g, yield: 52%) was obtained as an oil by a method similar to that in Example 1G.

### (61B) 2-{7-[(3R)-1-(cyclopropylmethyl)piperidin-3-yl]-6,7-dihydro-5H-pyrrolo[2,3-c]pyridazin-3-yl}-3-methyl-5-(trifluoromethyl)phenol

Using 7-[(3R)-1-(cyclopropylmethyl)piperidin-3-yl]-3-[2-(methoxymethoxy)-6-methyl-4-(trifluoromethyl)phenyl]-6,7-dihydro-5H-pyrrolo[2,3-c]pyridazine (0.044 g, 0.092 mmol) of Example 61A, and 4 M hydrogen chloride-1,4-dioxane (1 mL), the title compound (32 mg, yield: 80%) was obtained as a solid by a method similar to that in Example 3D.

### (Example 62)

### 5-ethyl-2-{7-[(1s,3s)-3-hydroxy-3-methylcyclobutyl]-5-methyl-7H-pyrrolo[2,3-c]pyridazin-3-yl}-3-methylphenol (also referred to as 5-ethyl-2-[7-(cis-3-hydroxy-3-methylcyclobutyl)-5-methyl-7H-pyrrolo[2,3-c]pyridazin-3-yl]-3-methylphenol)

### (62A) (1s,3s)-3-{3-[4-ethenyl-2-(methoxymethoxy)-6-methylphenyl]-5-methyl-7H-pyrrolo[2,3-c]pyridazin-7-yl}-1-methylcyclobutanol

(1s,3s)-3-{3-[4-Chloro-2-(methoxymethoxy)-6-methylphenyl]-5-methyl-7H-pyrrolo[2,3-c]pyridazin-7-yl}-1-methylcyclobutanol (0.055 g, 0.14 mmol) of Example 9D, potassium vinyltrifluoroborate (0.037 g, 0.28 mmol) and potassium carbonate (0.057 g, 0.41 mmol) were dissolved in a mixed solvent of 1,4-dioxane (1 mL) and water (0.25 mL), Pd(dppf)Cl₂ (0.010 g, 0.013 mmol) was added, and the mixture was stirred in the nitrogen-substituted system at 110°C for 1 hr. The reaction solution was allowed to room temperature, saturated aqueous sodium bicarbonate solution was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (0.028 g, yield: 52%) as an amorphous form.

### (62B) (1s,3s)-3-{3-[4-ethyl-2-(methoxymethoxy)-6-methylphenyl]-5-methyl-7H-pyrrolo[2,3-c]pyridazin-7-yl}-1-methylcyclobutanol

(1s,3s)-3-{3-[4-Ethenyl-2-(methoxymethoxy)-6-methylphenyl]-5-methyl-7H-pyrrolo[2,3-c]pyridazin-7-yl}-1-methylcyclobutanol (0.020 g, 0.051 mmol) of Example 62A was dissolved in ethanol (1 mL), 10% palladium on carbon (0.067 g) was added, and the mixture was stirred in the hydrogen-substituted system at room temperature for 1 hr. The reaction solution was filtered through Celite, the filtrate was concentrated under reduced pressure, and the obtained residue was purified by silica gel column chromatography [eluent: n-hexane/ethyl acetate =50/50-0/100(V/V)] to give the title compound (0.011 g, yield: 55%) as an oil.

### (62C) 5-ethyl-2-{7-[(1s,3s)-3-hydroxy-3-methylcyclobutyl]-5-methyl-7H-pyrrolo[2,3-c]pyridazin-3-yl}-3-methylphenol

Using (1s,3s)-3-{3-[4-ethyl-2-(methoxymethoxy)-6-methylphenyl]-5-methyl-7H-pyrrolo[2,3-c]pyridazin-7-yl}-1-methylcyclobutanol (0.011 g, 0.028 mmol) of Example 62B, and TFA (1.0 mL), the title compound (5.2 mg, yield: 55%) was obtained as a solid by a method similar to that in Example 6B.

### (Example 63)

### 3,5-dichloro-2-{7-[(1s,3s)-3-hydroxy-3-methylcyclobutyl]-5-methyl-7H-pyrrolo[2,3-c]pyridazin-3-yl}phenol (also referred to as 3,5-dichloro-2-[7-(cis-3-hydroxy-3-methylcyclobutyl)-5-methyl-7H-pyrrolo[2,3-c]pyridazin-3-yl]phenol)

### (63A) 3,5-dichloro-2-iodophenol

Using 3,5-dichlorophenol (0.999 g, 6.1 mmol), 55% sodium hydride (0.538 g, 12 mmol) and iodine (1.56 g, 6.2 mmol), the title compound (1.62 g, yield: 92%) was obtained as an oil by a method similar to that in Example 9A.

### (63B) 1,5-dichloro-2-iodo-3-(methoxymethoxy)benzene

Using 3,5-dichloro-2-iodophenol (1.62 g, 5.6 mmol) of Example 63A, DIPEA (2.0 mL, 11 mmol) and chloromethyl methyl ether (0.63 mL, 8.4 mmol), the title compound (1.81 g, yield: 97%) was obtained as an oil by a method similar to that in Example 1A.

### (63C) 2-[2,4-dichloro-6-(methoxymethoxy)phenyl]-4,4,5,5-tetramethyl-1,3,2-dioxaborolane

Using 1,5-dichloro-2-iodo-3-(methoxymethoxy)benzene (1.81 g, 5.4 mmol) of Example 63B, n-butyllithium (1.6 M n-hexane solution) (5.1 mL, 8.1 mmol) and iPrOBPin (2.2 mL, 11 mmol), the title compound (0.940 g, yield: 52%) was obtained as a solid by a method similar to that in Example 9C.

### (63D) (1s,3s)-3-{3-[2,4-dichloro-6-(methoxymethoxy)phenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (0.123 g, 0.49 mmol) of Example 4C, 2-[2,4-dichloro-6-(methoxymethoxy)phenyl]-4,4,5,5-tetramethyl-1,3,2-dioxaborolane (0.631 g, 1.9 mmol) of Example 63C, potassium carbonate (0.137 g, 0.99 mmol) and XPhos Pd G2 (0.080 g, 0.10 mmol), the title compound (0.039 g, yield: 19%) was obtained as an oil by a method similar to that in Example 9D.

### (63E) 3,5-dichloro-2-{7-[(1s,3s)-3-hydroxy-3-methylcyclobutyl]-5-methyl-7H-pyrrolo[2,3-c]pyridazin-3-yl}phenol

Using (1s,3s)-3-{3-[2,4-dichloro-6-(methoxymethoxy)phenyl]-5-methyl-7H-pyrrolo[2,3-c]pyridazin-7-yl}-1-methylcyclobutanol (0.039 g, 0.092 mmol) of Example 63D, and 4 M hydrogen chloride-1,4-dioxane (1 mL), the title compound (6.4 mg, yield: 18%) was obtained as a solid by a method similar to that in Example 3D.

### (Example 64)

### 7-[(3R)-1-ethylpiperidin-3-yl]-3-[2-hydroxy-6-methyl-4-(trifluoromethyl)phenyl]-7H-pyrrolo[2,3-c]pyridazine-5-carbonitrile

### (64A) tert-butyl (3R)-3-{5-cyano-3-[2-(methoxymethoxy)-6-methyl-4-(trifluoromethyl)phenyl]-7H-pyrrolo[2,3-c]pyridazin-7-yl}piperidine-1-carboxylate

tert-Butyl (3R)-3-{5-bromo-3-[2-(methoxymethoxy)-6-methyl-4-(trifluoromethyl)phenyl]-7H-pyrrolo[2,3-c]pyridazin-7-yl}piperidine-1-carboxylate (100.5 mg, 0.17 mmol) of Example 56B, potassium hexacyanoferrate(II) trihydrate (38.2 mg, 0.090 mmol), XPhos (14.1 mg, 0.033 mmol) and potassium carbonate (49.3 mg, 0.05 mmol) were dissolved in a mixed solvent of 1,4-dioxane (1.2 mL) and water (1.2 mL), XPhos Pd G2 (25.6 mg, 0.037 mmol) was added, and the mixture was stirred in the nitrogen-substituted system at 100°C for 3 hr. The reaction solution was filtered through Celite, to the filtrate was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =67/33-46/54(V/V)] to give the title compound (78.3 mg, yield: 86%) as an amorphous form.

### (64B) 3-[2-hydroxy-6-methyl-4-(trifluoromethyl)phenyl]-7-[(3R)-piperidin-3-yl]-7H-pyrrolo[2,3-c]pyridazine-5-carbonitrile

tert-Butyl (3R)-3-{5-cyano-3-[2-(methoxymethoxy)-6-methyl-4-(trifluoromethyl)phenyl]-7H-pyrrolo[2,3-c]pyridazin-7-yl}piperidine-1-carboxylate (78.1 mg, 0.14 mmol) of Example 64A was dissolved in DCM (1.0 mL), TFA (0.5 mL) was added, and the mixture was stirred at room temperature for one day. The reaction solution was concentrated under reduced pressure, and to the residue was added saturated aqueous sodium bicarbonate solution. The mixture was subjected to extraction with ethyl acetate. The organic layer was washed with saturated brine, and dried over anhydrous magnesium sulfate. After filtration, the filtrate was concentrated under reduced pressure, and the obtained residue was purified by reverse-phase preparative HPLC [mobile phase: 0.1% formic acid aqueous solution/0.1% formic acid-acetonitrile =87/13-58/42(V/V)] to give the title compound (39.8 mg, yield: 69%) as a solid.

### (64C) 7-[(3R)-1-ethylpiperidin-3-yl]-3-[2-hydroxy-6-methyl-4-(trifluoromethyl)phenyl]-7H-pyrrolo[2,3-c]pyridazine-5-carbonitrile

Using 3-[2-hydroxy-6-methyl-4-(trifluoromethyl)phenyl]-7-[(3R)-piperidin-3-yl]-7H-pyrrolo[2,3-c]pyridazine-5-carbonitrile (39.7 mg, 0.099 mmol) of Example 64B, acetaldehyde (0.020 mL, 0.40 mmol) and NaBH(OAc)₃ (113.9 mg, 0.54 mmol), the title compound (31.4 mg, yield: 74%) was obtained as a solid by a method similar to that in Example 1G.

### (Example 65)

### 5-{3-[2-hydroxy-6-methyl-4-(trifluoromethyl)phenyl]-5-methyl-7H-pyrrolo[2,3-c]pyridazin-7-yl}-3-oxabicyclo[3.1.1]heptan-1-ol

### (65A) ethyl 1-[(tert-butoxycarbonyl)amino]-3-methylidenecyclobutanecarboxylate

Methyltriphenylphosphonium bromide (18.5 g, 51 mmol) was suspended in 1,4-dioxane (85 mL), potassium tert-butoxide (5.7 g, 57 mmol) was added, and the mixture was stirred at 40°C for 30 min. A solution of ethyl 1-(tert-butoxycarbonylamino)-3-oxocyclobutane-1-carboxylate (CAS registration number: 129287-91-8, 11 g, 43 mmol) in 1,4-dioxane (85 mL) was added at 0°C, and the mixture was stirred at room temperature for 2.5 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =86/14-78/22(V/V)] to give the title compound (4.73 g, yield: 43%) as a solid.

### (65B) ethyl 1-[(tert-butoxycarbonyl)amino]-3-hydroxy-3-(hydroxymethyl)cyclobutanecarboxylate

Ethyl 1-[(tert-butoxycarbonyl)amino]-3-methylidenecyclobutanecarboxylate (4.73 g, 19 mmol) of Example 65A was dissolved in a mixed solvent of acetone (60 mL) and water (30 mL), then osmium tetroxide (11.8 g, 1.9 mmol) and 4-methylmorpholine N-oxide (8.68 g, 74 mmol) were added, and the mixture was stirred at room temperature for 30 min. To the reaction solution was added sodium thiosulfate aqueous solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: ethyl acetate/methanol=100/0-97/3(V/V)] to give the title compound (5.2 g, yield: 97%) as a solid.

### (65C) tert-butyl [(1s,3s)-3-hydroxy-1,3-bis(hydroxymethyl)cyclobutyl]carbamate

Ethyl 1-[(tert-butoxycarbonyl)amino]-3-hydroxy-3-(hydroxymethyl)cyclobutanecarboxylate (5.17 g, 18 mmol) of Example 65B was dissolved in THF (90 mL), lithium borohydride (1.56 g, 72 mmol) was added at 0°C, and the mixture was stirred at room temperature for 30 min. To the reaction solution was added saturated ammonium chloride aqueous solution at 0°C, the mixture was subjected to extraction with chloroform/2-propanol, and the organic layer was dried over anhydrous sodium sulfate. After filtration, the filtrate was concentrated under reduced pressure, and the obtained residue was purified by silica gel column chromatography [eluent: ethyl acetate/acetonitrile/methanol=85/15/5(V/V/V)] to give the title compound (2.26 g, yield: 51%) as a solid.

### (65D) {(1s,3s)-3-[(tert-butoxycarbonyl)amino]-1-hydroxy-3-(hydroxymethyl)cyclobutyl}methyl 4-methylbenzenesulfonate

tert-Butyl [(1s,3s)-3-hydroxy-1,3-bis(hydroxymethyl)cyclobutyl]carbamate (1.27 g, 5.1 mmol) of Example 65C was dissolved in pyridine (17 mL), p-toluenesulfonyl chloride (1.37 g, 7.2 mmol) was added at 0°C, and the mixture was stirred at 0°C for 1.5 hr. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (1.26 g, yield: 61%) as an amorphous form.

### (65E) tert-butyl (5-hydroxy-3-oxabicyclo[3.1.1]heptan-1-yl)carbamate

{(1s,3s)-3-[(tert-Butoxycarbonyl)amino]-1-hydroxy-3-(hydroxymethyl)cyclobutyl}methyl 4-methylbenzenesulfonate (1.26 g, 3.1 mmol) of Example 65D was dissolved in THF (60 mL), potassium tert-butoxide (740 mg, 6.6 mmol) was added at 0°C, and the mixture was stirred for 15 min. To the reaction solution was added saturated ammonium chloride aqueous solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =30/70-10/90(V/V)] to give the title compound (474 mg, yield: 66%) as an oil.

### (65F) 5-amino-3-oxabicyclo[3.1.1]heptan-1-ol

tert-Butyl (5-hydroxy-3-oxabicyclo[3.1.1]heptan-1-yl)carbamate (474 mg, 2.1 mmol) of Example 65E was dissolved in DCM (5.0 mL), TFA (5.0 mL) was added, and the mixture was stirred at room temperature for 30 min. The reaction solution was concentrated under reduced pressure, and the obtained residue was purified by NH silica gel column chromatography [eluent: DCM/methanol=90/10-80/20(V/V)] to give the title compound (267 mg, yield: quantitative) as an oil.

### (65G) 5-(3-chloro-5,6-dihydro-7H-pyrrolo[2,3-c]pyridazin-7-yl)-3-oxabicyclo[3.1.1]heptan-1-ol

Using 3,6-dichloro-4-ethenylpyridazine (345 mg, 2.0 mmol) of Example 1C, 5-amino-3-oxabicyclo[3.1.1]heptan-1-ol (267 mg, 2.1 mmol) of Example 65F, and DIPEA (1.3 mL, 7.9 mmol), the title compound (164 mg, yield 31%) was added as an oil by a method similar to that in Example 1D.

### (65H) 7-(5-{[tert-butyl(dimethyl)silyl]oxy}-3-oxabicyclo[3.1.1]heptan-1-yl)-3-chloro-6,7-dihydro-5H-pyrrolo[2,3-c]pyridazine

5-(3-Chloro-5,6-dihydro-7H-pyrrolo[2,3-c]pyridazin-7-yl)-3-oxabicyclo[3.1.1]heptan-1-ol (164 mg, 0.61 mmol) of Example 65G was dissolved in DMF (3.0 mL), then imidazole (208 mg, 3.1 mmol) and tert-butyldimethylsilyl chloride (369 mg, 2.5 mmol) were added, and the mixture was stirred at room temperature for 1 hr. To the reaction solution was added water at 0°C, and the mixture was subjected to extraction with ethyl acetate. The organic layer was washed with water and saturated brine, and dried over anhydrous sodium sulfate. After filtration, the filtrate was concentrated under reduced pressure, and the obtained residue was purified by silica gel column chromatography [eluent: n-hexane/ethyl acetate =60/40-50/50(V/V)] to give the title compound (90 mg, yield: 38%) as a solid.

### (65I) 7-(5-{[tert-butyl(dimethyl)silyl]oxy}-3-oxabicyclo[3.1.1]heptan-1-yl)-3-chloro-7H-pyrrolo[2,3-c]pyridazine

Using 7-(5-{[tert-butyl(dimethyl)silyl]oxy}-3-oxabicyclo[3.1.1]heptan-1-yl)-3-chloro-6,7-dihydro-5H-pyrrolo[2,3-c]pyridazine (90 mg, 0.24 mmol) of Example 65H, and DDQ (64 mg, 0.28 mmol), the title compound (66 mg, yield 74%) was obtained as an oil by a method similar to that in Example 2A.

### (65J) 7-(5-{[tert-butyl(dimethyl)silyl]oxy}-3-oxabicyclo[3.1.1]heptan-1-yl)-3-[2-(methoxymethoxy)-6-methyl-4-(trifluoromethyl)phenyl]-7H-pyrrolo[2,3-c]pyridazine

Using 7-(5-{[tert-butyl(dimethyl)silyl]oxy}-3-oxabicyclo[3.1.1]heptan-1-yl)-3-chloro-7H-pyrrolo[2,3-c]pyridazine (66 mg, 0.17 mmol) of Example 65I, 2-[2-(methoxymethoxy)-6-methyl-4-(trifluoromethyl)phenyl]-4,4,5,5-tetramethyl-1,3,2-dioxaborolane (66 mg, 0.19 mmol) of Example 1B, potassium carbonate (48 mg, 0.35 mmol) and XPhos Pd G2 (27 mg, 0.035 mmol), the title compound (72 mg, yield: 74%) was obtained as an oil by a method similar to that in Example 1E.

### (65K) 5-bromo-7-(5-{[tert-butyl(dimethyl)silyl]oxy}-3-oxabicyclo[3.1.1]heptan-1-yl)-3-[2-(methoxymethoxy)-6-methyl-4-(trifluoromethyl)phenyl]-7H-pyrrolo[2,3-c]pyridazine

7-(5-{[tert-Butyl(dimethyl)silyl]oxy}-3-oxabicyclo[3.1.1]heptan-1-yl)-3-[2-(methoxymethoxy)-6-methyl-4-(trifluoromethyl)phenyl]-7H-pyrrolo[2,3-c]pyridazine (28 mg, 0.049 mmol) of Example 65J was dissolved in DCE (0.5 mL), N-bromosuccinimide (11 mg, 0.059 mmol) was added, and the mixture was stirred at room temperature for 45 min. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-67/33(V/V)] to give the title compound (18 mg, yield: 57%) as an oil.

### (65L) 7-(5-{[tert-butyl(dimethyl)silyl]oxy}-3-oxabicyclo[3.1.1]heptan-1-yl)-3-[2-(methoxymethoxy)-6-methyl-4-(trifluoromethyl)phenyl]-5-methyl-7H-pyrrolo[2,3-c]pyridazine

5-Bromo-7-(5-{[tert-butyl(dimethyl)silyl]oxy}-3-oxabicyclo[3.1.1]heptan-1-yl)-3-[2-(methoxymethoxy)-6-methyl-4-(trifluoromethyl)phenyl]-7H-pyrrolo[2,3-c]pyridazine (18 mg, 0.028 mmol) of Example 65K, methylboronic acid (6 mg, 0.10 mmol) and potassium carbonate (7.0 mg, 0.056 mmol) were dissolved in a mixed solvent of 1,4-dioxane (0.3 mL) and water (0.1 mL), XPhos Pd G2 (4 mg, 0.0056 mmol) was added, and the mixture was stirred in the nitrogen-substituted system at 110°C for 2 hr. The reaction solution was allowed to room temperature, water was added, and the mixture was subjected to extraction with ethyl acetate. The organic layer was washed with saturated brine, and dried over anhydrous sodium sulfate. After filtration, the solvent was evaporated under reduced pressure, and the obtained residue was purified by silica gel column chromatography [eluent: n-hexane/ethyl acetate =75/25-67/33(V/V)] to give the title compound (7.5 mg, yield: 46%) as an oil.

### (65M) 5-{3-[2-hydroxy-6-methyl-4-(trifluoromethyl)phenyl]-5-methyl-7H-pyrrolo[2,3-c]pyridazin-7-yl}-3-oxabicyclo[3.1.1]heptan-1-ol

Using 7-(5-{[tert-butyl(dimethyl)silyl]oxy}-3-oxabicyclo[3.1.1]heptan-1-yl)-3-[2-(methoxymethoxy)-6-methyl-4-(trifluoromethyl)phenyl]-5-methyl-7H-pyrrolo[2,3-c]pyridazine (7.5 mg, 0.013 mmol) of Example 65L, and TFA (0.2 mL), the title compound (4 mg, yield: 73%) was obtained as a solid by a method similar to that in Example 21F.

### (Example 66)

### 2-{7-[(3R,5R)-1-ethyl-5-fluoropiperidin-3-yl]-6,7-dihydro-5H-pyrrolo[2,3-c]pyridazin-3-yl}-3-methyl-5-(trifluoromethyl)phenol

Using 2-{7-[(3R,5R)-5-fluoropiperidin-3-yl]-6,7-dihydro-5H-pyrrolo[2,3-c]pyridazin-3-yl}-3-methyl-5-(trifluoromethyl)phenol (22.0 mg, 0.056 mmol) of Example 60C, acetaldehyde (0.030 mL, 0.60 mmol) and NaBH(OAc)₃ (158.2 mg, 0.75 mmol), the title compound (10.0 mg, yield: 43%) was obtained as a solid by a method similar to that in Example 1G.

### (Example 67)

### 3-[4-(difluoromethoxy)-2-hydroxy-6-methylphenyl]-7-[(3R)-1-ethylpiperidin-3-yl]-7H-pyrrolo[2,3-c]pyridazine-5-carbonitrile

### (67A) tert-butyl (3R)-3-(3-chloro-5-iodo-7H-pyrrolo[2,3-c]pyridazin-7-yl)piperidine-1-carboxylate

tert-Butyl (3R)-3-(3-chloro-7H-pyrrolo[2,3-c]pyridazin-7-yl)piperidine-1-carboxylate (228 mg, 0.68 mmol) of Example 2A was dissolved in DCE (6.7 mL), N-iodosuccinimide (183 mg, 0.81 mmol) was added, and the mixture was stirred at 80°C for 1 hr. To the reaction solution was added saturated aqueous sodium bicarbonate solution at room temperature, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-50/50(V/V)] to give the title compound (261 mg, yield: 83%) as an amorphous form.

### (67B) tert-butyl (3R)-3-(3-chloro-5-cyano-7H-pyrrolo[2,3-c]pyridazin-7-yl)piperidine-1-carboxylate

tert-Butyl (3R)-3-(3-chloro-5-iodo-7H-pyrrolo[2,3-c]pyridazin-7-yl)piperidine-1-carboxylate (548 mg, 1.2 mmol) of Example 67A was dissolved in DMF (11 mL), then Pd(PPh₃)₄ (273 mg, 0.24 mmol) and zinc cyanide (321 mg, 2.6 mmol) were added, and the mixture was stirred at 100°C for 2 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =70/30-0/100(V/V)] to give the title compound (284 mg, yield: 66%) as an amorphous form.

### (67C) tert-butyl (3R)-3-{5-cyano-3-[4-(difluoromethoxy)-2-(methoxymethoxy)-6-methylphenyl]-7H-pyrrolo[2,3-c]pyridazin-7-yl}piperidine-1-carboxylate

Using tert-butyl (3R)-3-(3-chloro-5-cyano-7H-pyrrolo[2,3-c]pyridazin-7-yl)piperidine-1-carboxylate (0.141 g, 0.39 mmol) of Example 67B, 2-[4-(difluoromethoxy)-2-(methoxymethoxy)-6-methylphenyl]-4,4,5,5-tetramethyl-1,3,2-dioxaborolane (0.160 g, 0.47 mmol) of Example 13F, potassium carbonate (0.107 g, 0.77 mmol) and XPhos Pd G2 (0.061 g, 0.078 mmol), the title compound (0.156 g, yield: 74%) was obtained as an amorphous form by a method similar to that in Example 1E.

### (67D) 3-[4-(difluoromethoxy)-2-hydroxy-6-methylphenyl]-7-[(3R)-piperidin-3-yl]-7H-pyrrolo[2,3-c]pyridazine-5-carbonitrile

Using tert-butyl (3R)-3-{5-cyano-3-[4-(difluoromethoxy)-2-(methoxymethoxy)-6-methylphenyl]-7H-pyrrolo[2,3-c]pyridazin-7-yl}piperidine-1-carboxylate (0.156 g, 0.29 mmol) of Example 67C, and TFA (1 mL), a crude product (0.131 g, yield: quantitative) of the title compound was obtained as a solid by a method similar to that in Example 64B.

### (67E) 3-[4-(difluoromethoxy)-2-hydroxy-6-methylphenyl]-7-[(3R)-1-ethylpiperidin-3-yl]-7H-pyrrolo[2,3-c]pyridazine-5-carbonitrile

Using 3-[4-(difluoromethoxy)-2-hydroxy-6-methylphenyl]-7-[(3R)-piperidin-3-yl]-7H-pyrrolo[2,3-c]pyridazine-5-carbonitrile (71 mg, 0.18 mmol) of Example 67D, acetaldehyde (0.50 mL, 10 mmol) and NaBH(OAc)₃ (0.18 g, 0.85 mmol), the title compound (33 mg, yield: 43%) was obtained as a solid by a method similar to that in Example 1G.

### (Example 68)

### 3-[4-(difluoromethoxy)-2-hydroxy-6-methylphenyl]-7-[(3R)-1-(propan-2-yl)piperidin-3-yl]-7H-pyrrolo[2,3-c]pyridazine-5-carbonitrile

Using 3-[4-(difluoromethoxy)-2-hydroxy-6-methylphenyl]-7-[(3R)-piperidin-3-yl]-7H-pyrrolo[2,3-c]pyridazine-5-carbonitrile (60 mg, 0.15 mmol) of Example 67D, acetone (0.55 mL, 7.5 mmol), NaBH(OAc)₃ (0.16 g, 0.75 mmol) and acetic acid (0.018 mL, 0.31 mmol), the title compound (28 mg, yield: 42%) was obtained as a solid by a method similar to that in Example 1G.

### (Example 69)

### 5-(1,1-difluoroethyl)-2-{7-[(1s,3s)-3-hydroxy-3-methylcyclobutyl]-5-methyl-7H-pyrrolo[2,3-c]pyridazin-3-yl}-3-methylphenol (also referred to as 5-(1,1-difluoroethyl)-2-[7-(cis-3-hydroxy-3-methylcyclobutyl)-5-methyl-7H-pyrrolo[2,3-c]pyridazin-3-yl]-3-methylphenol)

### (69A) 1-[3-(methoxymethoxy)-5-methylphenyl]ethanone

Using 1-(3-hydroxy-5-methylphenyl)ethanone (8 g, 53 mmol), cesium carbonate (34.7 g, 110 mmol) and chloromethyl methyl ether (10.5 mL, 140 mmol), the title compound (6.5 g, yield: 63%) was obtained as an oil by a method similar to that in Example 1A.

### (69B) 3-methyl-5-(2-methyl-1,3-dithiolan-2-yl)phenol

1-[3-(Methoxymethoxy)-5-methylphenyl]ethanone (1.8 g, 9.3 mmol) of Example 69A was dissolved in DCE (20 mL), then ethane-1,2-dithiol (2.05 mL, 24 mmol) and boron trifluoride diethyl ether complex (2.35 mL, 19 mmol) were added in the nitrogen-substituted system at 0°C, and the mixture was stirred at 70°C for 12 hr. To the reaction solution was added saturated aqueous sodium bicarbonate solution at 0°C, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: petroleum ether/ethyl acetate =100/0-90/10(V/V)] to give the title compound (1.6 g, yield: 76%) as an oil.

### (69C) 2-[3-(methoxymethoxy)-5-methylphenyl]-2-methyl-1,3-dithiolane

Using 3-methyl-5-(2-methyl-1,3-dithiolan-2-yl)phenol (6.5 g, 29 mmol) of Example 69B, cesium carbonate (19 g, 58 mmol) and chloromethyl methyl ether (7.3 mL, 96 mmol), the title compound (5 g, yield: 64%) was obtained as an oil by a method similar to that in Example 1A.

### (69D) 1-(1,1-difluoroethyl)-3-(methoxymethoxy)-5-methylbenzene

N-Iodosuccinimide (2.0 g, 9.1 mmol) was dissolved in DCM (8 mL), pyridine hydrogen fluoride (0.62 mL, 4.5 mmol) was added at -30°C, and the mixture was stirred at -30°C for 30 min. Then, a solution of 2-[3-(methoxymethoxy)-5-methylphenyl]-2-methyl-1,3-dithiolane (600 mg, 2.2 mmol) of Example 69C in DCM (5 mL) was added, and the mixture was stirred at -30°C for 2 hr. To the reaction solution was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by preparative thin layer silica gel column chromatography [eluent: petroleum ether/ethyl acetate =90/10(V/V)] to give the title compound (400 mg, yield: 83%) as an oil.

### (69E) 3-(1,1-difluoroethyl)-5-methylphenol

Using 1-(1,1-difluoroethyl)-3-(methoxymethoxy)-5-methylbenzene (3.6 g, 17 mmol) of Example 69D, and 6 M hydrochloric acid (10 mL), the title compound (2.8 g, yield: 98%) was obtained as an oil by a method similar to that in Example 3D.

### (69F) 5-(1,1-difluoroethyl)-2-iodo-3-methylphenol

Using 3-(1,1-difluoroethyl)-5-methylphenol (320 mg, 1.9 mmol) of Example 69E, 60% sodium hydride (160 mg, 4.0 mmol) and iodine (236 mg, 0.93 mmol), the title compound (320 mg, yield: 58%) was obtained as an oil by a method similar to that in Example 9A.

### (69G) 5-(1,1-difluoroethyl)-2-iodo-1-(methoxymethoxy)-3-methylbenzene

Using 5-(1,1-difluoroethyl)-2-iodo-3-methylphenol (470 mg, 1.6 mmol) of Example 69F, cesium carbonate (1.03 g, 3.2 mmol) and chloromethyl methyl ether (0.31 mL, 4.1 mmol), the title compound (490 mg, yield: 91%) was obtained as an oil by a method similar to that in Example 1A.

### (69H) 2-[4-(1,1-difluoroethyl)-2-(methoxymethoxy)-6-methylphenyl]-4,4,5,5-tetramethyl-1,3,2-dioxaborolane

5-(1,1-Difluoroethyl)-2-iodo-1-(methoxymethoxy)-3-methylbenzene (1 g, 2.9 mmol) of Example 69G was dissolved in 1,4-dioxane (20 mL), then TEA (0.82 mL, 5.9 mmol), palladium(II) acetate (71 mg, 0.31 mmol), 2-(dicyclohexylphosphino)biphenyl (112 mg, 0.32 mmol) and 4,4,5,5-tetramethyl-1,3,2-dioxaborolane (1.13 g, 8.9 mmol) were added in the nitrogen-substituted system at room temperature, and the mixture was stirred at 80°C for 12 hr. The reaction solution was concentrated under reduced pressure, and the obtained residue was purified by silica gel column chromatography [eluent: n-hexane/ethyl acetate =100/0-90/10(V/V)] to give the title compound (157 mg, yield: 15%) as an oil.

### (69I) (1s,3s)-3-{3-[4-(1,1-difluoroethyl)-2-(methoxymethoxy)-6-methylphenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (0.050 g, 0.20 mmol) of Example 4C, 2-[4-(1,1-difluoroethyl)-2-(methoxymethoxy)-6-methylphenyl]-4,4,5,5-tetramethyl-1,3,2-dioxaborolane (0.086 g, 0.25 mmol) of Example 69H, potassium carbonate (0.061 g, 0.44 mmol) and XPhos Pd G2 (0.034 g, 0.043 mmol), the title compound (0.035 g, yield: 41%) was obtained as an oil by a method similar to that in Example 1E.

### (69J) 5-(1,1-difluoroethyl)-2-{7-[(1s,3s)-3-hydroxy-3-methylcyclobutyl]-5-methyl-7H-pyrrolo[2,3-c]pyridazin-3-yl}-3-methylphenol

Using (1s,3s)-3-{3-[4-(1,1-difluoroethyl)-2-(methoxymethoxy)-6-methylphenyl]-5-methyl-7H-pyrrolo[2,3-c]pyridazin-7-yl}-1-methylcyclobutanol (35 mg, 0.081 mmol) of Example 69I, and 4 M hydrogen chloride-1,4-dioxane (0.8 mL), the title compound (29 mg, yield: 92%) was obtained as a solid by a method similar to that in Example 3D.

### (Example 70)

### 4-{3-[2-hydroxy-6-methyl-4-(trifluoromethyl)phenyl]-5-methyl-7H-pyrrolo[2,3-c]pyridazin-7-yl}bicyclo[2.1.1]hexan-1-ol

### (70A) 4-(3-chloro-5,6-dihydro-7H-pyrrolo[2,3-c]pyridazin-7-yl)bicyclo[2.1.1]hexan-1-ol

Using 3,6-dichloro-4-ethenylpyridazine (500 mg, 2.9 mmol) of Example 1C, 4-aminobicyclo[2.1.1]hexanol hydrochloride (450 mg, 3.0 mmol) and DIPEA (2.0 mL, 11 mmol), the title compound (455 mg, yield 63%) was obtained as a solid by a method similar to that in Example 1D.

### (70B) 7-(4-{[tert-butyl(dimethyl)silyl]oxy}bicyclo[2.1.1]hexan-1-yl)-3-chloro-6,7-dihydro-5H-pyrrolo[2,3-c]pyridazine

4-(3-Chloro-5,6-dihydro-7H-pyrrolo[2,3-c]pyridazin-7-yl)bicyclo[2.1.1]hexan-1-ol (455 mg, 1.8 mmol) of Example 70A was dissolved in DMF (9.0 mL), then imidazole (615 mg, 9.0 mmol) and tert-butyldimethylsilyl chloride (1.1 g, 7.2 mmol) were added, and the mixture was stirred at room temperature for 2.5 hr. To the reaction solution was added water at 0°C, and the mixture was subjected to extraction with ethyl acetate. The organic layer was washed with water and saturated brine, and dried over anhydrous sodium sulfate. After filtration, the filtrate was concentrated under reduced pressure, and the obtained residue was purified by silica gel column chromatography [eluent: n-hexane/ethyl acetate =90/10-70/30(V/V)] to give the title compound (596 mg, yield: 90%) as a solid.

### (70C) 7-(4-{[tert-butyl(dimethyl)silyl]oxy}bicyclo[2.1.1]hexan-1-yl)-3-chloro-7H-pyrrolo[2,3-c]pyridazine

Using 7-(4-{[tert-butyl(dimethyl)silyl]oxy}bicyclo[2.1.1]hexan-1-yl)-3-chloro-6,7-dihydro-5H-pyrrolo[2,3-c]pyridazine (596 mg, 1.6 mmol) of Example 70B, and DDQ (443 mg, 2.0 mmol), the title compound (526 mg, yield: 89%) was obtained as an oil by a method similar to that in Example 2A.

### (70D) 7-(4-{[tert-butyl(dimethyl)silyl]oxy}bicyclo[2.1.1]hexan-1-yl)-3-[2-(methoxymethoxy)-6-methyl-4-(trifluoromethyl)phenyl]-7H-pyrrolo[2,3-c]pyridazine

Using 7-(4-{[tert-butyl(dimethyl)silyl]oxy}bicyclo[2.1.1]hexan-1-yl)-3-chloro-7H-pyrrolo[2,3-c]pyridazine (282 mg, 0.77 mmol) of Example 70C, 2-[2-(methoxymethoxy)-6-methyl-4-(trifluoromethyl)phenyl]-4,4,5,5-tetramethyl-1,3,2-dioxaborolane (295 mg, 0.85 mmol) of Example 1B, potassium carbonate (214 mg, 1.5 mmol) and XPhos Pd G2 (121 mg, 0.15 mmol), the title compound (374 mg, yield: 88%) was obtained as an oil by a method similar to that in Example 1E.

### (70E) 5-bromo-7-(4-{[tert-butyl(dimethyl)silyl]oxy}bicyclo[2.1.1]hexan-1-yl)-3-[2-(methoxymethoxy)-6-methyl-4-(trifluoromethyl)phenyl]-7H-pyrrolo[2,3-c]pyridazine

7-(4-{[tert-Butyl(dimethyl)silyl]oxy}bicyclo[2.1.1]hexan-1-yl)-3-[2-(methoxymethoxy)-6-methyl-4-(trifluoromethyl)phenyl]-7H-pyrrolo[2,3-c]pyridazine (173 mg, 0.32 mmol) of Example 70D was dissolved in DCE (2 mL), N-bromosuccinimide (61 mg, 0.35 mmol) was added, and the mixture was stirred at room temperature for 2 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-80/20(V/V)] to give the title compound (135 mg, yield: 68%) as an amorphous form.

### (70F) 7-(4-{[tert-butyl(dimethyl)silyl]oxy}bicyclo[2.1.1]hexan-1-yl)-3-[2-(methoxymethoxy)-6-methyl-4-(trifluoromethyl)phenyl]-5-methyl-7H-pyrrolo[2,3-c]pyridazine

5-Bromo-7-(4-{[tert-butyl(dimethyl)silyl]oxy}bicyclo[2.1.1]hexan-1-yl)-3-[2-(methoxymethoxy)-6-methyl-4-(trifluoromethyl)phenyl]-7H-pyrrolo[2,3-c]pyridazine (135 mg, 0.22 mmol) of Example 70E, methylboronic acid (38 mg, 0.65 mmol) and potassium carbonate (59 mg, 0.43 mmol) were dissolved in a mixed solvent of 1,4-dioxane (2.0 mL) and water (0.7 mL), XPhos Pd G2 (33 mg, 0.043 mmol) was added, and the mixture was stirred in the nitrogen-substituted system at 110°C for 1 hr. The reaction solution was allowed to room temperature, water was added, and the mixture was subjected to extraction with ethyl acetate. The organic layer was washed with saturated brine, and dried over anhydrous sodium sulfate. After filtration, the solvent was evaporated under reduced pressure, and the obtained residue was purified by NH silica gel column chromatography [eluent: n-hexane/ethyl acetate =90/10-72/28(V/V)] to give the title compound (73 mg, yield: 60%) as an amorphous form.

### (70G) 4-{3-[2-hydroxy-6-methyl-4-(trifluoromethyl)phenyl]-5-methyl-7H-pyrrolo[2,3-c]pyridazin-7-yl}bicyclo[2.1.1]hexan-1-ol

Using 7-(4-{[tert-butyl(dimethyl)silyl]oxy}bicyclo[2.1.1]hexan-1-yl)-3-[2-(methoxymethoxy)-6-methyl-4-(trifluoromethyl)phenyl]-5-methyl-7H-pyrrolo[2,3-c]pyridazine (73 mg, 0.13 mmol) of Example 70F, and TFA (0.7 mL), the title compound (24 mg, yield: 46%) was obtained as a solid by a method similar to that in Example 21F.

### (Example 71)

### 5-(difluoromethoxy)-2-{7-[(3R)-1-ethylpiperidin-3-yl]-5-methyl-7H-pyrrolo[2,3-c]pyridazin-3-yl}-3-methylphenol

### (71A) tert-butyl (3R)-3-{3-[4-(difluoromethoxy)-2-(methoxymethoxy)-6-methylphenyl]-5-methyl-7H-pyrrolo[2,3-c]pyridazin-7-yl}piperidine-1-carboxylate

Using tert-butyl (3R)-3-(3-chloro-5-methyl-7H-pyrrolo[2,3-c]pyridazin-7-yl)piperidine-1-carboxylate (0.153 g, 0.44 mmol) of Example 20B, 2-[4-(difluoromethoxy)-2-(methoxymethoxy)-6-methylphenyl]-4,4,5,5-tetramethyl-1,3,2-dioxaborolane (0.180 g, 0.52 mmol) of Example 13F, potassium carbonate (0.120 g, 0.87 mmol) and XPhos Pd G2 (0.070 g, 0.089 mmol), the title compound (0.183 g, yield: 79%) was obtained as an amorphous form by a method similar to that in Example 1E.

### (71B) 5-(difluoromethoxy)-2-{7-[(3R)-1-ethylpiperidin-3-yl]-5-methyl-7H-pyrrolo[2,3-c]pyridazin-3-yl}-3-methylphenol

Using tert-butyl (3R)-3-{3-[4-(difluoromethoxy)-2-(methoxymethoxy)-6-methylphenyl]-5-methyl-7H-pyrrolo[2,3-c]pyridazin-7-yl}piperidine-1-carboxylate (0.183 g, 0.34 mmol) of Example 71A, 4 M hydrogen chloride-1,4-dioxane (3.5 mL), acetaldehyde (0.20 mL, 4.0 mmol) and NaBH(OAc)₃ (0.374 g, 1.8 mmol), the title compound (0.128 g, yield: 89%) was obtained as a solid by a method similar to that in Example 20D.

### (Example 72)

### 5-(difluoromethoxy)-2-{7-[(1s,3s)-3-hydroxy-3-methylcyclobutyl]-5-methoxy-7H-pyrrolo[2,3-c]pyridazin-3-yl}-3-methylphenol (also referred to as 5-(difluoromethoxy)-2-[7-(cis-3-hydroxy-3-methylcyclobutyl)-5-methoxy-7H-pyrrolo[2,3-c]pyridazin-3-yl]-3-methylphenol)

### (72A) 7-((1s,3s)-3-{[tert-butyl(dimethyl)silyl]oxy}-3-methylcyclobutyl)-3-[4-(difluoromethoxy)-2-(methoxymethoxy)-6-methylphenyl]-5-methoxy-7H-pyrrolo[2,3-c]pyridazine

Using 7-((1s,3s)-3-{[tert-butyl(dimethyl)silyl]oxy}-3-methylcyclobutyl)-3-chloro-5-methoxy-7H-pyrrolo[2,3-c]pyridazine (0.150 g, 0.39 mmol) of Example 28A, 2-[4-(difluoromethoxy)-2-(methoxymethoxy)-6-methylphenyl]-4,4,5,5-tetramethyl-1,3,2-dioxaborolane (0.162 g, 0.47 mmol) of Example 13F, potassium carbonate (0.111 g, 0.80 mmol) and XPhos Pd G2 (0.068 g, 0.086 mmol), the title compound (0.092 g, yield: 42%) was obtained as an oil by a method similar to that in Example 1E.

### (72B) 5-(difluoromethoxy)-2-{7-[(1s,3s)-3-hydroxy-3-methylcyclobutyl]-5-methoxy-7H-pyrrolo[2,3-c]pyridazin-3-yl}-3-methylphenol

Using 7-((1s,3s)-3-{[tert-butyl(dimethyl)silyl]oxy}-3-methylcyclobutyl)-3-[4-(difluoromethoxy)-2-(methoxymethoxy)-6-methylphenyl]-5-methoxy-7H-pyrrolo[2,3-c]pyridazine (0.092 g, 0.16 mmol) of Example 72A, and TFA (0.5 mL), the title compound (0.036 g, yield: 54%) was obtained as a solid by a method similar to that in Example 21F.

### (Example 73)

### 7-[(1R,2R,5S or 1S,2S,5R)-8-ethyl-8-azabicyclo[3.2.1]octan-2-yl]-3-[2-hydroxy-6-methyl-4-(trifluoromethyl)phenyl]-7H-pyrrolo[2,3-c]pyridazine-5-carbonitrile

### (73A) tert-butyl (1S*,2S*,5S*)-2-(3-chloro-7H-pyrrolo[2,3-c]pyridazin-7-yl)-8-azabicyclo[3.2.1]octane-8-carboxylate

Using tert-butyl (1S*,2S*,5S*)-2-(3-chloro-5,6-dihydro-7H-pyrrolo[2,3-c]pyridazin-7-yl)-8-azabicyclo[3.2.1]octane-8-carboxylate (200 mg, 0.55 mmol) of Example 17E, and manganese dioxide (1.15 g, 13 mmol), the title compound (121 mg, yield: 61%) was obtained as a racemate (a solid) by a method similar to that in Example 4C.

### (73B) tert-butyl (1S*,2S*,5S*)-2-{3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate

Using tert-butyl (1S*,2S*,5S*)-2-(3-chloro-7H-pyrrolo[2,3-c]pyridazin-7-yl)-8-azabicyclo[3.2.1]octane-8-carboxylate (121 mg, 0.33 mmol) of Example 73A, 2-[2-(methoxymethoxy)-6-methyl-4-(trifluoromethyl)phenyl]-4,4,5,5-tetramethyl-1,3,2-dioxaborolane (164 mg, 0.47 mmol) of Example 1B, XPhos Pd G2 (26 mg, 0.033 mmol) and potassium carbonate (92 mg, 0.67 mmol), the title compound (184 mg, yield: quantitative) was obtained as a racemate (a solid) by a method similar to that in Example 9D.

### (73C) tert-butyl (1S*,2S*,5S*)-2-{5-bromo-3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate

To a solution of tert-butyl (1S*,2S*,5S*)-2-{3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate (184 mg, 0.34 mmol) of Example 73B in DCE (3.4 mL) was added N-bromosuccinimide (63 mg, 0.35 mmol) at 0°C, and the mixture was stirred at room temperature for 30 min. To the reaction solution were added DCM and saturated aqueous sodium bicarbonate solution at 0°C, and the mixture was stirred. The organic layer was separated through a phase separator (Biotage), and concentrated under reduced pressure, and the obtained residue was purified by silica gel column chromatography [eluent: n-hexane/ethyl acetate =80/20-50/50(V/V)] to give the title compound (163 mg, yield: 77%) as a racemate (a solid).

### (73D) tert-butyl (1R,2R,5R or 1S,2S,5S)-2-{5-bromo-3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate

tert-Butyl (1S*,2S*,5S*)-2-{5-bromo-3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate (163 mg, 0.26 mmol) of Example 73C was subjected to chiral HPLC [column: CHIRALPAK IE (20 mm I.D.× 250 mm), mobile phase: n-hexane/2-propanol=80/20(V/V), temperature: 40°C] to give the title compound eluting later (80.4 mg, yield: 49%) as a single enantiomer (a solid).

### (73E) tert-butyl (1R,2R,5R or 1S,2S,5S)-2-{5-cyano-3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate

A mixture of tert-butyl (1R,2R,5R or 1S,2S,5S)-2-{5-bromo-3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate (47.0 mg, 0.076 mmol) of Example 73D, tBuXPhos Pd G1 (5.2 mg, 0.0076 mmol), tBuXPhos (3.5 mg, 0.0082 mmol), potassium hexacyanoferrate(II) trihydrate (16.7 mg, 0.039 mmol), potassium acetate (2.0 mg, 0.020 mmol), 1,4-dioxane (0.5 mL) and water (0.5 mL) was stirred in the nitrogen-substituted system at 100°C for 40 min. The reaction solution was allowed to room temperature, to the reaction solution were added DCM and water, and the mixture was stirred. The organic layer was separated through a phase separator (Biotage), and concentrated under reduced pressure, and the obtained residue was purified by silica gel column chromatography [eluent: n-hexane/ethyl acetate =75/25-50/50(V/V)] and reverse-phase preparative HPLC [mobile phase: 0.1% formic acid aqueous solution/0.1% formic acid-acetonitrile =42/58-14/86(V/V)] to give the title compound (32.8 mg, yield: 76%) as a single enantiomer (a solid).

### (73F) 7-[(1R,2R,5S or 1S,2S,5R)-8-azabicyclo[3.2.1]octan-2-yl]-3-[2-hydroxy-6-methyl-4-(trifluoromethyl)phenyl]-7H-pyrrolo[2,3-c]pyridazine-5-carbonitrile

Using tert-butyl (1R,2R,5R or 1S,2S,5S)-2-{5-cyano-3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate (32.8 mg, 0.057 mmol) of Example 73E, and 4 M hydrogen chloride-1,4-dioxane solution (1.5 mL), the title compound (7.7 mg, yield: 31%) was obtained as a single enantiomer (a solid) by a method similar to that in Example 1F.

### (73G) 7-[(1R,2R,5S or 1S,2S,5R)-8-ethyl-8-azabicyclo[3.2.1]octan-2-yl]-3-[2-hydroxy-6-methyl-4-(trifluoromethyl)phenyl]-7H-pyrrolo[2,3-c]pyridazine-5-carbonitrile

Using 7-[(1R,2R,5S or 1S,2S,5R)-8-azabicyclo[3.2.1]octan-2-yl]-3-[2-hydroxy-6-methyl-4-(trifluoromethyl)phenyl]-7H-pyrrolo[2,3-c]pyridazine-5-carbonitrile (7.7 mg, 0.018 mmol) of Example 73F, acetaldehyde (4 drops) and NaBH(OAc)₃ (26 mg, 0.13 mmol), the title compound (5.9 mg, yield: 72%) was obtained as a single enantiomer (a solid) by a method similar to that in Example 1G.

### (Example 74)

### 3-[4-(difluoromethoxy)-2-hydroxy-6-methylphenyl]-7-[(1s,3s)-3-hydroxy-3-methylcyclobutyl]-7H-pyrrolo[2,3-c]pyridazine-5-carbonitrile (also referred to as 3-[4-(difluoromethoxy)-2-hydroxy-6-methylphenyl]-7-(cis-3-hydroxy-3-methylcyclobutyl)-7H-pyrrolo[2,3-c]pyridazine-5-carbonitrile)

### (74A) 3-chloro-5-iodo-7-{(1s,3s)-3-[(4-methoxybenzyl)oxy]-3-methylcyclobutyl}-7H-pyrrolo[2,3-c]pyridazine

Using 3-chloro-7-{(1s,3s)-3-[(4-methoxybenzyl)oxy]-3-methylcyclobutyl}-7H-pyrrolo[2,3-c]pyridazine (1.49 g, 4.2 mmol) of Example 5A, and N-iodosuccinimide (1.13 g, 5.0 mmol), the title compound (1.81 g, yield: 90%) was obtained as a solid by a method similar to that in Example 16E.

### (74B) 3-chloro-7-{(1s,3s)-3-[(4-methoxybenzyl)oxy]-3-methylcyclobutyl}-7H-pyrrolo[2,3-c]pyridazine-5-carbonitrile

3-Chloro-5-iodo-7-{(1s,3s)-3-[(4-methoxybenzyl)oxy]-3-methylcyclobutyl}-7H-pyrrolo[2,3-c]pyridazine (0.100 g, 0.21 mmol) of Example 74A was dissolved in DMF (2 mL), then zinc cyanide (0.050 g, 0.43 mmol) and Pd(PPh₃)₄ (0.049 g, 0.042 mmol) were added, and the mixture was stirred in the nitrogen-substituted system at 100°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-30/70(V/V)] to give the title compound (0.062 g, yield: 78%) as a solid.

### (74C) 3-[4-(difluoromethoxy)-2-(methoxymethoxy)-6-methylphenyl]-7-{(1s,3s)-3-[(4-methoxybenzyl)oxy]-3-methylcyclobutyl}-7H-pyrrolo[2,3-c]pyridazine-5-carbonitrile

Using 3-Chloro-7-{(1s,3s)-3-[(4-methoxybenzyl)oxy]-3-methylcyclobutyl}-7H-pyrrolo[2,3-c]pyridazine-5-carbonitrile (0.062 g, 0.16 mmol) of Example 74B, 2-[4-(difluoromethoxy)-2-(methoxymethoxy)-6-methylphenyl]-4,4,5,5-tetramethyl-1,3,2-dioxaborolane (0.073 g, 0.21 mmol) of Example 13F, potassium carbonate (0.051 g, 0.37 mmol) and XPhos Pd G2 (0.025 g, 0.032 mmol), the title compound (0.033 g, yield: 36%) was obtained as an oil by a method similar to that in Example 1E.

### (74D) 3-[4-(difluoromethoxy)-2-hydroxy-6-methylphenyl]-7-[(1s,3s)-3-hydroxy-3-methylcyclobutyl]-7H-pyrrolo[2,3-c]pyridazine-5-carbonitrile

Using 3-[4-(difluoromethoxy)-2-(methoxymethoxy)-6-methylphenyl]-7-{(1s,3s)-3-[(4-methoxybenzyl)oxy]-3-methylcyclobutyl}-7H-pyrrolo[2,3-c]pyridazine-5-carbonitrile (0.033 g, 0.058 mmol) of Example 74C, and TFA (0.5 mL), the title compound (0.017 g, yield: 73%) was obtained as a solid by a method similar to that in Example 5D.

### (Example 75)

### 3-ethyl-5-{3-[2-hydroxy-6-methyl-4-(trifluoromethyl)phenyl]-5-methyl-7H-pyrrolo[2,3-c]pyridazin-7-yl}-3-azabicyclo[3.1.1]heptan-1-ol

### (75A) (1s,3s)-3-amino-1,3-bis(hydroxymethyl)cyclobutanol monohydrochloride

Using tert-butyl [(1s,3s)-3-hydroxy-1,3-bis(hydroxymethyl)cyclobutyl]carbamate (813 mg, 3.3 mmol) of Example 65C, and 4 M hydrogen chloride-1,4-dioxane (10 mL), a crude product (603 mg, yield: quantitative) of the title compound was obtained as a solid by a method similar to that in Example 2B.

### (75B) benzyl [(1s,3s)-3-hydroxy-1,3-bis(hydroxymethyl)cyclobutyl]carbamate

(1s,3s)-3-Amino-1,3-bis(hydroxymethyl)cyclobutanol monohydrochloride (603 mg, 3.3 mmol) of Example 75A was dissolved in a mixed solvent of THF (16 mL) and water (8 mL), then sodium hydrogencarbonate (827 mg, 9.9 mmol) and benzyl chloroformate (0.536 mL, 3.8 mmol) were added, and the mixture was stirred at room temperature for 3 hr. To the reaction solution was added water at 0°C, and the mixture was subjected to extraction with chloroform/2-propanol. The organic layer was washed with water and saturated brine, and dried over anhydrous sodium sulfate. After filtration, the filtrate was concentrated under reduced pressure, and the obtained residue was purified by silica gel column chromatography [eluent: ethyl acetate/methanol=95/5-85/15(V/V)] to give the title compound (880 mg, yield: 95%) as an amorphous form.

### (75C) ((1s,3s)-1-{[(benzyloxy)carbonyl]amino}-3-hydroxycyclobutane-1,3-diyl)dimethanediyl bis(4-methylbenzenesulfonate)

Benzyl [(1s,3s)-3-hydroxy-1,3-bis(hydroxymethyl)cyclobutyl]carbamate (940 mg, 3.3 mmol) of Example 75B was dissolved in pyridine (11 mL), p-toluenesulfonyl chloride (1.9 g, 10 mmol) was added at 0°C, and the mixture was stirred at room temperature for 2.5 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =58/42-40/60(V/V)] to give the title compound (1.68 g, yield: 85%) as a solid.

### (75D) benzyl (5-hydroxy-3-azabicyclo[3.1.1]heptan-1-yl)carbamate

((1s,3s)-1-{[(Benzyloxy)carbonyl]amino}-3-hydroxycyclobutane-1,3-diyl)dimethanediyl bis(4-methylbenzenesulfonate) (1.68 g, 2.9 mmol) of Example 75C was dissolved in acetonitrile (14 mL), 28% ammonia aqueous solution (7.0 mL, 105 mmol) was added, and the mixture was stirred 80°C for 3.5 hr. The reaction solution was concentrated under reduced pressure, and the obtained residue was purified by NH silica gel column chromatography [eluent: DCM/methanol=97/3-80/20(V/V)] to give the title compound (375 mg, yield: 50%) as a solid.

### (75E) tert-butyl 1-{[(benzyloxy)carbonyl]amino}-5-hydroxy-3-azabicyclo[3.1.1]heptane-3-carboxylate

Benzyl (5-hydroxy-3-azabicyclo[3.1.1]heptan-1-yl)carbamate (375 mg, 1.4 mmol) of Example 75D was dissolved in a mixed solvent of THF (7.0 mL) and water (3.5 mL), then sodium hydrogencarbonate (360 mg, 4.3 mmol) and di-tert-butyl dicarbonate (343 mg, 1.6 mmol) were added, and the mixture was stirred at room temperature for 1 hr. To the reaction solution was added water, and the mixture was subjected to extraction with ethyl acetate. The organic layer was washed with water and saturated brine, and dried over anhydrous sodium sulfate. After filtration, the filtrate was concentrated under reduced pressure, and the obtained residue was purified by silica gel column chromatography [eluent: n-hexane/ethyl acetate =50/50-33/67(V/V)] to give the title compound (465 mg, yield: 90%) as an amorphous form.

### (75F) tert-butyl 1-{[(benzyloxy)carbonyl]amino}-5-(methoxymethoxy)-3-azabicyclo[3.1.1]heptane-3-carboxylate

Using tert-butyl 1-{[(benzyloxy)carbonyl]amino}-5-hydroxy-3-azabicyclo[3.1.1]heptane-3-carboxylate (465 mg, 1.3 mmol) of Example 75E, DIPEA (1.6 mL, 9.0 mmol) and chloromethyl methyl ether (0.443 mL, 5.9 mmol), the title compound (413 mg, yield: 79%) was obtained as an oil by a method similar to that in Example 1A.

### (75G) tert-butyl 1-amino-5-(methoxymethoxy)-3-azabicyclo[3.1.1]heptane-3-carboxylate

tert-Butyl 1-{[(benzyloxy)carbonyl]amino}-5-(methoxymethoxy)-3-azabicyclo[3.1.1]heptane-3-carboxylate (413 mg, 1.0 mmol) of Example 75F was dissolved in methanol (5.0 mL), ASCA-2 (100 mg) was added, and the mixture was stirred in the hydrogen-substituted system at room temperature for 70 min. The reaction solution was filtered through Celite, and the filtrate was concentrated under reduced pressure to give a crude product of the title compound as an oil.

### (75H) tert-butyl 1-(3-chloro-5,6-dihydro-7H-pyrrolo[2,3-c]pyridazin-7-yl)-5-(methoxymethoxy)-3-azabicyclo[3.1.1]heptane-3-carboxylate

Using tert-butyl 1-amino-5-(methoxymethoxy)-3-azabicyclo[3.1.1]heptane-3-carboxylate (233 mg, 0.86 mmol) of Example 75G, 3,6-dichloro-4-ethenylpyridazine (358 mg, 2.0 mmol) of Example 1C, and DIPEA (0.447 mL, 2.6 mmol), the title compound (188 mg, yield: 45% in two steps) was obtained as an amorphous form by a method similar to that in Example 1D.

### (75I) tert-butyl 1-(3-chloro-7H-pyrrolo[2,3-c]pyridazin-7-yl)-5-(methoxymethoxy)-3-azabicyclo[3.1.1]heptane-3-carboxylate

Using tert-butyl 1-(3-chloro-5,6-dihydro-7H-pyrrolo[2,3-c]pyridazin-7-yl)-5-(methoxymethoxy)-3-azabicyclo[3.1.1]heptane-3-carboxylate (188 mg, 0.46 mmol) of Example 75H, and DDQ (124 mg, 0.55 mmol), the title compound (91 mg, yield: 49%) was obtained as an amorphous form by a method similar to that in Example 2A.

### (75J) tert-butyl 1-(methoxymethoxy)-5-{3-[2-(methoxymethoxy)-6-methyl-4-(trifluoromethyl)phenyl]-7H-pyrrolo[2,3-c]pyridazin-7-yl}-3-azabicyclo[3.1.1]heptane-3-carboxylate

Using tert-butyl 1-(3-chloro-7H-pyrrolo[2,3-c]pyridazin-7-yl)-5-(methoxymethoxy)-3-azabicyclo[3.1.1]heptane-3-carboxylate (91 mg, 0.22 mmol) of Example 75I, 2-[2-(methoxymethoxy)-6-methyl-4-(trifluoromethyl)phenyl]-4,4,5,5-tetramethyl-1,3,2-dioxaborolane (92 mg, 0.27 mmol) of Example 1B, potassium carbonate (61 mg, 0.45 mmol) and XPhos Pd G2 (35 mg, 0.045 mmol), the title compound (83 mg, yield: 63%) was obtained as a solid by a method similar to that in Example 1E.

### (75K) tert-butyl 1-{5-bromo-3-[2-(methoxymethoxy)-6-methyl-4-(trifluoromethyl)phenyl]-7H-pyrrolo[2,3-c]pyridazin-7-yl}-5-(methoxymethoxy)-3-azabicyclo[3.1.1]heptane-3-carboxylate

tert-Butyl 1-(methoxymethoxy)-5-{3-[2-(methoxymethoxy)-6-methyl-4-(trifluoromethyl)phenyl]-7H-pyrrolo[2,3-c]pyridazin-7-yl}-3-azabicyclo[3.1.1]heptane-3-carboxylate (83 mg, 0.14 mmol) of Example 75J was dissolved in DCE (1.0 mL), N-bromosuccinimide (26 mg, 0.15 mmol) was added, and the mixture was stirred at room temperature for 1.5 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =75/25-50/50(V/V)] to give the title compound (69 mg, yield: 73%) as an amorphous form.

### (75L) tert-butyl 1-(methoxymethoxy)-5-{3-[2-(methoxymethoxy)-6-methyl-4-(trifluoromethyl)phenyl]-5-methyl-7H-pyrrolo[2,3-c]pyridazin-7-yl}-3-azabicyclo[3.1.1]heptane-3-carboxylate

tert-Butyl 1-{5-bromo-3-[2-(methoxymethoxy)-6-methyl-4-(trifluoromethyl)phenyl]-7H-pyrrolo[2,3-c]pyridazin-7-yl}-5-(methoxymethoxy)-3-azabicyclo[3.1.1]heptane-3-carboxylate (68 mg, 0.10 mmol) of Example 75K, methylboronic acid (18 mg, 0.30 mmol) and potassium carbonate (27 mg, 0.20 mmol) were dissolved in a mixed solvent of 1,4-dioxane (1.0 mL) and water (0.3 mL), XPhos Pd G2 (15 mg, 0.020 mmol) was added, and the mixture was stirred in the nitrogen-substituted system at 120°C for 1 hr. The reaction solution was purified by NH silica gel column chromatography [eluent: n-hexane/ethyl acetate =75/25-65/35(V/V)] to give the title compound (43 mg, yield: 70%) as a solid.

### (75M) 5-{3-[2-hydroxy-6-methyl-4-(trifluoromethyl)phenyl]-5-methyl-7H-pyrrolo[2,3-c]pyridazin-7-yl}-3-azabicyclo[3.1.1]heptan-1-ol

Using tert-butyl 1-(methoxymethoxy)-5-{3-[2-(methoxymethoxy)-6-methyl-4-(trifluoromethyl)phenyl]-5-methyl-7H-pyrrolo[2,3-c]pyridazin-7-yl}-3-azabicyclo[3.1.1]heptane-3-carboxylate (43 mg, 0.070 mmol) of Example 75L, and TFA (0.4 mL), the title compound (22 mg, yield: 74%) was obtained as a solid by a method similar to that in Example 64B.

### (75N) 3-ethyl-5-{3-[2-hydroxy-6-methyl-4-(trifluoromethyl)phenyl]-5-methyl-7H-pyrrolo[2,3-c]pyridazin-7-yl}-3-azabicyclo[3.1.1]heptan-1-ol

Using 5-{3-[2-hydroxy-6-methyl-4-(trifluoromethyl)phenyl]-5-methyl-7H-pyrrolo[2,3-c]pyridazin-7-yl}-3-azabicyclo[3.1.1]heptan-1-ol (22 mg, 0.053 mmol) of Example 75M, acetaldehyde (0.030 mL, 0.54 mmol) and NaBH(OAc)₃ (55 mg, 0.26 mmol), the title compound (3.5 mg, yield: 15%) was obtained as a solid by a method similar to that in Example 1G.

### (Example 76)

### 2-{7-[(1R,2R,5S or 1S,2S,5R)-8-ethyl-8-azabicyclo[3.2.1]octan-2-yl]-5-methyl-7H-pyrrolo[2,3-c]pyridazin-3-yl}-3-methyl-5-(trifluoromethyl)phenol

### (76A) tert-butyl (1R,2R,5R or 1S,2S,5S)-2-{3-[2-(methoxymethoxy)-6-methyl-4-(trifluoromethyl)phenyl]-5-methyl-7H-pyrrolo[2,3-c]pyridazin-7-yl}-8-azabicyclo[3.2.1]octane-8-carboxylate

Using tert-butyl (1R,2R,5R or 1S,2S,5S)-2-{5-bromo-3-[2-(methoxymethoxy)-6-methyl-4-(trifluoromethyl)phenyl]-7H-pyrrolo[2,3-c]pyridazin-7-yl}-8-azabicyclo[3.2.1]octane-8-carboxylate (33 mg, 0.053 mmol) of Example 73D, methylboronic acid (9.4 mg, 0.16 mmol), XPhos Pd G2 (8.6 mg, 0.011 mmol) and potassium carbonate (15.1 mg, 0.11 mmol), the title compound (20.8 mg, yield: 70%) was obtained as a single enantiomer (a solid) by a method similar to that in Example 9D.

### (76B) 2-{7-[(1R,2R,5S or 1S,2S,5R)-8-azabicyclo[3.2.1]octan-2-yl]-5-methyl-7H-pyrrolo[2,3-c]pyridazin-3-yl}-3-methyl-5-(trifluoromethyl)phenol

Using tert-butyl (1R,2R,5R or 1S,2S,5S)-2-{3-[2-(methoxymethoxy)-6-methyl-4-(trifluoromethyl)phenyl]-5-methyl-7H-pyrrolo[2,3-c]pyridazin-7-yl}-8-azabicyclo[3.2.1]octane-8-carboxylate (20.8 mg, 0.037 mmol) of Example 76A, and 4 M hydrogen chloride-1,4-dioxane (1.5 mL), the title compound (15.6 mg, yield: quantitative) was obtained as a single enantiomer (a solid) by a method similar to that in Example 1F.

### (76C) 2-{7-[(1R,2R,5S or 1S,2S,5R)-8-ethyl-8-azabicyclo[3.2.1]octan-2-yl]-5-methyl-7H-pyrrolo[2,3-c]pyridazin-3-yl}-3-methyl-5-(trifluoromethyl)phenol

Using 2-{7-[(1R,2R,5S or 1S,2S,5R)-8-azabicyclo[3.2.1]octan-2-yl]-5-methyl-7H-pyrrolo[2,3-c]pyridazin-3-yl}-3-methyl-5-(trifluoromethyl)phenol (15.6 mg, 0.038 mmol) of Example 76B, acetaldehyde (4 drops) and NaBH(OAc)₃ (63 mg, 0.30 mmol), the title compound (8.0 mg, yield: 48%) was obtained as a single enantiomer (a solid) by a method similar to that in Example 1G.

### (Example 77)

### 2-{7-[(3R)-1-ethylpiperidin-3-yl]-5-methoxy-7H-pyrrolo[2,3-c]pyridazin-3-yl}-3-methyl-5-(trifluoromethyl)phenol

### (77A) tert-butyl (3R)-3-[5-(acetyloxy)-3-chloro-7H-pyrrolo[2,3-c]pyridazin-7-yl]piperidine-1-carboxylate

tert-Butyl (3R)-3-(3-chloro-7H-pyrrolo[2,3-c]pyridazin-7-yl)piperidine-1-carboxylate (189 mg, 0.56 mmol) of Example 2A was dissolved in acetic acid (1.4 mL), then palladium(II) acetate (25.4 mg, 0.11 mmol) and diacetoxyiodobenzene (274 mg, 0.84 mmol) were added, and the mixture was stirred in the nitrogen-substituted system at 100°C for 1 hr. The reaction solution was allowed to room temperature, and the solvent was evaporated under reduced pressure. To the obtained residue were added saturated aqueous sodium bicarbonate solution and DCM, the mixture was stirred, and the organic layer was separated through a phase separator (Biotage). The solvent was evaporated under reduced pressure, and the obtained residue was purified by silica gel chromatography [eluent: n-hexane/ethyl acetate =90/10-50/50(V/V)] to give the title compound (94 mg, yield: 42%) as a solid.

### (77B) tert-butyl (3R)-3-(3-chloro-5-hydroxy-7H-pyrrolo[2,3-c]pyridazin-7-yl)piperidine-1-carboxylate

tert-Butyl (3R)-3-[5-(acetyloxy)-3-chloro-7H-pyrrolo[2,3-c]pyridazin-7-yl]piperidine-1-carboxylate (103 mg, 0.26 mmol) of Example 77A was dissolved in methanol (1.3 mL), potassium carbonate (91 mg, 0.65 mmol) was added, and the mixture was stirred at room temperature for 30 min. To the reaction solution were added saturated ammonium chloride aqueous solution and DCM at 0°C, and the mixture was stirred. The organic layer was separated through a phase separator (Biotage), and concentrated under reduced pressure, and the obtained residue was purified by silica gel chromatography [eluent: n-hexane/ethyl acetate =70/30-40/60(V/V)] to give the title compound (78 mg, yield: 85%) as a solid.

### (77C) tert-butyl (3R)-3-(3-chloro-5-methoxy-7H-pyrrolo[2,3-c]pyridazin-7-yl)piperidine-1-carboxylate

tert-Butyl (3R)-3-(3-chloro-5-hydroxy-7H-pyrrolo[2,3-c]pyridazin-7-yl)piperidine-1-carboxylate (78 mg, 0.22 mmol) of Example 77B was dissolved in DMF (1.1 mL), then potassium carbonate (58 mg, 0.42 mmol) and iodomethane (0.021 mL, 0.33 mmol) were added, and the mixture was stirred at room temperature for 1.5 hr. To the reaction solution was added water, and the mixture was subjected to extraction with ethyl acetate. The organic layer was washed with saturated brine, and dried over anhydrous sodium sulfate. After filtration, concentrated under reduced pressure, and the obtained residue was purified by silica gel chromatography [eluent: n-hexane/ethyl acetate =75/25-50/50(V/V)] to give the title compound (63.4 mg, yield: 78%) as a solid.

### (77D) tert-butyl (3R)-3-{5-methoxy-3-[2-(methoxymethoxy)-6-methyl-4-(trifluoromethyl)phenyl]-7H-pyrrolo[2,3-c]pyridazin-7-yl}piperidine-1-carboxylate

Using tert-butyl (3R)-3-(3-chloro-5-methoxy-7H-pyrrolo[2,3-c]pyridazin-7-yl)piperidine-1-carboxylate (63.4 mg, 0.17 mmol) of Example 77C, 2-[2-(methoxymethoxy)-6-methyl-4-(trifluoromethyl)phenyl]-4,4,5,5-tetramethyl-1,3,2-dioxaborolane (81 mg, 0.23 mmol) of Example 1B, XPhos Pd G2 (28 mg, 0.035 mmol) and potassium carbonate (49 mg, 0.35 mmol), the title compound (86.6 mg, yield: 91%) was obtained as a solid by a method similar to that in Example 9D.

### (77E) 2-{5-methoxy-7-[(3R)-piperidin-3-yl]-7H-pyrrolo[2,3-c]pyridazin-3-yl}-3-methyl-5-(trifluoromethyl)phenol

Using tert-butyl (3R)-3-{5-methoxy-3-[2-(methoxymethoxy)-6-methyl-4-(trifluoromethyl)phenyl]-7H-pyrrolo[2,3-c]pyridazin-7-yl}piperidine-1-carboxylate (86.6 mg, 0.16 mmol) of Example 77D, and TFA (1 mL), the title compound (63.9 mg, yield: quantitative) was obtained as a solid by a method similar to that in Example 64B.

### (77F) 2-{7-[(3R)-1-ethylpiperidin-3-yl]-5-methoxy-7H-pyrrolo[2,3-c]pyridazin-3-yl}-3-methyl-5-(trifluoromethyl)phenol

Using 2-{5-methoxy-7-[(3R)-piperidin-3-yl]-7H-pyrrolo[2,3-c]pyridazin-3-yl}-3-methyl-5-(trifluoromethyl)phenol (63.9 mg, 0.16 mmol) of Example 77E, acetaldehyde (5 drops) and NaBH(OAc)₃ (264 mg, 1.3 mmol), the title compound (24.9 mg, yield: 36%) was obtained as obtained as a solid by a method similar to that in Example 1G.

### (Example 78)

### 5-(difluoromethoxy)-2-[5-(difluoromethoxy)-7-[(1s,3s)-3-hydroxy-3-methylcyclobutyl]-7H-pyrrolo[2,3-c]pyridazin-3-yl]-3-methylphenol (also referred to as 5-(difluoromethoxy)-2-[5-(difluoromethoxy)-7-(cis-3-hydroxy-3-methylcyclobutyl)-7H-pyrrolo[2,3-c]pyridazin-3-yl]-3-methylphenol)

### (78A) 7-[(1s,3s)-3-(benzyloxy)-3-methylcyclobutyl]-3-chloro-7H-pyrrolo[2,3-c]pyridazine

(1s,3s)-3-(3-Chloro-7H-pyrrolo[2,3-c]pyridazin-7-yl)-1-methylcyclobutanol (4.0 g, 17 mmol) of Example 3B was dissolved in DMF (42 mL), then 63% sodium hydride (1.0 g, 24 mmol) and benzyl bromide (2.6 mL, 22 mmol) were added at 0°C, and the mixture was stirred at room temperature for 2.5 hr. To the reaction solution was added water at 0°C, and the mixture was subjected to extraction with ethyl acetate. The organic layer was washed successively with water and saturated brine, and dried over anhydrous sodium sulfate. After filtration, the filtrate was concentrated under reduced pressure, and the obtained residue was purified by silica gel column chromatography [eluent: n-hexane/ethyl acetate =80/20-65/35(V/V)] to give the title compound (5.47 g, yield: 99%) as an oil.

### (78B) 7-[(1s,3s)-3-(benzyloxy)-3-methylcyclobutyl]-3-chloro-7H-pyrrolo[2,3-c]pyridazin-5-yl acetate

7-[(1s,3s)-3-(Benzyloxy)-3-methylcyclobutyl]-3-chloro-7H-pyrrolo[2,3-c]pyridazine (5.47 g, 17 mmol) of Example 78A was dissolved in acetic acid (33 mL), then palladium(II) acetate (750 mg, 3.3 mmol) and diacetoxyiodobenzene (8.0 g, 25 mmol) were added, and the mixture was stirred at 100°C for 30 min. The reaction solution was concentrated under reduced pressure, to the obtained residue was added saturated sodium hydrogencarbonate aqueous solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-65/35(V/V)] to give the title compound (2.1 g, yield: 33%) as an oil.

### (78C) 7-[(1s,3s)-3-(benzyloxy)-3-methylcyclobutyl]-3-chloro-7H-pyrrolo[2,3-c]pyridazin-5-ol

7-[(1s,3s)-3-(Benzyloxy)-3-methylcyclobutyl]-3-chloro-7H-pyrrolo[2,3-c]pyridazin-5-yl acetate (2.1 g, 5.4 mmol) of Example 78B was dissolved in methanol (27 mL), potassium carbonate (1.9 g, 14 mmol) was added, and the mixture was stirred at room temperature for 30 min. Saturated ammonium chloride aqueous solution was added at 0°C, and the mixture was subjected to extraction with ethyl acetate. The organic layer was washed with saturated brine, and dried over anhydrous sodium sulfate. After filtration, the filtrate was concentrated under reduced pressure, to the obtained residue was added ethyl acetate, and the solid was collected by filtration to give the title compound (1.4 g, yield: 75%) as a solid.

### (78D) 7-[(1s,3s)-3-(benzyloxy)-3-methylcyclobutyl]-3-chloro-5-(difluoromethoxy)-7H-pyrrolo[2,3-c]pyridazine

7-[(1s,3s)-3-(Benzyloxy)-3-methylcyclobutyl]-3-chloro-7H-pyrrolo[2,3-c]pyridazin-5-ol (400 mg, 1.2 mmol) of Example 78C was dissolved in a mixed solvent of acetonitrile (6 mL) and water (6 mL), then potassium hydroxide (652 mg, 12 mmol) and diethyl (bromodifluoromethyl)phosphonate (0.413 mL, 2.3 mmol) were added, and the mixture was stirred at room temperature for 4 hr. To the reaction solution was added saturated ammonium chloride aqueous solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =82/18-75/25(V/V)] to give the title compound (224 mg, yield: 49%) as an oil.

### (78E) 7-[(1s,3s)-3-(benzyloxy)-3-methylcyclobutyl]-5-(difluoromethoxy)-3-[4-(difluoromethoxy)-2-(methoxymethoxy)-6-methylphenyl]-7H-pyrrolo[2,3-c]pyridazine

Using 7-[(1s,3s)-3-(benzyloxy)-3-methylcyclobutyl]-3-chloro-5-(difluoromethoxy)-7H-pyrrolo[2,3-c]pyridazine (0.109 g, 0.28 mmol) of Example 78D, 2-[4-(difluoromethoxy)-2-(methoxymethoxy)-6-methylphenyl]-4,4,5,5-tetramethyl-1,3,2-dioxaborolane (0.114 g, 0.33 mmol) of Example 13F, potassium carbonate (0.077 g, 0.56 mmol) and XPhos Pd G2 (0.044 g, 0.056 mmol), the title compound (0.150 g, yield: 94%) was obtained as an oil by a method similar to that in Example 1E.

### (78F) (1s,3s)-3-(5-(difluoromethoxy)-3-[4-(difluoromethoxy)-2-(methoxymethoxy)-6-methylphenyl]-7H-pyrrolo[2,3-c]pyridazin-7-yl}-1-methylcyclobutanol

Using 7-[(1s,3s)-3-(benzyloxy)-3-methylcyclobutyl]-5-(difluoromethoxy)-3-[4-(difluoromethoxy)-2-(methoxymethoxy)-6-methylphenyl]-7H-pyrrolo[2,3-c]pyridazine (0.150 g, 0.26 mmol) of Example 78E, and ASCA-2 (0.083 g), the title compound (0.077 g, yield: 61%) was obtained as an oil by a method similar to that in Example 24F.

### (78G) 5-(difluoromethoxy)-2-[5-(difluoromethoxy)-7-[(1s,3s)-3-hydroxy-3-methylcyclobutyl]-7H-pyrrolo[2,3-c]pyridazin-3-yl]-3-methylphenol

Using (1s,3s)-3-{5-(difluoromethoxy)-3-[4-(difluoromethoxy)-2-(methoxymethoxy)-6-methylphenyl]-7H-pyrrolo[2,3-c]pyridazin-7-yl}-1-methylcyclobutanol (0.077 g, 0.16 mmol) of Example 78F, and TFA (0.5 mL), the title compound (0.040 g, yield: 57%) was obtained as a solid by a method similar to that in Example 6B.

### (Example 79)

### 5-(difluoromethoxy)-2-{7-[(1S,2S,5R or 1R,2R,5S)-8-ethyl-8-azabicyclo[3.2.1]octan-2-yl]-6,7-dihydro-5H-pyrrolo[2,3-c]pyridazin-3-yl}-3-methylphenol

### (79A) tert-butyl (1S,2S,5S or 1R,2R,5R)-2-(3-chloro-5,6-dihydro-7H-pyrrolo[2,3-c]pyridazin-7-yl)-8-azabicyclo[3.2.1]octane-8-carboxylate

tert-Butyl (1S*,2S*,5S*)-2-(3-chloro-5,6-dihydro-7H-pyrrolo[2,3-c]pyridazin-7-yl)-8-azabicyclo[3.2.1]octane-8-carboxylate (160.6 mg, 0.44 mmol) of Example 17E was subjected to chiral HPLC [column: CHIRALPAK IG (20 mm I.D.× 250 mm), mobile phase: n-hexane/ethanol=50/50(V/V), temperature: 40°C] to give the title compound eluting earlier (63.5 mg, yield: 40%) as a single enantiomer (a solid).

### (79B) tert-butyl (1S,2S,5S or 1R,2R,5R)-2-{3-[4-(difluoromethoxy)-2-(methoxymethoxy)-6-methylphenyl]-5,6-dihydro-7H-pyrrolo[2,3-c]pyridazin-7-yl}-8-azabicyclo[3.2.1]octane-8-carboxylate

Using tert-butyl (1S,2S,5S or 1R,2R,5R)-2-(3-chloro-5,6-dihydro-7H-pyrrolo[2,3-c]pyridazin-7-yl)-8-azabicyclo[3.2.1]octane-8-carboxylate (39.3 mg, 0.11 mmol) of Example 79A, 2-[4-(difluoromethoxy)-2-(methoxymethoxy)-6-methylphenyl]-4,4,5,5-tetramethyl-1,3,2-dioxaborolane (52 mg, 0.15 mmol) of Example 13F, XPhos Pd G2 (17 mg, 0.022 mmol) and potassium carbonate (30 mg, 0.22 mmol), the title compound (37.7 mg, yield: 64%) was obtained as a single enantiomer (a solid) by a method similar to that in Example 9D.

### (79C) 2-{7-[(1S,2S,5R or 1R,2R,5S)-8-azabicyclo[3.2.1]octan-2-yl]-6,7-dihydro-5H-pyrrolo[2,3-c]pyridazin-3-yl}-5-(difluoromethoxy)-3-methylphenol

Using tert-butyl (1S,2S,5S or 1R,2R,5R)-2-{3-[4-(difluoromethoxy)-2-(methoxymethoxy)-6-methylphenyl]-5,6-dihydro-7H-pyrrolo[2,3-c]pyridazin-7-yl}-8-azabicyclo[3.2.1]octane-8-carboxylate (37 mg, 0.068 mmol) of Example 79B, and TFA (1 mL), the title compound (27.2 mg, yield: quantitative) was obtained as a single enantiomer (a solid) by a method similar to that in Example 64B.

### (79D) 5-(difluoromethoxy)-2-{7-[(1S,2S,5R or 1R,2R,5S)-8-ethyl-8-azabicyclo[3.2.1]octan-2-yl]-6,7-dihydro-5H-pyrrolo[2,3-c]pyridazin-3-yl}-3-methylphenol formate

Using 2-{7-[(1S,2S,5R or 1R,2R,5S)-8-azabicyclo[3.2.1]octan-2-yl]-6,7-dihydro-5H-pyrrolo[2,3-c]pyridazin-3-yl}-5-(difluoromethoxy)-3-methylphenol (27.2 mg, 0.068 mmol) of Example 79C, acetaldehyde (4 drops) and NaBH(OAc)₃ (89 mg, 0.44 mmol), a crude product was obtained by a method similar to that in Example 1G, and the crude product was purified by reverse-phase preparative HPLC [mobile phase: 0.1% formic acid aqueous solution/0.1% formic acid-acetonitrile =86/14-58/42(V/V)] to give the title compound (10.8 mg, yield: 34%) as a single enantiomer (a solid).

### (Example 80)

### 5-(difluoromethoxy)-2-{8-[(1s,3s)-3-hydroxy-3-methylcyclobutyl]-5,6,7,8-tetrahydrocyclopenta[4,5]pyrrolo[2,3-c]pyridazin-3-yl}-3-methylphenol (also referred to as 5-(difluoromethoxy)-2-[8-(cis-3-hydroxy-3-methylcyclobutyl)-5,6,7,8-tetrahydrocyclopenta[4,5]pyrrolo[2,3-c]pyridazin-3-yl]-3-methylphenol)

### (80A) 8-((1s,3s)-3-{[tert-butyl(dimethyl)silyl]oxy}-3-methylcyclobutyl)-3-[4-(difluoromethoxy)-2-(methoxymethoxy)-6-methylphenyl]-5,6,7,8-tetrahydrocyclopenta[4,5]pyrrolo[2,3-c]pyridazine

Using 8-((1s,3s)-3-{[tert-butyl(dimethyl)silyl]oxy}-3-methylcyclobutyl)-3-chloro-5,6,7,8-tetrahydrocyclopenta[4,5]pyrrolo[2,3-c]pyridazine (0.150 g, 0.38 mmol) of Example 12C, 2-[4-(difluoromethoxy)-2-(methoxymethoxy)-6-methylphenyl]-4,4,5,5-tetramethyl-1,3,2-dioxaborolane (0.158 g, 0.46 mmol) of Example 13F, potassium carbonate (0.130 g, 0.94 mmol) and XPhos Pd G2 (0.060 g, 0.076 mmol), the title compound (0.141 g, yield: 64%) was obtained as an amorphous form by a method similar to that in Example 1E.

### (80B) 5-(difluoromethoxy)-2-{8-[(1s,3s)-3-hydroxy-3-methylcyclobutyl]-5,6,7,8-tetrahydrocyclopenta[4,5]pyrrolo[2,3-c]pyridazin-3-yl}-3-methylphenol

Using 8-((1s,3s)-3-([tert-butyl(dimethyl)silyl]oxyl-3-methylcyclobutyl)-3-[4-(difluoromethoxy)-2-(methoxymethoxy)-6-methylphenyl]-5,6,7,8-tetrahydrocyclopenta[4,5]pyrrolo[2,3-c]pyridazine (0.141 g, 0.25 mmol) of Example 80A, and 4 M hydrogen chloride-1,4-dioxane (2 mL), the title compound (0.085 g, yield: 83%) was obtained as a solid by a method similar to that in Example 12E.

### (Example 81)

### 2-{9-[(1s,3s)-3-hydroxy-3-methylcyclobutyl]-9H-pyridazino[3,4-b]indol-3-yl}-3-methyl-5-(trifluoromethyl)phenol (also referred to as 2-[9-(cis-3-hydroxy-3-methylcyclobutyl)-9H-pyridazino[3,4-b]indol-3-yl]-3-methyl-5-(trifluoromethyl)phenol)

### (81A) 4-(2-bromophenyl)-3,6-dichloropyridazine

Using 4-bromo-3,6-dichloropyridazine (1.03 g, 4.5 mmol), 2-bromophenylboronic acid (0.908 g, 4.5 mmol), potassium carbonate (1.33 g, 9.6 mmol) and Pd(dppf)Cl₂ (0.253 g, 0.35 mmol), the title compound (0.644 g, yield: 47%) was obtained as a solid by a method similar to that in Example 1C.

### (81B) tert-butyl [(1s,3s)-3-(benzyloxy)-3-methylcyclobutyl]carbamate

tert-Butyl (1s,3s)-3-hydroxy-3-methylcyclobutylcarbamate (1.0 g, 5.0 mmol) was dissolved in DMF (18 mL), then 55% sodium hydride (230 mg, 6.0 mmol) and benzyl bromide (0.890 mL, 7.5 mmol) were added at 0°C, and the mixture was stirred at room temperature for 5.5 hr. To the reaction solution was added water at 0°C, and the mixture was subjected to extraction with n-hexane/ethyl acetate. The organic layer was washed successively with water and saturated brine, and dried over anhydrous sodium sulfate. After filtration, the filtrate was concentrated under reduced pressure, and the residue was purified by silica gel column chromatography [eluent: n-hexane/ethyl acetate =80/20-50/50(V/V)] to give the title compound (160 mg, yield: 11%) as a solid.

### (81C) (1s,3s)-3-(benzyloxy)-3-methylcyclobutanamine monohydrochloride

Using tert-butyl [(1s,3s)-3-(benzyloxy)-3-methylcyclobutyl]carbamate (1.17 g, 4.0 mmol) of Example 81B, and 4 M hydrogen chloride-1,4-dioxane (20 mL), a crude product (890 mg, yield: 97%) of the title compound was obtained as a solid by a method similar to that in Example 2B.

### (81D) 9-[(1s,3s)-3-(benzyloxy)-3-methylcyclobutyl]-3-chloro-9H-pyridazino[3,4-b]indole

Using 4-(2-bromophenyl)-3,6-dichloropyridazine (0.200 g, 0.66 mmol) of Example 81A, (ls,3s)-3-(benzyloxy)-3-methylcyclobutanamine monohydrochloride (0.150 g, 0.66 mmol) of Example 81C, Pd₂(dba)₃ (0.121 g, 0.13 mmol), RuPhos (0.071 g, 0.15 mmol) and sodium tert-butoxide (0.190 g, 2.0 mmol), the title compound (0.014 g, yield: 6%) was obtained as a solid by a method similar to that in Example 12C.

### (81E) 9-[(1s,3s)-3-(benzyloxy)-3-methylcyclobutyl]-3-[2-(methoxymethoxy)-6-methyl-4-(trifluoromethyl)phenyl]-9H-pyridazino[3,4-b]indole

Using 9-[(1s,3s)-3-(benzyloxy)-3-methylcyclobutyl]-3-chloro-9H-pyridazino[3,4-b]indole (14 mg, 0.037 mmol) of Example 81D, 2-[2-(methoxymethoxy)-6-methyl-4-(trifluoromethyl)phenyl]-4,4,5,5-tetramethyl-1,3,2-dioxaborolane (15 mg, 0.043 mmol) of Example 1B, potassium carbonate (10 mg, 0.072 mmol) and XPhos Pd G2 (6 mg, 0.0080 mmol), the title compound (0.017 g, yield: 82%) was obtained by a method similar to that in Example 1E.

### (81F) (1s,3s)-3-{3-[2-(methoxymethoxy)-6-methyl-4-(trifluoromethyl)phenyl]-9H-pyridazino[3,4-b]indol-9-yl}-1-methylcyclobutanol

9-[(1s,3s)-3-(Benzyloxy)-3-methylcyclobutyl]-3-[2-(methoxymethoxy)-6-methyl-4-(trifluoromethyl)phenyl]-9H-pyridazino[3,4-b]indole (17 mg, 0.030 mmol) of Example 81E was dissolved in ethanol (1 mL), ASCA-2 (23 mg) was added, and the mixture was stirred in the hydrogen-substituted system at 60°C for 5 hr. The reaction solution was filtered through Celite, the filtrate was concentrated under reduced pressure, and the obtained residue was purified by silica gel column chromatography [eluent: n-hexane/ethyl acetate =90/10-50/50(V/V)] to give the title compound (6 mg, yield: 40%) as an oil.

### (81G) 2-{9-[(1s,3s)-3-hydroxy-3-methylcyclobutyl]-9H-pyridazino[3,4-b]indol-3-yl}-3-methyl-5-(trifluoromethyl)phenol

Using (1s,3s)-3-{3-[2-(methoxymethoxy)-6-methyl-4-(trifluoromethyl)phenyl]-9H-pyridazino[3,4-b]indol-9-yl}-1-methylcyclobutanol (0.028 g, 0.057 mmol) of Example 81F, and 4 M hydrogen chloride-1,4-dioxane (1 mL), the title compound (0.021 g, yield: 83%) was obtained as a solid by a method similar to that in Example 3D.

### (Example 82)

### 5-chloro-2-{8-[(1s,3s)-3-hydroxy-3-methylcyclobutyl]-5,6,7,8-tetrahydrocyclopenta[4,5]pyrrolo[2,3-c]pyridazin-3-yl}-3-methylphenol (also referred to as 5-chloro-2-[8-(cis-3-hydroxy-3-methylcyclobutyl)-5,6,7,8-tetrahydrocyclopenta[4,5]pyrrolo[2,3-c]pyridazin-3-yl]-3-methylphenol)

### (82A) 8-((1s,3s)-3-{[tert-butyl(dimethyl)silyl]oxy}-3-methylcyclobutyl)-3-[4-chloro-2-(methoxymethoxy)-6-methylphenyl]-5,6,7,8-tetrahydrocyclopenta[4,5]pyrrolo[2,3-c]pyridazine

Using 8-((1s,3s)-3-{[tert-butyl(dimethyl)silyl]oxy}-3-methylcyclobutyl)-3-chloro-5,6,7,8-tetrahydrocyclopenta[4,5]pyrrolo[2,3-c]pyridazine (0.236 g, 0.60 mmol) of Example 12C, 2-[4-chloro-2-(methoxymethoxy)-6-methylphenyl]-4,4,5,5-tetramethyl-1,3,2-dioxaborolane (0.230 g, 0.74 mmol) of Example 9C, potassium carbonate (0.177 g, 1.3 mmol) and XPhos Pd G2 (0.051 g, 0.065 mmol), the title compound (0.107 g, yield: 33%) was obtained as an amorphous form by a method similar to that in Example 9D.

### (82B) 5-chloro-2-{8-[(1s,3s)-3-hydroxy-3-methylcyclobutyl]-5,6,7,8-tetrahydrocyclopenta[4,5]pyrrolo[2,3-c]pyridazin-3-yl}-3-methylphenol

Using 8-((1s,3s)-3-{[tert-butyl(dimethyl)silyl]oxy}-3-methylcyclobutyl)-3-[4-chloro-2-(methoxymethoxy)-6-methylphenyl]-5,6,7,8-tetrahydrocyclopenta[4,5]pyrrolo[2,3-c]pyridazine (0.107 g, 0.20 mmol) of Example 82A, and 4 M hydrogen chloride-1,4-dioxane (2 mL), the title compound (0.062 g, yield: 82%) was obtained as a solid by a method similar to that in Example 12E.

### (Example 83)

### 2-{8-[(1s,3s)-3-hydroxy-3-methylcyclobutyl]-7,8-dihydro-6H-furo[2',3':4,5]pyrrolo[2,3-c]pyridazin-3-yl}-3-methyl-5-(trifluoromethyl)phenol (also referred to as 2-[8-(cis-3-hydroxy-3-methylcyclobutyl)-7,8-dihydro-6H-furo[2',3':4,5]pyrrolo[2,3-c]pyridazin-3-yl]-3-methyl-5-(trifluoromethyl)phenol)

### (83A) 4-(3,6-dichloropyridazin-4-yl)but-3-yn-1-ol

4-Bromo-3,6-dichloropyridazine (18.0 g, 79 mmol), bis(triphenylphosphine)palladium(II) dichloride (1.39 g, 2.0 mmol) and copper(I) iodide (755 mg, 4.0 mmol) were dissolved in DMF (150 mL), then 3-butyn-1-ol (7.15 mL, 95 mmol) and TEA (32.9 mL, 240 mmol) were added, and the mixture was stirred in the nitrogen-substituted system at room temperature for 1 hr and 10 min. To the reaction solution was added water, and the mixture was subjected to extraction with ethyl acetate. The organic layer was washed with saturated brine/water (1/1), and dried over anhydrous sodium sulfate. After filtration, the filtrate was concentrated under reduced pressure, and the obtained residue was purified by silica gel column chromatography [eluent: n-hexane/ethyl acetate =55/45-29/71(V/V)] to give the title compound (9.05 g, yield: 53%) as a solid.

### (83B) 3,6-dichloro-4-(4,5-dihydrofuran-2-yl)pyridazine

Silver trifluoromethanesulfonate (256 mg, 1.0 mmol) and (triphenylphosphine)gold(I) chloride (492 mg, 0.99 mmol) were suspended in DCE (155 mL), 4-(3,6-dichloropyridazin-4-yl)but-3-yn-1-ol (2.05 g, 9.5 mmol) of Example 83A was added, and the mixture was stirred in the nitrogen-substituted system at 80°C for 1 hr and 15 min. The reaction solution was allowed to room temperature, and filtered, the filtrate was concentrated under reduced pressure, and the obtained residue was purified by silica gel column chromatography [eluent: n-hexane/ethyl acetate =97/3-63/37(V/V)] to give the title compound (1.44 g, yield: 70%) as a solid.

### (83C) 2-(3,6-dichloropyridazin-4-yl)-3-iodotetrahydrofuran-2-yl acetate

3,6-Dichloro-4-(4,5-dihydrofuran-2-yl)pyridazine (640 mg, 2.9 mmol) of Example 83Bb was dissolved in acetic acid (5.0 mL), N-iodosuccinimide (762 mg, 3.4 mmol) was added, and the mixture was stirred at room temperature for 20 min. To the reaction solution was added saturated aqueous sodium bicarbonate solution at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =70/30-50/50(V/V)] to give the title compound (1.06 g, yield: 89%) as a solid.

### (83D) 3,6-dichloro-4-(3-iodo-4,5-dihydrofuran-2-yl)pyridazine

2-(3,6-Dichloropyridazin-4-yl)-3-iodotetrahydrofuran-2-yl acetate (1.06 g, 2.6 mmol) of Example 83C was dissolved in DCM (8.0 mL), trimethylsilyl trifluoromethanesulfonate (1.1 mL, 6.3 mmol) was added at 0°C, and the mixture was stirred at room temperature for 5 min. To the reaction solution were added tert-butanol, methanol and water at 0°C, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-80/20(V/V)] to give the title compound (652 mg, yield: 73%) as a solid.

### (83E) 8-((1s,3s)-3-{[tert-butyl(dimethyl)silyl]oxy}-3-methylcyclobutyl)-3-chloro-7,8-dihydro-6H-furo[2',3':4,5]pyrrolo[2,3-c]pyridazine

3,6-Dichloro-4-(3-iodo-4,5-dihydrofuran-2-yl)pyridazine (652 mg, 1.9 mmol) of Example 83D was dissolved in 1,4-dioxane (10 mL), then (1s,3s)-3-{[tert-butyl(dimethyl)silyl]oxy}-3-methylcyclobutanamine (491 mg, 2.3 mmol) of Example 12B, Pd₂(dba)₃ (348 mg, 0.38 mmol), RuPhos (177 mg, 0.38 mmol) and sodium tert-butoxide (548 mg, 5.7 mmol) were added, and the mixture was stirred in the nitrogen-substituted system at 110°C for 1 hr. The reaction solution was filtered through Celite, the filtrate was concentrated under reduced pressure, and the obtained residue was purified by silica gel column chromatography [eluent: n-hexane/ethyl acetate =80/20-70/30(V/V)] and NH silica gel column chromatography [eluent: n-hexane/ethyl acetate =85/15-80/20(V/V)] to give the title compound (48 mg, yield: 6%) as a solid.

### (83F) 8-((1s,3s)-3-{[tert-butyl(dimethyl)silyl]oxy}-3-methylcyclobutyl)-3-[2-(methoxymethoxy)-6-methyl-4-(trifluoromethyl)phenyl]-7,8-dihydro-6H-furo[2',3':4,5]pyrrolo[2,3-c]pyridazine

8-((1s,3s)-3-{[tert-Butyl(dimethyl)silyl]oxy}-3-methylcyclobutyl)-3-chloro-7,8-dihydro-6H-furo[2',3':4,5]pyrrolo[2,3-c]pyridazine (48 mg, 0.12 mmol) of Example 83E, 2-[2-(methoxymethoxy)-6-methyl-4-(trifluoromethyl)phenyl]-4,4,5,5-tetramethyl-1,3,2-dioxaborolane (63 mg, 0.18 mmol) of Example 1B, and potassium carbonate (33 mg, 0.24 mmol) were dissolved in a mixed solvent of 1,4-dioxane (1.2 mL) and water (0.3 mL), XPhos Pd G2 (28 mg, 0.037 mmol) was added, and the mixture was stirred under microwave irradiation in the nitrogen-substituted system at 120°C for 50 min. The reaction solution was purified by silica gel column chromatography [eluent: n-hexane/ethyl acetate =85/15-70/30(V/V)] to give the title compound (52 mg, yield: 74%) as a solid.

### (83G) 2-{8-[(1s,3s)-3-hydroxy-3-methylcyclobutyl]-7,8-dihydro-6H-furo[2',3':4,5]pyrrolo[2,3-c]pyridazin-3-yl}-3-methyl-5-(trifluoromethyl)phenol

Using 8-((1s,3s)-3-{[tert-butyl(dimethyl)silyl]oxy}-3-methylcyclobutyl)-3-[2-(methoxymethoxy)-6-methyl-4-(trifluoromethyl)phenyl]-7,8-dihydro-6H-furo[2',3':4,5]pyrrolo[2,3-c]pyridazine (20 mg, 0.035 mmol) of Example 83F, and TFA (0.4 mL), the title compound (11 mg, yield: 76%) was obtained as a solid by a method similar to that in Example 21F.

### (Example 84)

### 2-[7-(5-fluoropyrimidin-2-yl)-7H-pyrrolo[2,3-c]pyridazin-3-yl]-3-methyl-5-(trifluoromethyl)phenol

### (84A) 3-chloro-7-(5-fluoropyrimidin-2-yl)-7H-pyrrolo[2,3-c]pyridazine

3-Chloro-7H-pyrrolo[2,3-c]pyridazine (300 mg, 2.0 mmol) and potassium carbonate (540 mg, 3.9 mmol) were dissolved in DMF (10 mL), 2-chloro-5-fluoropyrimidine (0.48 mL, 3.9 mmol) was added, and the mixture was stirred in the nitrogen-substituted system at 70°C for 12 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by reverse-phase silica gel column chromatography [eluent: 0.225% formic acid aqueous solution/acetonitrile =80/20-45/55(V/V)] to give the title compound (170 mg, yield: 35%) as a solid.

### (84B) 7-(5-fluoropyrimidin-2-yl)-3-[2-(methoxymethoxy)-6-methyl-4-(trifluoromethyl)phenyl]-7H-pyrrolo[2,3-c]pyridazine

Using 3-chloro-7-(5-fluoropyrimidin-2-yl)-7H-pyrrolo[2,3-c]pyridazine (0.023 g, 0.092 mmol) of Example 84A, 2-[2-(methoxymethoxy)-6-methyl-4-(trifluoromethyl)phenyl]-4,4,5,5-tetramethyl-1,3,2-dioxaborolane (0.048 g, 0.14 mmol) of Example 1B, potassium carbonate (0.034 g, 0.25 mmol) and XPhos Pd G2 (0.017 g, 0.022 mmol), the title compound (0.018 g, yield: 45%) was obtained as an oil by a method similar to that in Example 1E.

### (84C) 2-[7-(5-fluoropyrimidin-2-yl)-7H-pyrrolo[2,3-c]pyridazin-3-yl]-3-methyl-5-(trifluoromethyl)phenol

Using 7-(5-fluoropyrimidin-2-yl)-3-[2-(methoxymethoxy)-6-methyl-4-(trifluoromethyl)phenyl]-7H-pyrrolo[2,3-c]pyridazine (0.018 g, 0.042 mmol) of Example 84B, and 4 M hydrogen chloride-1,4-dioxane (0.4 mL), the title compound (0.013 g, yield: 80%) was obtained as a solid by a method similar to that in Example 3D.

### (Example 85)

### 2-{7-[(1s,3s)-3-hydroxy-3-methylcyclobutyl]-5-methyl-6,7-dihydro-5H-pyrrolo[2,3-c]pyridazin-3-yl}-3-methyl-5-(trifluoromethyl)phenol (also referred to as 2-[7-(cis-3-hydroxy-3-methylcyclobutyl)-5-methyl-6,7-dihydro-5H-pyrrolo[2,3-c]pyridazin-3-yl]-3-methyl-5-(trifluoromethyl)phenol)

### (85A) (1s,3s)-3-{3-[2-(methoxymethoxy)-6-methyl-4-(trifluoromethyl)phenyl]-5-methyl-5,6-dihydro-7H-pyrrolo[2,3-c]pyridazin-7-yl}-1-methylcyclobutanol

Using (1s,3s)-3-(3-chloro-5-methyl-5,6-dihydro-7H-pyrrolo[2,3-c]pyridazin-7-yl)-1-methylcyclobutanol (100 mg, 0.39 mmol) of Example 4B, 2-[2-(methoxymethoxy)-6-methyl-4-(trifluoromethyl)phenyl]-4,4,5,5-tetramethyl-1,3,2-dioxaborolane (274 mg, 0.79 mmol) of Example 1B, XPhos Pd G3 (33 mg, 0.039 mmol) and sodium carbonate (126 mg, 1.2 mmol), the title compound (80 mg, yield: 46%) was obtained as a racemate (an oil) by a method similar to that in Example 9D.

### (85B) 2-{7-[(1s,3s)-3-hydroxy-3-methylcyclobutyl]-5-methyl-6,7-dihydro-5H-pyrrolo[2,3-c]pyridazin-3-yl}-3-methyl-5-(trifluoromethyl)phenol

Using (1s,3s)-3-{3-[2-(methoxymethoxy)-6-methyl-4-(trifluoromethyl)phenyl]-5-methyl-5,6-dihydro-7H-pyrrolo[2,3-c]pyridazin-7-yl}-1-methylcyclobutanol (30 mg, 0.069 mmol) of Example 85A, and 6 M hydrochloric acid (4.0 mL), the title compound (17 mg, yield: 64%) was obtained as a racemate (a solid) by a method similar to that in Example 3D.

### (Example 86)

### 2-{7-[(1s,3s)-3-hydroxy-3-methylcyclobutyl]-6-methyl-6,7-dihydro-5H-pyrrolo[2,3-c]pyridazin-3-yl}-3-methyl-5-(trifluoromethyl)phenol (also referred to as 2-[7-(cis-3-hydroxy-3-methylcyclobutyl)-6-methyl-6,7-dihydro-5H-pyrrolo[2,3-c]pyridazin-3-yl]-3-methyl-5-(trifluoromethyl)phenol)

### (86A) (1s,3s)-3-{3-[2-(methoxymethoxy)-6-methyl-4-(trifluoromethyl)phenyl]-6-methyl-5,6-dihydro-7H-pyrrolo[2,3-c]pyridazin-7-yl}-1-methylcyclobutanol

Using (1s,3s)-3-(3-chloro-6-methyl-5,6-dihydro-7H-pyrrolo[2,3-c]pyridazin-7-yl)-1-methylcyclobutanol (100 mg, 0.39 mmol) of Example 51B, 2-[2-(methoxymethoxy)-6-methyl-4-(trifluoromethyl)phenyl]-4,4,5,5-tetramethyl-1,3,2-dioxaborolane (274 mg, 0.79 mmol) of Example 1B, XPhos Pd G3 (33 mg, 0.039 mmol) and sodium carbonate (84 mg, 0.79 mmol), the title compound (118 mg, yield: 51%) was obtained as a racemate (an oil) by a method similar to that in Example 9D.

### (86B) 2-{7-[(1s,3s)-3-hydroxy-3-methylcyclobutyl]-6-methyl-6,7-dihydro-5H-pyrrolo[2,3-c]pyridazin-3-yl}-3-methyl-5-(trifluoromethyl)phenol

Using (1s,3s)-3-{3-[2-(methoxymethoxy)-6-methyl-4-(trifluoromethyl)phenyl]-6-methyl-5,6-dihydro-7H-pyrrolo[2,3-c]pyridazin-7-yl}-1-methylcyclobutanol (41 mg, 0.094 mmol) of Example 86A, and 6 M hydrochloric acid (4.0 mL), the title compound (9.46 mg, yield: 25%) was obtained as a racemate (a solid) by a method similar to that in Example 3D.

### (Example 87)

### 2-{7-[(1s,3s)-3-hydroxy-3-methylcyclobutyl]-6-methyl-7H-pyrrolo[2,3-c]pyridazin-3-yl}-3-methyl-5-(trifluoromethyl)phenol (also referred to as 2-[7-(cis-3-hydroxy-3-methylcyclobutyl)-6-methyl-7H-pyrrolo[2,3-c]pyridazin-3-yl]-3-methyl-5-(trifluoromethyl)phenol)

### (87A) (1s,3s)-3-{3-[2-(methoxymethoxy)-6-methyl-4-(trifluoromethyl)phenyl]-6-methyl-7H-pyrrolo[2,3-c]pyridazin-7-yl}-1-methylcyclobutanol

Using (1s,3s)-3-{3-[2-(methoxymethoxy)-6-methyl-4-(trifluoromethyl)phenyl]-6-methyl-5,6-dihydro-7H-pyrrolo[2,3-c]pyridazin-7-yl}-1-methylcyclobutanol (40 mg, 0.091 mmol) of Example 86A, and manganese dioxide (40 mg, 0.46 mmol), a crude product (36 mg, yield: 90%) of the title compound was obtained as an oil by a method similar to that in Example 4C.

### (87B) 2-{7-[(1s,3s)-3-hydroxy-3-methylcyclobutyl]-6-methyl-7H-pyrrolo[2,3-c]pyridazin-3-yl}-3-methyl-5-(trifluoromethyl)phenol

Using (1s,3s)-3-{3-[2-(methoxymethoxy)-6-methyl-4-(trifluoromethyl)phenyl]-6-methyl-7H-pyrrolo[2,3-c]pyridazin-7-yl}-1-methylcyclobutanol (36 mg, 0.083 mmol) of Example 87A, and 6 M hydrochloric acid (4.0 mL), the title compound (6.09 mg, yield: 19%) was obtained as a solid by a method similar to that in Example 3D.

### (Example 88)

### 5-{3-[2-hydroxy-6-methyl-4-(trifluoromethyl)phenyl]-7H-pyrrolo[2,3-c]pyridazin-7-yl}bicyclo[3.1.1]heptan-1-ol

Using 5-{3-[2-(methoxymethoxy)-6-methyl-4-(trifluoromethyl)phenyl]-7H-pyrrolo[2,3-c]pyridazin-7-yl}bicyclo[3.1.1]heptan-1-ol (30 mg, 0.067 mmol) of Example 16C, and 6 M hydrochloric acid (4.0 mL), the title compound (6 mg, yield: 22%) was obtained as a solid by a method similar to that in Example 3D.

### (Example 89)

### 2-{7-[(2S)-2-hydroxypropyl]-7H-pyrrolo[2,3-c]pyridazin-3-yl}-3-methyl-5-(trifluoromethyl)phenol

### (89A) (2S)-1-(3-chloro-5,6-dihydro-7H-pyrrolo[2,3-c]pyridazin-7-yl)propan-2-ol

Using 3,6-dichloro-4-ethenylpyridazine (200 mg, 1.1 mmol) of Example 1C, (2S)-1-aminopropan-2-ol (94 mg, 1.3 mmol) and DIPEA (1 mL, 5.7 mmol), the title compound (35 mg, yield: 14%) was obtained as a solid by a method similar to that in Example 1D.

### (89B) (2S)-1-{3-[2-(methoxymethoxy)-6-methyl-4-(trifluoromethyl)phenyl]-5,6-dihydro-7H-pyrrolo[2,3-c]pyridazin-7-yl}propan-2-ol

Using (2S)-1-(3-chloro-5,6-dihydro-7H-pyrrolo[2,3-c]pyridazin-7-yl)propan-2-ol (30 mg, 0.14 mmol) of Example 89A, 2-[2-(methoxymethoxy)-6-methyl-4-(trifluoromethyl)phenyl]-4,4,5,5-tetramethyl-1,3,2-dioxaborolane (140 mg, 0.40 mmol) of Example 1B, XPhos Pd G3 (12 mg, 0.014 mmol) and sodium carbonate (30 mg, 0.28 mmol), the title compound (26 mg, yield: 47%) was obtained as a solid by a method similar to that in Example 9D.

### (89C) 7-[(2S)-2-{[tert-butyl(dimethyl)silyl]oxy}propyl]-3-[2-(methoxymethoxy)-6-methyl-4-(trifluoromethyl)phenyl]-6,7-dihydro-5H-pyrrolo[2,3-c]pyridazine

(2S)-1-{3-[2-(Methoxymethoxy)-6-methyl-4-(trifluoromethyl)phenyl]-5,6-dihydro-7H-pyrrolo[2,3-c]pyridazin-7-yl}propan-2-ol (25 mg, 0.063 mmol) of Example 89B was dissolved in DMF (2 mL), then imidazole (10 mg, 0.15 mmol) and tert-butyldimethylsilyl chloride (15 mg, 0.10 mmol) were added, and the mixture was stirred in the nitrogen-substituted system at room temperature for 12 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by preparative thin layer silica gel column chromatography [eluent: DCM/methanol=10/1(V/V)] to give the title compound (30 mg, yield: 89%) as an oil.

### (89D) 7-[(2S)-2-{[tert-butyl(dimethyl)silyl]oxy}propyl]-3-[2-(methoxymethoxy)-6-methyl-4-(trifluoromethyl)phenyl]-7H-pyrrolo[2,3-c]pyridazine

Using 7-[(2S)-2-{[tert-butyl(dimethyl)silyl]oxy}propyl]-3-[2-(methoxymethoxy)-6-methyl-4-(trifluoromethyl)phenyl]-6,7-dihydro-5H-pyrrolo[2,3-c]pyridazine (30 mg, 0.056 mmol) of Example 89C, and manganese dioxide (60 mg, 0.69 mmol), the title compound (25 mg, yield: 88%) was obtained as a solid by a method similar to that in Example 4C.

### (89E) 2-{7-[(2S)-2-hydroxypropyl]-7H-pyrrolo[2,3-c]pyridazin-3-yl}-3-methyl-5-(trifluoromethyl)phenol

Using 7-[(2S)-2-{[tert-butyl(dimethyl)silyl]oxy}propyl]-3-[2-(methoxymethoxy)-6-methyl-4-(trifluoromethyl)phenyl]-7H-pyrrolo[2,3-c]pyridazine (25 mg, 0.049 mmol) of Example 89D, and 6 M hydrochloric acid (4.0 mL), the title compound (6.81 mg, yield: 39%) was obtained as a solid by a method similar to that in Example 12E.

### (Example 90)

### 2-{7-[(1s,3s)-3-hydroxy-3-methylcyclobutyl]-5-methyl-7H-pyrrolo[2,3-c]pyridazin-3-yl}-3,5-dimethylphenol (also referred to as 2-[7-(cis-3-hydroxy-3-methylcyclobutyl)-5-methyl-7H-pyrrolo[2,3-c]pyridazin-3-yl]-3,5-dimethylphenol)

### (90A) (1s,3s)-3-{3-[2-(methoxymethoxy)-4,6-dimethylphenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (50 mg, 0.20 mmol) of Example 4C, 2-[2-(methoxymethoxy)-4,6-dimethyl-phenyl]-4,4,5,5-tetramethyl-1,3,2-dioxaborolane (US20200361898) (117 mg, 0.40 mmol), XPhos Pd G3 (17 mg, 0.020 mmol) and sodium carbonate (63 mg, 0.60 mmol), the title compound (60 mg, yield: 79%) was obtained as a solid by a method similar to that in Example 1E.

### (90B) 2-{7-[(1s,3s)-3-hydroxy-3-methylcyclobutyl]-5-methyl-7H-pyrrolo[2,3-c]pyridazin-3-yl}-3,5-dimethylphenol

Using (1s,3s)-3-{3-[2-(methoxymethoxy)-4,6-dimethylphenyl]-5-methyl-7H-pyrrolo[2,3-c]pyridazin-7-yl}-1-methylcyclobutanol (60 mg, 0.16 mmol) of Example 90A, and 6 M hydrochloric acid (6.0 mL), the title compound (40.7 mg, yield: 76%) was obtained as an oil by a method similar to that in Example 3D.

### (Example 91)

### 5-fluoro-2-{7-[(1s,3s)-3-hydroxy-3-methylcyclobutyl]-5-methyl-7H-pyrrolo[2,3-c]pyridazin-3-yl}-3-methylphenol (also referred to as 5-fluoro-2-[7-(cis-3-hydroxy-3-methylcyclobutyl)-5-methyl-7H-pyrrolo[2,3-c]pyridazin-3-yl]-3-methylphenol)

### (91A) 5-fluoro-2-iodo-3-methylphenol

Using 3-fluoro-5-methylphenol (2 g, 16 mmol), 60% sodium hydride (1.6 g, 40 mmol) and iodine (2.4 g, 9.5 mmol), the title compound (1.8 g, yield: 45%) was obtained as a solid by a method similar to that in Example 9A.

### (91B) 5-fluoro-2-iodo-1-(methoxymethoxy)-3-methylbenzene

Using 5-fluoro-2-iodo-3-methylphenol (1.8 g, 7.1 mmol) of Example 91A, cesium carbonate (4.7 g, 14 mmol) and chloromethyl methyl ether (0.82 mL, 11 mmol), the title compound (1.7 g, yield: 80%) was obtained as a solid by a method similar to that in Example 1A.

### (91C) 2-[2-(methoxymethoxy)-5-fluoro-3-methylphenyl]-4,4,5,5-tetramethyl-1,3,2-dioxaborolane

5-Fluoro-2-iodo-1-(methoxymethoxy)-3-methylbenzene (800 mg, 2.7 mmol) of Example 91B was dissolved in 1,4-dioxane (10 mL), then TEA (0.75 mL, 5.4 mmol), palladium(II) acetate (61 mg, 0.27 mmol), 2-(dicyclohexylphosphino)biphenyl (115 mg, 0.33 mmol) and 4,4,5,5-tetramethyl-1,3,2-dioxaborolane (700 mg, 5.5 mmol) were added in the nitrogen-substituted system at room temperature, and the mixture was stirred at 80°C for 16 hr. The reaction solution was concentrated under reduced pressure, and the obtained residue was purified by silica gel column chromatography [eluent: n-hexane/ethyl acetate =100/0-90/10(V/V)] to give the title compound (460 mg, yield: 57%) as an oil.

### (91D) (1s,3s)-3-{3-[2-(methoxymethoxy)-5-fluoro-3-methylphenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (40 mg, 0.16 mmol) of Example 4C, 2-[2-(methoxymethoxy)-5-fluoro-3-methylphenyl]-4,4,5,5-tetramethyl-1,3,2-dioxaborolane (95 mg, 0.32 mmol) of Example 91C, XPhos Pd G3 (14 mg, 0.017 mmol) and sodium carbonate (51 mg, 0.48 mmol), the title compound (50 mg, yield: 82%) was obtained as a solid substance by a method similar to that in Example 9D.

### (91E) 5-fluoro-2-{7-[(1s,3s)-3-hydroxy-3-methylcyclobutyl]-5-methyl-7H-pyrrolo[2,3-c]pyridazin-3-yl}-3-methylphenol

Using (1s,3s)-3-{3-[2-(methoxymethoxy)-5-fluoro-3-methylphenyl]-5-methyl-7H-pyrrolo[2,3-c]pyridazin-7-yl}-1-methylcyclobutanol (50 mg, 0.13 mmol) of Example 91D, and 6 M hydrochloric acid (4 mL), the title compound (14.6 mg, yield: 33%) was obtained as a solid by a method similar to that in Example 3D.

### (Example 92)

### 5-(difluoromethyl)-2-{7-[(1s,3s)-3-hydroxy-3-methylcyclobutyl]-5-methyl-7H-pyrrolo[2,3-c]pyridazin-3-yl}-3-methylphenol (also referred to as 5-(difluoromethyl)-2-[7-(cis-3-hydroxy-3-methylcyclobutyl)-5-methyl-7H-pyrrolo[2,3-c]pyridazin-3-yl]-3-methylphenol)

### (92A) 3-(difluoromethyl)-5-methylphenol

3-Hydroxy-5-methyl-benzaldehyde (1 g, 7.4 mmol) was dissolved in DCM (10 mL), N,N-diethylaminosulfur trifluoride (6 g, 37 mmol) was added at 0°C, and the mixture was stirred at room temperature for 24 hr. To the reaction solution was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: petroleum ether/ethyl acetate =100/0-90/10(V/V)] to give the title compound (1 g, yield: 86%) as an oil.

### (92B) 5-(difluoromethyl)-2-iodo-3-methylphenol

Using 3-(difluoromethyl)-5-methylphenol (300 mg, 1.9 mmol) of Example 92A, 60% sodium hydride (152 mg, 3.8 mmol) and iodine (289 mg, 1.1 mmol), the title compound (400 mg, yield: 25%) was obtained as an oil by a method similar to that in Example 9A.

### (92C) 5-(difluoromethyl)-2-iodo-1-(methoxymethoxy)-3-methylbenzene

Using 5-(difluoromethyl)-2-iodo-3-methylphenol (400 mg, 1.4 mmol) of Example 92B, cesium carbonate (918 mg, 2.8 mmol) and chloromethyl methyl ether (0.22 mL, 2.9 mmol), the title compound (450 mg, yield: 97%) was obtained as an oil by a method similar to that in Example 1A.

### (92D) 2-[4-(difluoromethyl)-2-(methoxymethoxy)-6-methylphenyl]-4,4,5,5-tetramethyl-1,3,2-dioxaborolane

5-(Difluoromethyl)-2-iodo-1-(methoxymethoxy)-3-methylbenzene (100 mg, 0.30 mmol) of Example 92C was dissolved in 1,4-dioxane (6 mL), then TEA (0.085 mL, 0.61 mmol), palladium(II) acetate (7 mg, 0.031 mmol), 2-(dicyclohexylphosphino)biphenyl (11 mg, 0.031 mmol) and 4,4,5,5-tetramethyl-1,3,2-dioxaborolane (0.13 mL, 0.91 mmol) were added in the nitrogen-substituted system at room temperature, and the mixture was stirred at 80°C for 12 hr. The reaction solution was concentrated under reduced pressure, and the obtained residue was purified by silica gel column chromatography [eluent: n-hexane/ethyl acetate =100/0-90/10(V/V)] to give the title compound (50 mg, yield: 50%) as an oil.

### (92E) (1s,3s)-3-{3-[4-(difluoromethyl)-2-(methoxymethoxy)-6-methylphenyl]-5-methyl-7H-pyrrolo[2,3-c]pyridazin-7-yl}-1-methylcyclobutanol

Using (1s,3s)-3-(3-chloro-5-methyl-7H-pyrrolo[2,3-c]pyridazin-7-yl)-1-methylcyclobutanol (30 mg, 0.12 mmol) of Example 4C, 2-[4-(difluoromethyl)-2-(methoxymethoxy)-6-methylphenyl]-4,4,5,5-tetramethyl-1,3,2-dioxaborolane (50 mg, 0.15 mmol) of Example 92D, XPhos Pd G3 (11 mg, 0.013 mmol) and sodium carbonate (13 mg, 0.12 mmol), the title compound (30 mg, yield: 82%) was obtained as a solid substance by a method similar to that in Example 9D.

### (92F) 5-(difluoromethyl)-2-{7-[(1s,3s)-3-hydroxy-3-methylcyclobutyl]-5-methyl-7H-pyrrolo[2,3-c]pyridazin-3-yl}-3-methylphenol

Using (1s,3s)-3-{3-[4-(difluoromethyl)-2-(methoxymethoxy)-6-methylphenyl]-5-methyl-7H-pyrrolo[2,3-c]pyridazin-7-yl}-1-methylcyclobutanol (30 mg, 0.071 mmol) of Example 92E, and 6 M hydrochloric acid (4 mL), the title compound (6.48 mg, yield: 24%) was obtained as a solid by a method similar to that in Example 3D.

### (Example 93)

### 3-methyl-2-{6-[(1-methylpiperidin-3-yl)methyl]pyridazin-3-yl}-5-(trifluoromethyl)phenol

### (93A) 3-chloro-6-[2-(methoxymethoxy)-6-methyl-4-(trifluoromethyl)phenyl]pyridazine

Using 3,6-dichloropyridazine (2.15 g, 14 mmol), 2-[2-(methoxymethoxy)-6-methyl-4-(trifluoromethyl)phenyl]-4,4,5,5-tetramethyl-1,3,2-dioxaborolane (5 g, 14 mmol) of Example 1B, Pd(dppf)Cl₂ (1.06 g, 1.4 mmol) and cesium carbonate (14.1 g, 43 mmol), the title compound (1.0 g, yield: 21%) was obtained as a solid by a method similar to that in Example 9D.

### (93B) tert-butyl (3E)-3-({6-[2-(methoxymethoxy)-6-methyl-4-(trifluoromethyl)phenyl]pyridazin-3-yl}methylidene)piperidine-1-carboxylate

Using 3-chloro-6-[2-(methoxymethoxy)-6-methyl-4-(trifluoromethyl)phenyl]pyridazine (250 mg, 0.75 mmol) of Example 93A, tert-butyl (3E)-3-[(4,4,5,5-tetramethyl-1,3,2-dioxaborolan-2-yl]piperidine-1-carboxylate (500 mg, 1.6 mmol), XPhos Pd G3 (38 mg, 0.044 mmol) and sodium carbonate (240 mg, 2.26 mmol), the title compound (320 mg, yield: 86%) was obtained as an oil by a method similar to that in Example 9D.

### (93C) 3-methyl-2-[6-(piperidin-3-ylmethyl)pyridazin-3-yl]-5-(trifluoromethyl)phenol

tert-Butyl (3E)-3-({6-[2-(methoxymethoxy)-6-methyl-4-(trifluoromethyl)phenyl]pyridazin-3-yl}methylidene)piperidine-1-carboxylate (303 mg, 0.61 mmol) of Example 93B was dissolved in ethyl acetate (3 mL), 6 M hydrochloric acid (0.10 mL) was added at room temperature, and the mixture was stirred at room temperature for 24 hr. The reaction solution was concentrated under reduced pressure, and the obtained residue was purified by reverse-phase silica gel column chromatography [eluent: water/acetonitrile =86/14-56/44(V/V)] to give the title compound (20 mg, yield: 9%) as a racemate (a solid).

### (93D) 3-methyl-2-{6-[(1-methylpiperidin-3-yl)methyl]pyridazin-3-yl}-5-(trifluoromethyl)phenol

Using 3-methyl-2-[6-(piperidin-3-ylmethyl)pyridazin-3-yl]-5-(trifluoromethyl)phenol (15 mg, 0.043 mmol) of Example 93C, 37% formaldehyde (0.0059 mL, 0.21 mmol), acetic acid (0.00244 mL, 0.043 mmol) and sodium cyanoborohydride (2.68 mg, 0.043 mmol), the title compound (3.96 mg, yield: 25%) was obtained as a racemate (a solid) by a method similar to that in Example 1G.

### (Example 94)

### 3-methyl-2-[5-methyl-7-(1-methylpiperidin-3-yl)-5H-pyrrolo[3,2-c]pyridazin-3-yl]-5-(trifluoromethyl)phenol

### (94A) tert-butyl 3-[2-(methoxymethoxy)-6-methyl-4-(trifluoromethyl)phenyl]-5H-pyrrolo[3,2-c]pyridazine-5-carboxylate

Using tert-butyl 3-chloropyrrolo[3,2-c]pyridazine-5-carboxylate (WO2020005873) (409 mg, 1.6 mmol), 2-[2-(methoxymethoxy)-6-methyl-4-(trifluoromethyl)phenyl]-4,4,5,5-tetramethyl-1,3,2-dioxaborolane (781 mg, 2.3 mmol) of Example 1B, XPhos Pd G2 (127 mg, 0.16 mmol) and potassium carbonate (668 mg, 4.8 mmol), the title compound (465 mg, yield: 66%) was obtained as a solid by a method similar to that in Example 1E.

### (94B) 3-[2-(methoxymethoxy)-6-methyl-4-(trifluoromethyl)phenyl]-5H-pyrrolo[3,2-c]pyridazine

tert-Butyl 3-[2-(methoxymethoxy)-6-methyl-4-(trifluoromethyl)phenyl]-5H-pyrrolo[3,2-c]pyridazine-5-carboxylate (199 mg, 0.45 mmol) of Example 94A was dissolved in THF (5 mL), 5 M sodium hydroxide aqueous solution (0.64 mL, 3.2 mmol) was added, and the mixture was stirred in the nitrogen-substituted system at 55°C for 2 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (88 mg, yield: 57%) as a solid.

### (94C) 7-iodo-3-[2-(methoxymethoxy)-6-methyl-4-(trifluoromethyl)phenyl]-5H-pyrrolo[3,2-c]pyridazine

3-[2-(Methoxymethoxy)-6-methyl-4-(trifluoromethyl)phenyl]-5H-pyrrolo[3,2-c]pyridazine (88 mg, 0.26 mmol) of Example 94B was dissolved in DMF (2.0 mL), N-iodosuccinimide (64 mg, 0.29 mmol) was added, and the mixture was stirred in the nitrogen-substituted system at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-0/100(V/V)] to give the title compound (81 mg, yield: 67%) as a solid.

### (94D) 7-iodo-3-[2-(methoxymethoxy)-6-methyl-4-(trifluoromethyl)phenyl]-5-methyl-5H-pyrrolo[3,2-c]pyridazine

7-Iodo-3-[2-(methoxymethoxy)-6-methyl-4-(trifluoromethyl)phenyl]-5H-pyrrolo[3,2-c]pyridazine (81 mg, 0.17 mmol) of Example 94C was dissolved in DMF (5.0 mL), 50% sodium hydride (10 mg, 0.21 mmol) was added, and the mixture was stirred in the nitrogen-substituted system at 0°C for 2 hr. To the reaction solution was added iodomethane (0.013 mL, 0.21 mmol), and the mixture was stirred at 0°C for 2 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (56 mg, yield: 67%) as an oil.

### (94E) tert-butyl 5-{3-[2-(methoxymethoxy)-6-methyl-4-(trifluoromethyl)phenyl]-5-methyl-5H-pyrrolo[3,2-c]pyridazin-7-yl}-3,6-dihydropyridine-1(2H)-carboxylate

7-Iodo-3-[2-(methoxymethoxy)-6-methyl-4-(trifluoromethyl)phenyl]-5-methyl-5H-pyrrolo[3,2-c]pyridazine (55 mg, 0.12 mmol) of Example 94D, tert-butyl 3-(4,4,5,5-tetramethyl-1,3,2-dioxaborolan-2-yl)-5,6-dihydropyridine-1(2H)-carboxylate (43 mg, 0.14 mmol) and potassium carbonate (48 mg, 0.34 mmol) were dissolved in a mixed solvent of 1,4-dioxane (2.0 mL) and water (1.0 mL), Pd(dppf)Cl₂ (19 mg, 0.023 mmol) was added, and the mixture was stirred in the nitrogen-substituted system at 90°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (52 mg, yield: 85%) as an oil.

### (94F) tert-butyl 3-{3-[2-(methoxymethoxy)-6-methyl-4-(trifluoromethyl)phenyl]-5-methyl-5H-pyrrolo[3,2-c]pyridazin-7-yl}piperidine-1-carboxylate

tert-Butyl 5-{3-[2-(methoxymethoxy)-6-methyl-4-(trifluoromethyl)phenyl]-5-methyl-5H-pyrrolo[3,2-c]pyridazin-7-yl}-3,6-dihydropyridine-1(2H)-carboxylate (52 mg, 0.098 mmol) of Example 94E was dissolved in methanol (1.0 mL), 10% palladium on carbon (21 mg) was added, and the mixture was stirred in the hydrogen-substituted system at room temperature for 1 hr. The reaction solution was filtered through Celite, the filtrate was concentrated under reduced pressure, and the obtained residue was purified by silica gel column chromatography [eluent: n-hexane/ethyl acetate =90/10-10/90(V/V)] to give the title compound (40 mg, yield: 76%) as a racemate (an oil).

### (94G) 3-methyl-2-[5-methyl-7-(piperidin-3-yl)-5H-pyrrolo[3,2-c]pyridazin-3-yl]-5-(trifluoromethyl)phenol

tert-Butyl 3-{3-[2-(methoxymethoxy)-6-methyl-4-(trifluoromethyl)phenyl]-5-methyl-5H-pyrrolo[3,2-c]pyridazin-7-yl}piperidine-1-carboxylate (40 mg, 0.074 mmol) of Example 94F was dissolved in 1,4-dioxane (2.0 mL), 4 M hydrogen chloride-1,4-dioxane (1.0 mL) was added, and the mixture was stirred at room temperature for 1 hr. The reaction solution was concentrated under reduced pressure, to the obtained residue was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=100/0-80/20(V/V)] to give the title compound (22 mg, yield 76%) as a racemate (a solid).

### (94H) 3-methyl-2-[5-methyl-7-(1-methylpiperidin-3-yl)-5H-pyrrolo[3,2-c]pyridazin-3-yl]-5-(trifluoromethyl)phenol

3-Methyl-2-[5-methyl-7-(piperidin-3-yl)-5H-pyrrolo[3,2-c]pyridazin-3-yl]-5-(trifluoromethyl)phenol (22 mg, 0.056 mmol) of Example 94G was dissolved in DCM (5.0 mL), then 37% formaldehyde (0.0062 mL, 0.084 mmol), acetic acid (0.0064 mL, 0.11 mmol) and NaBH(OAc)₃ (48 mg, 0.22 mmol) were added at 0°C, and the mixture was stirred at 0°C for 1 hr. To the reaction solution was added water, and the reaction solution was subjected to extraction with a mixed solvent of chloroform:2-propanol=3:1. The organic layer was washed with saturated brine, and dried over anhydrous sodium sulfate. After filtration, the filtrate was concentrated under reduced pressure, and the obtained residue was purified by silica gel column chromatography [eluent: ethyl acetate/methanol=100/0-50/50(V/V)] to give the title compound (10 mg, yield: 45%) as a racemate (a solid) .

### (Example 95)

### 3-methyl-2-[7-(1-methylpiperidin-3-yl)thieno[3,2-c]pyridazin-3-yl]-5-(trifluoromethyl)phenol

### (95A) methyl 3-[2-(methoxymethoxy)-6-methyl-4-(trifluoromethyl)phenyl]-7-triisopropylsilyloxy-thieno[3,2-c]pyridazine-6-carboxylate

Methyl 3-chloro-7-triisopropylsilyloxythieno[3,2-c]pyridazine-6-carboxylate (WO2020005873) (800 mg, 1.7 mmol), 2-[2-(methoxymethoxy)-6-methyl-4-(trifluoromethyl)phenyl]-4,4,5,5-tetramethyl-1,3,2-dioxaborolane (651 mg, 1.9 mmol) of Example 1B, and potassium carbonate (475 mg, 3.4 mmol) were dissolved in a mixed solvent of 1,4-dioxane (14 mL) and water (3.5 mL), Pd(dppf)Cl₂ (140 mg, 0.17 mmol) was added, and the mixture was stirred in the nitrogen-substituted system at 90°C for 4 hr. The reaction solution was allowed to room temperature, 1 M hydrochloric acid was added, and the mixture was subjected to extraction with DCM. The organic layer was washed with saturated brine, and dried over anhydrous sodium sulfate. After filtration, the filtrate was concentrated under reduced pressure to give a crude product (1.0 g, yield: quantitative) of the title compound as a solid.

### (95B) 3-[2-(methoxymethoxy)-6-methyl-4-(trifluoromethyl)phenyl]thieno[3,2-c]pyridazin-7-ol

Methyl 3-[2-(methoxymethoxy)-6-methyl-4-(trifluoromethyl)phenyl]-7-triisopropylsilyloxy-thieno[3,2-c]pyridazine-6-carboxylate (1.0 g, 1.7 mmol) of Example 95A was dissolved in methanol (12 mL), 5 M sodium hydroxide (2.4 mL, 12 mmol) was added, and the mixture was stirred in the nitrogen-substituted system at 90°C for 11 hr. To the reaction solution was added 1 M hydrochloric acid, and the mixture was subjected to extraction with a mixed solvent of chloroform:2-propanol=3:1. The organic layer was washed with saturated brine, and dried over anhydrous sodium sulfate. After filtration, the filtrate was concentrated under reduced pressure, the obtained residue was dissolved in DMSO (9.0 mL), and the solution was stirred in the nitrogen-substituted system at 80°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: DCM/methanol=100/0-95/5(V/V)] to give the title compound (419 mg, yield: 66%) as a solid.

### (95C) 3-[2-(methoxymethoxy)-6-methyl-4-(trifluoromethyl)phenyl]thieno[3,2-c]pyridazin-7-yl trifluorosulfonate

3-[2-(Methoxymethoxy)-6-methyl-4-(trifluoromethyl)phenyl]thieno[3,2-c]pyridazin-7-ol (419 mg, 1.1 mmol) of Example 95B, and DIPEA (0.40 mL, 2.3 mmol) were dissolved in DCM (5.6 mL), Tf₂O (0.23 mL, 1.4 mmol) was added, and the mixture was stirred in the nitrogen-substituted system at 0°C for 1.5 hr. To the reaction solution was added water, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =90/10-70/30(V/V)] to give the title compound (109 mg, yield: 19%) as an oil.

### (95D) tert-butyl 5-{3-[2-(methoxymethoxy)-6-methyl-4-(trifluoromethyl)phenyl]thieno[3,2-c]pyridazin-7-yl}-3,6-dihydropyridine-1(2H)-carboxylate

3-[2-(Methoxymethoxy)-6-methyl-4-(trifluoromethyl)phenyl]thieno[3,2-c]pyridazin-7-yl trifluorosulfonate (109 mg, 0.22 mmol) of Example 95C, tert-butyl 3-(4,4,5,5-tetramethyl-1,3,2-dioxaborolan-2-yl)-5,6-dihydropyridine-1(2H)-carboxylate (84 mg, 0.26 mmol) and potassium carbonate (100 mg, 0.71 mmol) were dissolved in a mixed solvent of 1,4-dioxane (1.1 mL) and water (0.72 mL), Pd(dppf)Cl₂ (17.8 mg, 0.022 mmol) was added, and the mixture was stirred in the nitrogen-substituted system at 90°C for 10 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =90/10-70/30(V/V)] to give the title compound (66 mg, yield: 37%) as an amorphous form.

### (95E) tert-butyl 3-{3-[2-(methoxymethoxy)-6-methyl-4-(trifluoromethyl)phenyl]thieno[3,2-c]pyridazin-7-yl}piperidine-1-carboxylate

tert-Butyl 5-{3-[2-(methoxymethoxy)-6-methyl-4-(trifluoromethyl)phenyl]thieno[3,2-c]pyridazin-7-yl}-3,6-dihydropyridine-1(2H)-carboxylate (28 mg, 0.053 mmol) of Example 95D was dissolved in acetic acid (1.0 mL), 10% palladium on carbon (10 mg) was added, and the mixture was stirred in the hydrogen-substituted system at 60°C for 1 hr. The reaction solution was filtered through Celite, and the filtrate was concentrated under reduced pressure. To the obtained residue was added saturated aqueous sodium bicarbonate solution, and the mixture was subjected to extraction with chloroform. The organic layer was washed with saturated brine, and dried over anhydrous sodium sulfate. After filtration, the filtrate was concentrated under reduced pressure, and the obtained residue was purified by silica gel column chromatography [eluent: n-hexane/DCM/ethyl acetate =50/50/0-40/40/20(V/V)] to give the title compound (10 mg, yield: 36%) as a racemate (an amorphous form).

### (95F) 3-methyl-2-[7-(piperidin-3-yl)thieno[3,2-c]pyridazin-3-yl]-5-(trifluoromethyl)phenol dihydrochloride

tert-Butyl 3-{3-[2-(methoxymethoxy)-6-methyl-4-(trifluoromethyl)phenyl]thieno[3,2-c]pyridazin-7-yl}piperidine-1-carboxylate (10 mg, 0.019 mmol) of Example 95E was dissolved in methanol (0.50 mL), 4 M hydrogen chloride-1,4-dioxane (0.50 mL) was added, and the mixture was stirred at room temperature for 4 hr. The reaction solution was concentrated under reduced pressure to give a crude product (10 mg, yield: quantitative) of the title compound as a racemate (a solid).

### (95G) 3-methyl-2-[7-(1-methylpiperidin-3-yl)thieno[3,2-c]pyridazin-3-yl]-5-(trifluoromethyl)phenol

3-Methyl-2-[7-(piperidin-3-yl)thieno[3,2-c]pyridazin-3-yl]-5-(trifluoromethyl)phenol dihydrochloride (10 mg, 0.019 mmol) of Example 95F was dissolved in DCM (0.40 mL), then DIPEA (0.0065 mL, 0.038 mmol), 37% formaldehyde (0.0021 mL, 0.028 mmol), acetic acid (0.0022 mL, 0.038 mmol) and NaBH(OAc)₃ (16 mg, 0.076 mmol) were added at 0°C, and the mixture was stirred at room temperature for 1 hr. To the reaction solution was added saturated aqueous sodium bicarbonate solution, and the reaction solution was subjected to extraction with a mixed solvent of chloroform:2-propanol=3:1. The organic layer was washed with saturated brine, and dried over anhydrous sodium sulfate. After filtration, the filtrate was concentrated under reduced pressure, and the obtained residue was purified by silica gel column chromatography [eluent: ethyl acetate/methanol=100/0-50/50(V/V)] to give the title compound (10 mg, yield: 45%) as a racemate (a solid).

### (Example 96)

### 3-methyl-2-{8-[(3R or 3S)-1-methylpiperidin-3-yl]cinnolin-3-yl}-5-(trifluoromethyl)phenol

### (96A) 3-chloro-5,6,7,8-tetrahydrocinnoline

To 5,6,7,8-tetrahydrocinnolin-3-ol (11.3 g, 75 mmol) was added phosphorus oxychloride (69 mL, 750 mmol) at 0°C, and the mixture was stirred in the nitrogen-substituted system at 90°C for 5 hr. The reaction solution was concentrated under reduced pressure, to the obtained residue was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =70/30-0/100(V/V)] to give the title compound (8.88 g, yield: 70%) as an oil.

### (96B) 3-chloro-5,6,7,8-tetrahydrocinnoline-1-oxide

3-Chloro-5,6,7,8-tetrahydrocinnoline (8.76 g, 52 mmol) of Example 96A was dissolved in DCM (260 mL), metachloroperbenzoic acid (13.8 g, 52 mmol) was added, and the mixture was stirred in the nitrogen-substituted system at room temperature for 3 hr. To the reaction solution was added saturated aqueous sodium bicarbonate solution, and the mixture was subjected to extraction with ethyl acetate. The organic layer was washed with sodium thiosulfate aqueous solution and saturated brine, and dried over anhydrous sodium sulfate. After filtration, the filtrate was concentrated under reduced pressure, and the obtained residue was purified by NH silica gel column chromatography [eluent: n-hexane/ethyl acetate =80/20-20/80(V/V)] to give the title compound (8.26 g, yield: 86%) as a solid.

### (96C) 3-chloro-5,6,7,8-tetrahydrocinnolin-8-ol

3-Chloro-5,6,7,8-tetrahydrocinnoline-1-oxide (8.22 g, 45 mmol) of Example 96B was dissolved in THF (223 mL), trifluoroacetic anhydride (124 mL, 890 mmol) was added at 0°C, and the mixture was stirred in the nitrogen-substituted system at room temperature for 2 hr. The reaction solution was concentrated under reduced pressure, to the residue was added 1 M sodium hydroxide aqueous solution (89 mL, 89 mmol), and the mixture was stirred at room temperature for 2 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =80/20-20/80(V/V)] to give the title compound (5.36 g, yield: 65%) as a solid.

### (96D) 3-chloro-6,7-dihydrocinnolin-8(5H)-one

3-Chloro-5,6,7,8-tetrahydrocinnolin-8-ol (1.21 g, 6.6 mmol) of Example 96C was dissolved in DCM (33 mL), Dess-Martin periodinane (3.06 g, 7.2 mmol) was added, and the mixture was stirred in the nitrogen-substituted system at room temperature for 1 hr. The reaction solution was diluted with ethyl acetate, and the mixture was filtered through Celite. The filtrate was concentrated under reduced pressure, and the obtained residue was purified by silica gel column chromatography [eluent: n-hexane/ethyl acetate =60/40-0/100(V/V)] to give the title compound (620 mg, yield: 52%) as a solid.

### (96E) (3-chloro-5,6-dihydrocinnolin-8-yl)oxy-triisopropylsilane

3-Chloro-6,7-dihydrocinnolin-8(5H)-one (613 mg, 3.4 mmol) of Example 96D, and TEA (2.32 mL, 17 mmol) were dissolved in 1,4-dioxane (33 mL), triisopropylsilyl triflate (1.35 mL, 5.0 mmol) was added, and the mixture was stirred in the nitrogen-substituted system at room temperature for 1 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure to give a crude product (1.12 g, yield: 98%) of the title compound as an amorphous form.

### (96F) (3-chlorocinnolin-8-yl)oxy-triisopropylsilane

(3-Chloro-5,6-dihydrocinnolin-8-yl)oxy-triisopropylsilane (1.12 g, 3.3 mmol) of Example 96E was dissolved in 1,4-dioxane (17 mL), DDQ (900 mg, 4.0 mmol) was added, and the mixture was stirred in the nitrogen-substituted system at 70°C for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-80/20(V/V)] to give the title compound (887 mg, yield: 80%) as an oil.

### (96G) triisopropyl-[3-[2-(methoxymethoxy)-6-methyl-4-(trifluoromethyl)phenyl]cinnolin-8-yl]silane

(3-Chlorocinnolin-8-yl)oxy-triisopropylsilane (314 mg, 1.3 mmol) of Example 96F, 2-[2-(methoxymethoxy)-6-methyl-4-(trifluoromethyl)phenyl]-4,4,5,5-tetramethyl-1,3,2-dioxaborolane (595 mg, 1.9 mmol) of Example 1B, and potassium carbonate (347 mg, 2.5 mmol) were dissolved in a mixed solvent of 1,4-dioxane (6.0 mL) and water (1.5 mL), XPhos Pd G2 (98 mg, 0.13 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =40/60-20/80(V/V)] to give the title compound (497 mg, yield: 98%) as an amorphous form.

### (96H) 3-[2-(methoxymethoxy)-6-methyl-4-(trifluoromethyl)phenyl]cinnolin-8-ol

Triisopropyl-[3-[2-(methoxymethoxy)-6-methyl-4-(trifluoromethyl)phenyl]cinnolin-8-yl]silane (920 mg, 1.8 mmol) of Example 96G was dissolved in THF (18 mL), TBAF (1.0 M THF solution) (2.3 mL, 2.3 mmol) was added at 0°C, and the mixture was stirred in the nitrogen-substituted system at room temperature for 1 hr. To the reaction solution was added saturated aqueous sodium bicarbonate solution, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =100/0-65/35(V/V)] to give the title compound (489 mg, yield: 76%) as a solid.

### (961) 3-[2-(methoxymethoxy)-6-methyl-4-(trifluoromethyl)phenyl]cinnolin-8-yl trifluoromethanesulfonate

3-[2-(Methoxymethoxy)-6-methyl-4-(trifluoromethyl)phenyl]cinnolin-8-ol (486 mg, 1.3 mmol) of Example 96H, and TEA (0.74 mL, 5.3 mmol) were dissolved in DCM (13 mL), Tf₂O (0.34 mL, 2.0 mmol) was added, and the mixture was stirred in the nitrogen-substituted system at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-60/40(V/V)] to give the title compound (565 mg, yield: 85%) as an amorphous form.

### (96J) tert-butyl 5-{3-[2-(methoxymethoxy)-6-methyl-4-(trifluoromethyl)phenyl]cinnolin-8-yl}-3,6-dihydropyridine-1(2H)-carboxylate

3-[2-(Methoxymethoxy)-6-methyl-4-(trifluoromethyl)phenyl]cinnolin-8-yl trifluoromethanesulfonate (565 mg, 1.1 mmol) of Example 96I, tert-butyl 3-(4,4,5,5-tetramethyl-1,3,2-dioxaborolan-2-yl)-5,6-dihydropyridine-1(2H)-carboxylate (422 mg, 1.4 mmol) and potassium carbonate (315 mg, 2.3 mmol) were dissolved in a mixed solvent of 1,4-dioxane (11 mL) and water (4.0 mL), Pd(dppf)Cl₂ (93 mg, 0.11 mmol) was added, and the mixture was stirred under microwave irradiation in the nitrogen-substituted system at 120°C for 1 hr. The reaction solution was allowed to room temperature, water was added, and the mixture was subjected to extraction with ethyl acetate. The organic layer was washed with saturated brine, and dried over anhydrous sodium sulfate. After filtration, the filtrate was concentrated under reduced pressure, and the obtained residue was purified by NH silica gel column chromatography [eluent: n-hexane/ethyl acetate =100/0-60/40(V/V)] to give the title compound (583 mg, yield: 97%) as an amorphous form.

### (96K) tert-butyl 3-{3-[2-(methoxymethoxy)-6-methyl-4-(trifluoromethyl)phenyl]-7,8-dihydrocinnolin-8-yl}piperidine-1-carboxylate

tert-Butyl 5-{3-[2-(methoxymethoxy)-6-methyl-4-(trifluoromethyl)phenyl]cinnolin-8-yl}-3,6-dihydropyridine-1(2H)-carboxylate (581 mg, 1.1 mmol) of Example 96J was dissolved in ethanol (11 mL), 20% palladium hydroxide carbon (155 mg) was added, and the mixture was stirred in the hydrogen-substituted system at room temperature for 1 hr. The reaction solution was filtered through Celite, and the filtrate was concentrated under reduced pressure to give a crude product (585 mg, yield: 99%) of the title compound as an oil.

### (96L) tert-butyl 3-{3-[2-(methoxymethoxy)-6-methyl-4-(trifluoromethyl)phenyl]cinnolin-8-yl}piperidine-1-carboxylate

tert-Butyl 3-{3-[2-(methoxymethoxy)-6-methyl-4-(trifluoromethyl)phenyl]-7,8-dihydrocinnolin-8-yl}piperidine-1-carboxylate (585 mg, 1.1 mmol) of Example 96K was dissolved in 1,4-dioxane (10 mL), DDQ (299 mg, 1.3 mmol) was added, and the mixture was stirred in the nitrogen-substituted system at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =100/0-60/40(V/V)] to give the title compound (428 mg, yield: 73%) as a racemate (an amorphous form).

### (96M) tert-butyl (3S or 3R)-3-{3-[2-(methoxymethoxy)-6-methyl-4-(trifluoromethyl)phenyl]cinnolin-8-yl}piperidine-1-carboxylate

tert-Butyl 3-{3-[2-(methoxymethoxy)-6-methyl-4-(trifluoromethyl)phenyl]cinnolin-8-yl}piperidine-1-carboxylate (423 mg, 0.80 mmol) of Example 96L was subjected to chiral HPLC [column: CHIRALPAK IG (20 mm I.D.× 250 mm), mobile phase: n-hexane/2-propanol=85/15(V/V), temperature: 40°C] to give the title compound eluting earlier (196 mg, yield: 46%) as a single enantiomer (a solid).

### (96N) 3-methyl-2-{8-[(3S or 3R)-piperidin-3-yl]cinnolin-3-yl}-5-(trifluoromethyl)phenol

To tert-butyl (3S or 3R)-3-{3-[2-(methoxymethoxy)-6-methyl-4-(trifluoromethyl)phenyl]cinnolin-8-yl}piperidine-1-carboxylate (196 mg, 0.37 mmol) of Example 96M was added 4 M hydrogen chloride-1,4-dioxane (3 mL), and the mixture was stirred at room temperature for 1 hr. The reaction solution was concentrated under reduced pressure, to the obtained residue was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure to give a crude product (136 mg, yield: 95%) of the title compound as a single enantiomer (a solid).

### (960) 3-methyl-2-{8-[(3R or 3S)-1-methylpiperidin-3-yl]cinnolin-3-yl}-5-(trifluoromethyl)phenol

3-Methyl-2-{8-[(3S or 3R)-piperidin-3-yl]cinnolin-3-yl}-5-(trifluoromethyl)phenol (104 mg, 0.27 mmol) of Example 96N was dissolved in a mixed solvent of methanol (0.2 mL) and DCM (2 mL), then 37% formaldehyde (0.040 mL, 0.54 mmol) and NaBH(OAc)₃ (228 mg, 1.1 mmol) were added, and the mixture was stirred at room temperature for 40 min. To the reaction solution was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=100/0-90/10(V/V)] to give the title compound (82 mg, yield: 76%) as a single enantiomer (a solid).

### (Example 97)

### 2-{8-[(3R or 3S)-1-ethylpiperidin-3-yl]cinnolin-3-yl}-3-methyl-5-(trifluoromethyl)phenol

3-Methyl-2-{8-[(3S or 3R)-piperidin-3-yl]cinnolin-3-yl}-5-(trifluoromethyl)phenol (32 mg, 0.056 mmol) of Example 96N was dissolved in a mixed solvent of methanol (0.1 mL) and DCM (1 mL), then acetaldehyde (0.041 mL, 0.82 mmol) and NaBH(OAc)₃ (70 mg, 0.33 mmol) were added, and the mixture was stirred at room temperature for 1 hr. To the reaction solution was added saturated aqueous sodium bicarbonate solution, and the mixture was subjected to extraction with DCM. The organic layer was washed with saturated brine, and dried over anhydrous sodium sulfate. After filtration, the filtrate was concentrated under reduced pressure, and the obtained residue was purified by NH silica gel column chromatography [eluent: ethyl acetate/methanol=100/0-20/10(V/V)] to give the title compound (20 mg, yield: 58%) as a single enantiomer (a solid).

### (Example 98)

### 2-[(4bR,7aR or 4bS,7aS)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol

### (98A) 3-chloro-4-[(2,4-dimethoxyphenyl)methyl]-7,8-dihydro-6H-furo[2',3':4,5]pyrrolo[2,3-c]pyridazine

3,6-Dichloro-4-(3-iodo-4,5-dihydrofuran-2-yl)pyridazine (1.11 g, 3.2 mmol) of Example 83D was dissolved in NMP (33 mL), then 2,4-dimethoxybenzylamine (0.98 mL, 6.5 mmol), L-proline (0.077 g, 0.67 mmol), copper(I) iodide (0.117 g, 0.61 mmol) and potassium carbonate (1.34 g, 9.7 mmol) were added, and the mixture was stirred in the nitrogen-substituted system at 120°C for 1 hr. To the reaction solution was added water at 0°C, and the mixture was subjected to extraction with DCM. The organic layer was washed with saturated ammonium chloride aqueous solution and saturated brine, and dried over anhydrous sodium sulfate. After filtration, the filtrate was concentrated under reduced pressure, and the obtained residue was purified by NH silica gel column chromatography [eluent: n-hexane/ethyl acetate =75/25-65/35(V/V)]. To the obtained solid were added ethyl acetate and n-hexane, and the mixture was subjected to ultrasonic treatment. The solid was collected by filtration to give the title compound (422 mg, yield: 38%) as a solid.

### (98B) 3-chloro-7,8-dihydro-6H-furo[2',3':4,5]pyrrolo[2,3-c]pyridazine

3-Chloro-4-[(2,4-dimethoxyphenyl)methyl]-7,8-dihydro-6H-furo[2',3':4,5]pyrrolo[2,3-c]pyridazine (422 mg, 1.2 mmol) of Example 98A was dissolved in TFA (6.0 mL), trifluoromethanesulfonic acid (0.32 mL, 3.6 mmol) was added, and the mixture was stirred at room temperature for 7 hr. The reaction solution was added dropwise to saturated aqueous sodium bicarbonate solution at 0°C for neutralization, and the mixture was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: DCM/methanol=100/0-90/10(V/V)] to give the title compound (124 mg, yield: 52%) as a solid.

### (98C) 3-chloro-8-(5-fluoropyrimidin-2-yl)-7,8-dihydro-6H-furo[2',3':4,5]pyrrolo[2,3-c]pyridazine

3-Chloro-7,8-dihydro-6H-furo[2',3':4,5]pyrrolo[2,3-c]pyridazine (104 mg, 0.53 mmol) of Example 98B was dissolved in toluene (5.3 mL), then 2-bromo-5-fluoropyrimidine (188 mg, 1.1 mmol), cesium carbonate (0.346 g, 1.1 mmol), 4,5-bis(diphenylphosphino)-9,9-dimethylxanthene (0.062 g, 0.11 mmol) and Pd2(dba)3 (0.049 g, 0.054 mmol) were added, and the mixture was stirred at 100°C for 4 hr. To the reaction solution was added saturated ammonium chloride, and the mixture was subjected to extraction with chloroform/methanol. The organic layer was washed with saturated brine, and dried over anhydrous sodium sulfate. After filtration, the filtrate was concentrated under reduced pressure, and the obtained residue was purified by silica gel column chromatography [eluent: DCM/methanol=100/0-95/5(V/V)] to give the title compound (119 mg, yield: 77%) as a solid.

### (98D) 8-(5-fluoropyrimidin-2-yl)-3-[2-(methoxymethoxy)-6-methyl-4-(trifluoromethyl)phenyl]-7,8-dihydro-6H-furo[2',3':4,5]pyrrolo[2,3-c]pyridazine

3-Chloro-8-(5-fluoropyrimidin-2-yl)-7,8-dihydro-6H-furo[2',3':4,5]pyrrolo[2,3-c]pyridazine (109 mg, 0.37 mmol) of Example 98C, 2-[2-(methoxymethoxy)-6-methyl-4-(trifluoromethyl)phenyl]-4,4,5,5-tetramethyl-1,3,2-dioxaborolane (323 mg, 0.93 mmol) of Example 1B, and potassium carbonate (155 mg, 1.1 mmol) were dissolved in a mixed solvent of 1,4-dioxane (3.7 mL) and water (0.75 mL), XPhos Pd G2 (59 mg, 0.075 mmol) was added, and the mixture was stirred under microwave irradiation in the nitrogen-substituted system at 120°C for 2 hr. The reaction solution was concentrated under reduced pressure, and the obtained residue was purified by NH silica gel column chromatography [eluent: DCM/ethyl acetate =0/100-95/5(V/V)] and silica gel column chromatography [eluent: DCM/ethyl acetate =20/80-35/65(V/V)] to give the title compound (107 mg, yield: 60%) as a solid.

### (98E) 2-[8-(5-fluoropyrimidin-2-yl)-7,8-dihydro-6H-furo[2',3':4,5]pyrrolo[2,3-c]pyridazin-3-yl]-3-methyl-5-(trifluoromethyl)phenol

To 8-(5-fluoropyrimidin-2-yl)-3-[2-(methoxymethoxy)-6-methyl-4-(trifluoromethyl)phenyl]-7,8-dihydro-6H-furo[2',3':4,5]pyrrolo[2,3-c]pyridazine (0.107 g, 0.23 mmol) of Example 98D was added 4 M hydrogen chloride-1,4-dioxane (2.2 mL), and the mixture was stirred at room temperature for 1 hr. To the reaction solution was added saturated aqueous sodium bicarbonate solution at 0°C, the mixture was subjected to extraction with DCM, and the organic layer was dried over anhydrous sodium sulfate. After filtration, the filtrate was concentrated under reduced pressure, and the obtained residue was purified by NH silica gel column chromatography [eluent: DCM/methanol=100/0-90/10(V/V)] to give the title compound (0.091 g, yield: 94%) as a solid.

### (98F) 2-[(4bR*,7aR*)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol

2-[8-(5-Fluoropyrimidin-2-yl)-7,8-dihydro-6H-furo[2',3':4,5]pyrrolo[2,3-c]pyridazin-3-yl]-3-methyl-5-(trifluoromethyl)phenol (5.3 mg, 0.012 mmol) of Example 98E was dissolved in THF (0.5 mL), ASCA-2 (5.3 mg) was added, and the mixture was stirred in the hydrogen-substituted system at room temperature for 2.5 hr. The reaction solution was filtered through Celite, the filtrate was concentrated under reduced pressure, and the obtained residue was purified by NH silica gel column chromatography [eluent: DCM/methanol=100/0-90/10(V/V)] to give the title compound (3.7 mg, yield: 69%) as a racemate (an amorphous form).

### (98G) 2-[(4bR,7aR or 4bS,7aS)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol

2-[(4bR*,7aR*)-8-(5-Fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol (25 mg, 0.058 mmol) of Example 98F was subjected to chiral HPLC [column: CHIRALPAK IG (20 mm I.D.× 250 mm), mobile phase: n-hexane/2-propanol=60/40(V/V), temperature: 40°C] to give the title compound eluting later (11 mg, yield: 44%) as a single enantiomer (a solid).

### (Example 99)

### 2-{8-[(1s,3s)-3-hydroxy-3-methylcyclobutyl]-7,8-dihydro-5H-furo[3',4':4,5]pyrrolo[2,3-c]pyridazin-3-yl}-3-methyl-5-(trifluoromethyl)phenol (also referred to as 2-[8-(cis-3-hydroxy-3-methylcyclobutyl)-7,8-dihydro-5H-furo[3',4':4,5]pyrrolo[2,3-c]pyridazin-3-yl]-3-methyl-5-(trifluoromethyl)phenol)

### (99A) 6-chloro-4-(2,5-dihydrofuran-3-yl)pyridazin-3-amine

3-Amino-4-bromo-6-chloropyridazine (201 mg, 0.96 mmol), 2-(2,5-dihydrofuran-3-yl)-4,4,5,5-tetramethyl-1,3,2-dioxaborolane (216 mg, 1.1 mmol) and potassium carbonate (265 mg, 1.9 mmol) were dissolved in a mixed solvent of 1,4-dioxane (4 mL) and water (1 mL), Pd(dppf)Cl₂ (78 mg, 0.095 mmol) was added, and the mixture was stirred in the nitrogen-substituted system at 100°C for 1 hr. To the reaction solution was added water, and the mixture was subjected to extraction with chloroform. The organic layer was washed with saturated brine, and dried over anhydrous sodium sulfate. After filtration, the filtrate was concentrated under reduced pressure, and the obtained residue was purified by NH silica gel column chromatography [eluent: DCM/methanol=100/0-90/10(V/V)]. To the obtained residue was added DEE, and the solid was collected by filtration to give the title compound (193 mg, yield: 98%) as a solid.

### (99B) N-[6-chloro-4-(2,5-dihydrofuran-3-yl)pyridazin-3-yl]-4-methylbenzenesulfonamide

6-Chloro-4-(2,5-dihydrofuran-3-yl)pyridazin-3-amine (55 mg, 0.28 mmol) of Example 99A was suspended in THF (1 mL), 55% sodium hydride (24 mg, 0.55 mmol) was added at 0°C, and the mixture was stirred for 30 min. To the reaction solution was added p-toluenesulfonyl chloride (74 mg, 0.39 mmol), and the mixture was stirred at room temperature for 1.5 hr. To the reaction solution was added saturated ammonium chloride aqueous solution at 0°C, and the mixture was subjected to extraction with ethyl acetate. The organic layer was washed successively with water and saturated brine, and dried over anhydrous sodium sulfate. After filtration, the filtrate was concentrated under reduced pressure, and the obtained residue was purified by silica gel column chromatography [eluent: n-hexane/ethyl acetate =50/50-71/29(V/V)] to give the title compound (37 mg, yield: 38%) as a solid substance.

### (99C) 3-chloro-8-[(4-methylphenyl)sulfonyl]-7,8-dihydro-5H-furo[3',4':4,5]pyrrolo[2,3-c]pyridazine

N-[6-Chloro-4-(2,5-dihydrofuran-3-yl)pyridazin-3-yl]-4-methylbenzenesulfonamide (37 mg, 0.11 mmol) of Example 99B was dissolved in toluene (2.0 mL), then molecular sieve 4A (20 mg), N-fluorobenzenesulfonimide (35 mg, 0.11 mmol) and diphenyl diselenide (6 mg, 0.02 mmol) were added, and the mixture was stirred at 100°C for 3.5 hr. N-Fluorobenzenesulfonimide (37 mg, 0.12 mmol) was added again, and the mixture was stirred at 100°C for 2.5 hr. The reaction solution was concentrated under reduced pressure, and the obtained residue was purified by silica gel column chromatography [eluent: n-hexane/ethyl acetate =70/30-50/50(V/V)] to give the title compound (13 mg, yield: 35%) as a solid.

### (99D) 3-chloro-7,8-dihydro-5H-furo[3',4':4,5]pyrrolo[2,3-c]pyridazine

3-Chloro-8-[(4-methylphenyl)sulfonyl]-7,8-dihydro-5H-furo[3',4':4,5]pyrrolo[2,3-c]pyridazine (1.48 g, 4.2 mmol) of Example 99C was suspended in ethanol (50 mL), potassium hydroxide (2.3 g, 42 mmol) was added, and the mixture was stirred at 90°C for 1 hr. The reaction solution was allowed to room temperature, and neutralized with acetic acid. The mixture was concentrated under reduced pressure, to the obtained residue was added water, and the solid was collected by filtration to give the title compound (737 mg, yield: 89%) as a solid substance.

### (99E) 3-(3-chloro-5,7-dihydro-8H-furo[3',4':4,5]pyrrolo[2,3-c]pyridazin-8-yl)cyclobutanone

3-Oxocyclobutanecarboxylic acid (224 mg, 2.0 mmol) was dissolved in toluene (65 mL), iodomesitylene diacetate (358 mg, 0.98 mmol) was added, and the mixture was concentrated under reduced pressure. The obtained residue was dissolved in dioxane (16 mL), then 3-chloro-7,8-dihydro-5H-furo[3',4':4,5]pyrrolo[2,3-c]pyridazine (102 mg, 0.52 mmol) of Example 99D, tris(2-phenylpyridinato)iridium(III) (7 mg, 0.01 mmol), copper(I) 2-thiophenecarboxylate (20 mg, 0.10 mmol), 1,10-phenanthroline (52 mg, 0.16 mmol) and 2-tert-butyl-1,1,3,3-tetramethylguanidine (0.12 mL, 1.1 mmol) were added, and the mixture was stirred under blue light irradiation (450 nm) at room temperature for 1 hr. To the reaction solution was added water, and the mixture was subjected to extraction with ethyl acetate. The organic layer was washed with saturated brine, and dried over anhydrous sodium sulfate. After filtration, the filtrate was concentrated under reduced pressure, and the obtained residue was purified by silica gel column chromatography [eluent: n-hexane/ethyl acetate =50/50-75/25(V/V)] to give the title compound (71 mg, yield: 52%) as a solid substance.

### (99F) (1s,3s)-3-(3-chloro-5,7-dihydro-8H-furo[3',4':4,5]pyrrolo[2,3-c]pyridazin-8-yl)-1-methylcyclobutanol

3-(3-Chloro-5,7-dihydro-8H-furo[3',4':4,5]pyrrolo[2,3-c]pyridazin-8-yl)cyclobutanone (93 mg, 0.35 mmol) of Example 99E was dissolved in DCM (3.5 mL), cerium(III) chloride (0.203 g, 0.82 mmol) was added at 0°C, and the mixture was stirred at 0°C for 20 min. Methylmagnesium iodide (3.0 M DEE solution) (0.23 mL, 0.70 mmol) was added at 0°C, and the mixture was stirred at 0°C for 1.5 hr. To the reaction solution were added DCM and saturated ammonium chloride aqueous solution, the mixture was filtered through Celite, and the filtrate was subjected to extraction with DCM. The organic layer was washed with saturated brine, and dried over anhydrous sodium sulfate. After filtration, the filtrate was concentrated under reduced pressure, and the obtained residue was purified by silica gel column chromatography [eluent: DCM/ethyl acetate =50/50-100/0(V/V)] and NH silica gel column chromatography [eluent: DCM/2-propanol=100/0-96/4(V/V)] to give the title compound eluting earlier (28 mg, yield: 28%) as a single diastereomer (a solid).

### (99G) (1s,3s)-3-{3-[2-(methoxymethoxy)-6-methyl-4-(trifluoromethyl)phenyl]-5,7-dihydro-8H-furo[3',4':4,5]pyrrolo[2,3-c]pyridazin-8-yl}-1-methylcyclobutanol

(1s,3s)-3-(3-Chloro-5,7-dihydro-8H-furo[3',4':4,5]pyrrolo[2,3-c]pyridazin-8-yl)-1-methylcyclobutanol (28 mg, 0.10 mmol) of Example 99F, 2-[2-(methoxymethoxy)-6-methyl-4-(trifluoromethyl)phenyl]-4,4,5,5-tetramethyl-1,3,2-dioxaborolane (88 mg, 0.25 mmol) of Example 1B, and potassium carbonate (43 mg, 0.31 mmol) were dissolved in a mixed solvent of 1,4-dioxane (1.0 mL) and water (0.20 mL), XPhos Pd G2 (18 mg, 0.023 mmol) was added, and the mixture was stirred under microwave irradiation in the nitrogen-substituted system at 120°C for 2 hr. The reaction solution was allowed to room temperature, and dried over anhydrous sodium sulfate. After filtration, the filtrate was concentrated under reduced pressure, and the obtained residue was purified by silica gel column chromatography [eluent: n-hexane/ethyl acetate =50/50-0/100(V/V)] to give the title compound (19 mg, yield: 41%) as an amorphous form.

### (99H) 2-{8-[(1s,3s)-3-hydroxy-3-methylcyclobutyl]-7,8-dihydro-5H-furo[3',4':4,5]pyrrolo[2,3-c]pyridazin-3-yl}-3-methyl-5-(trifluoromethyl)phenol

To (1s,3s)-3-{3-[2-(methoxymethoxy)-6-methyl-4-(trifluoromethyl)phenyl]-5,7-dihydro-8H-furo[3',4':4,5]pyrrolo[2,3-c]pyridazin-8-yl}-1-methylcyclobutanol (19 mg, 0.23 mmol) of Example 99G was added 4 M hydrogen chloride-1,4-dioxane (0.50 mL), and the mixture was stirred at room temperature for 1 hr. To the reaction solution were added DCM, methanol and saturated aqueous sodium bicarbonate solution at 0°C, the mixture was concentrated under reduced pressure, and the obtained residue was purified by NH silica gel column chromatography [eluent: DCM/methanol=100/0-90/10(V/V)] to give the title compound (19 mg, yield: quantitative) as a solid.

Physicochemical data of the compounds described in the examples are shown below.

In the tables, Ex indicates the example number, and Data indicates the physicochemical data.

**[Table 3]**

| Ex | Data |
|---|---|
| 1A | 1H-NMR (CDCl3) δ: 7.17 (1H, s), 7.09 (1H, s), 5.28 (2H, s), 3.53 (3H, s), 2.53 (3H, s). |
| 1B | 1H-NMR (CDCl3) δ: 7.05 (2H, s), 5.16 (2H, s), 3.47 (3H, s), 2.39 (3H, s), 1.39 (12H, s). |
| | MS(ESI/APCI) m/z: 347 [M - H]- |
| 1C | 1H-NMR (CDCl3) δ: 7.60 (1H, s), 6.92 (1H, dd, J = 17.5, 11.1 Hz), 6.11 (1H, d, J = 17.5 Hz), 5.86 (1H, d, J = 11.1 Hz). |
| | MS(ESI/APCI) m/z: 175 [M + H]+ |
| 1D | 1H-NMR (CDCl3) δ: 6.90 (1H, s), 4.17-3.84 (3H, m), 3.72-3.59 (2H, m), 3.12-2.98 (3H, m), 2.87-2.69 (1H, m), 2.00-1.90 (1H, m), 1.87-1.72 (2H, m), 1.65-1.54 (1H, m), 1.45 (9H, s). |
| | MS(ESI/APCI) m/z: 339 [M + H]+ |
| 1E | 1H-NMR (CDCl3) δ: 7.28 (1H, s), 7.21 (1H, s), 6.91 (1H, s), 5.13-5.07 (2H, m), 4.27-3.93 (3H, m), 3.74-3.61 (2H, m), 3.38 (3H, s), 3.15-3.05 (3H, m), 2.87-2.72 (1H, m), 2.23 (3H, s), 2.07-1.99 (1H, m), 1.90-1.73 (2H, m), 1.70-1.60 (1H, m), 1.46 (9H, s). |
| | MS(ESI/APCI) m/z: 523 [M + H]+ |
| 1F | MS(ESI/APCI) m/z: 379 [M + H]+ |
| 1G | 1H-NMR (CDCl3) δ: 7.18 (1H, s), 7.15 (1H, s), 7.02 (1H, s), 4.32-4.22 (1H, m), 3.81-3.66 (2H, m), 3.12 (2H, t, J = 8.2 Hz), 2.97 (1H, d, J = 7.3 Hz), 2.77 (1H, d, J = 11.0 Hz), 2.46 (3H, s), 2.31 (3H, s), 2.20 (1H, t, J = 10.4 Hz), 2.01-1.89 (2H, m), 1.84-1.71 (2H, m), 1.66-1.57 (1H, m). |
| | MS(ESI/APCI) m/z: 393 [M + H]+ |
| 2A | 1H-NMR (CDCl3) δ: 7.70 (1H, s), 7.61 (1H, d, J = 3.1 Hz), 6.47 (1H, d, J = 3.1 Hz), 4.99-4.96 (1H, m), 4.25-4.22 (1H, m), 3.99-3.96 (1H, m), 3.55-3.45 (1H, m), 3.13-3.09 (1H, m), 2.25-2.22 (2H, m), 1.84-1.74 (2H, m), 1.47 (9H, s). |
| | MS(ESI/APCI) m/z: 281 [M - tBu + H]+ |
| 2B | MS(ESI/APCI) m/z: 237 [M + H]+ |
| 2C | 1H-NMR (CDCl3) δ: 7.92-7.90 (1H, m), 7.67 (1H, s), 6.42 (1H, d, J = 3.7 Hz), 5.18-5.14 (1H, m), 3.04-3.02 (1H, m), 2.66-2.61 (2H, m), 2.35 (3H, s), 2.34-2.24 (1H, m), 2.04-2.00 (2H, m), 1.84-1.76 (2H, m). |
| | MS(ESI/APCI) m/z: 251 [M + H]+ |
| 2D | 1H-NMR (CDCl3) δ: 7.84-7.82 (1H, m), 7.61 (1H, s), 7.22 (1H, s), 7.06 (1H, s), 6.49 (1H, d, J = 3.6 Hz), 5.28-5.27 (1H, m), 3.74 (3H, s), 3.13-3.10 (1H, m), 2.74-2.71 (1H, m), 2.63-2.61 (1H, m), 2.35 (3H, s), 2.31-2.28 (1H, m), 2.17 (3H, s), 2.03-1.99 (2H, m), 1.86-1.82 (2H, m). |
| | MS(ESI/APCI) m/z: 405 [M + H]+ |
| 2E | 1H-NMR (CDCl3) δ: 8.02-7.98 (1H, m), 7.90 (1H, s), 7.21 (1H, s), 7.12 (1H, s), 6.49 (1H, d, J = 3.6 Hz), 5.30-5.25 (1H, m), 3.75 (3H, s), 3.13-3.11 (1H, m), 2.74-2.73 (1H, m), 2.64-2.61 (1H, m), 2.36 (3H, s), 2.32-2.30 (1H, m), 2.17 (3H, s), 2.14-2.13 (1H, m), 2.05-2.03 (1H, m), 1.86-1.81 (2H, m). |
| | MS(ESI/APCI) m/z: 391 [M + H]+ |
| 3A | 1H-NMR (CDCl3) δ: 6.90 (1H, s), 4.18-4.09 (1H, m), 3.68 (2H, t, J = 8.2 Hz), 3.06-3.00 (2H, m), 2.56-2.42 (4H, m), 1.43 (3H, s). |
| | MS(ESI/APCI) m/z: 240 [M + H]+ |

**[Table 4 ]**

| | |
|---|---|
| 3B | 1H-NMR (CDCl3) δ: 7.70 (1H, s), 7.64 (1H, d, J = 3.1 Hz), 6.45 (1H, d, J = 3.7 Hz), 5.04-4.94 (1H, m), 2.90-2.80 (4H, m), 1.55 (3H, s). |
| | MS(ESI/APCI) m/z: 238 [M + H]+ |
| 3C | 1H-NMR (CDCl3) δ: 7.65 (1H, s), 7.64 (1H, d, J = 3.1 Hz), 7.34 (1H, s), 7.28-7.27 (1H, m), 6.52 (1H, d, J = 3.7 Hz), 5.13-5.08 (1H, m), 5.07 (2H, s), 3.33 (3H, s), 2.97-2.85 (4H, m), 2.16 (3H, s), 1.56 (3H, s). |
| | MS(ESI/APCI) m/z: 422 [M + H]+ |
| 3D | 1H-NMR (CDCl3) δ: 11.09 (1H, br s), 7.93 (1H, s), 7.74 (1H, d, J = 3.6 Hz), 7.22 (1H, s), 7.13 (1H, s), 6.62 (1H, d, J = 3.0 Hz), 5.17-5.05 (1H, m), 2.96-2.89 (2H, m), 2.88-2.80 (2H, m), 2.53 (3H, s), 1.56 (3H, s). |
| | MS(ESI/APCI) m/z: 378 [M + H]+ |
| 4A | 1H-NMR (CDCl3) δ: 7.36 (1H, s), 5.47 (1H, d, J = 1.2 Hz), 5.24 (1H, s), 2.14 (3H, d, J = 1.2 Hz). |
| | MS(ESI/APCI) m/z: 189 [M + H]+ |
| 4B | 1H-NMR (CDCl3) δ: 6.87 (1H, d, J = 1.2 Hz), 4.16-4.08 (1H, m), 3.82 (1H, t, J = 8.9 Hz), 3.37-3.30 (1H, m), 3.26-3.19 (1H, m), 2.53-2.43 (4H, m), 1.43 (3H, s), 1.35 (3H, d, J = 6.7 Hz). |
| | MS(ESI/APCI) m/z: 254 [M + H]+ |
| 4C | 1H-NMR (CDCl3) δ: 7.63 (1H, s), 7.40 (1H, s), 4.97-4.90 (1H, m), 2.87-2.74 (4H, m), 2.28 (3H, d, J = 1.2 Hz), 1.53 (3H, s). |
| | MS(ESI/APCI) m/z: 252 [M + H]+ |
| 4D | 1H-NMR (CDCl3) δ: 7.59 (1H, s), 7.40 (1H, s), 7.33 (1H, s), 7.28-7.27 (1H, m), 5.09-5.04 (3H, m), 3.33 (3H, s), 2.87 (4H, d, J = 8.5 Hz), 2.32 (3H, s), 2.15 (3H, s), 1.55 (3H, s). |
| | MS(ESI/APCI) m/z: 436 [M + H]+ |
| 4E | 1H-NMR (CDCl3) δ: 7.86 (1H, s), 7.51 (1H, s), 7.21 (1H, s), 7.13 (1H, s), 5.12-5.02 (1H, m), 2.92-2.85 (2H, m), 2.83-2.76 (2H, m), 2.54 (3H, s), 2.36 (3H, s), 1.57 (3H, s). |
| | MS(ESI/APCI) m/z: 392 [M + H]+ |
| 5A | 1H-NMR (CDCl3) δ: 7.72 (1H, d, J = 3.6 Hz), 7.66 (1H, s), 7.30 (2H, d, J = 6.8 Hz), 6.90 (2H, d, J = 6.8 Hz), 6.47 (1H, d, J = 3.6 Hz), 5.31-5.30 (1H, m), 3.81 (3H, s), 2.80-2.78 (2H, m), 2.65-2.63 (2H, m), 1.61 (3H, s). |
| | MS(ESI/APCI) m/z: 358 [M + H]+ |
| 5B | 1H-NMR (CDCl3) δ: 7.70 (1H, s), 7.53 (1H, d, J = 2.4 Hz), 7.30 (2H, d, J = 8.8 Hz), 6.90 (2H, d, J = 8.8 Hz), 5.44-5.31 (1H, m), 4.40 (2H, s), 3.80 (3H, s), 2.81-2.73 (2H, m), 2.62-2.49 (2H, m), 1.56 (3H, s). |
| | MS(ESI/APCI) m/z: 376 [M + H]+ |
| 5C | 1H-NMR (CDCl3) δ: 7.66 (1H, s), 7.53 (1H, d, J = 1.6 Hz), 7.34 (1H, s), 7.31 (2H, d, J = 8.8 Hz), 7.26 (1H, s), 6.90 (2H, d, J = 8.8 Hz), 5.59-5.48 (1H, m), 5.07 (2H, s), 4.41 (2H, s), 3.81 (3H, s), 3.33 (3H, s), 2.85-2.75 (2H, m), 2.66-2.55 (2H, m), 2.15 (3H, s), 1.62 (3H, s). |
| | MS(ESI/APCI) m/z: 560 [M + H]+ |
| 5D | 1H-NMR (CDCl3) δ: 7.94 (1H, s), 7.58 (1H, d, J = 2.4 Hz), 7.22 (1H, s), 7.14 (1H, s), 5.18-5.16 (1H, m), 2.92-2.87 (2H, m), 2.75-2.72 (2H, m), 2.53 (3H, s), 1.58 (3H, s). |
| | MS(ESI/APCI) m/z: 396 [M + H]+ |
| 6A | 1H-NMR (CDCl3) δ: 7.68 (1H, s), 7.64 (1H, s), 7.34 (1H, s), 7.27 (1H, s), 5.15-5.13 (1H, m), 5.08 (2H, s), 3.33 (3H, s), 2.90-2.86 (4H, m), 2.15 (3H, s). |
| | MS(ESI/APCI) m/z: 456 [M + H]+ |

**[Table 5]**

| | |
|---|---|
| 6B | 1H-NMR (CDCl3) δ: 7.93 (1H, s), 7.74 (1H, s), 7.21 (1H, s), 7.14 (1H, s), 5.15-5.13 (1H, m), 2.92-2.90 (2H, m), 2.79-2.76 (2H, m), 2.54 (3H, s), 1.58 (3H, s). |
| | MS(ESI/APCI) m/z: 412 [M + H]+ |
| 7A | 1H-NMR (CDCl3) δ: 6.88 (1H, t, J = 1.8 Hz), 4.66-4.58 (1H, m), 3.68 (2H, t, J = 8.3 Hz), 3.43 (2H, d, J = 5.5 Hz), 3.04-2.99 (2H, m), 2.48 (2H, td, J = 9.2, 2.5 Hz), 2.23 (1H, t, J = 5.5 Hz), 1.97-1.91 (2H, m), 1.21 (3H, s). |
| | MS(ESI/APCI) m/z: 254 [M + H]+ |
| 7B | 1H-NMR (CDCl3) δ: 6.85 (1H, d, J = 1.8 Hz), 4.87-4.76 (1H, m), 3.68 (2H, t, J = 8.2 Hz), 3.30 (2H, s), 3.02-2.98 (2H, m), 2.37-2.32 (2H, m), 1.85-1.80 (2H, m), 1.16 (3H, s), 0.92 (9H, s), 0.06 (6H, s). |
| | MS(ESI/APCI) m/z: 368 [M + H]+ |
| 7C | 1H-NMR (CDCl3) δ: 7.83 (1H, d, J = 3.7 Hz), 7.65 (1H, s), 6.45 (1H, d, J = 3.7 Hz), 5.69-5.58 (1H, m), 3.40 (2H, s), 2.64-2.58 (2H, m), 2.27-2.22 (2H, m), 1.27 (3H, s), 0.95 (9H, s), 0.11 (6H, s). |
| | MS(ESI/APCI) m/z: 366 [M + H]+ |
| 7D | 1H-NMR (CDCl3) δ: 7.85 (1H, d, J = 3.1 Hz), 7.61 (1H, s), 7.37-7.23 (2H, m), 6.54 (1H, d, J = 3.7 Hz), 5.84-5.78 (1H, m), 5.05 (2H, s), 3.43 (2H, s), 3.32 (3H, s), 2.70-2.63 (2H, m), 2.31-2.25 (2H, m), 2.15 (3H, s), 1.28 (3H, s), 0.98 (9H, s), 0.13 (6H, s). |
| | MS(ESI/APCI) m/z: 550 [M + H]+ |
| 7E | 1H-NMR (CDCl3) δ: 7.91 (1H, s), 7.88 (1H, d, J = 3.1 Hz), 7.22 (1H, s), 7.13 (1H, s), 6.62 (1H, d, J = 3.7 Hz), 5.67-5.58 (1H, m), 3.56 (2H, s), 2.84-2.77 (2H, m), 2.53 (3H, s), 2.42-2.35 (2H, m), 2.12 (1H, br s), 1.35 (3H, s). |
| | MS(ESI/APCI) m/z: 392 [M + H]+ |
| 8A | 1H-NMR (CDCl3) δ: 6.84 (1H, s), 3.67 (1H, t, J = 8.0 Hz), 3.29 (1H, t, J = 8.0 Hz), 3.11-3.09 (1H, m), 2.43-2.41 (1H, m), 2.32-2.30 (1H, m), 2.11-2.09 (2H, m), 1.85 (6H, m), 1.32 (3H, d, J = 6.4 Hz). |
| | MS(ESI/APCI) m/z: 280 [M + H]+ |
| 8B | 1H-NMR (CDCl3) δ: 7.59 (1H, s), 7.19 (1H, s), 2.72-2.71 (2H, m), 2.42-2.40 (2H, m), 2.26 (3H, s), 2.16-2.13 (2H, m), 1.99-1.98 (4H, m). |
| | MS(ESI/APCI) m/z: 278 [M + H]+ |
| 8C | 1H-NMR (CDCl3) δ: 7.55 (1H, s), 7.32 (1H, s), 7.26 (1H, s), 7.19 (1H, s), 5.06 (2H, s), 3.33 (3H, s), 2.78-2.76 (2H, m), 2.51-2.49 (2H, m), 2.30 (3H, s), 2.24-2.22 (2H, m), 2.16 (3H, s), 2.01-1.98 (4H, m). |
| | MS(ESI/APCI) m/z: 462 [M + H]+ |
| 8D | 1H-NMR (CDCl3) δ: 7.82 (1H, s), 7.26 (1H, s), 7.21 (1H, s), 7.12 (1H, s), 2.78-2.75 (2H, m), 2.54 (3H, s), 2.48-2.46 (2H, m), 2.34 (3H, s), 2.23-2.21 (2H, m), 2.04-2.02 (4H, m). |
| | MS(ESI/APCI) m/z: 418 [M + H]+ |
| 9A | 1H-NMR (CDCl3) δ: 6.87-6.82 (2H, m), 2.42 (3H, s). |
| 9B | 1H-NMR (CDCl3) δ: 6.94 (1H, d, J = 2.4 Hz), 6.90 (1H, d, J = 2.4 Hz), 5.23 (2H, s), 3.51 (3H, s), 2.44 (3H, s). |
| 9C | 1H-NMR (CDCl3) δ: 6.85 (1H, s), 6.81 (1H, s), 5.12 (2H, s), 3.46 (3H, s), 2.32 (3H, s), 1.38 (12H, s). |
| 9D | 1H-NMR (CDCl3) δ: 7.58 (1H, s), 7.38 (1H, s), 7.12 (1H, s), 7.01 (1H, s), 5.13-4.96 (1H, m), 5.02 (2H, s), 3.32 (3H, s), 2.96-2.78 (4H, m), 2.31 (3H, s), 2.08 (3H, s), 1.54 (3H, s). |
| | MS(ESI/APCI) m/z: 402 [M + H]+ |

**[Table 6]**

| | |
|---|---|
| 9E | 1H-NMR (CDCl3) δ: 7.81 (1H, s), 7.48 (1H, s), 6.98 (1H, d, J = 1.8 Hz), 6.89 (1H, d, J = 2.4 Hz), 5.14-4.94 (1H, m), 2.93-2.71 (4H, m), 2.47 (3H, s), 2.35 (3H, s), 1.57 (3H, s). |
| | MS(ESI/APCI) m/z: 358 [M + H]+ |
| 10A | 1H-NMR (CDCl3) δ: 7.72 (1H, d, J = 3.6 Hz), 7.67 (1H, s), 7.37-7.37 (5H, m), 6.47 (1H, d, J = 3.6 Hz), 5.31-5.30 (1H, m), 4.48 (2H, s), 2.82-2.77 (2H, m), 2.67-2.65 (2H, m), 1.62 (3H, s). |
| | MS(ESI/APCI) m/z: 328 [M + H]+ |
| 10B | 1H-NMR (CDCl3) δ: 7.85 (1H, s), 7.63 (1H, s), 7.38-7.29 (5H, m), 5.35-5.32 (1H, m), 4.47 (2H, s), 2.81-2.75 (2H, m), 2.66-2.64 (2H, m), 2.36 (3H, s). |
| | MS(ESI/APCI) m/z: 386 [M + H]+ |
| 10C | 1H-NMR (CDCl3) δ: 7.71 (1H, s), 7.36-7.31 (6H, m), 5.35-5.33 (1H, m), 4.47 (2H, s), 2.77-2.72 (2H, m), 2.58-2.55 (2H, m). |
| | MS(ESI/APCI) m/z: 344 [M + H]+ |
| 10D | 1H-NMR (CDCl3) δ: 7.67 (1H, s), 7.38-7.33 (5H, m), 7.18 (1H, s), 5.39-5.37 (1H, m), 4.48 (2H, s), 3.87 (3H, s), 2.78-2.73 (2H, m), 2.61-2.58 (2H, m), 1.61 (3H, s). |
| | MS(ESI/APCI) m/z: 358 [M + H]+ |
| 10E | 1H-NMR (CDCl3) δ: 7.63 (1H, s), 7.43-7.33 (7H, m), 7.18 (1H, s), 5.56-5.54 (1H, m), 5.06 (2H, s), 4.50 (2H, s), 3.90 (3H, s), 3.32 (3H, s), 2.81-2.78 (2H, m), 2.66-2.63 (2H, m), 2.15 (3H, s), 1.62 (3H, s). |
| | MS(ESI/APCI) m/z: 542 [M + H]+ |
| 10F | MS(ESI/APCI) m/z: 452 [M + H]+ |
| 10G | 1H-NMR (CDCl3) δ: 7.94 (1H, s), 7.20 (2H, s), 7.12 (1H, s), 5.16-5.14 (1H, m), 3.95 (3H, s), 2.85-2.84 (2H, m), 2.78-2.76 (3H, m), 2.53 (3H, s), 1.57 (3H, s). |
| | MS(ESI/APCI) m/z: 408 [M + H]+ |
| 11 | 1H-NMR (CDCl3) δ: 7.18 (1H, t, J = 1.5 Hz), 7.14 (1H, d, J = 1.2 Hz), 7.02 (1H, s), 4.32-4.22 (1H, m), 3.81-3.66 (2H, m), 3.12 (2H, td, J = 8.3, 1.2 Hz), 3.07-3.01 (1H, m), 2.86 (1H, d, J = 11.0 Hz), 2.50-2.40 (5H, m), 2.20 (1H, t, J = 10.4 Hz), 2.01-1.91 (2H, m), 1.84-1.59 (4H, m), 1.09 (3H, t, J = 7.1 Hz). |
| | MS(ESI/APCI) m/z: 407 [M + H]+ |
| 12A | 1H-NMR (CDCl3) δ: 7.44 (1H, s), 2.94-2.83 (2H, m), 2.80-2.68 (2H, m), 2.25-2.11 (2H, m). |
| | MS(ESI/APCI) m/z: 293 [M + H]+ |
| 12B | 1H-NMR (CDCl3) δ: 3.04-2.92 (1H, m), 2.44-2.35 (2H, m), 1.86-1.73 (2H, m), 1.30 (3H, s), 0.87 (9H, s), 0.06 (6H, s). |
| | MS(ESI/APCI) m/z: 216 [M + H]+ |
| 12C | 1H-NMR (CDCl3) δ: 7.41 (1H, s), 5.22-5.06 (1H, m), 3.16 (2H, t, J = 7.3 Hz), 2.79 (2H, t, J = 7.3 Hz), 2.75-2.63 (4H, m), 2.63-2.52 (2H, m), 1.52 (3H, s), 0.91 (9H, s), 0.11 (6H, s). |
| | MS(ESI/APCI) m/z: 392 [M + H]+ |
| 12D | 1H-NMR (CDCl3) δ: 7.38 (1H, s), 7.32 (1H, s), 7.25 (1H, s), 5.45-5.31 (1H, m), 5.05 (2H, s), 3.32 (3H, s), 3.21 (2H, t, J = 7.3 Hz), 2.84 (2H, t, J = 7.0 Hz), 2.80-2.53 (6H, m), 2.14 (3H, s), 1.54 (3H, s), 0.93 (9H, s), 0.13 (6H, s).MS(ESI/APCI) m/z: 576 [M + H]+ |
| | MS(ESI/APCI) m/z: 576 [M + H]+ |
| 12E | 1H-NMR (CDCl3) δ: 7.68 (1H, s), 7.20 (1H, s), 7.11 (1H, s), 4.95-4.82 (1H, m), 3.11 (2H, t, J = 7.3 Hz), 3.07-2.97 (2H, m), 2.93-2.80 (4H, m), 2.66-2.55 (2H, m), 2.53 (3H, s), 1.54 (3H, s). |
| | MS(ESI/APCI) m/z: 418 [M + H]+ |

**[Table 7]**

| | |
|---|---|
| 13A | 1H-NMR (CDCl3) δ: 7.47-7.28 (5H, m), 6.41 (1H, s), 6.31-6.28 (1H, m), 6.27 (1H, s), 5.01 (2H, s), 2.27 (3H, s). |
| 13B | 1H-NMR (CDCl3) δ: 7.46-7.31 (5H, m), 6.65 (1H, s), 6.57-6.52 (2H, m), 6.47 (1H, t, J = 74.4 Hz), 5.03 (2H, s), 2.32 (3H, s). |
| 13C | 1H-NMR (CDCl3) δ: 6.53-6.49 (2H, m), 6.47 (1H, t, J = 74.1 Hz), 6.45-6.41 (1H, m), 2.30 (3H, s). |
| 13D | 1H-NMR (CDCl3) δ: 6.65 (2H, s), 6.48 (1H, t, J = 73.2 Hz), 2.44 (3H, s). |
| 13E | 1H-NMR (CDCl3) δ: 6.74 (1H, d, J = 2.4 Hz), 6.71 (1H, d, J = 2.4 Hz), 6.48 (1H, t, J = 73.5 Hz), 5.23 (2H, s), 3.51 (3H, s), 2.47 (3H, s). |
| 13F | 1H-NMR (CDCl3) δ: 6.61 (1H, d, J = 1.8 Hz), 6.56 (1H, s), 6.46 (1H, t, J = 74.4 Hz), 5.12 (2H, s), 3.46 (3H, s), 2.35 (3H, s), 1.38 (12H, s). |
| 13G | 1H-NMR (CDCl3) δ: 7.58 (1H, s), 7.38 (1H, s), 6.88 (1H, d, J = 1.8 Hz), 6.77 (1H, s), 6.56 (1H, t, J = 74.1 Hz), 5.11-4.97 (1H, m), 5.03 (2H, s), 3.32 (3H, s), 2.95-2.79 (4H, m), 2.31 (3H, s), 2.10 (3H, s), 1.54 (3H, s). |
| | MS(ESI/APCI) m/z: 434 [M + H]+ |
| 13H | 1H-NMR (CDCl3) δ: 7.81 (1H, s), 7.48 (1H, s), 6.73 (1H, d, J = 2.4 Hz), 6.64 (1H, d, J = 2.4 Hz), 6.57 (1H, t, J = 74.1 Hz), 5.11-4.97 (1H, m), 2.92-2.73 (4H, m), 2.50 (3H, s), 2.35 (3H, s), 1.57 (3H, s). |
| | MS(ESI/APCI) m/z: 390 [M + H]+ |
| 14A | 1H-NMR (CDCl3) δ: 7.36-7.19 (5H, m), 6.84 (1H, s), 4.32-4.19 (1H, m), 3.69 (1H, q, J = 8.7 Hz), 3.60 (1H, q, J = 8.5 Hz), 3.52 (2H, s), 3.02-2.90 (3H, m), 2.82-2.70 (2H, m), 2.19 (1H, t, J = 10.3 Hz), 2.03-1.92 (1H, m), 1.91-1.81 (1H, m), 1.78-1.65 (1H, m), 1.65-1.46 (1H, m). |
| | MS(ESI/APCI) m/z: 329 [M + H]+ |
| 14B | 1H-NMR (CDCl3) δ: 7.38-7.15 (7H, m), 6.85 (1H, s), 5.08 (2H, s), 4.48-4.36 (1H, m), 3.70 (1H, q, J = 8.5 Hz), 3.62 (1H, q, J = 8.3 Hz), 3.55 (2H, s), 3.37 (3H, s), 3.08-2.98 (3H, m), 2.79 (1H, d, J = 10.9 Hz), 2.23 (1H, t, J = 10.3 Hz), 2.23 (3H, s), 2.04-1.88 (2H, m), 1.80-1.69 (2H, m), 1.67-1.51 (1H, m). |
| | MS(ESI/APCI) m/z: 513 [M + H]+ |
| 14C | 1H-NMR (CDCl3) δ: 7.27 (1H, s), 7.21 (1H, s), 6.88 (1H, s), 5.10 (2H, s), 4.26-4.13 (1H, m), 3.77-3.59 (2H, m), 3.38 (3H, s), 3.31-3.22 (1H, m), 3.13-2.99 (3H, m), 2.77 (1H, t, J = 11.5 Hz), 2.56 (1H, t, J = 11.5 Hz), 2.23 (3H, s), 2.10-2.00 (1H, m), 1.90-1.51 (4H, m). |
| | MS(ESI/APCI) m/z: 423 [M + H]+ |
| 14D | 1H-NMR (CDCl3) δ: 7.27 (1H, s), 7.21 (1H, s), 6.87 (1H, s), 5.09 (2H, s), 4.41-4.27 (1H, m), 3.77-3.61 (2H, m), 3.38 (3H, s), 3.11-2.99 (3H, m), 2.83 (1H, d, J = 11.5 Hz), 2.42-2.26 (2H, m), 2.27-2.15 (1H, m), 2.23 (3H, s), 2.00-1.88 (3H, m), 1.82-1.70 (2H, m), 1.70-1.58 (1H, m), 1.58-1.42 (1H, m), 0.89 (3H, t, J = 7.3 Hz). |
| | MS(ESI/APCI) m/z: 465 [M + H]+ |
| 14E | 1H-NMR (CDCl3) δ: 7.19-7.16 (1H, m), 7.16-7.13 (1H, m), 7.03-6.99 (1H, m), 4.34-4.22 (1H, m), 3.78 (1H, q, J = 8.5 Hz), 3.71 (1H, q, J = 8.5 Hz), 3.12 (2H, t, J = 8.5 Hz), 3.04-2.95 (1H, m), 2.86-2.77 (1H, m), 2.46 (3H, s), 2.40-2.25 (2H, m), 2.21 (1H, t, J = 10.3 Hz), 2.04-1.86 (2H, m), 1.85-1.42 (5H, m), 0.90 (3H, t, J = 7.6 Hz). |
| | MS(ESI/APCI) m/z: 421 [M + H]+ |

**[Table 8]**

| | |
|---|---|
| 15A | 1H-NMR (CDCl3) δ: 7.27 (1H, s), 7.21 (1H, s), 6.87 (1H, s), 5.10 (2H, s), 4.37-4.24 (1H, m), 3.79-3.61 (2H, m), 3.38 (3H, s), 3.06 (2H, t, J = 7.6 Hz), 3.03-2.98 (1H, m), 2.84-2.73 (2H, m), 2.38 (1H, t, J = 10.3 Hz), 2.23 (3H, s), 2.15 (1H, td, J = 10.9, 3.0 Hz), 1.98-1.88 (1H, m), 1.84-1.54 (3H, m), 1.04 (3H, d, J = 3.6 Hz), 1.02 (3H, d, J = 3.6 Hz). |
| | MS(ESI/APCI) m/z: 465 [M + H]+ |
| 15B | 1H-NMR (CDCl3) δ: 7.19-7.16 (1H, m), 7.16-7.12 (1H, m), 7.03-7.00 (1H, m), 4.32-4.16 (1H, m), 3.79 (1H, q, J = 8.7 Hz), 3.72 (1H, q, J = 8.5 Hz), 3.12 (2H, t, J = 8.2 Hz), 3.01-2.91 (1H, m), 2.85-2.70 (2H, m), 2.46 (3H, s), 2.39 (1H, t, J = 10.0 Hz), 2.22-2.11 (1H, m), 1.98-1.87 (1H, m), 1.85-1.38 (3H, m), 1.03 (6H, d, J = 6.1 Hz). |
| | MS(ESI/APCI) m/z: 421 [M + H]+ |
| 16A | 1H-NMR (CDCl3) δ: 6.88 (1H, t, J = 1.5 Hz), 3.55 (2H, t, J = 8.1 Hz), 2.99 (2H, td, J = 8.1 1.5 Hz), 2.38 (2H, dd, J = 6.4, 2.7 Hz), 2.17-2.06 (2H, m), 1.95-1.80 (7H, m). |
| | MS(ESI/APCI) m/z: 266 [M + H]+ |
| 16B | 1H-NMR (CDCl3) δ: 7.65 (1H, s), 7.43 (1H, d, J = 3.6 Hz), 6.39 (1H, d, J = 3.6 Hz), 2.75-2.74 (2H, m), 2.45-2.44 (2H, m), 2.18-2.15 (2H, m), 2.04-1.99 (4H, m). |
| | MS(ESI/APCI) m/z: 264 [M + H]+ |
| 16C | 1H-NMR (CDCl3) δ: 7.61 (1H, s), 7.44 (1H, d, J = 3.6 Hz), 7.33 (1H, s), 7.26 (1H, s), 6.47 (1H, d, J = 3.6 Hz), 5.06 (2H, s), 3.32 (3H, s), 2.81-2.79 (2H, m), 2.55-2.53 (2H, m), 2.28-2.26 (2H, m), 2.16 (3H, s), 2.01-2.01 (4H, m). |
| | MS(ESI/APCI) m/z: 448 [M + H]+ |
| 16D | 1H-NMR (CDCl3) δ: 7.61 (1H, s), 7.44 (1H, d, J = 3.6 Hz), 7.32-7.28 (3H, m), 6.90-6.86 (2H, m), 6.47 (1H, d, J = 3.6 Hz), 5.06 (2H, s), 4.45 (2H, s), 3.81 (3H, s), 3.33 (3H, s), 2.80-2.78 (2H, m), 2.63-2.61 (2H, m), 2.31-2.29 (2H, m), 2.17 (3H, s), 2.04-2.02 (4H, m). |
| | MS(ESI/APCI) m/z: 568 [M + H]+ |
| 16E | 1H-NMR (CDCl3) δ: 7.52 (1H, s), 7.43 (1H, s), 7.31 (2H, d, J = 8.4 Hz), 7.27-7.25 (2H, m), 6.86 (2H, d, J = 8.4 Hz), 5.08 (2H, s), 4.45 (2H, s), 3.79 (3H, s), 3.35 (3H, s), 2.77-2.75 (2H, m), 2.63-2.60 (2H, m), 2.30-2.28 (2H, m), 2.17 (3H, s), 2.08-2.04 (4H, m). |
| | MS(ESI/APCI) m/z: 694 [M + H]+ |
| 16F | 1H-NMR (CDCl3) δ: 7.93 (1H, s), 7.82 (1H, s), 7.36 (1H, s), 7.27-7.25 (3H, m), 6.88-6.86 (2H, m), 5.09 (2H, s), 4.45 (2H, s), 3.79 (3H, s), 3.35 (3H, s), 2.82-2.80 (2H, m), 2.61-2.59 (2H, m), 2.34-2.33 (2H, m), 2.17-2.04 (4H, m), 2.17 (3H, s). |
| | MS(ESI/APCI) m/z: 593 [M + H]+ |
| 16G | 1H-NMR (CDCl3) δ: 8.05 (1H, s), 8.00 (1 H, s), 7.22 (1H, s), 7.16 (1H, s), 2.83-2.81 (2H, m), 2.52 (3H, s), 2.51-2.48 (2H, m), 2.30-2.28 (2H, m), 2.07-2.06 (4H, m). |
| | MS(ESI/APCI) m/z: 429 [M + H]+ |
| 17A | 1H-NMR (CDCl3) δ: 4.49-4.08 (2H, m), 3.67 (3H, s), 2.88-2.64 (1H, m), 2.03-1.70 (6H, m), 1.64-1.57 (1H, m), 1.52-1.49 (1H, m), 1.48 (9H, s). |
| | MS(ESI/APCI) m/z: 214 [M - tBu + H]+ |
| 17B | MS(ESI/APCI) m/z: 200 [M - tBu + H]+ |

**[Table 9]**

| | |
|---|---|
| 17C | 1H-NMR (CDCl3) δ: 7.40-7.30 (5H, m), 5.16-5.04 (2H, m), 4.59-4.50 (1H, m), 4.27-4.16 (2H, m), 3.88-3.73 (1H, m), 2.02-1.75 (4H, m), 1.69-1.29 (13H, m). |
| | MS(ESI/APCI) m/z: 305 [M - tBu + H]+ |
| 17D | MS(ESI/APCI) m/z: 171 [M - tBu + H]+ |
| 17E | 1H-NMR (CDCl3) δ: 6.89 (1H, s), 4.43 (1H, s), 4.30-4.09 (2H, m), 3.77-3.71 (2H, m), 3.02 (2H, t, J = 7.4 Hz), 2.20-1.84 (6H, m), 1.74-1.42 (11 H, m). |
| | MS(ESI/APCI) m/z: 365 [M + H]+ |
| 17F | 1H-NMR (CDCl3) δ: 7.27 (1H, s), 7.21 (1H, s), 6.89 (1H, s), 5.10 (2H, s), 4.54-4.18 (3H, m), 3.79-3.65 (2H, m), 3.38 (3H, s), 3.11-3.02 (2H, m), 2.22 (3H, s), 2.17-1.81 (6H, m), 1.76-1.42 (11H, m). |
| | MS(ESI/APCI) m/z: 549 [M + H]+ |
| 17G | 1H-NMR (CDCl3) δ: 7.18 (1H, s), 7.13 (1H, s), 7.01 (1H, s), 4.21-4.14 (1H, m), 3.89-3.76 (3H, m), 3.55-3.52 (1H, m), 3.15-3.11 (2H, m), 2.44 (3H, s), 1.99-1.83 (5H, m), 1.79-1.58 (3H, m). |
| | MS(ESI/APCI) m/z: 405 [M + H]+ |
| 17H | 1H-NMR (CDCl3) δ: 7.16 (1H, s), 7.14 (1H, s), 7.01 (1H, s), 4.36-4.29 (1H, m), 3.88-3.74 (2H, m), 3.68-3.61 (1H, m), 3.30-3.26 (1H, m), 3.11 (2H, t, J = 7.4 Hz), 2.52 (2H, q, J = 7.2 Hz), 2.45 (3H, s), 2.07-1.75 (6H, m), 1.61-1.54 (1H, m), 1.53-1.48 (1H, m), 1.14 (3H, t, J = 6.7 Hz). |
| | MS(ESI/APCI) m/z: 433 [M + H]+ |
| 18A | 1H-NMR (CDCl3) δ: 7.27 (1H, s), 7.21 (1H, s), 6.87 (1H, s), 5.10 (2H, s), 4.33-4.20 (1H, m), 3.77-3.59 (2H, m), 3.39 (3H, s), 3.22-3.13 (1H, m), 3.06 (2H, t, J = 8.2 Hz), 2.96 (1H, d, J = 10.9 Hz), 2.43 (1H, t, J = 10.6 Hz), 2.27-2.06 (2H, m), 2.23 (3H, s), 1.98-1.82 (2H, m), 1.82-1.53 (2H, m), 0.53-0.33 (4H, m). |
| | MS(ESI/APCI) m/z: 463 [M + H]+ |
| 18B | 1H-NMR (CDCl3) δ: 7.17 (1H, s), 7.14 (1H, s), 7.01 (1H, s), 4.27-4.17 (1H, m), 3.77 (1H, q, J = 8.5 Hz), 3.70 (1H, dd, J = 17.3, 8.8 Hz), 3.17-3.06 (3H, m), 2.99-2.88 (1H, m), 2.50-2.38 (1H, m), 2.46 (3H, s), 2.27-2.14 (1H, m), 1.96-1.85 (1H, m), 1.85-1.49 (4H, m), 0.52-0.33 (4H, m). |
| | MS(ESI/APCI) m/z: 419 [M + H]+ |
| 19A | 1H-NMR (CDCl3) δ: 7.17 (1H, t, J = 1.5 Hz), 7.15-7.14 (1H, m), 7.02-7.01 (1H, m), 4.30-4.24 (1H, m), 3.87-3.74 (2H, m), 3.57-3.54 (1H, m), 3.18-3.09 (3H, m), 2.46 (3H, s), 2.37 (3H, s), 2.15-1.48 (8H, m). |
| | MS(ESI/APCI) m/z: 419 [M + H]+ |
| 19B | MS(ESI/APCI) m/z: 419 [M + H]+ |
| 20A | 1H-NMR (CDCl3) δ: 6.87 (1H, s), 4.20-3.86 (3H, m), 3.86-3.71 (1H, m), 3.38-3.26 (1H, m), 3.26-3.14 (1H, m), 3.14-3.00 (1H, m), 2.88-2.66 (1H, m), 2.00-1.89 (1H, m), 1.89-1.68 (2H, m), 1.46 (9H, s), 1.37-1.30 (4H, m). |
| | MS(ESI/APCI) m/z: 353 [M + H]+ |
| 20B | 1H-NMR (CDCl3) δ: 7.62 (1H, s), 7.36 (1H, s), 5.02-4.77 (1H, m), 4.25-4.14 (1H, m), 4.06-3.82 (1H, m), 3.55-3.32 (1H, m), 3.12-2.97 (1H, m), 2.27 (3H, s), 2.24-2.09 (2H, m), 1.91-1.64 (2H, m), 1.45 (9H, s). |
| | MS(ESI/APCI) m/z: 295 [M - tBu + H]+ |

**[Table 10]**

| | |
|---|---|
| 20C | 1H-NMR (CDCl3) δ: 7.58 (1H, s), 7.35 (1H, s), 7.34 (1H, s), 7.26 (1H, s), 5.14-4.91 (1H, m), 5.08 (2H, s), 4.37-4.18 (1H, m), 4.08-3.91 (1H, m), 3.47 (1H, dd, J = 12.8, 9.7 Hz), 3.34 (3H, s), 3.11-2.99 (1H, m), 2.32 (3H, s), 2.29-2.21 (1H, m), 2.16 (3H, s), 1.97-1.92 (1H, m), 1.92-1.80 (1H, m), 1.80-1.66 (1H, m), 1.46 (9H, s). |
| | MS(ESI/APCI) m/z: 535 [M + H]+ |
| 200 | 1H-NMR (CDCl3) δ: 7.83 (1H, s), 7.77-9.69 (1H, m), 7.21 (1H, s), 7.13 (1H, s), 5.22-5.09 (1H, m), 3.17-3.04 (1H, m), 2.86-2.74 (1H, m), 2.73-2.59 (1H, m), 2.58-2.43 (5H, m), 2.35 (4H, s), 2.21-1.95 (2H, m), 1.91-1.73 (2H, m), 1.12 (3H, t, J = 7.3 Hz). |
| | MS(ESI/APCI) m/z: 419 [M + H]+ |
| 21A | 1H-NMR (CDCl3) δ: 7.81 (1H, s), 7.66 (1H, s), 5.01-4.99 (1H, m), 2.88-2.82 (4H, m), 2.37 (3H, s), 1.53 (3H, s). |
| | MS(ESI/APCI) m/z: 296 [M + H]+ |
| 21B | 1H-NMR (CDCl3) δ: 7.79 (1H, s), 7.62 (1H, s), 5.18-5.16 (1H, m), 2.81-2.78 (2H, m), 2.54-2.51 (2H, m), 2.37 (3H, s), 1.55 (3H, s), 0.91 (9H, s), 0.10 (6H, s). |
| | MS(ESI/APCI) m/z: 410 [M + H]+ |
| 21C | 1H-NMR (CDCl3) δ: 7.69 (1H, s), 7.31 (1H, s), 5.19-5.17 (1H, m), 2.77-2.75 (2H, m), 2.48-2.46 (2H, m), 1.52 (3H, s), 0.92 (9H, s), 0.11 (6H, s). |
| | MS(ESI/APCI) m/z: 368 [M + H]+ |
| 21D | 1H-NMR (CDCl3) δ: 7.69 (1H, s), 7.55 (1H, s), 6.52 (1H, t, J = 73.2 Hz), 5.19-5.17 (1H, m), 2.84-2.79 (2H, m), 2.53-2.48 (2H, m), 1.53 (3H, s), 0.91 (9H, s), 0.11 (6H, s). |
| | MS(ESI/APCI) m/z: 418 [M + H]+ |
| 21E | 1H-NMR (CDCl3) δ: 7.68 (1H, s), 7.57 (1H, s), 7.33 (1H, s), 7.28-7.26 (1H, m), 6.54 (1H, t, J = 73.3 Hz), 5.41-5.32 (1H, m), 5.07 (2H, s), 3.33 (3H, s), 2.89-2.82 (2H, m), 2.59-2.51 (2H, m), 2.17 (3H, s), 1.53 (3H, s), 0.94 (9H, s), 0.14 (6H, s). |
| | MS(ESI/APCI) m/z: 602 [M + H]+ |
| 21F | 1H-NMR (CDCl3) δ: 10.81 (1H, br s), 7.96 (1H, s), 7.66 (1H, s), 7.22 (1H, s), 7.15 (1H, s), 6.57 (1H, t, J = 72.7 Hz), 5.21-5.11 (1H, m), 2.95-2.88 (2H, m), 2.83-2.75 (2H, m), 2.52 (3H, s), 1.59 (3H, s). |
| | MS(ESI/APCI) m/z: 444 [M + H]+ |
| 22A | 1H-NMR (CDCl3) δ: 6.90 (1H, s), 6.83 (1H, d, J = 1.8 Hz), 6.70 (1H, s), 6.53 (1H, t, J = 74.1 Hz), 5.06 (2H, s), 4.31-3.84 (3H, m), 3.75-3.57 (2H, m), 3.37 (3H, s), 3.17-3.00 (3H, m), 2.87-2.68 (1H, m), 2.18 (3H, s), 2.08-1.97 (1H, m), 1.88-1.70 (2H, m), 1.69-1.56 (1H, m), 1.46 (9H, s). |
| | MS(ESI/APCI) m/z: 521 [M + H]+ |
| 22B | 1H-NMR (CDCl3) δ: 7.17-7.14 (1H, m), 6.67-6.63 (1H, m), 6.54-6.51 (1H, m), 6.53 (1H, t, J = 74.1 Hz), 4.31-4.18 (1H, m), 3.80-3.63 (2H, m), 3.10 (2H, t, J = 8.5 Hz), 3.07-3.00 (1H, m), 2.91-2.82 (1H, m), 2.52-2.37 (5H, m), 2.20 (1H, t, J = 10.3 Hz), 2.02-1.86 (2H, m), 1.86-1.55 (3H, m), 1.09 (3H, t, J = 7.0 Hz). |
| | MS(ESI/APCI) m/z: 405 [M + H]+ |
| 23A | 1H-NMR (CDCl3) δ: 6.61 (1H, s), 6.57 (1H, s), 6.52 (1H, s), 5.11 (1H, br s), 2.32 (3H, s). |
| | MS(ESI/APCI) m/z: 191 [M - H]- |
| 23B | MS(ESI/APCI) m/z: 317 [M - H]- |

**[Table 11]**

| | |
|---|---|
| 23C | 1H-NMR (CDCI3) δ: 6.84 (1H, s), 6.80 (1H, s), 5.24 (2H, s), 3.52 (3H, s), 2.49 (3H, s). |
| 23D | 1H-NMR (CDCI3) δ: 6.71 (1H, s), 6.67 (1H, s), 5.12 (2H, s), 3.46 (3H, s), 2.36 (3H, s), 1.38 (12H, s). |
| 23E | 1H-NMR (CDCl3) δ: 6.92 (1H, s), 6.91 (1H, s), 6.82 (1H, s), 5.06 (2H, s), 4.28-3.90 (2H, m), 3.74-3.56 (2H, m), 3.38 (3H, s), 3.17-3.01 (3H, m), 2.88-2.66 (1H, m), 2.19 (3H, s), 2.08-1.97 (1H, m), 1.92-1.71 (2H, m), 1.71-1.54 (2H, m), 1.46 (9H, s). |
| | MS(ESI/APCI) m/z: 539 [M + H]+ |
| 23F | 1H-NMR (CDCI3) δ: 7.17-7.14 (1H, m), 6.80-6.75 (1H, m), 6.65-6.60 (1H, m), 4.32-4.19 (1H, m), 3.81-3.62 (2H, m), 3.11 (2H, t, J = 7.9 Hz), 3.07-2.99 (1H, m), 2.90-2.81 (1H, m), 2.53-2.34 (5H, m), 2.19 (1H, t, J = 10.3 Hz), 2.02-1.87 (2H, m), 1.86-1.44 (3H, m), 1.08 (3H, t, J = 7.3 Hz). |
| | MS(ESI/APCI) m/z: 423 [M + H]+ |
| 24A | 1H-NMR (CDCI3) δ: 7.65 (1H, s), 7.44 (1H, d, J = 3.6 Hz), 7.33-7.32 (5H, m), 6.40 (1H, d, J = 3.6 Hz), 4.50 (2H, s), 2.74-2.72 (2H, m), 2.56-2.54 (2H, m), 2.23-2.21 (2H, m), 2.12-2.10 (2H, m), 2.03-2.01 (2H, m). |
| | MS(ESI/APCI) m/z: 354 [M + H]+ |
| 24B | 1H-NMR (CDCI3) δ: 7.63 (1H, s), 7.59 (1H, s), 7.33-7.32 (5H, m), 4.50 (2H, s), 2.71-2.69 (2H, m), 2.56-2.54 (2H, m), 2.22-2.20 (2H, m), 2.10 (3H, s), 2.10-2.08 (2H, m), 2.03-2.00 (2H, m). |
| | MS(ESI/APCI) m/z: 412 [M + H]+ |
| 24C | 1H-NMR (CDCI3) δ: 7.68 (1H, s), 7.32-7.31 (5H, m), 7.07 (1H, s), 4.49 (2H, s), 2.68-2.64 (2H, m), 2.53-2.51 (2H, m), 2.17-2.15 (2H, m), 2.09-2.07 (2H, m), 1.99-1.97 (2H, m). |
| | MS(ESI/APCI) m/z: 370 [M + H]+ |
| 24D | 1H-NMR (CDCI3) δ: 7.67 (1H, s), 7.33-7.29 (5H, m), 6.89 (1H, s), 4.50 (2H, s), 3.88 (3H, s), 2.70-2.68 (2H, m), 2.56-2.54 (2H, m), 2.18-2.16 (2H, m), 2.10-2.08 (3H, m), 1.99-1.97 (2H, m). |
| | MS(ESI/APCI) m/z: 384 [M + H]+ |
| 24E | 1H-NMR (CDCI3) δ: 7.63 (1H, s), 7.34-7.33 (6H, m), 7.26 (1H, s), 6.88 (1H, s), 5.06 (2H, s), 4.52 (2H, s), 3.91 (3H, s), 3.33 (3H, s), 2.77-2.75 (2H, m), 2.65-2.63 (2H, m), 2.25-2.23 (2H, m), 2.17 (3H, s), 2.11-2.09 (2H, m), 2.03-2.02 (2H, m). |
| | MS(ESI/APCI) m/z: 568 [M + H]+ |
| 24F | 1H-NMR (CDCI3) δ: 7.63 (1H, s), 7.33 (1H, s), 7.29 (1H, s), 6.88 (1H, s), 5.06 (2H, s), 3.91 (3H, s), 3.33 (3H, s), 2.77-2.74 (2H, m), 2.55-2.52 (2H, m), 2.17-2.16 (2H, m), 2.17 (3H, s), 2.01-1.98 (4H, m). |
| | MS(ESI/APCI) m/z: 478 [M + H]+ |
| 24G | 1H-NMR (CDCI3) δ: 7.92 (1H, s), 7.21 (1H, s), 7.12 (1H, s), 6.94 (1H, s), 3.93 (3H, s), 2.78-2.76 (2H, m), 2.54 (3H, s), 2.51-2.49 (2H, m), 2.22-2.20 (2H, m), 2.03-2.01 (4H, m). |
| | MS(ESI/APCI) m/z: 434 [M + H]+ |
| 25A | 1H-NMR (CDCI3) δ: 7.67 (1H, s), 7.39-7.30 (5H, m), 7.18 (1H, s), 5.39-5.35 (1H, m), 4.47 (2H, s), 4.00-4.04 (2H, m), 2.78-2.72 (2H, m), 2.63-2.55 (2H, m), 1.60 (3H, s). 1.40-1.46 (3H, m). |
| | MS(ESI/APCI) m/z: 372 [M + H]+ |

**[Table 12]**

| | |
|---|---|
| 25B | 1H-NMR (CDCl3) δ: 7.65 (1H, s), 7.40-7.32 (7H, m), 7.19 (1H, s), 5.55-5.52 (1H, m), 5.05 (2H, s), 4.50 (2H, s), 4.08 (2H, q, J = 7.0 Hz), 3.32 (3H, s), 2.78-2.76 (2H, m), 2.66-2.63 (2H, m), 2.14 (3H, s), 1.61 (3H, s), 1.45 (3H, t, J = 7.0 Hz). |
| | MS(ESI/APCI) m/z: 556 [M + H]+ |
| 25C | 1H-NMR (CDCI3) δ: 7.68 (1H, s), 7.33 (1H, s), 7.29 (1H, s), 7.11 (1H, s), 5.13-5.05 (1H, m), 5.06 (2H, s), 4.11-4.09 (2H, m), 3.32 (3H, s), 2.84-2.82 (4H, m), 2.14 (3H, s), 1.61 (3H, s), 1.46 (3H, t, J = 7.0 Hz). |
| | MS(ESI/APCI) m/z: 466 [M + H]+ |
| 25D | 1H-NMR (CDCI3) δ: 7.95 (1H, s), 7.21 (1H, s), 7.20 (1H, s), 7.12 (1H, s), 5.22-5.07 (1H, m), 4.12 (2H, q, J = 6.8 Hz), 2.94-2.69 (4H, m), 2.54 (3H, s), 1.49 (3H, t, J = 6.8 Hz). |
| | MS(ESI/APCI) m/z: 422 [M + H]+ |
| 26A | 1H-NMR (CDCI3) δ: 7.07-7.02 (1H, m), 6.97-6.93 (1H, m), 6.91-6.86 (1H, m), 5.04 (2H, s), 4.29-3.86 (3H, m), 3.74-3.59 (2H, m), 3.37 (3H, s), 3.15-3.03 (3H, m), 2.88-2.69 (1H, m), 2.15 (3H, s), 2.08-1.97 (1H, m), 1.88-1.57 (3H, m), 1.46 (9H, s). |
| | MS(ESI/APCI) m/z: 490[M + H]+ |
| 26B | MS(ESI/APCI) m/z: 345[M + H]+ |
| 26C | 1H-NMR (CDCI3) δ: 7.16-7.13 (1H, m), 6.92-6.89 (1H, m), 6.79-6.75 (1H, m), 4.29-4.18 (1H, m), 3.79-3.63 (3H, m), 3.14-3.06 (2H, m), 3.06-3.00 (1H, m), 2.89-2.82 (1H, m), 2.50-2.36 (5H, m), 2.23-2.15 (1H, m), 2.01-1.89 (2H, m), 1.86-1.56 (3H, m), 1.27-1.05 (3H, m). |
| | MS(ESI/APCI) m/z: 373[M + H]+ |
| 27A | 1H-NMR (CDCI3) δ: 7.64 (1H, s), 7.52-7.51 (1H, m), 7.31 (2H, d, J = 8.5 Hz), 7.12 (1H, s), 7.01 (1H, s), 6.91 (2H, d, J = 8.5 Hz), 5.53-5.50 (1H, m), 5.02 (2H, s), 4.42 (2H, s), 3.82 (3H, s), 3.32 (3H, s), 2.81-2.78 (2H, m), 2.61-2.58 (2H, m), 2.08 (3H, s), 1.61 (3H, s). |
| | MS(ESI/APCI) m/z: 526 [M + H]+ |
| 27B | 1H-NMR (CDCI3) δ: 7.90 (1H, s), 7.55 (1H, s), 6.98 (1H, s), 6.90 (1H, s), 5.16-5.14 (1H, m), 2.91-2.86 (2H, m), 2.74-2.71 (2H, m), 2.46 (3H, s), 1.58 (3H, s). |
| | MS(ESI/APCI) m/z: 362 [M + H]+ |
| 28A | 1H-NMR (CDCI3) δ: 7.66 (1H, s), 7.11 (1H, s), 5.19-5.17 (1H, m), 3.91 (3H, s), 2.77-2.75 (2H, m), 2.51-2.49 (2H, m), 1.52 (3H, s), 0.92 (9H, s), 0.12 (6H, s). |
| | MS(ESI/APCI) m/z: 382 [M + H]+ |
| 28B | 1H-NMR (CDCI3) δ: 7.62 (1H, s), 7.11 (2H, s), 6.99 (1H, s), 5.34-5.29 (1H, m), 5.01 (2H, s), 3.31 (3H, s), 2.81-2.78 (2H, m), 2.58-2.54 (2H, m), 2.06 (3H, s), 1.51 (3H, s), 0.94 (9H, s), 0.14 (6H, s). |
| | MS(ESI/APCI) m/z: 532 [M + H]+ |
| 28C | 1H-NMR (CDCI3) δ: 7.89 (1H, s), 7.18 (1H, s), 6.97 (1H, s), 6.88 (1H, s), 5.15-5.12 (1H, m), 3.94 (3H, s), 2.85-2.83 (2H, m), 2.79-2.77 (2H, m), 1.57 (6H, s). |
| | MS(ESI/APCI) m/z: 374 [M + H]+ |
| 29A | 1H-NMR (CDCI3) δ: 7.65 (1H, s), 7.54 (1H, s), 7.12 (1H, d, J = 1.8 Hz), 7.01 (1H, d, J = 1.8 Hz), 6.53 (1H, t, J = 73.3 Hz), 5.38-5.33 (1H, m), 5.02 (2H, s), 3.32 (3H, s), 2.89-2.82 (2H, m), 2.58-2.51 (2H, m), 2.09 (3H, s), 0.93 (9H, s), 0.13 (6H, s). |
| | MS(ESI/APCI) m/z: 568 [M + H]+ |

**[Table 13]**

| | |
|---|---|
| 29B | 1H-NMR (CDCI3) δ: 10.90 (1H, br s), 7.91 (1H, s), 7.63 (1H, s), 6.99 (1H, d, J = 2.5 Hz), 6.90 (1H, d, J = 1.8 Hz), 6.56 (1H, t, J = 73.0 Hz), 5.18-5.10 (1H, m), 2.95-2.87 (2H, m), 2.82-2.74 (3H, m), 2.45 (3H, s), 1.58 (3H, s). |
| | MS(ESI/APCI) m/z: 410 [M + H]+ |
| 30A | MS(ESI/APCI) m/z: 189 [M + H]+ |
| 30B | MS(ESI/APCI) m/z: 353 [M + H]+ |
| 30C | MS(ESI/APCI) m/z: 253 [M + H]+ |
| 300 | MS(ESI/APCI) m/z: 267 [M + H]+ |
| 30E | MS(ESI/APCI) m/z: 421 [M + H]+ |
| 30F | 1H-NMR (DMSO-D6) δ: 7.09-7.00 (3H, m), 4.13-3.98 (1H, m), 3.93-3.68 (1H, m), 3.33-3.22 (2H, m), 2.93-2.54 (4H, m), 2.24-2.17 (3H, m), 2.13-2.09 (3H, m), 2.00-1.50 (4H, m), 1.34-1.26 (3H, m). |
| | MS(ESI/APCI) m/z: 407 [M + H]+ |
| 31A | MS(ESI/APCI) m/z: 294 [M - tBu + H]+ |
| 31B | MS(ESI/APCI) m/z: 160 [M - tBu + H]+ |
| 31C | MS(ESI/APCI) m/z: 298 [M - tBu + H]+ |
| 31D | MS(ESI/APCI) m/z: 452 [M - tBu + H]+ |
| 31E | MS(ESI/APCI) m/z: 584 [M - tBu + H]+ |
| 31F | MS(ESI/APCI) m/z: 436 [M - tBu + H]+ |
| 31G | MS(ESI/APCI) m/z: 392 [M + H]+ |
| 31H | MS(ESI/APCI) m/z: 406 [M + H]+ |
| 311 | 1H-NMR (CDCI3) δ: 8.24 (1H, s), 8.07 (1H, s), 7.25 (1H, s), 7.18 (1H, s), 5.40-5.38 (1H, m), 3.24-3.21 (1H, m), 2.95-2.92 (1H, m), 2.75-2.72 (1H, m), 2.51 (3H, s), 2.40 (3H, s), 2.25-2.13 (3H, m), 1.97-1.94 (2H, m). |
| | MS(ESI/APCI) m/z: 392 [M + H]+ |
| 32A | MS(ESI/APCI) m/z: 328 [M + H]+ |
| 32B | MS(ESI/APCI) m/z: 282 [M - tBu + H]+ |
| 32C | MS(ESI/APCI) m/z: 238 [M + H]+ |
| 32D | MS(ESI/APCI) m/z: 252 [M + H]+ |
| 32E | MS(ESI/APCI) m/z: 406 [M + H]+ |
| 32F | 1H-NMR (CDCI3) δ: 8.97 (1H, s), 8.09 (1H, s), 7.23 (1H, s), 7.16 (1H, s), 5.16-5.15 (1H, m), 3.01-2.98 (2H, m), 2.66-2.60 (2H, m), 2.53 (3H, s), 2.41 (3H, s), 2.26-2.23 (1H, m), 2.10-2.05 (1H, m), 1.82-1.80 (2H, m). |
| | MS(ESI/APCI) m/z: 392 [M + H]+ |
| 33A | MS(ESI/APCI) m/z: 353 [M + H]+ |
| 33B | MS(ESI/APCI) m/z: 253 [M + H]+ |
| 33C | MS(ESI/APCI) m/z: 267 [M + H]+ |
| 33D | MS(ESI/APCI) m/z: 421 [M + H]+ |
| 33E | 1H-NMR (CDCI3) δ: 7.15 (2H, s), 7.03 (1H, s), 4.29-4.26 (1H, m), 3.85 (1H, t, J = 7.2 Hz), 3.43-3.40 (1H, m), 3.32-3.30 (1H, m), 2.99-2.97 (1H, m), 2.80-2.77 (1H, m), 2.47 (3H, s), 2.32 (3H, s), 2.21-2.19 (1H, m), 1.98-1.94 (2H, m), 1.79-1.53 (3H, m), 1.38 (3H, d, J = 7.2 Hz). |
| | MS(ESI/APCI) m/z: 407 [M + H]+ |
| 34A | MS(ESI/APCI) m/z: 353 [M + H]+ |
| 34B | MS(ESI/APCI) m/z: 253 [M + H]+ |
| 34C | MS(ESI/APCI) m/z: 267 [M + H]+ |

**[Table 14]**

| | |
|---|---|
| 34D | MS(ESI/APCI) m/z: 421 [M + H]+ |
| 34E | 1H-NMR (CDCI3) δ: 7.15 (2H, s), 7.03 (1H, s), 4.28-4.26 (1H, m), 3.92 (1H, t, J = 7.2 Hz), 3.43-3.41 (1H, m), 3.27-3.25 (1H, m), 2.98-2.95 (1H, m), 2.79-2.77 (1H, m), 2.47 (3H, s), 2.32 (3H, s), 2.20-2.17 (1H, m), 1.97-1.94 (2H, m), 1.79-1.77 (3H, m), 1.38 (3H, d, J = 7.2 Hz). |
| | MS(ESI/APCI) m/z: 407 [M + H]+ |
| 35A | MS(ESI/APCI) m/z: 336 [M + H]+ |
| 35B | MS(ESI/APCI) m/z: 222 [M + H]+ |
| 35C | MS(ESI/APCI) m/z: 376 [M + H]+ |
| 350 | MS(ESI/APCI) m/z: 368 [M + H]+ |
| 35E | MS(ESI/APCI) m/z: 282 [M + H]+ |
| 35F | MS(ESI/APCI) m/z: 480 [M + H]+ |
| 35G | 1H-NMR (CDCI3) δ: 7.13 (1H, s), 7.00 (1H, s), 6.39 (1H, s), 5.01-4.92 (1H, m), 3.68-3.61 (1H, m), 3.68-3.30 (1H, m), 3.57-3.50 (1H, m), 3.47-3.41 (2H, m), 2.98-2.91 (4H, m), 2.81-2.73 (1H, m), 2.51 (3H, s), 2.29 (3H, s), 2.10 (1H, t, J = 10.1 Hz), 1.97-1.75 (4H, m), 1.64-1.53 (1H, m). |
| | MS(ESI/APCI) m/z: 422 [M + H]+ |
| 36A | MS(ESI/APCI) m/z: 369 [M + H]+ |
| 36B | MS(ESI/APCI) m/z: 553 [M + H]+ |
| 36C | MS(ESI/APCI) m/z: 689 [M + H]+ |
| 36D | MS(ESI/APCI) m/z: 537 [M + H]+ |
| 36E | MS(ESI/APCI) m/z: 393 [M + H]+ |
| 36F | 1H-NMR (CDCI3) δ: 7.15 (2H, s), 7.01 (1H, s), 5.07-4.98 (1H, m), 4.22-4.10 (1H, m), 3.70-3.45 (3H, m), 3.05-2.97 (1H, m), 2.93-2.83 (1H, m), 2.82-2.76 (2H, m), 2.46 (3H, s), 2.38-2.32 (4H, m), 2.04-1.62 (5H, m). |
| | MS(ESI/APCI) m/z: 407 [M + H]+ |
| 37A | MS(ESI/APCI) m/z: 236 [M + H]+ |
| 37B | MS(ESI/APCI) m/z: 390 [M + H]+ |
| 37C | MS(ESI/APCI) m/z: 382 [M + H]+ |
| 370 | MS(ESI/APCI) m/z: 282 [M + H]+ |
| 37E | MS(ESI/APCI) m/z: 296 [M + H]+ |
| 37F | MS(ESI/APCI) m/z: 480 [M + H]+ |
| 37G | 1H-NMR (CDCI3) δ: 7.14 (1H, s), 7.00 (1H, s), 6.45 (1H, s), 4.63-4.60 (1H, m), 3.65-3.51 (4H, m), 3.12-3.09 (1H, m), 2.90 (3H, s), 2.85-2.83 (1H, m), 2.51 (3H, s), 2.31 (3H, s), 1.98-1.93 (3H, m), 1.87-1.77 (3H, m), 1.47-1.45 (2H, m). |
| | MS(ESI/APCI) m/z: 436 [M + H]+ |
| 38A | MS(ESI/APCI) m/z: 258 [M + H]+ |
| 38B | MS(ESI/APCI) m/z: 274 [M + H]+ |
| 38C | MS(ESI/APCI) m/z: 288 [M + H]+ |
| 380 | MS(ESI/APCI) m/z: 362 [M + H]+ |
| 38E | MS(ESI/APCI) m/z: 248 [M + H]+ |
| 38F | MS(ESI/APCI) m/z: 402 [M + H]+ |
| 38G | MS(ESI/APCI) m/z: 308 [M + H]+ |
| 38H | 1H-NMR (CDCI3) δ: 7.45 (1H, d, J = 8.0 Hz), 7.30 (1H, s), 7.07 (1H, d, J = 8.0 Hz), 4.94-4.88 (1H, m), 3.59-3.56 (1H, m), 3.50-3.44 (1H, m), 3.38-3.33 (4H, m), 2.95-2.93 (1H, m), 2.85-2.77 (3H, m), 2.29 (3H, s), 2.10-2.07 (1H, m), 1.96-1.76 (6H, m), 1.58-1.55 (1H, m). |
| | MS(ESI/APCI) m/z: 434 [M + H]+ |

**[Table 15]**

| | |
|---|---|
| 39A | MS(ESI/APCI) m/z: 197 [M + H]+ |
| 39B | MS(ESI/APCI) m/z: 189 [M + H]+ |
| 39C | MS(ESI/APCI) m/z: 254 [M + H]+ |
| 39D | 1H-NMR (CDCI3) δ: 7.47 (1H, d, J = 8.0 Hz), 7.32 (1H, s), 7.12 (1H, d, J = 8.0 Hz), 4.26-4.23 (1H, m), 3.76 (2H, t, J = 8.0 Hz), 3.07 (2H, t, J = 8.0 Hz), 2.50-2.48 (4H, m), 2.31 (3H, s), 1.46 (3H, s). |
| | MS(ESI/APCI) m/z: 380 [M + H]+ |
| 40A | MS(ESI/APCI) m/z: 424 [M + H]+ |
| 40B | 1H-NMR (DMSO-D6) δ: 7.49 (1H, s), 7.15 (1H, s), 7.10 (1H, s), 4.18-4.23 (1H, m), 3.92 (2H, t, J = 7.6 Hz), 3.25 (2H, t, J = 7.6 Hz), 2.34 (2H, t, J = 9.2 Hz), 2.26 (2H, t, J = 9.2 Hz), 2.15 (3H, s), 1.26 (3H, s). |
| | MS(ESI/APCI) m/z: 380 [M + H]+ |
| 41A | 1H-NMR (CDCI3) δ: 7.10 (1H, t, J = 7.8 Hz), 6.89 (2H, dd, J = 13.1, 7.8 Hz), 5.20 (2H, s), 3.49 (3H, s), 2.96-2.86 (4H, m), 2.12-2.02 (2H, m). |
| 41B | 1H-NMR (CDCI3) δ: 7.57 (1H, d, J = 7.3 Hz), 7.01 (1H, d, J = 7.3 Hz), 5.09 (2H, s), 3.58 (3H, s), 3.02-2.90 (4H, m), 2.10-2.01 (2H, m), 1.33 (12H, s). |
| 41C | MS(ESI/APCI) m/z: 252 [M + H]+ |
| 41D | MS(ESI/APCI) m/z: 394 [M + H]+ |
| 41E | 1H-NMR (CDCI3) δ: 7.62 (1H, d, J = 3.1 Hz), 7.28-7.25 (1H, m), 6.88 (1H, d, J = 7.9 Hz), 6.59 (1H, d, J = 3.7 Hz), 5.08-5.00 (1H, m), 3.07-2.94 (4H, m), 2.93-2.79 (4H, m), 2.71 (3H, s), 2.21-2.10 (2H, m), 1.56 (3H, s). |
| | MS(ESI/APCI) m/z: 350 [M + H]+ |
| 42A | MS(ESI/APCI) m/z: 144 [M - tBu + H]+ |
| 42B | MS(ESI/APCI) m/z: 142 [M - tBu - H2O + H]+ |
| 42C | MS(ESI/APCI) m/z: 116 [M + H]+ |
| 420 | MS(ESI/APCI) m/z: 254 [M + H]+ |
| 42E | MS(ESI/APCI) m/z: 438 [M + H]+ |
| 42F | MS(ESI/APCI) m/z: 436 [M + H]+ |
| 42G | 1H-NMR (CDCl3) δ: 7.94 (1H, s), 7.77 (1H, d, J = 3.6 Hz), 7.22 (1H, s), 7.13 (1H, s), 6.62 (1H, d, J = 3.6 Hz), 5.35-5.32 (1H, m), 3.08-3.04 (1H, m), 2.93-2.90 (1H, m), 2.84-2.78 (1H, m), 2.55 (3H, s), 1.60 (3H, s), 0.80 (3H, d, J = 7.6 Hz). |
| | MS(ESI/APCI) m/z: 392 [M + H]+ |
| 43A | MS(ESI/APCI) m/z: 234 [M + H]+ |
| 43B | MS(ESI/APCI) m/z: 218 [M - H₂O + H]+ |
| 43C | MS(ESI/APCI) m/z: 218 [M + H]+ |
| 430 | MS(ESI/APCI) m/z: 220 [M + H]+ |
| 43E | MS(ESI/APCI) m/z: 298, 300 [M + H]+ |
| 43F | 1H-NMR (CDCI3) δ: 6.70 (1H, s), 5.57 (1H, d, J = 6.7 Hz), 2.95-2.79 (4H, m), 2.34 (3H, s), 2.14-2.00 (2H, m). |
| 43G | 1H-NMR (CDCI3) δ: 6.91 (1H, s), 5.11 (2H, s), 3.63 (3H, s), 2.98 (2H, t, J = 7.3 Hz), 2.84 (2H, t, J = 7.6 Hz), 2.38 (3H, s), 2.11-2.01 (2H, m). |
| 43H | 1H-NMR (CDCI3) δ: 6.81 (1H, s), 5.06 (2H, s), 3.54 (3H, s), 2.93 (2H, t, J = 7.5 Hz), 2.84 (2H, t, J = 7.5 Hz), 2.35 (3H, s), 2.03-1.98 (2H, m), 1.37 (12H, s). |
| 431 | 1H-NMR (CDCI3) δ: 7.63 (1H, s), 7.37 (1H, s), 7.00 (1H, s), 5.08-4.98 (1H, m), 4.71 (2H, s), 3.08 (3H, s), 3.04-2.93 (4H, m), 2.90-2.79 (4H, m), 2.28 (3H, s), 2.16-2.06 (5H, m), 1.53 (3H, s).. |

**[Table 16]**

| | |
|---|---|
| 43J | 1H-NMR (CDCl3) δ: 7.79 (1H, s), 7.44 (1H, s), 6.81 (1H, s), 5.09-4.97 (1H, m), 3.00-2.90 (4H, m), 2.89-2.76 (4H, m), 2.44 (3H, s), 2.32 (3H, d, J = 1.2 Hz), 2.18-2.08 (2H, m), 1.55 (3H, s). |
| | MS(ESI/APCI) m/z: 364 [M + H]+ |
| 44A | MS(ESI/APCI) m/z: 500 [M + H]+ |
| 44B | MS(ESI/APCI) m/z: 448 [M + H]+ |
| 44C | MS(ESI/APCI) m/z: 450 [M + H]+ |
| 44D | 1H-NMR (CDCI3) δ: 7.87 (1H, s), 7.50 (1H, s), 7.20 (1H, s), 7.12 (1H, s), 5.08-5.06 (1H, m), 2.85-2.81 (6H, m), 2.52 (3H, s), 1.57 (3H, s), 1.34 (3H, t, J = 8.0 Hz). |
| | MS(ESI/APCI) m/z: 406 [M + H]+ |
| 45A | 1H-NMR (CDCI3) δ: 8.19 (1H, s), 7.85 (1H, s), 7.37 (1H, s), 7.29 (1H, s), 5.21-5.18 (1H, m), 5.09 (2H, s), 3.34 (3H, s), 2.96-2.94 (2H, m), 2.88-2.86 (2H, m), 2.15 (3H, s), 1.58 (3H, s). |
| | MS(ESI/APCI) m/z: 447 [M + H]+ |
| 45B | 1H-NMR (CDCI3) δ: 8.27 (1H, s), 8.08 (1H, s), 7.23 (1H, s), 7.17 (1H, s), 5.19-5.17 (1H, m), 2.99-2.96 (2H, m), 2.82-2.79 (2H, m), 2.53 (3H, s), 1.60 (3H, s). |
| | MS(ESI/APCI) m/z: 403 [M + H]+ |
| 46A | MS(ESI/APCI) m/z: 462 [M + H]+ |
| 46B | 1H-NMR (CDCI3) δ: 7.96 (1H, s), 7.44 (1H, s), 7.21 (1H, s), 7.13 (1H, s), 5.06-5.04 (1H, m), 2.89-2.83 (2H, m), 2.79-2.76 (2H, m), 2.54 (3H, s), 1.93-1.89 (1H, m), 1.56 (3H, s), 0.99-0.96 (2H, m), 0.69-0.68 (2H, m). |
| | MS(ESI/APCI) m/z: 418 [M + H]+ |
| 47A | 1H-NMR (CDCI3) δ: 7.29 (1H, s), 7.11 (1H, s). |
| 47B | 1H-NMR (CDCI3) δ: 7.56 (1H, s), 7.34 (1H, s), 3.20 (3H, s), 3.06 (3H, s). |
| 47C | 1H-NMR (CDCI3) δ: 7.40 (1H, s), 7.16 (1H, s), 5.30 (2H, s), 3.53 (3H, s). |
| 47D | 1H-NMR (CDCI3) δ: 7.24 (1H, s), 7.15 (1H, s), 5.18 (2H, s), 3.47 (3H, s), 1.41 (12H, s). |
| 47E | MS(ESI/APCI) m/z: 456 [M + H]+ |
| 47F | 1H-NMR (CDCI3) δ: 8.45 (1H, s), 7.53 (1H, s), 7.32 (1H, s), 7.29 (1H, s), 5.12-4.99 (1H, m), 2.95-2.72 (4H, m), 2.38 (3H, s), 1.57 (3H, s). |
| | MS(ESI/APCI) m/z: 412 [M + H]+ |
| 48A | 1H-NMR (CDCI3) δ: 7.01 (1H, s), 6.88 (1H, s), 6.82 (1H, s), 5.07 (1H, br s), 2.36 (3H, s). |
| 48B | 1H-NMR (CDCI3) δ: 7.10-7.05 (2H, m), 7.02 (1H, s), 5.19 (2H, s), 3.48 (3H, s), 2.38 (3H, s). |
| 48C | 1H-NMR (CDCl3) δ: 7.19 (1H, d, J = 7.3 Hz), 7.02 (1H, d, J = 5.5 Hz), 5.22 (2H, s), 3.53 (3H, s), 2.35 (3H, s). |
| 48D | 1H-NMR (CDCI3) δ: 7.09 (1H, d, J = 6.1 Hz), 5.15 (2H, s), 3.58 (3H, s), 2.37 (3H, s), 1.39 (12H, s). |
| 48E | MS(ESI/APCI) m/z: 454 [M + H]+ |
| 48F | 1H-NMR (CDCI3) δ: 7.86 (1H, s), 7.53 (1H, s), 7.05 (1H, d, J = 6.7 Hz), 5.19-4.99 (1H, m), 2.92-2.82 (2H, m), 2.82-2.71 (2H, m), 2.48 (3H, s), 2.37 (3H, s), 1.57 (3H, s). |
| | MS(ESI/APCI) m/z: 410 [M + H]+ |
| 49A | MS(ESI/APCI) m/z: 355 [M + H]+ |
| 49B | MS(ESI/APCI) m/z: 539 [M + H]+ |
| 49C | MS(ESI/APCI) m/z: 395 [M + H]+ |

**[Table 17]**

| | |
|---|---|
| 49D | 1H-NMR (CDCI3) δ: 7.24 (1H, s), 7.15 (1H, s), 7.04 (1H, s), 6.94 (1H, br s), 4.49-4.42 (1H, m), 4.33-4.26 (1H, m), 3.91-3.82 (2H, m), 3.58-3.49 (1H, m), 3.25-3.12 (2H, m), 2.89-2.76 (2H, m), 2.75 (3H, s), 2.52-2.45 (1H, m), 2.44 (3H, s), 1.93-1.84 (1H, m), 1.64-1.53 (2H, m), 1.34-1.22 (1H, m), 0.91-0.84 (1H, m). |
| | MS(ESI/APCI) m/z: 409 [M + H]+ |
| 50A | 1H-NMR (CDCI3) δ: 7.11 (1H, d, J = 6.1 Hz), 2.44 (3H, d, J = 3.0 Hz). |
| 50B | 1H-NMR (CDCI3) δ: 7.15 (1H, d, J = 6.1 Hz), 5.23 (2H, s), 3.52 (3H, s), 2.46 (3H, d, J = 2.4 Hz). |
| 50C | 1H-NMR (CDCI3) δ: 7.05 (1H, d, J = 5.5 Hz), 5.11 (2H, s), 3.47 (3H, s), 2.29 (3H, d, J = 2.4 Hz), 1.39 (12H, s). |
| 500 | MS(ESI/APCI) m/z: 454 [M + H]+ |
| 50E | 1H-NMR (CDCI3) δ: 7.83-7.78 (1H, m), 7.54 (1H, s), 7.22-7.15 (1H, m), 5.16-4.97 (1H, m), 2.93-2.73 (4H, m), 2.45-2.38 (3H, m), 2.36 (3H, s), 1.57 (3H, s). |
| | MS(ESI/APCI) m/z: 410 [M + H]+ |
| 51A | MS(ESI/APCI) m/z: 189 [M + H]+ |
| 51B | MS(ESI/APCI) m/z: 254 [M + H]+ |
| 51C | MS(ESI/APCI) m/z: 252 [M + H]+ |
| 510 | MS(ESI/APCI) m/z: 436 [M + H]+ |
| 51E | MS(ESI/APCI) m/z: 514 [M + H]+ |
| 51F | MS(ESI/APCI) m/z: 450 [M + H]+ |
| 51G | 1H-NMR (CDCI3) δ: 7.76 (1H, s), 7.21 (1H, s), 7.12 (1H, s), 4.86-4.84 (1H, m), 3.34-3.32 (2H, m), 2.94-2.92 (2H, m), 2.54 (3H, s), 2.49 (3H, s), 2.26 (3H, s), 1.51 (3H, s). |
| | MS(ESI/APCI) m/z: 406 [M + H]+ |
| 52A | MS(ESI/APCI) m/z: 393 [M + H]+ |
| 52B | 1H-NMR (CD3OD) δ: 7.82 (1H, d, J = 1.2 Hz), 7.82 (1H, s), 7.20 (1H, s), 7.08 (1H, s), 5.18-5.09 (1H, m), 2.82-2.72 (2H, m), 2.72-2.62 (2H, m), 2.36 (3H, d, J = 1.2 Hz), 2.08 (3H, s), 1.51 (3H, s). |
| | MS(ESI/APCI) m/z: 349 [M + H]+ |
| 53A | 1H-NMR (CDCI3) δ: 7.49 (1H, s), 7.34-7.32 (1H, m), 7.32-7.29 (1H, m). |
| 53B | 1H-NMR (CDCI3) δ: 7.54 (1H, s), 7.51 (2H, s), 5.24 (2H, s), 3.50 (3H, s). |
| 53C | 1H-NMR (CDCI3) δ: 7.54 (1H, s), 7.48 (1H, s), 5.23 (2H, s), 3.49 (3H, s), 1.43 (12H, s). |
| 530 | MS(ESI/APCI) m/z: 447 [M + H]+ |
| 53E | 1H-NMR (CDCI3) δ: 8.76-8.46 (1H, m), 7.63-7.37 (3H, m), 5.14-4.88 (1H, m), 2.96-2.67 (4H, m), 2.41 (3H, s), 1.54 (3H, s). |
| | MS(ESI/APCI) m/z: 403 [M + H]+ |
| 54A | MS(ESI/APCI) m/z: 268 [M + H]+ |
| 54B | MS(ESI/APCI) m/z: 382 [M + H]+ |
| 54C | MS(ESI/APCI) m/z: 380 [M + H]+ |
| 54D | MS(ESI/APCI) m/z: 564 [M + H]+ |
| 54E | 1H-NMR (CDCI3) δ: 7.83 (1H, s), 7.43-7.41 (1H, m), 7.18 (1H, s), 7.11 (1H, s), 5.34 (1H, tt, J = 12.0, 3.8 Hz), 4.45-4.42 (1H, m), 2.52 (3H, s), 2.37-2.30 (1H, m), 2.34 (3H, s), 2.23-1.58 (8H, m). |
| | MS(ESI/APCI) m/z: 406 [M + H]+ |

**[Table 18]**

| | |
|---|---|
| 55A | MS(ESI/APCI) m/z: 353 [M + H]+ |
| 55B | MS(ESI/APCI) m/z: 537 [M + H]+ |
| 55C | MS(ESI/APCI) m/z: 393 [M + H]+ |
| 55D | 1H-NMR (CDCl3) δ: 7.16-7.14 (2H, m), 7.02 (1H, s), 4.15-4.06 (1H, m), 3.84-3.75 (1H, m), 3.36-3.27 (1H, m), 2.95 (1H, dd, J = 10.4, 3.7 Hz), 2.82 (1H, d, J = 11.0 Hz), 2.68 (1H, ddd, J = 17.9, 5.4, 1.4 Hz), 2.59 (1H, t, J = 10.7 Hz), 2.46 (3H, s), 2.37-2.31 (4H, m), 2.08-2.00 (1H, m), 1.88-1.80 (2H, m), 1.77-1.69 (1H, m), 1.38 (3H, d, J = 6.1 Hz). |
| | MS(ESI/APCI) m/z: 407 [M + H]+ |
| 56A | MS(ESI/APCI) m/z: 521 [M + H]+ |
| 56B | MS(ESI/APCI) m/z: 599 [M + H]+ |
| 56C | MS(ESI/APCI) m/z: 535 [M + H]+ |
| 56D | MS(ESI/APCI) m/z: 391 [M + H]+ |
| 56E | 1H-NMR (CDCl3) δ: 7.83 (1H, s), 7.74-7.70 (1H, m), 7.22 (1H, s), 7.12 (1H, s), 5.22-5.11 (1H, m), 3.77-3.69 (1H, m), 3.10-3.00 (1H, m), 2.77-2.57 (2H, m), 2.54 (3H, s), 2.42-2.27 (7H, m), 2.20-1.93 (2H, m), 1.93-1.71 (2H, m). |
| | MS(ESI/APCI) m/z: 405 [M + H]+ |
| 57A | MS(ESI/APCI) m/z: 493 [M + H]+ |
| 57B | 1H-NMR (CDCl3) δ: 7.20-7.17 (1H, m), 7.16-7.13 (1H, m), 7.04-7.01 (1H, m), 4.36-4.20 (1H, m), 4.01-3.61 (6H, m), 3.20-2.63 (5H, m), 2.46 (3H, s), 2.39-2.21 (1H, m), 2.16-1.42 (7H, m). |
| | MS(ESI/APCI) m/z: 449 [M + H]+ |
| 58A | MS(ESI/APCI) m/z: 495 [M + H]+ |
| 58B | 1H-NMR (CDCl3) δ: 7.21-7.17 (1H, m), 7.17-7.13 (1H, m), 7.03-7.01 (1H, m), 4.42-4.27 (1H, m), 3.79-3.60 (2H, m), 3.19-3.02 (3H, m), 2.93-2.83 (1H, m), 2.55 (1H, t, J = 10.6 Hz), 2.46 (3H, s), 2.39-2.27 (1H, m), 2.37 (2H, dd, J = 22.8, 13.7 Hz), 2.00-1.89 (1H, m), 1.84-1.72 (2H, m), 1.71-1.42 (2H, m), 1.18 (3H, s), 1.17 (3H, s). |
| | MS(ESI/APCI) m/z: 451 [M + H]+ |
| 59A | MS(ESI/APCI) m/z: 542 [M + H]+ |
| 59B | MS(ESI/APCI) m/z: 668 [M + H]+ |
| 59C | MS(ESI/APCI) m/z: 610 [M + H]+ |
| 59D | 1H-NMR (CDCl3) δ: 8.09 (1H, s), 8.03 (1H, s), 7.21 (1H, s), 7.14 (1H, s), 5.16-5.14 (1H, m), 2.97-2.93 (2H, m), 2.83-2.81 (2H, m), 2.49 (3H, s), 1.59 (3H, s). |
| | MS(ESI/APCI) m/z: 446 [M + H]+ |
| 60A | MS(ESI/APCI) m/z: 357 [M + H]+ |
| 60B | MS(ESI/APCI) m/z: 541 [M + H]+ |
| 60C | MS(ESI/APCI) m/z: 397 [M + H]+ |
| 60D | 1H-NMR (CDCl3) δ: 11.42 (1H, br s), 7.21-7.18 (1H, m), 7.14 (1H, s), 7.02 (1H, s), 5.06-4.88 (1H, m), 4.42-4.30 (1H, m), 3.81-3.68 (2H, m), 3.18-3.10 (2H, m), 3.08-2.92 (2H, m), 2.64-2.56 (1H, m), 2.45 (3H, s), 2.40-2.33 (4H, m), 2.33-2.16 (2H, m). |
| | MS(ESI/APCI) m/z: 411 [M + H]+ |

**[Table 19]**

| | |
|---|---|
| 61A | MS(ESI/APCI) m/z: 477 [M + H]+ |
| 61B | 1H-NMR (CDCl3) δ: 7.18 (1H, s), 7.14 (1H, s), 7.02 (1H, s), 4.36-4.23 (1H, m), 3.78 (1H, q, J = 8.8 Hz), 3.71 (1H, q, J = 8.6 Hz), 3.18-3.08 (3H, m), 3.01-2.89 (1H, m), 2.46 (3H, s), 2.34-2.21 (3H, m), 2.08-1.99 (1H, m), 1.99-1.87 (1H, m), 1.86-1.39 (3H, m), 0.95-0.81 (1H, m), 0.55-0.47 (2H, m), 0.13-0.04 (2H, m). |
| | MS(ESI/APCI) m/z: 433 [M + H]+ |
| 62A | MS(ESI/APCI) m/z: 394 [M + H]+ |
| 62B | MS(ESI/APCI) m/z: 396 [M + H]+ |
| 62C | 1H-NMR (CDCl3) δ: 7.82 (1H, s), 7.44 (1H, s), 6.83 (1H, s), 6.74 (1H, s), 5.15-4.97 (1H, m), 2.93-2.73 (4H, m), 2.63 (2H, q, J = 7.7 Hz), 2.47 (3H, s), 2.33 (3H, s), 1.56 (3H, s), 1.27 (3H, t, J = 7.6 Hz). |
| | MS(ESI/APCI) m/z: 352 [M + H]+ |
| 63A | 1H-NMR (CDCl3) δ: 7.08 (1H, d, J = 2.4 Hz), 6.91 (1H, d, J = 2.4 Hz). |
| 63B | 1H-NMR (CDCl3) δ: 7.19-7.16 (1H, m), 6.98-6.96 (1H, m), 5.24 (2H, s), 3.51 (3H, s). |
| 63C | 1H-NMR (CDCl3) δ: 7.01-6.99 (1H, m), 6.95 (1H, d, J = 1.8 Hz), 5.12 (2H, s), 3.46 (3H, s), 1.39 (12H, s). |
| 63D | MS(ESI/APCI) m/z: 422 [M + H]+ |
| 63E | 1H-NMR (CDCl3) δ: 8.40 (1H, s), 7.49 (1H, s), 7.09 (1H, d, J = 2.4 Hz), 7.05 (1H, d, J = 2.4 Hz), 5.12-4.91 (1H, m), 2.95-2.70 (4H, m), 2.36 (3H, s), 1.56 (3H, s). |
| | MS(ESI/APCI) m/z: 378 [M + H]+ |
| 64A | MS(ESI/APCI) m/z: 546 [M + H]+ |
| 64B | MS(ESI/APCI) m/z: 402 [M + H]+ |
| 64C | 1H-NMR (CDCl3) δ: 10.51 (1H, br s), 9.02 (1H, br s), 8.05 (1H, s), 7.22 (1H, s), 7.16 (1H, s), 5.44-5.34 (1H, m), 3.09-2.66 (2H, m), 2.60-2.38 (6H, m), 2.20-2.00 (2H, m), 1.83-1.67 (2H, m), 1.61-1.49 (1H, m), 1.20-1.11 (3H, m). |
| | MS(ESI/APCI) m/z: 430 [M + H]+ |
| 65A | MS(ESI/APCI) m/z: 200 [M - tBu + H]+ |
| 65B | MS(ESI/APCI) m/z: 190 [M - tBuCO2 + H]+ |
| 65C | MS(ESI/APCI) m/z: 148 [M - tBuCO2 + H]+ |
| 65D | MS(ESI/APCI) m/z: 302 [M - tBuCO2 + H]+ |
| 65E | MS(ESI/APCI) m/z: 174 [M - tBu + H]+ |
| 65F | 1H-NMR (CDCl3) δ: 3.82 (2H, s), 3.61 (2H, s), 1.85-1.83 (2H, m), 1.64-1.63 (2H, m). |
| 65G | MS(ESI/APCI) m/z: 268 [M + H]+ |
| 65H | MS(ESI/APCI) m/z: 382 [M + H]+ |
| 65I | MS(ESI/APCI) m/z: 380 [M + H]+ |
| 65J | MS(ESI/APCI) m/z: 564 [M + H]+ |
| 65K | MS(ESI/APCI) m/z: 642 [M + H]+ |
| 65L | MS(ESI/APCI) m/z: 578 [M + H]+ |
| 65M | 1H-NMR (CDCl3) δ: 7.85 (1H, s), 7.32 (1H, s), 7.21 (1H, s), 7.13 (1H, s), 4.35 (2H, s), 4.02 (2H, s), 2.54-2.53 (7H, m), 2.35 (3H, s). |
| | MS(ESI/APCI) m/z: 420 [M + H]+ |

**[Table 20]**

| | |
|---|---|
| 66 | 1H-NMR (CDCl3) δ: 7.20 (1H, s), 7.15 (1H, s), 7.02 (1H, s), 5.06-4.88 (1H, m), 4.42-4.31 (1H, m), 3.84-3.68 (2H, m), 3.19-2.97 (4H, m), 2.67-2.58 (1H, m), 2.54 (2H, q, J = 7.2 Hz), 2.46 (3H, s), 2.44-2.14 (3H, m), 1.11 (3H, t, J = 7.2 Hz). |
| | MS(ESI/APCI) m/z: 425 [M + H]+ |
| 67A | MS(ESI/APCI) m/z: 407 [M - tBu + H]+ |
| 67B | MS(ESI/APCI) m/z: 306 [M - tBu + H]+ |
| 67C | MS(ESI/APCI) m/z: 544 [M + H]+ |
| 67D | MS(ESI/APCI) m/z: 400 [M + H]+ |
| 67E | 1H-NMR (CDCl3) δ: 9.09-8.85 (1H, m), 8.02 (1H, s), 6.73 (1H, s), 6.67 (1H, s), 6.58 (1H, t, J = 74.4 Hz), 5.42-5.28 (1H, m), 3.11-2.86 (2H, m), 2.86-2.65 (1H, m), 2.57-2.43 (1H, m), 2.53 (2H, q, J = 7.9 Hz), 2.50 (3H, s), 2.21-1.99 (2H, m), 1.83-1.70 (2H, m), 1.15 (3H, t, J = 7.3 Hz). |
| | MS(ESI/APCI) m/z: 428 [M + H]+ |
| 68 | 1H-NMR (CDCl3) δ: 9.13-8.79 (1H, m), 8.02 (1H, s), 6.74 (1H, s), 6.68 (1H, s), 6.58 (1H, t, J = 73.8 Hz), 5.45-5.30 (1H, m), 3.11-3.03 (1H, m), 3.03-2.94 (1H, m), 2.94-2.83 (1H, m), 2.83-2.71 (1H, m), 2.68-2.55 (1H, m), 2.50 (3H, s), 2.17-1.99 (2H, m), 1.80-1.68 (2H, m), 1.11 (3H, d, J = 4.3 Hz), 1.09 (3H, d, J = 3.6 Hz). |
| | MS(ESI/APCI) m/z: 442 [M + H]+ |
| 69A | 1H-NMR (CDCl3) δ: 7.42 (2H, d, J = 1.6 Hz), 7.09-7.05 (1H, m), 5.22-5.19 (2H, m), 3.51-3.48 (3H, m), 2.60-2.56 (3H, m), 2.40 (3H, m). |
| 69B | 1H-NMR (CDCl3) δ: 7.17-7.11 (1H, m), 7.09-7.06 (1H, m), 6.57-6.53 (1H, m), 4.99-4.65 (1H, m), 3.50-3.34 (4H, m), 2.32 (3H, s), 2.15-2.11 (3H, m). |
| 69C | 1H-NMR (CDCl3) δ: 7.25-7.23 (1H, m), 7.21 (1H, s), 6.77-6.75 (1H, m), 5.17 (2H, s), 3.50 (3H, s), 3.48-3.36 (4H, m), 2.38-2.30 (3H, m), 2.13 (3H, s). |
| 69D | 1H-NMR (CDCl3) δ: 7.00-6.95 (2H, m), 6.93-6.90 (1H, m), 5.20-5.18 (2H, m), 3.51-3.49 (3H, m), 2.39-2.35 (3H, m), 1.97-1.83 (3H, m). |
| 69E | 1H-NMR (CDCl3) δ: 6.88 (1H, s), 6.80-6.77 (1H, m), 6.74-6.69 (1H, m), 5.21-5.14 (1H, m), 2.36-2.32 (3H, m), 1.95-1.83 (3H, m). |
| 69F | 1H-NMR (CDCl3) δ: 6.96 (2H, s), 5.57 (1H, s), 2.49 (3H, s), 1.89 (3H, t, J = 18.2 Hz). |
| 69G | 1H-NMR (CDCl3) δ: 7.08-7.04 (1H, m), 7.01-6.98 (1H, m), 5.29-5.26 (2H, m), 3.55-3.52 (3H, m), 2.55-2.49 (3H, m), 1.98-1.82 (3H, m). |
| 69H | 1H-NMR (CDCl3) δ: 6.96-6.94 (1H, m), 6.94-6.92 (1H, m), 5.17-5.16 (2H, m), 3.49-3.47 (3H, m), 2.40-2.37 (3H, m), 1.91-1.82 (3H, m), 1.40 (12H, s). |
| 69I | MS(ESI/APCI) m/z: 432 [M + H]+ |
| 69J | 1H-NMR (CDCl3) δ: 7.84 (1H, s), 7.49 (1H, s), 7.09 (1H, s), 7.03 (1H, s), 5.15-4.95 (1H, m), 2.93-2.72 (4H, m), 2.52 (3H, s), 2.35 (3H, s), 1.94 (3H, t, J = 18.2 Hz), 1.57 (3H, s). |
| | MS(ESI/APCI) m/z: 388 [M + H]+ |
| 70A | MS(ESI/APCI) m/z: 252 [M + H]+ |
| 70B | MS(ESI/APCI) m/z: 366 [M + H]+ |
| 70C | MS(ESI/APCI) m/z: 364 [M + H]+ |
| 70D | MS(ESI/APCI) m/z: 548 [M + H]+ |
| 70E | MS(ESI/APCI) m/z: 626 [M + H]+ |
| 70F | MS(ESI/APCI) m/z: 562 [M + H]+ |

**[Table 21]**

| | |
|---|---|
| 70G | 1H-NMR (CDCl3) δ: 7.83 (1H, s), 7.30 (1H, s), 7.21 (1H, s), 7.12 (1H, s), 2.54 (3H, s), 2.45-2.35 (6H, m), 2.33 (3H, s), 2.04-2.02 (2H, m). |
| | MS(ESI/APCI) m/z: 404 [M + H]+ |
| 71A | MS(ESI/APCI) m/z: 533 [M + H]+ |
| 71B | 1H-NMR (CDCl3) δ: 7.79 (1H, s), 7.75-7.63 (1H, m), 6.73 (1H, d, J = 2.4 Hz), 6.64 (1H, d, J = 2.4 Hz), 6.57 (1H, t, J = 74.1 Hz), 5.21-5.08 (1H, m), 3.16-3.03 (1H, m), 2.84-2.71 (1H, m), 2.71-2.58 (1H, m), 2.58-2.42 (5H, m), 2.42-2.24 (4H, m), 2.22-1.94 (2H, m), 1.92-1.71 (2H, m), 1.12 (3H, t, J = 7.3 Hz). |
| | MS(ESI/APCI) m/z: 417 [M + H]+ |
| 72A | MS(ESI/APCI) m/z: 564 [M + H]+ |
| 72B | 1H-NMR (CDCl3) δ: 7.90 (1H, s), 7.18 (1H, s), 6.74-6.71 (1H, m), 6.66-6.61 (1H, m), 6.57 (1H, t, J = 74.1 Hz), 5.21-5.06 (1H, m), 3.95 (3H, s), 2.91-2.81 (2H, m), 2.81-2.71 (2H, m), 2.49 (3H, s), 1.58 (3H, s). |
| | MS(ESI/APCI) m/z: 406 [M + H]+ |
| 73A | MS(ESI/APCI) m/z: 363 [M + H]+ |
| 73B | MS(ESI/APCI) m/z: 547 [M + H]+ |
| 73C | MS(ESI/APCI) m/z: 625, 627 [M + H]+ |
| 73E | MS(ESI/APCI) m/z: 572 [M + H]+ |
| 73F | MS(ESI/APCI) m/z: 428 [M + H]+ |
| 73G | 1H-NMR (CDCl3) δ: 8.21 (1H, s), 8.05 (1H, s), 7.22 (1H, s), 7.16 (1H, s), 5.42-5.35 (1H, m), 3.86-3.82 (1H, m), 3.44-3.39 (1H, m), 2.61-2.53 (2H, m), 2.52 (3H, s), 2.19-1.55 (8H, m), 1.20 (3H, t, J = 7.4 Hz). |
| | MS(ESI/APCI) m/z: 456 [M + H]+ |
| 74A | MS(ESI/APCI) m/z: 484 [M + H]+ |
| 74B | MS(ESI/APCI) m/z: 383 [M + H]+ |
| 74C | MS(ESI/APCI) m/z: 565 [M + H]+ |
| 74D | 1H-NMR (CD3OD) δ: 8.76 (1H, s), 7.97 (1H, s), 6.84 (1H, t, J = 74.4 Hz), 6.68-6.62 (1H, m), 6.62-6.57 (1H, m), 5.26-5.08 (1H, m), 2.94-2.78 (2H, m), 2.78-2.66 (2H, m), 2.07 (3H, s), 1.52 (3H, s). |
| | MS(ESI/APCI) m/z: 401 [M + H]+ |
| 75A | MS(ESI/APCI) m/z: 148 [M + H]+ |
| 75B | MS(ESI/APCI) m/z: 282 [M + H]+ |
| 75C | MS(ESI/APCI) m/z: 590 [M + H]+ |
| 75D | MS(ESI/APCI) m/z: 263 [M + H]+ |
| 75E | MS(ESI/APCI) m/z: 263 [M - tBuCO₂ + H]+ |
| 75F | MS(ESI/APCI) m/z: 307 [M - tBuCO₂ + H]+ |
| 75G | MS(ESI/APCI) m/z: 217 [M - tBu + H]+ |
| 75H | MS(ESI/APCI) m/z: 411 [M + H]+ |
| 75I | MS(ESI/APCI) m/z: 409 [M + H]+ |
| 75J | MS(ESI/APCI) m/z: 593 [M + H]+ |
| 75K | MS(ESI/APCI) m/z: 671 [M + H]+ |
| 75L | MS(ESI/APCI) m/z: 607 [M + H]+ |
| 75M | MS(ESI/APCI) m/z: 419 [M + H]+ |
| 75N | 1H-NMR (CDCl3) δ: 7.82 (1H, s), 7.27 (1H, s), 7.20 (1H, s), 7.12 (1H, s), 3.12 (2H, s), 2.98-2.96 (2H, m), 2.92 (2H, s), 2.64-2.62 (2H, m), 2.53 (3H, s), 2.45-2.43 (2H, m), 2.33 (3H, s), 1.10 (3H, t, J = 7.6 Hz). |
| | MS(ESI/APCI) m/z: 447 [M + H]+ |

**[Table 22]**

| | |
|---|---|
| 76A | MS(ESI/APCI) m/z: 561 [M + H]+ |
| 76B | MS(ESI/APCI) m/z: 417 [M + H]+ |
| 76C | 1H-NMR (CDCl3) δ: 7.83 (1H, s), 7.51 (1H, s), 7.21 (1H, s), 7.12 (1H, s), 5.31-5.22 (1H, m), 3.75 (1H, s), 3.38 (1H, s), 2.65-2.50 (2H, m), 2.54 (3H, s), 2.35 (3H, s), 2.24-1.44 (8H, m), 1.30-1.16 (3H, m). |
| | MS(ESI/APCI) m/z: 445 [M + H]+ |
| 77A | MS(ESI/APCI) m/z: 395 [M + H]+ |
| 77B | MS(ESI/APCI) m/z: 353 [M + H]+ |
| 77C | MS(ESI/APCI) m/z: 367 [M + H]+ |
| 77D | MS(ESI/APCI) m/z: 551 [M + H]+ |
| 77E | MS(ESI/APCI) m/z: 407 [M + H]+ |
| 77F | 1H-NMR (CDCl3) δ: 7.92 (1H, s), 7.51-7.46 (1H, m), 7.21 (1H, s), 7.12 (1H, s), 5.26-5.17 (1H, m), 3.94 (3H, s), 3.11-3.05 (1H, m), 2.80-2.62 (2H, m), 2.54 (3H, s), 2.51 (2H, q, J = 7.2 Hz), 2.41-2.31 (1H, m), 2.17-1.72 (4H, m), 1.12 (3H, t, J = 7.1 Hz). |
| | MS(ESI/APCI) m/z: 435 [M + H]+ |
| 78A | 1H-NMR (CDCl3) δ: 7.72 (1H, d, J = 3.6 Hz), 7.67 (1H, s), 7.37-7.31 (5H, m), 6.47 (1H, d, J = 3.6 Hz), 5.33-5.30 (1H, m), 4.48 (2H, s), 2.82-2.77 (2H, m), 2.66-2.63 (2H, m), 1.62 (3H, s). |
| 78B | 1H-NMR (CDCl3) δ: 7.85 (1H, s), 7.63 (1H, s), 7.38-7.29 (5H, m), 5.35-5.32 (1H, m), 4.47 (2H, s), 2.81-2.75 (2H, m), 2.66-2.64 (2H, m), 2.36 (3H, s). |
| 78C | MS(ESI/APCI) m/z: 344 [M + H]+ |
| 78D | MS(ESI/APCI) m/z: 394 [M + H]+ |
| 78E | MS(ESI/APCI) m/z: 576 [M + H]+ |
| 78F | MS(ESI/APCI) m/z: 486 [M + H]+ |
| 78G | 1H-NMR (CDCl3) δ: 7.91 (1H, s), 7.63 (1H, s), 6.75-6.70 (1H, m), 6.67-6.64 (1H, m), 6.57 (1H, t, J = 74.1 Hz), 6.55 (1H, t, J = 72.0 Hz), 5.22-5.03 (1H, m), 2.95-2.84 (2H, m), 2.84-2.72 (2H, m), 2.47 (3H, s), 1.58 (3H, s). |
| | MS(ESI/APCI) m/z: 442 [M + H]+ |
| 79B | MS(ESI/APCI) m/z: 547 [M + H]+ |
| 79C | MS(ESI/APCI) m/z: 403 [M + H]+ |
| 79D | 1H-NMR (DMSO-D6) δ: 8.21 (1H, s), 7.20 (1H, t, J = 74.8 Hz), 7.02 (1H, s), 6.57-6.54 (2H, m), 4.12-4.05 (1H, m), 3.80-3.72 (1H, m), 3.66 (1H, q, J = 8.8 Hz), 3.60-3.56 (1H, m), 3.30-3.24 (1H, m), 3.01 (2H, t, J = 8.0 Hz), 2.47-2.38 (2H, m), 2.06 (3H, s), 1.97-1.88 (1H, m), 1.84-1.63 (5H, m), 1.61-1.45 (2H, m), 1.06 (3H, t, J = 7.1 Hz). |
| | MS(ESI/APCI) m/z: 431 [M + H]+ |
| 80A | MS(ESI/APCI) m/z: 574 [M + H]+ |
| 80B | 1H-NMR (CDCl3) δ: 7.64 (1H, s), 6.72 (1H, d, J = 2.4 Hz), 6.62 (1H, d, J = 2.4 Hz), 6.56 (1H, t, J = 74.1 Hz), 4.96-4.77 (1H, m), 3.09 (2H, t, J = 7.0 Hz), 3.05-2.96 (2H, m), 2.91-2.78 (4H, m), 2.66-2.54 (2H, m), 2.49 (3H, s), 1.53 (3H, s). |
| | MS(ESI/APCI) m/z: 416 [M + H]+ |
| 81A | MS(ESI/APCI) m/z: 303 [M + H]+ |
| 81B | MS(ESI/APCI) m/z: 192 [M - tBuCO₂ + H]+ |
| 81C | MS(ESI/APCI) m/z: 192 [M + H]+ |

**[Table 23]**

| | |
|---|---|
| 81D | MS(ESI/APCI) m/z: 378 [M + H]+ |
| 81E | MS(ESI/APCI) m/z: 562 [M + H]+ |
| 81F | MS(ESI/APCI) m/z: 472 [M + H]+ |
| 81G | 1H-NMR (CD3OD) δ: 8.35 (1H, s), 8.27 (1H, d, J = 7.9 Hz), 7.91 (1H, d, J = 8.5 Hz), 7.72 (1H, t, J = 7.6 Hz), 7.35 (1H, t, J = 7.3 Hz), 7.15 (1H, s), 7.09 (1H, s), 5.33-5.20 (1H, m), 3.42-3.33 (2H, m), 2.86-2.74 (2H, m), 2.17 (3H, s), 1.57 (3H, s). |
| | MS(ESI/APCI) m/z: 428 [M + H]+ |
| 82A | MS(ESI/APCI) m/z: 542 [M + H]+ |
| 82B | 1H-NMR (CD3OD) δ: 7.54 (1H, s), 6.84 (1H, d, J = 1.8 Hz), 6.80 (1H, d, J = 1.8 Hz), 5.12-4.97 (1H, m), 3.26-3.18 (2H, m), 2.95-2.80 (4H, m), 2.80-2.66 (2H, m), 2.66-2.52 (2H, m), 2.03 (3H, s), 1.50 (3H, s). |
| | MS(ESI/APCI) m/z: 384 [M + H]+ |
| 83A | 1H-NMR (CDCl3) δ: 7.51 (1H, s), 3.91 (2H, q, J = 6.5 Hz), 2.83 (2H, t, J = 6.5 Hz), 1.98 (1H, t, J = 6.5 Hz). |
| | MS(ESI/APCI) m/z: 217 [M + H]+ |
| 83B | 1H-NMR (CDCl3) δ: 7.75 (1H, s), 6.50 (1H, t, J = 3.6 Hz), 4.49 (2H, t, J = 9.6 Hz), 3.00 (2H, dt, J = 3.6, 9.6 Hz). |
| | MS(ESI/APCI) m/z: 217 [M + H]+ |
| 83C | 1H-NMR (CDCl3) δ: 7.98 (1H, s), 5.08 (1H, d, J = 5.2 Hz), 4.50-4.47 (1H, m), 4.39-4.37 (1H, m), 3.11-3.01 (1H, m), 2.56-2.52 (1H, m), 2.04 (3H, s). |
| | MS(ESI/APCI) m/z: 403 [M + H]+ |
| 83D | 1H-NMR (CDCl3) δ: 7.61 (1H, s), 4.57 (2H, t, J = 9.6 Hz), 3.11 (2H, t, J = 9.6 Hz). |
| | MS(ESI/APCI) m/z: 343 [M + H]+ |
| 83E | 1H-NMR (CDCl3) δ: 7.43 (1H, s), 5.28-5.25 (1H, m), 5.04 (2H, t, J = 8.4 Hz), 3.57 (2H, t, J = 8.4 Hz), 2.73-2.72 (2H, m), 2.52-2.50 (2H, m), 1.53 (3H, s), 0.90 (9H, s), 0.10 (6H, s). |
| | MS(ESI/APCI) m/z: 394 [M + H]+ |
| 83F | 1H-NMR (CDCl3) δ: 7.41 (1H, s), 7.32 (1H, s), 7.24 (1H, s), 5.47-5.45 (1H, m), 5.08 (2H, t, J = 8.8 Hz), 5.05 (2H, s), 3.61 (2H, t, J = 8.8 Hz), 3.32 (3H, s), 2.79-2.74 (2H, m), 2.56-2.53 (2H, m), 2.14 (3H, s), 1.54 (3H, s), 0.92 (9H, s), 0.12 (6H, s). |
| | MS(ESI/APCI) m/z: 578 [M + H]+ |
| 83G | 1H-NMR (CDCl3) δ: 7.69 (1H, s), 7.20 (1H, s), 7.11 (1H, s), 5.11-5.08 (3H, m), 3.61 (2H, t, J = 8.8 Hz), 2.84-2.81 (4H, m), 2.51 (3H, s), 2.51 (3H, s). MS(ESI/APCI) m/z: 420 [M + H]+ |
| 84A | 1H-NMR (CDCl3) δ: 8.75 (2H, s), 8.49 (1H, d, J = 4.0 Hz), 7.75 (1H, s), 6.67 (1H, d, J = 3.6 Hz). |
| | MS(ESI/APCI) m/z: 250 [M + H]+ |
| 84B | MS(ESI/APCI) m/z: 434 [M + H]+ |
| 84C | 1H-NMR (CD3OD) δ: 8.87 (2H, s), 8.64 (1H, d, J = 4.3 Hz), 8.01 (1H, s), 7.14 (1H, s), 7.08 (1H, s), 6.91 (1H, d, J = 4.3 Hz), 2.17 (3H, s). |
| | MS(ESI/APCI) m/z: 390 [M + H]+ |
| 85A | MS(ESI/APCI) m/z: 438 [M + H]+ |

**[Table 24]**

| | |
|---|---|
| 85B | 1H-NMR (CD3OD) δ: 7.34 (1H, d, J = 1.2 Hz), 7.10 (1H, s), 7.02 (1H, s), 4.24 (1H, q, J = 8.4 Hz), 4.04 (1H, t, J = 9.2 Hz), 3.57-3.50 (1H, m), 3.47-3.41 (1H, m), 2.48-2.39 (4H, m), 2.21 (3H, s), 1.47-1.38 (6H, m). |
| | MS(ESI/APCI) m/z: 394 [M + H]+ |
| 86A | MS(ESI/APCI) m/z: 438 [M + H]+ |
| 86B | 1H-NMR (CDCl3) δ: 7.24-7.22 (1H, m), 7.17 (1H, s), 7.05-7.02 (1H, m), 4.12-4.03 (1H, m), 3.90-3.80 (1H, m), 3.34-3.31 (1H, m), 2.91-2.54 (6H, m), 2.47 (3H, s), 1.44-1.42 (3H, m), 1.32-1.29 (3H, m). |
| | MS(ESI/APCI) m/z: 394 [M + H]+ |
| 87A | MS(ESI/APCI) m/z: 436 [M + H]+ |
| 87B | 1H-NMR (CDCl3) δ: 7.82-7.79 (1H, m), 7.24-7.21 (1H, m), 7.14-7.10 (1H, m), 6.35-6.31 (1H, m), 4.77-4.68 (1H, m), 3.37-3.31 (2H, m), 2.94-2.86 (2H, m), 2.57 (3H, s), 2.52 (3H, s), 1.52 (3H, s). |
| | MS(ESI/APCI) m/z: 392 [M + H]+ |
| 88 | 1H-NMR (CD3OD) δ: 7.84 (1H, s), 7.79 (1H, d, J = 3.5 Hz), 7.14 (1H, s), 7.07 (1H, s), 6.62 (1H, d, J = 3.5 Hz), 2.80-2.76 (2H, m), 2.51-2.45 (2H, m), 2.28-2.23 (2H, m), 2.14 (3H, s), 2.06-1.97 (4H, m). |
| | MS(ESI/APCI) m/z: 404 [M + H]+ |
| 89A | MS(ESI/APCI) m/z: 214 [M + H]+ |
| 89B | MS(ESI/APCI) m/z: 398 [M + H]+ |
| 89C | MS(ESI/APCI) m/z: 512 [M + H]+ |
| 89D | MS(ESI/APCI) m/z: 510 [M + H]+ |
| 89E | 1H-NMR (CD3OD) δ: 7.89-7.86 (2H, m), 7.13 (1H, s), 7.06 (1H, s), 6.65 (1H, d, J = 3.4 Hz), 4.57 (1H, dd, J = 13.6, 3.3 Hz), 4.39-4.29 (2H, m), 2.12 (3H, s), 1.26 (3H, d, J = 6.1 Hz). |
| | MS(ESI/APCI) m/z: 352 [M + H]+ |
| 90A | MS(ESI/APCI) m/z: 382 [M + H]+ |
| 90B | 1H-NMR (CD3OD) δ: 7.82 (1H, d, J = 0.8 Hz), 7.80 (1H, s), 6.68 (1H, s), 6.63 (1H, s), 5.11(1H, q, J = 8.4 Hz), 2.80-2.72 (2H, m), 2.72-2.64 (2H, m), 2.36 (3H, d, J = 0.8 Hz), 2.30 (3H, s), 2.02 (3H, s), 1.51 (3H, s). |
| | MS(ESI/APCI) m/z: 338 [M + H]+ |
| 91A | 1H-NMR (CDCl3) δ: 6.71-6.52 (2H, m), 5.71 (1H, s), 2.44 (3H, s). |
| 91B | 1H-NMR (CDCl3) δ: 6.74-6.67 (2H, s), 5.23 (2H, s), 3.52 (3H, s), 2.47 (3H, s). |
| 91C | 1H-NMR (CDCl3) δ: 6.59 (1H, dd, J = 10.8, 2.0 Hz), 6.53 (1H, dd, J = 9.6, 2.0 Hz), 5.12 (2H, s), 3.47 (3H, s), 2.35 (3H, s), 1.39 (12H, s). |
| 91D | MS(ESI/APCI) m/z: 382 [M + H]+ |
| 91E | 1H-NMR (CD3OD) δ: 7.82 (1H, s), 7.80 (1H, s), 6.59 (1H, dd, J = 9.6, 2.4 Hz), 6.53 (1H, dd, J = 10.4, 2.4 Hz), 5.12 (1H, q, J = 8.4 Hz), 2.81-2.72 (2H, m), 2.72-2.63 (2H, m), 2.36 (3H, d, J = 0.8 Hz), 2.05 (3H, s), 1.52 (3H, s). |
| | MS(ESI/APCI) m/z: 342 [M + H]+ |
| 92A | 1H-NMR (CDCl3) δ: 6.90-6.87 (1H, m), 6.81-6.76 (2H, m), 6.70-6.40 (1H, m), 5.52 (1H, s), 2.36-2.33 (3H, m). |
| 92B | 1H-NMR (CDCl3) δ: 7.00-6.92 (2H, m), 6.72-6.40 (1H, m), 5.57-5.62 (1H, m), 2.53-2.46 (3H, m). |
| 92C | 1H-NMR (CDCl3) δ: 7.09-7.06 (1H, m), 7.03-6.98 (1H, m), 6.73-6.42 (1H, m), 5.32-5.23 (2H, m), 3.60-3.45 (3H, m) 2.58-2.47 (3H, m). |

**[Table 25]**

| | |
|---|---|
| 92D | 1H-NMR (CDCl3) δ: 6.90-6.85 (2H, m), 6.63-6.31 (1H, m), 5.09 (2H, s), 3.42-3.38 (3H, m), 2.34-2.26 (3H, m), 1.34-1.30 (12H, m). |
| 92E | MS(ESI/APCI) m/z: 418 [M + H]+ |
| 92F | 1H-NMR (CDCl3) δ: 7.91-7.84 (1H, m), 7.63-7.57 (1H, m), 7.16-7.10 (1H, m), 7.06-7.01 (1H, m), 6.75-6.45 (1H, m), 5.11-5.02 (1H, m), 2.91-2.77 (4H, m) 2.51-2.46 (3H, m), 2.39-2.36 (3H, m), 1.57 (3H, s). |
| | MS(ESI/APCI) m/z: 374 [M + H]+ |
| 93A | MS(ESI/APCI) m/z: 333 [M + H]+ |
| 93B | MS(ESI/APCI) m/z: 494 [M + H]+ |
| 93C | MS(ESI/APCI) m/z: 352 [M + H]+ |
| 93D | 1H-NMR (CD3OD) δ: 8.51 (1H, s), 7.74 (2H, s), 7.14 (1H, s), 7.06 (1H, s), 3.51-3.38 (2H, m), 3.10-3.00 (2H, m), 2.95-2.72 (5H, m), 2.52-2.33 (1H, m), 2.16 (3H, s), 2.02-1.70 (3H, m), 1.27-1.49 (1H, m). |
| | MS(ESI/APCI) m/z: 366 [M + H]+ |
| 94A | 1H-NMR (CDCl3) δ: 8.07 (1H, s), 7.93 (1H, d, J = 3.7 Hz), 7.34 (1H, s), 7.28-7.26 (1H, m), 7.10 (1H, d, J = 3.1 Hz), 5.09 (2H, s), 3.34 (3H, s), 2.18 (3H, s), 1.67 (9H, s). |
| | MS(ESI/APCI) m/z: 438 [M + H]+ |
| 94B | 1H-NMR (CD3OD) δ: 7.75 (1H, q, J = 1.8 Hz), 7.58 (1H, s), 7.37 (1H, s), 7.30 (1H, s), 6.98 (1H, s), 5.10 (2H, s), 3.32 (3H, s), 2.14 (3H, s). |
| | MS(ESI/APCI) m/z: 338 [M + H]+ |
| 94C | 1H-NMR (CDCl3) δ: 7.79-7.75 (1H, m), 7.51-7.47 (1H, m), 7.36-7.32 (1H, m), 7.31-7.27 (1H, m), 5.09-5.05 (2H, m), 3.32-3.30 (3H, m), 2.15-2.12 (3H, m). |
| | MS(ESI/APCI) m/z: 464 [M + H]+ |
| 94D | 1H-NMR (CDCl3) δ: 7.54-7.49 (1H, m), 7.35-7.30 (2H, m), 7.27 (1H, s), 5.06-5.02 (2H, m), 3.89-3.82 (3H, m), 3.32-3.25 (3H, m), 2.16-2.10 (3H, m). |
| | MS(ESI/APCI) m/z: 478 [M + H]+ |
| 94E | 1H-NMR (CDCl3) δ: 7.72-7.54 (1H, m), 7.40-7.28 (4H, m), 5.06 (2H, s), 4.41-4.33 (2H, m), 3.85-3.77 (3H, m), 3.70-3.60 (2H, m), 3.37-3.28 (3H, m), 2.50-2.41 (2H, m), 2.22-2.15 (3H, m), 1.24 (9H, s). |
| | MS(ESI/APCI) m/z: 533 [M + H]+ |
| 94F | 1H-NMR (CDCl3) δ: 7.34 (1H, s), 7.29 (1H, s), 7.27 (1H, s), 7.23 (1H, s), 5.07 (2H, s), 4.25-4.17 (1H, m), 4.05-3.84 (1H, m), 3.78 (3H, s), 3.50-3.38 (2H, m), 3.33 (3H, s), 3.18-3.03 (1H, m), 2.32-2.24 (1H, m), 2.17 (3H, s), 1.80-1.64 (3H, m), 1.47 (9H, s). |
| | MS(ESI/APCI) m/z: 535 [M + H]+ |
| 94G | 1H-NMR (CD3OD) δ: 7.69 (1H, s), 7.53 (1H, s), 7.01 (2H, s), 3.82 (3H, s), 3.39-3.29 (2H, m), 3.12 (1H, d, J = 12.3 Hz), 2.89 (1H, t, J = 11.7 Hz), 2.73 (1H, td, J = 12.3, 3.1 Hz), 2.29-2.22 (1H, m), 2.09 (3H, s), 1.94-1.74 (2H, m). |
| | MS(ESI/APCI) m/z: 391 [M + H]+ |
| 94H | 1H-NMR (CDCl3) δ: 7.51 (1H, s), 7.30 (1H, s), 7.21 (1H, s), 7.11 (1H, s), 3.82 (3H, s), 3.52-3.43 (1H, m), 3.21-3.11 (1H, m), 2.89-2.77 (1H, m), 2.53 (3H, s), 2.50-2.41 (1H, m), 2.35 (3H, s), 2.23-2.13 (2H, m), 1.85-1.72 (3H, m). |
| | MS(ESI/APCI) m/z: 405 [M + H]+ |
| 95A | MS(ESI/APCI) m/z: 585 [M + H]+ |

**[Table 26]**

| | |
|---|---|
| 95B | 1H-NMR (CDCl3) δ: 7.93 (1H, s), 7.37-7.35 (1H, m), 7.29-7.28 (1H, m), 6.90 (1H, s), 5.09 (2H, s), 3.33 (3H, s), 2.18 (3H, s). |
| | MS(ESI/APCI) m/z: 371 [M + H]+ |
| 95C | MS(ESI/APCI) m/z: 503 [M + H]+ |
| 95D | MS(ESI/APCI) m/z: 536 [M + H]+ |
| 95E | 1H-NMR (CDCl3) δ: 7.96 (1H, s), 7.55 (1H, s), 7.37 (1H, s), 7.29 (1H, s), 5.09 (2H, s), 4.30 (1H, d, J = 12.9 Hz), 4.06-3.95 (1H, m), 3.77-3.64 (1H, m), 3.46-3.37 (1H, m), 3.34 (3H, s), 3.13-3.02 (1H, m), 2.39-2.27 (1H, m), 2.20 (3H, s), 2.06-1.90 (1H, m), 1.81-1.61 (2H, m), 1.46 (9H, s). |
| | MS(ESI/APCI) m/z: 538 [M + H]+ |
| 95F | MS(ESI/APCI) m/z: 394 [M + H]+ |
| 95G | 1H-NMR (CDCl3) δ: 8.20 (1H, s), 7.70 (1H, s), 7.24 (1H, s), 7.14 (1H, s), 3.82-3.75 (1H, m), 3.18 (1H, d, J = 9.8 Hz), 2.90-2.78 (1H, m), 2.53 (3H, s), 2.52-2.43 (1H, m), 2.37 (3H, s), 2.25-2.18 (2H, m), 1.87-1.80 (2H, m), 1.78-1.68 (1H, m). |
| | MS(ESI/APCI) m/z: 408 [M + H]+ |
| 96A | 1H-NMR (CDCl3) δ: 7.20 (1H, s), 3.12 (2H, t, J = 6.3 Hz), 2.81 (2H, t, J = 6.3 Hz), 1.99-1.91 (2H, m), 1.88-1.80 (2H, m). |
| | MS(ESI/APCI) m/z: 169 [M + H]+ |
| 96B | 1H-NMR (CDCl3) δ: 6.91 (1H, s), 2.85 (2H, t, J = 6.3 Hz), 2.78 (2H, t, J = 6.3 Hz), 1.97-1.88 (2H, m), 1.84-1.75 (2H, m). |
| | MS(ESI/APCI) m/z: 185 [M + H]+ |
| 96C | 1H-NMR (CDCl3) δ: 7.28 (1H, s), 4.98 (1H, dd, J = 8.0, 5.5 Hz), 3.73 (1H, br s), 2.94-2.77 (2H, m), 2.33-2.23 (1H, m), 2.13-2.01 (1H, m), 1.99-1.89 (1H, m), 1.87-1.78 (1H, m). |
| | MS(ESI/APCI) m/z: 185 [M + H]+ |
| 96D | 1H-NMR (CDCl3) δ: 7.52-7.51 (1H, m), 3.06 (2H, t, J = 6.1 Hz), 2.91 (2H, t, J = 6.7 Hz), 2.28-2.21 (2H, m). |
| | MS(ESI/APCI) m/z: 183 [M + H]+ |
| 96E | MS(ESI/APCI) m/z: 339 [M + H]+ |
| 96F | 1H-NMR (CDCl3) δ: 7.85 (1H, s), 7.65 (1H, t, J = 8.0 Hz), 7.31 (1H, d, J = 8.6 Hz), 7.23 (1H, d, J = 7.4 Hz), 1.51-1.41 (3H, m), 1.13 (18H, d, J = 7.4 Hz). |
| | MS(ESI/APCI) m/z: 337 [M + H]+ |
| 96G | 1H-NMR (CDCl3) δ: 7.77 (1H, s), 7.66 (1H, t, J = 8.0 Hz), 7.42-7.38 (1H, m), 7.35-7.33 (1H, m), 7.30-7.28 (1H, m), 7.10-7.07 (1H, m), 5.07 (2H, s), 3.28 (3H, s), 2.17 (3H, s), 1.54-1.47 (3H, m), 1.17-1.12 (18H, m). |
| | MS(ESI/APCI) m/z: 521 [M + H]+ |
| 96H | 1H-NMR (CDCl3) δ: 7.86 (1H, s), 7.74 (1H, t, J = 8.0 Hz), 7.39 (2H, t, J = 4.3 Hz), 7.33-7.29 (2H, m), 5.09 (2H, s), 3.32 (3H, s), 2.19 (3H, s). |
| | MS(ESI/APCI) m/z: 365 [M + H]+ |
| 96I | 1H-NMR (CDCl3) δ: 7.97-7.92 (2H, m), 7.85 (1H, t, J = 7.7 Hz), 7.81-7.78 (1H, m), 7.39 (1H, s), 7.32 (1H, s), 5.09 (2H, s), 3.30 (3H, s), 2.20 (3H, s). |
| | MS(ESI/APCI) m/z: 497 [M + H]+ |
| 96J | 1H-NMR (CDCl3) δ: 7.86-7.73 (4H, m), 7.38 (1H, s), 7.31 (1H, s), 6.28-6.22 (1H, m), 5.08 (2H, s), 4.70-4.63 (2H, m), 3.77-3.70 (2H, m), 3.32 (3H, s), 2.49-2.41 (2H, m), 2.20 (3H, s), 1.47 (9H, s). |
| | MS(ESI/APCI) m/z: 530 [M + H]+ |

**[Table 27]**

| | |
|---|---|
| 96K | MS(ESI/APCI) m/z: 534 [M + H]+ |
| 96L | 1H-NMR (CDCl3) δ: 7.83 (1H, s), 7.78-7.71 (3H, m), 7.40-7.37 (1H, m), 7.33-7.30 (1H, m), 5.08 (2H, s), 4.59-4.36 (2H, m), 4.31-4.15 (1H, m), 3.32 (3H, s), 3.25-3.16 (1H, m), 3.00-2.87 (1H, m), 2.35-2.26 (1H, m), 2.21 (3H, s), 1.94-1.79 (3H, m), 1.46 (9H, s). |
| | MS(ESI/APCI) m/z: 532 [M + H]+ |
| 96M | MS(ESI/APCI) m/z: 532 [M + H]+ |
| 96N | MS(ESI/APCI) m/z: 388 [M + H]+ |
| 96O | 1H-NMR (CDCl3) δ: 8.08 (1H, s), 7.87-7.72 (3H, m), 7.28-7.26 (1H, m), 7.17 (1H, s), 4.60-4.51 (1H, m), 3.19 (1H, d, J = 10.9 Hz), 2.96 (1H, d, J = 10.9 Hz), 2.57 (3H, s), 2.38-2.28 (4H, m), 2.19-2.09 (2H, m), 2.00-1.93 (1H, m), 1.91-1.83 (1H, m), 1.79-1.71 (1H, m). |
| | MS(ESI/APCI) m/z: 402 [M + H]+ |
| 97 | 1H-NMR (CDCl3) δ: 8.04 (1H, s), 7.78-7.70 (3H, m), 7.20 (1H, s), 7.12 (1H, s), 4.51-4.42 (1H, m), 3.21 (1H, d, J = 10.9 Hz), 3.06 (1H, d, J = 10.9 Hz), 2.54-2.47 (2H, m), 2.46 (3H, s), 2.35 (1H, t, J = 10.6 Hz), 2.17-2.08 (2H, m), 1.98-1.80 (2H, m), 1.79-1.71 (1H, m), 1.10 (3H, t, J = 7.3 Hz). |
| | MS(ESI/APCI) m/z: 416 [M + H]+ |
| 98A | 1H-NMR (CDCl3) δ: 7.41 (1H, s), 7.30 (1H, d, J = 7.9 Hz), 6.46-6.40 (2H, m), 5.49 (2H, s), 4.91 (2H, t, J = 8.5 Hz), 3.80 (3H, s), 3.79 (3H, s), 3.15 (2H, t, J = 8.5 Hz). |
| | MS(ESI/APCI) m/z: 346 [M + H]+ |
| 98B | 1H-NMR (CDCl3) δ: 7.52 (1H, s), 5.05 (2H, t, J = 8.5 Hz), 3.48 (2H, t, J = 8.5 Hz). |
| | MS(ESI/APCI) m/z: 196 [M + H]+ |
| 98C | 1H-NMR (DMSO-D6) δ: 9.05 (2H, s), 7.99 (1H, s), 5.07 (2H, t, J = 8.9 Hz), 3.74 (2H, t, J = 8.9 Hz). |
| | MS(ESI/APCI) m/z: 292 [M + H]+ |
| 98D | 1H-NMR (CDCl3) δ: 8.71 (2H, s), 7.51 (1H, s), 7.34 (1H, s), 7.27-7.27 (1H, m), 5.12 (2H, t, J = 8.6 Hz), 5.05 (2H, s), 3.82 (2H, t, J = 8.6 Hz), 3.32 (3H, s), 2.19 (3H, s). |
| | MS(ESI/APCI) m/z: 476 [M + H]+ |
| 98E | 1H-NMR (CD3OD) δ: 8.81 (2H, s), 7.72 (1H, s), 7.13 (1H, s), 7.06 (1H, s), 5.12 (2H, t, J = 8.8 Hz), 3.85 (2H, t, J = 8.8 Hz), 3.34 (3H, s). |
| | MS(ESI/APCI) m/z: 432 [M + H]+ |
| 98F | 1H-NMR (CDCl3) δ: 8.66 (2H, s), 7.80 (1H, s), 7.20 (1H, s), 7.09 (1H, s), 5.68 (1H, d, J = 7.3 Hz), 5.33 (1H, td, J = 7.4, 2.0 Hz), 4.11-4.04 (1H, m), 3.63-3.56 (1H, m), 2.61-2.52 (4H, m), 2.36-2.28 (1H, m). |
| | MS(ESI/APCI) m/z: 434 [M + H]+ |
| 98G | 1H-NMR (CDCl3) δ: 8.66 (2H, s), 7.80 (1H, s), 7.20 (1H, s), 7.09 (1H, s), 5.68 (1H, d, J = 7.4 Hz), 5.36-5.30 (1H, m), 4.11-4.04 (1H, m), 3.63-3.55 (1H, m), 2.58-2.51 (4H, m), 2.37-2.28 (1H, m). |
| | MS(ESI/APCI) m/z: 434 [M + H]+ |

**[Table 28]**

| | |
|---|---|
| 99A | 1H-NMR (DMSO-D6) δ: 7.24 (1H, s), 6.73-6.69 (1H, m), 6.62 (2H, s), 4.91-4.85 (2H, m), 4.81-4.75 (2H, m). |
| | MS(ESI/APCI) m/z: 198 [M + H]+ |
| 99B | 1H-NMR (CDCl3) δ: 7.85 (2H, d, J = 8.5 Hz), 7.57 (1H, s), 7.31 (2H, d, J = 7.9 Hz), 6.84 (1H, s), 4.91-4.84 (4H, m), 2.42 (3H, s). |
| | MS(ESI/APCI) m/z: 352 [M + H]+ |
| 99C | 1H-NMR (CDCl3) δ: 8.14 (2H, d, J = 8.5 Hz), 7.46 (1H, s), 7.34 (2H, d, J = 8.5 Hz), 5.43-5.35 (2H, m), 5.09-5.03 (2H, m), 2.41 (3H, s). |
| | MS(ESI/APCI) m/z: 350 [M + H]+ |
| 99D | MS(ESI/APCI) m/z: 196 [M + H]+ |
| 99E | 1H-NMR (CDCl3) δ: 7.53 (1H, s), 5.46-5.37 (1H, m), 5.22 (2H, t, J = 3.2 Hz), 5.12 (2H, t, J = 3.2 Hz), 3.93-3.84 (2H, m), 3.78-3.69 (2H, m). |
| | MS(ESI/APCI) m/z: 264 [M + H]+ |
| 99F | 1H-NMR (CDCl3) δ: 7.49 (1H, s), 5.25 (2H, t, J = 3.0 Hz), 5.09 (2H, t, J = 3.0 Hz), 4.91-4.81 (1H, m), 2.87-2.72 (4H, m), 1.53 (3H, s). |
| | MS(ESI/APCI) m/z: 280 [M + H]+ |
| 99G | 1H-NMR (CDCl3) δ: 7.45 (1H, s), 7.33 (1H, s), 7.26 (1H, s), 5.28 (2H, t, J = 3.2 Hz), 5.14 (2H, t, J = 3.2 Hz), 5.06 (2H, s), 5.03-4.94 (1H, m), 3.32 (3H, s), 2.84 (4H, d, J = 8.5 Hz), 2.14 (3H, s), 1.53 (3H, s). |
| | MS(ESI/APCI) m/z: 464 [M + H]+ |
| 99H | 1H-NMR (CDCl3) δ: 7.71 (1H, s), 7.21 (1H, s), 7.12 (1H, s), 5.31 (2H, t, J = 3.1 Hz), 5.18 (2H, t, J = 3.1 Hz), 5.07-4.96 (1H, m), 2.93-2.83 (2H, m), 2.78-2.69 (2H, m), 2.51 (3H, s), 1.57 (3H, s). |
| | MS(ESI/APCI) m/z: 420 [M + H]+ |

The structural formulas of the compounds described in the examples are shown below.

In the tables, Ex indicates the example number, and Structure indicates the structural formula.

The following symbols may be used in the structural formula.
MOM: a methoxymethyl group
Boc: a tert-butoxycarbonyl group
PMB: a p-methoxybenzyl group
TBS: a tert-butyldimethylsilyl group
Bn: a benzyl group
Ac: an acetyl group
Cbz: a benzyloxycarbonyl group
Tf: a trifluoromethanesulfonyl group
Ts: a p-toluenesulfonyl group
EOM: an ethoxymethoxy group
TIPS: a triisopropylsilyl group

**[Table 29]**

| Ex | Structure | Ex | Structure |
|---|---|---|---|
| 1A | 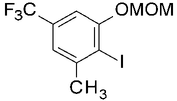 | 1B | 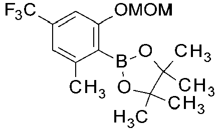 |
| 1C | 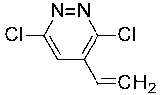 | 1D | 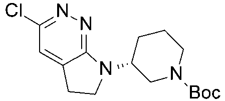 |
| 1E | 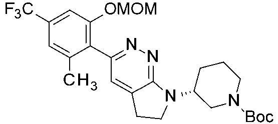 | 1F | 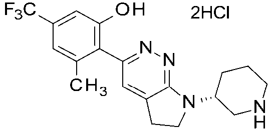 |
| 1G | 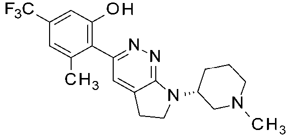 | 2A | 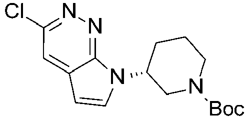 |
| 2B | 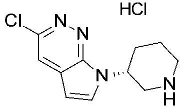 | 2C | 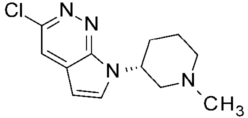 |
| 2D | 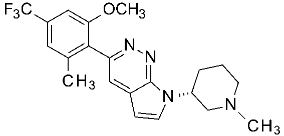 | 2E | 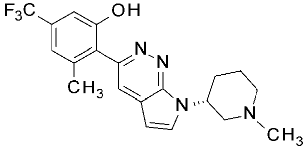 |

**[Table 30]**

| | | | |
|---|---|---|---|
| 3A | 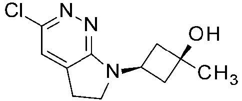 | 3B | 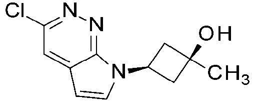 |
| 3C | 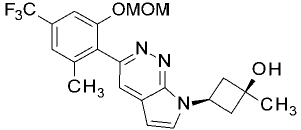 | 3D | 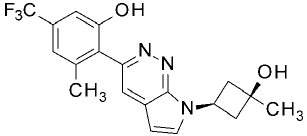 |
| 4A | 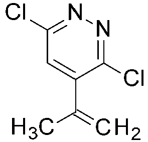 | 4B | 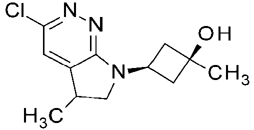 |
| 4C | 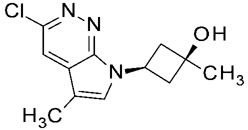 | 4D | 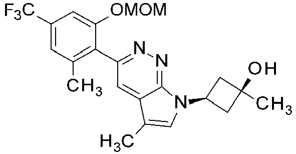 |
| 4E | 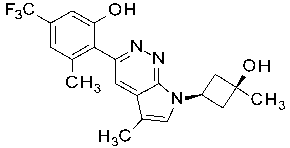 | 5A | 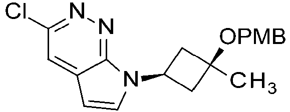 |
| 5B | 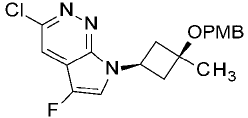 | 5C | 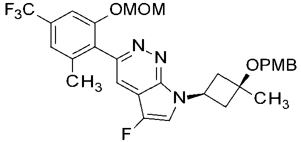 |
| 5D | 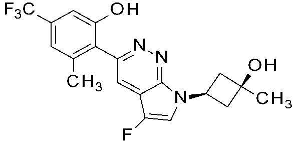 | 6A | 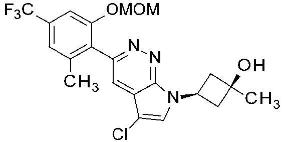 |

**[Table 31]**

| | | | |
|---|---|---|---|
| 6B | 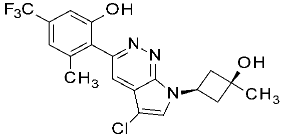 | 7A | 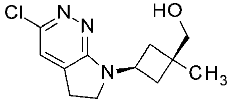 |
| 7B | 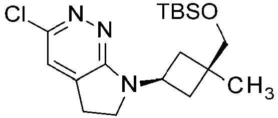 | 7C | 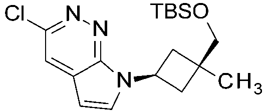 |
| 7D | 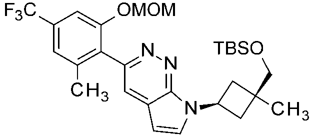 | 7E | 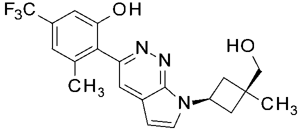 |
| 8A | 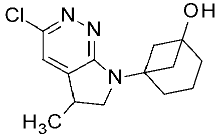 | 8B | 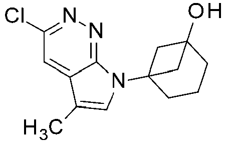 |
| 8C | 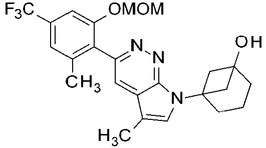 | 8D | 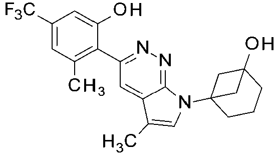 |

**[Table 32]**

| | | | |
|---|---|---|---|
| 9A | 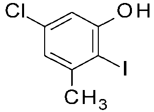 | 9B | 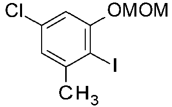 |
| 9C | 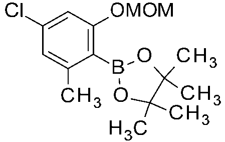 | 9D | 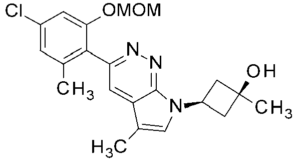 |
| 9E | 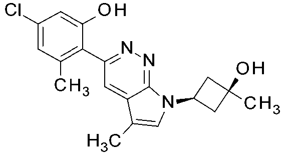 | 10A | 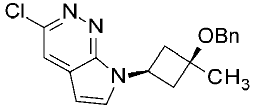 |
| 10B | 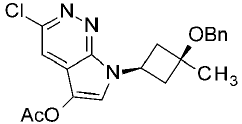 | 10C | 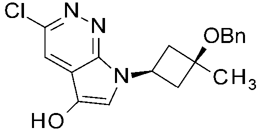 |
| 10D | 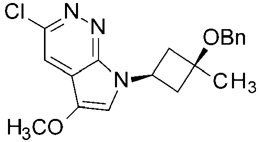 | 10E | 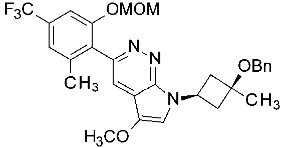 |
| 10F | 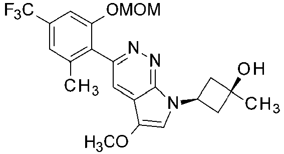 | 10G | 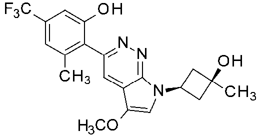 |
| 11 | 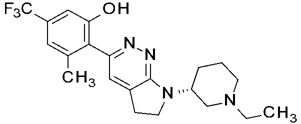 | 12A | 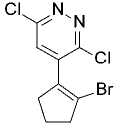 |

**[Table 33]**

| | | | |
|---|---|---|---|
| 12B | 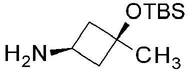 | 12C | 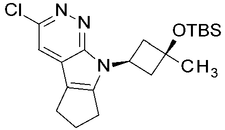 |
| 12D | 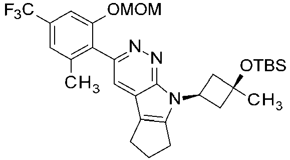 | 12E | 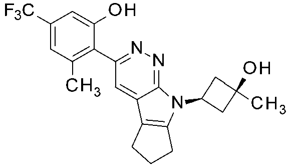 |
| 13A | 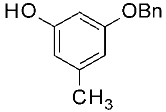 | 13B | 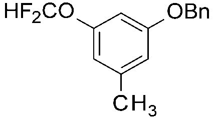 |
| 13C | 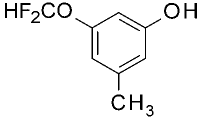 | 13D | 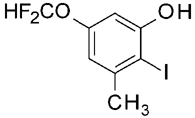 |
| 13E | 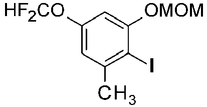 | 13F | 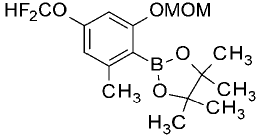 |
| 13G | 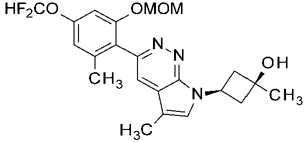 | 13H | 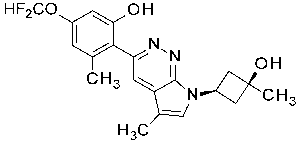 |
| 14A | 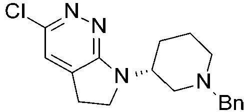 | 14B | 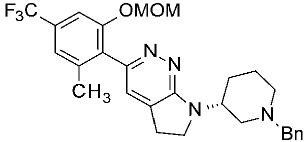 |

**[Table 34]**

| | | | |
|---|---|---|---|
| 14C | 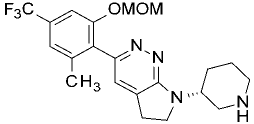 | 14D | 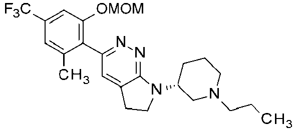 |
| 14E | 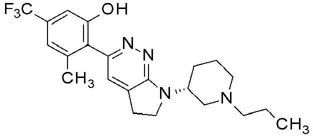 | 15A | 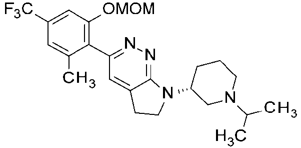 |
| 15B | 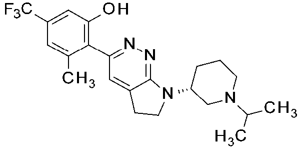 | 16A | 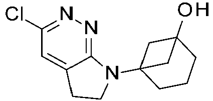 |
| 16B | 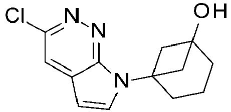 | 16C | 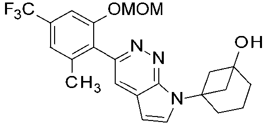 |
| 16D | 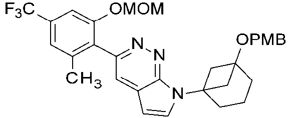 | 16E | 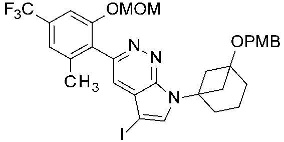 |
| 16F | 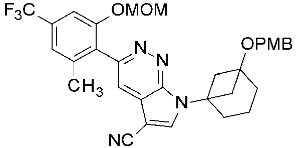 | 16G | 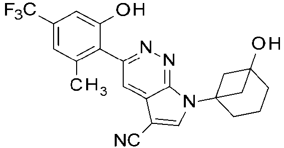 |
| 17A | 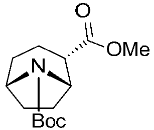 | 17B | 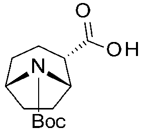 |

**[Table 35]**

| | | | |
|---|---|---|---|
| 17C | 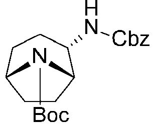 | 17D | 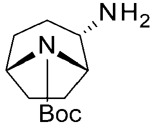 |
| 17E | 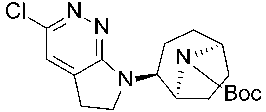 | 17F | 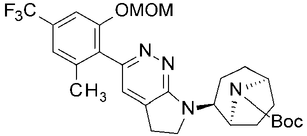 |
| 17G | 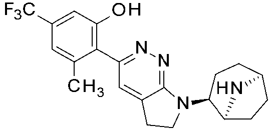 | 17H | 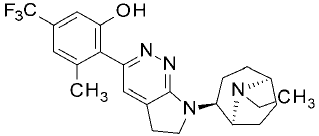 |
| 18A | 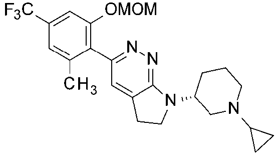 | 18B | 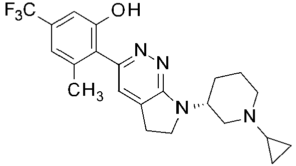 |
| 19A | 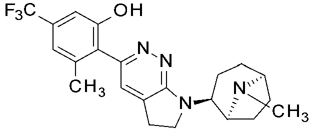 | 19B | 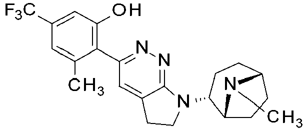 |
| 20A | 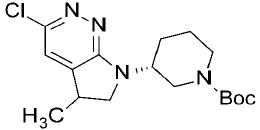 | 20B | 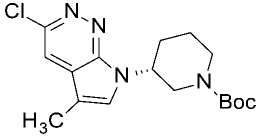 |

**[Table 36]**

| | | | |
|---|---|---|---|
| 20C | 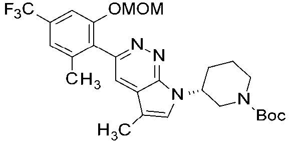 | 20D | 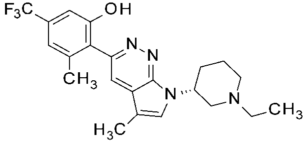 |
| 21A | 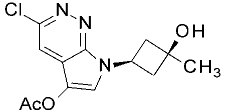 | 21B | 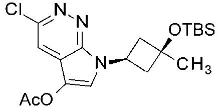 |
| 21C | 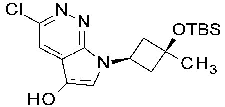 | 21D | 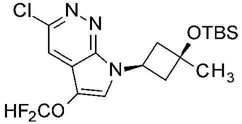 |
| 21E | 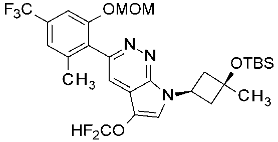 | 21F | 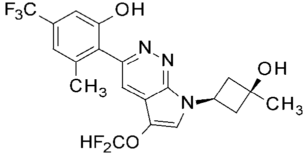 |
| 22A | 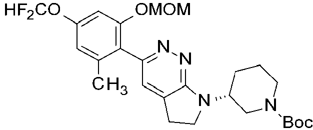 | 22B | 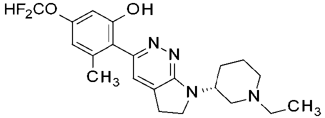 |
| 23A | 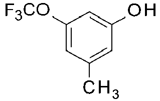 | 23B | 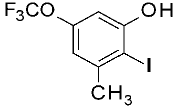 |
| 23C | 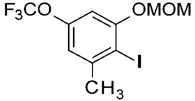 | 23D | 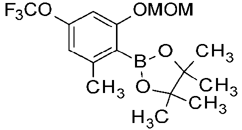 |

**[Table 37]**

| | | | |
|---|---|---|---|
| 23E | 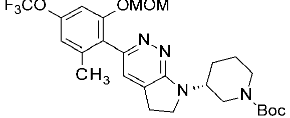 | 23F | 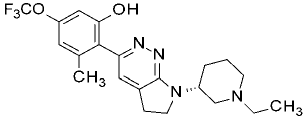 |
| 24A | 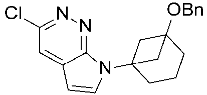 | 24B | 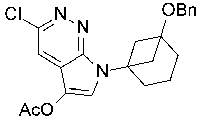 |
| 24C | 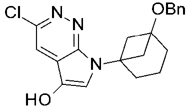 | 24D | 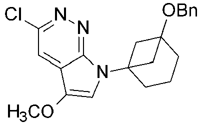 |
| 24E | 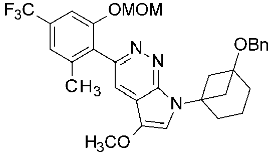 | 24F | 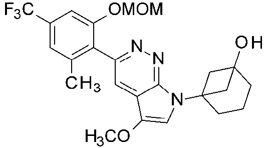 |
| 24G | 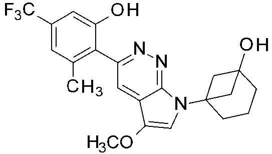 | 25A | 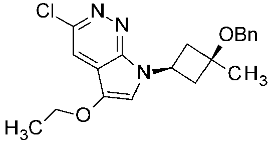 |
| 25B | 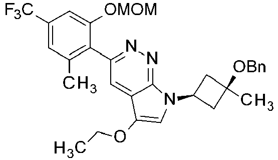 | 25C | 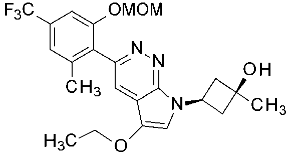 |
| 25D | 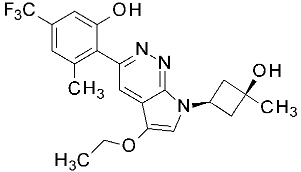 | 26A | 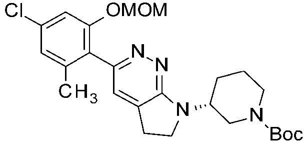 |

**[Table 38]**

| | | | |
|---|---|---|---|
| 26B | 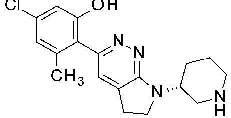 | 26C | 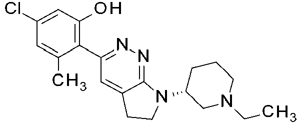 |
| 27A | 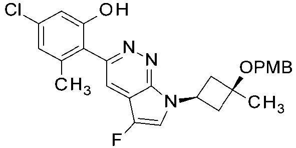 | 27B | 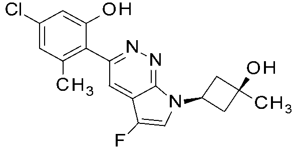 |
| 28A | 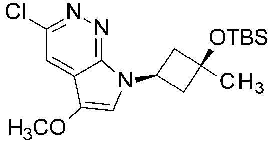 | 28B | 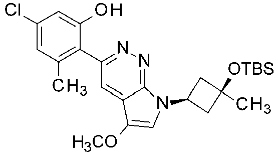 |
| 28C | 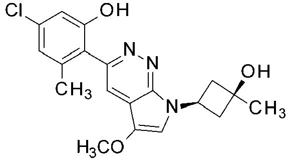 | 29A | 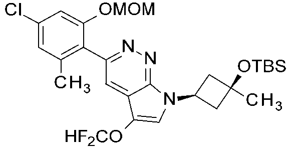 |
| 29B | 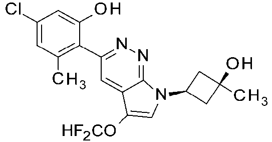 | 30A | 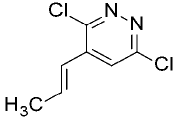 |
| 30B | 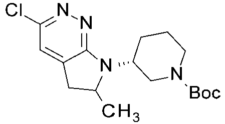 | 30C | 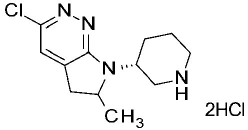 |

**[Table 39]**

| | | | |
|---|---|---|---|
| 30D | 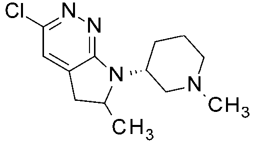 | 30 E | 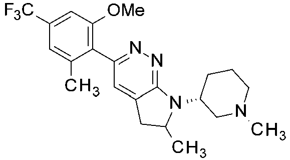 |
| 30F | 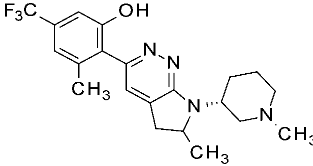 | 31 A | 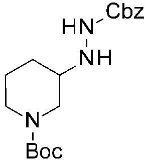 |
| 31B | 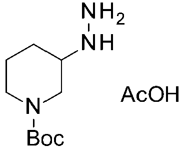 | 31 C | 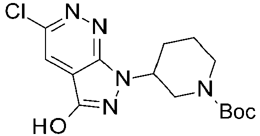 |
| 31D | 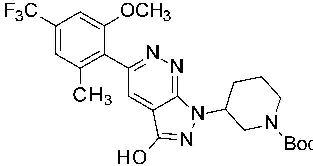 | 31 E | 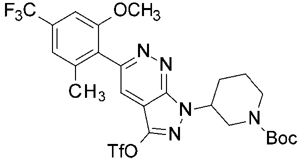 |
| 31F | 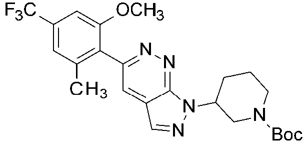 | 31 G | 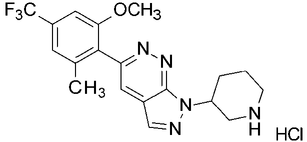 |
| 31H | 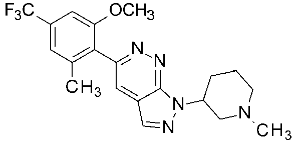 | 31I | 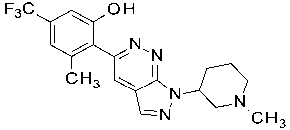 |

**[Table 40]**

| | | | |
|---|---|---|---|
| 32A | 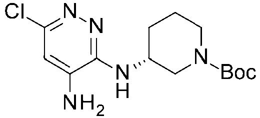 | 32B | 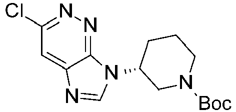 |
| 32C | 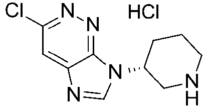 | 32D | 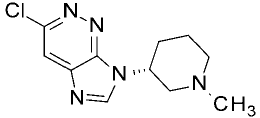 |
| 32E | 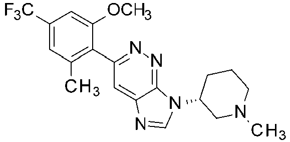 | 32F | 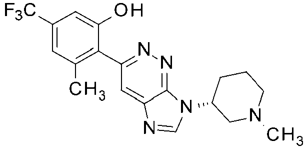 |
| 33A | 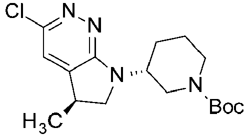 | 33B | 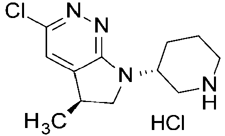 |
| 33C | 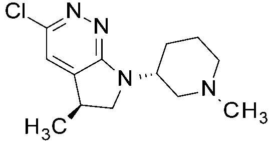 | 33D | 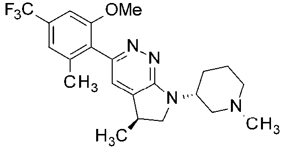 |
| 33E | 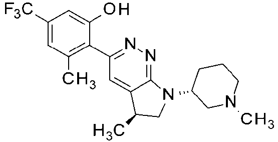 | 34A | 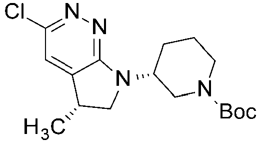 |

**[Table 41]**

| | | | |
|---|---|---|---|
| 34B | 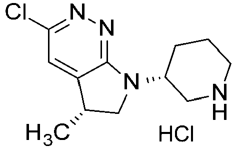 | 34C | 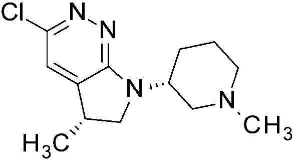 |
| 34D | 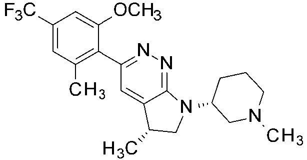 | 34E | 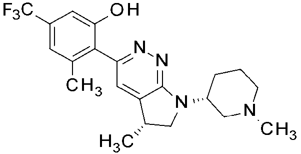 |
| 35A | 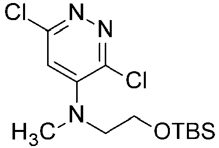 | 35B | 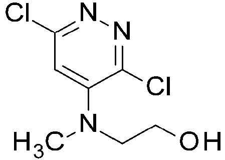 |
| 35C | 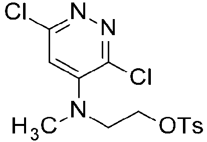 | 35D | 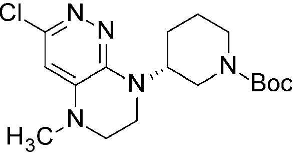 |
| 35E | 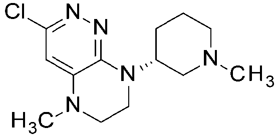 | 35F | 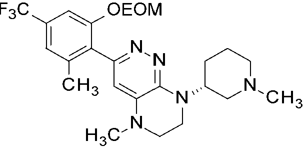 |
| 35G | 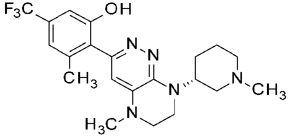 | 36A | 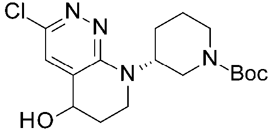 |

**[Table 42]**

| | | | |
|---|---|---|---|
| 36B | 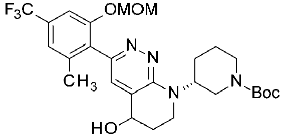 | 36C | 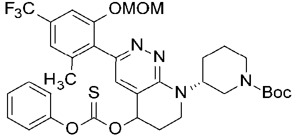 |
| 36D | 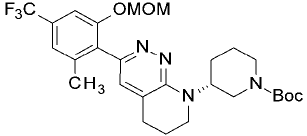 | 36E | 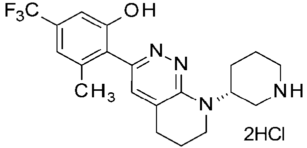 |
| 36F | 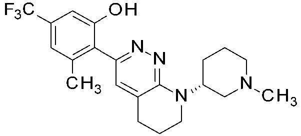 | 37A | 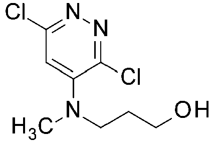 |
| 37B | 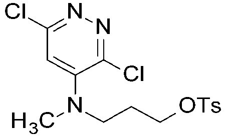 | 37C | 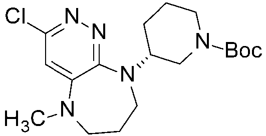 |
| 37D | 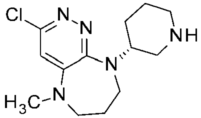 | 37E | 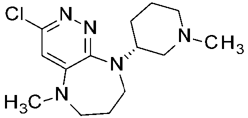 |
| 37F | 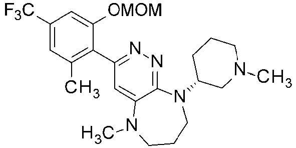 | 37G | 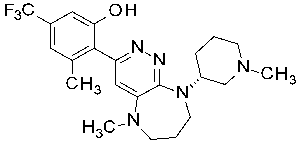 |
| 38A | 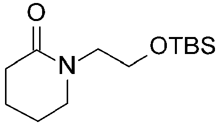 | 38B | 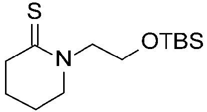 |

**[Table 43]**

| | | | |
|---|---|---|---|
| 38C | 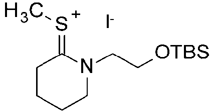 | 38D | 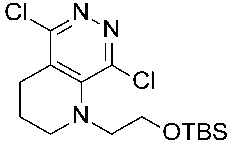 |
| 38E | 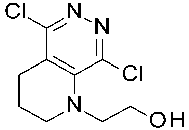 | 38F | 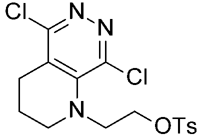 |
| 38G | 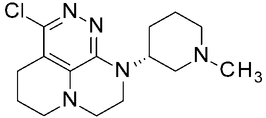 | 38H | 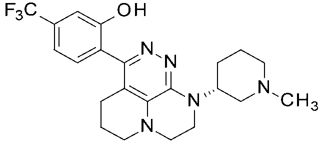 |
| 39A | 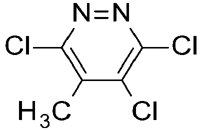 | 39B | 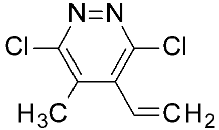 |
| 39C | 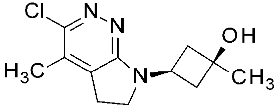 | 39D | 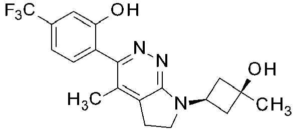 |
| 40A | 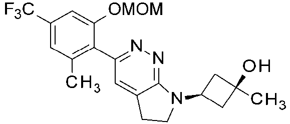 | 40B | 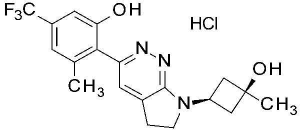 |

**[Table 44]**

| | | | |
|---|---|---|---|
| 41A | 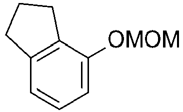 | 41B | 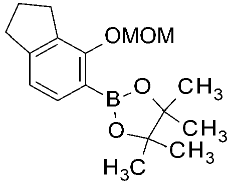 |
| 41C | 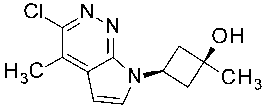 | 41D | 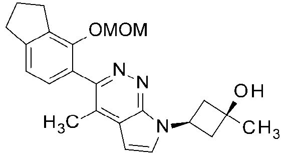 |
| 41E | 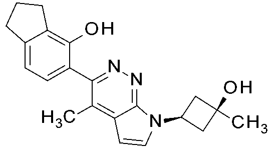 | 42A | 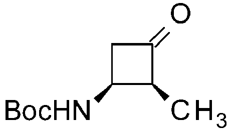 |
| 42B | 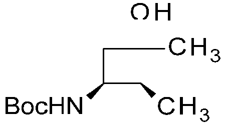 | 42C | 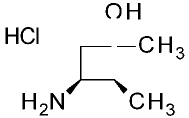 |
| 42D | 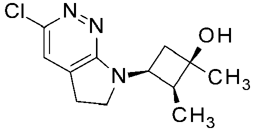 | 42E | 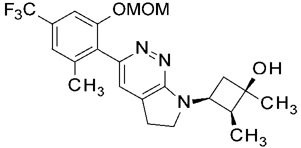 |
| 42F | 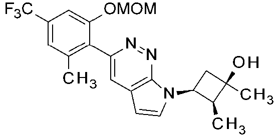 | 42G | 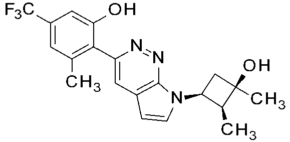 |

**[Table 45]**

| | | | |
|---|---|---|---|
| 43A | 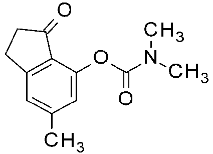 | 43B | 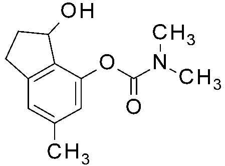 |
| 43C | 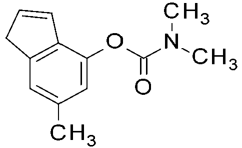 | 43D | 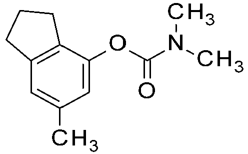 |
| 43E | 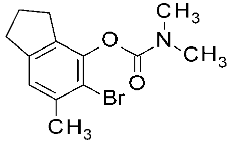 | 43F | 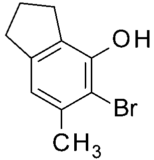 |
| 43G | 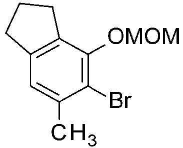 | 43H | 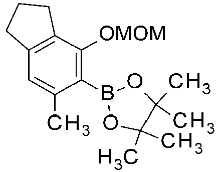 |
| 43I | 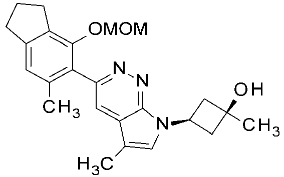 | 43J | 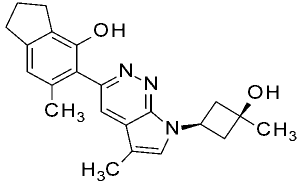 |
| 44A | 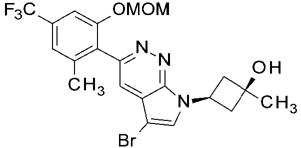 | 44B | 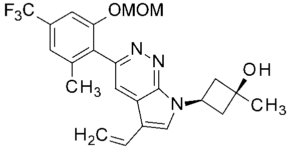 |

**[Table 46]**

| | | | |
|---|---|---|---|
| 44C | 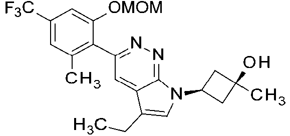 | 44D | 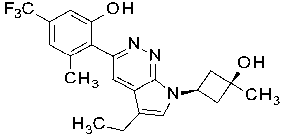 |
| 45A | 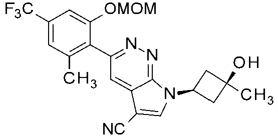 | 45B | 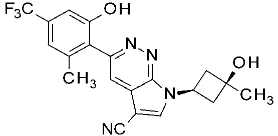 |
| 46A | 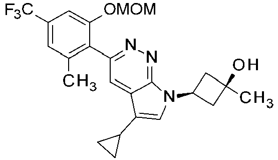 | 46B | 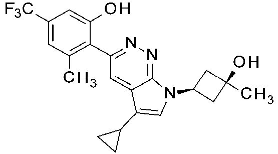 |
| 47A | 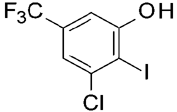 | 47B | 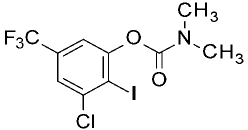 |
| 47C | 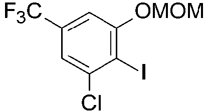 | 47D | 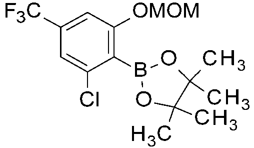 |
| 47E | 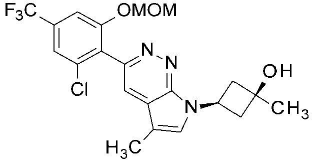 | 47F | 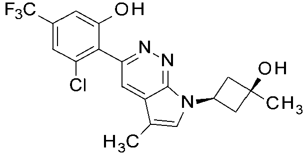 |

**[Table 47]**

| | | | |
|---|---|---|---|
| 48A | 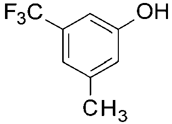 | 48B | 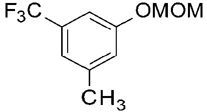 |
| 48C | 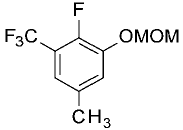 | 48D | 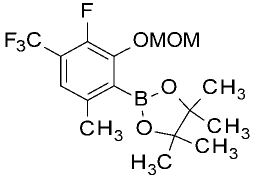 |
| 48E | 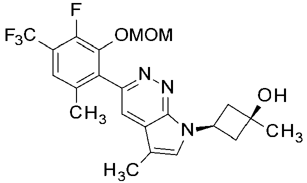 | 48F | 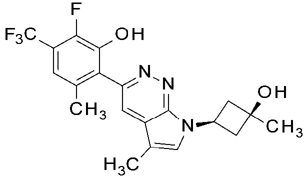 |
| 49A | 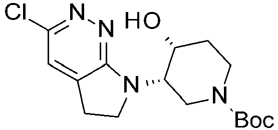 | 49B | 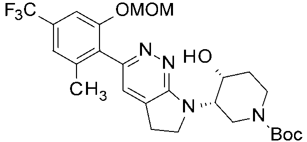 |
| 49C | 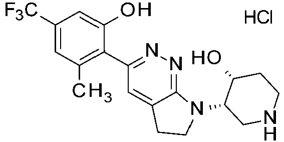 | 49D | 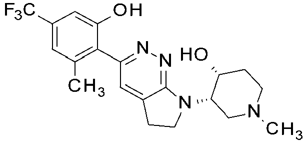 |

**[Table 48]**

| | | | |
|---|---|---|---|
| 50A | 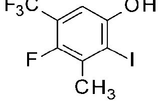 | 50B | 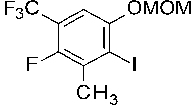 |
| 50C | 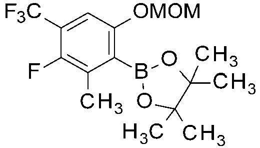 | 50D | 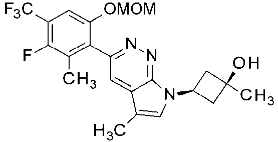 |
| 50E | 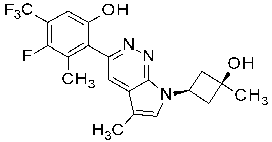 | 51A | 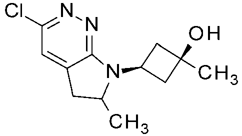 |
| 51B | 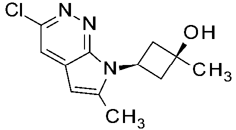 | 51C | 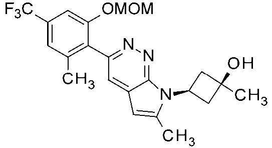 |
| 51D | 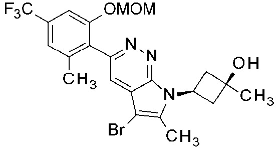 | 51E | 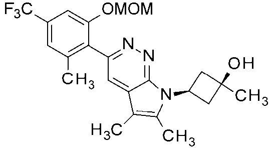 |
| 51F | 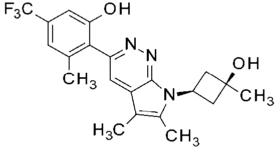 | 52A | 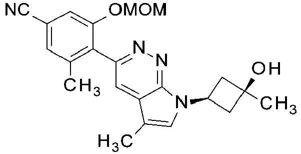 |

**[Table 49]**

| | | | |
|---|---|---|---|
| 52B | 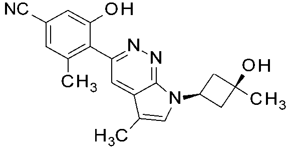 | 53A | 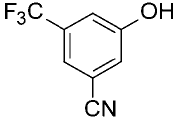 |
| 53B | 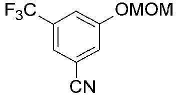 | 53C | 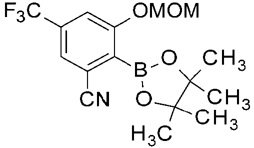 |
| 53D | 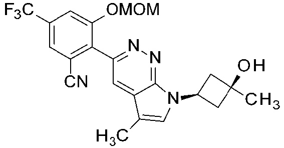 | 53E | 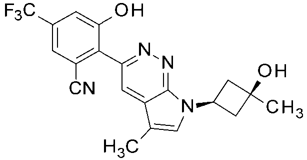 |
| 54A | 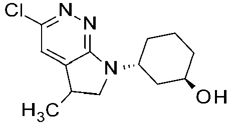 | 54B | 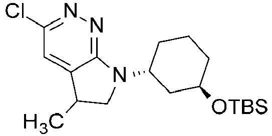 |
| 54C | 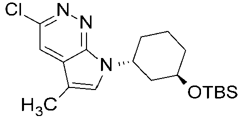 | 54D | 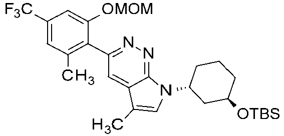 |
| 54E | 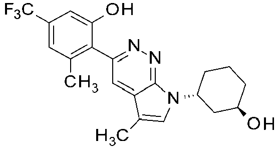 | 55A | 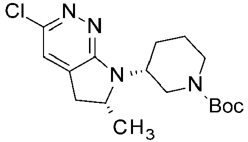 |

**[Table 50]**

| | | | |
|---|---|---|---|
| 55B | 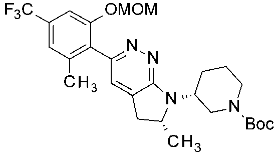 | 55C | 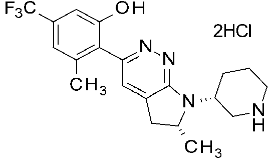 |
| 55D | 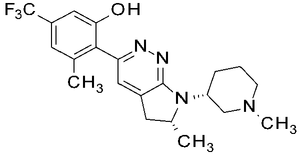 | 56A | 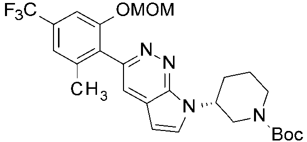 |
| 56B | 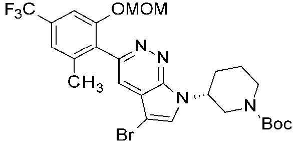 | 56C | 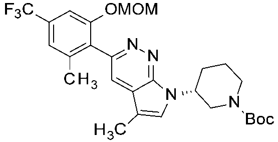 |
| 56D | 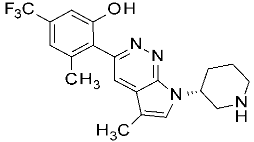 | 56E | 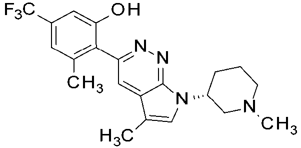 |
| 57A | 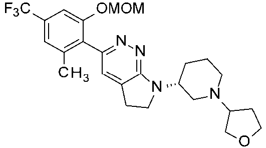 | 57B | 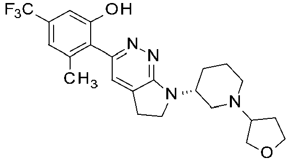 |
| 58A | 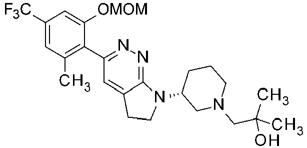 | 58B | 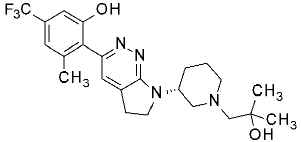 |

**[Table 51]**

| | | | |
|---|---|---|---|
| 59A | 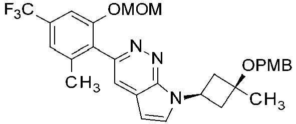 | 59B | 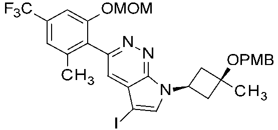 |
| 59C | 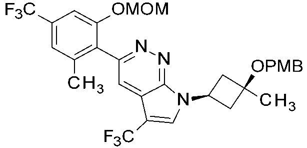 | 59D | 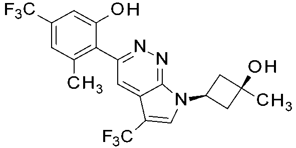 |
| 60A | 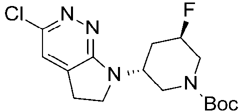 | 60B | 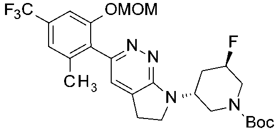 |
| 60C | 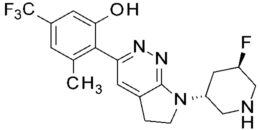 | 60D | 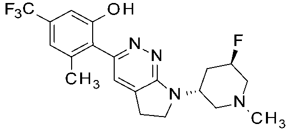 |
| 61A | 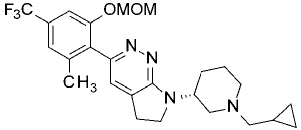 | 61B | 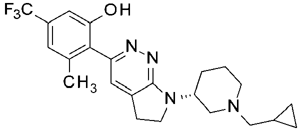 |
| 62A | 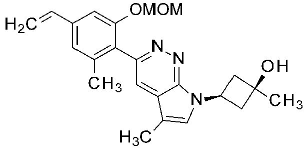 | 62B | 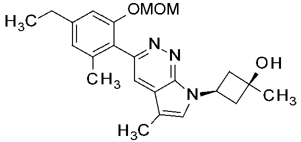 |
| 62C | 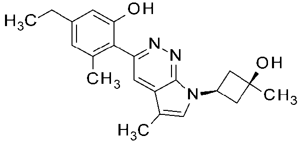 | 63A | 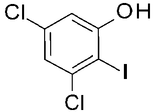 |

**[Table 52]**

| | | | |
|---|---|---|---|
| 63B | 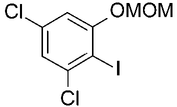 | 63C | 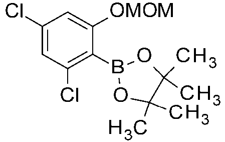 |
| 63D | 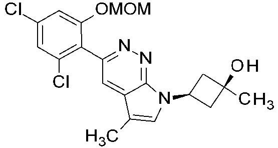 | 63E | 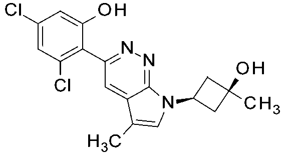 |
| 64A | 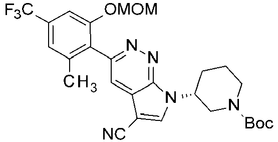 | 64B | 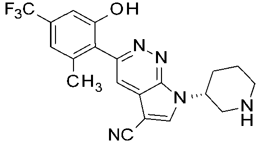 |
| 64C | 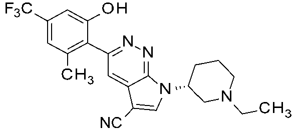 | 65A | 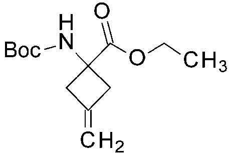 |
| 65B | 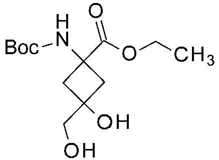 | 65C | 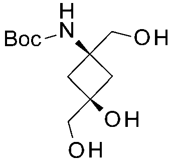 |
| 65D | 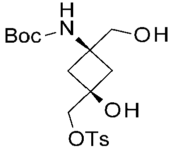 | 65E | 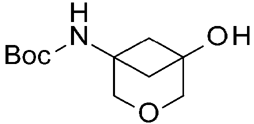 |

**[Table 53]**

| | | | |
|---|---|---|---|
| 65F | 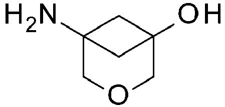 | 65G | 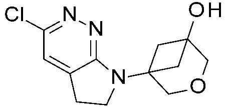 |
| 65H | 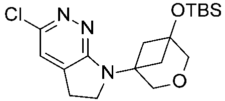 | 65I | 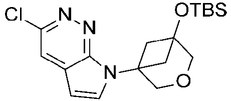 |
| 65J | 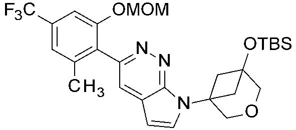 | 65K | 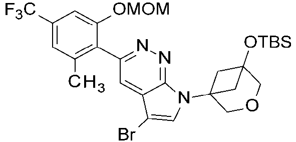 |
| 65L | 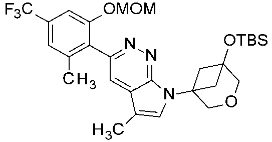 | 65M | 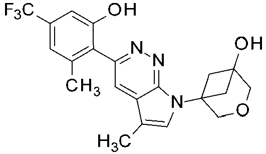 |
| 66 | 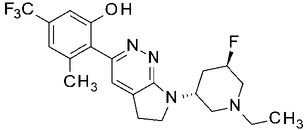 | 67A | 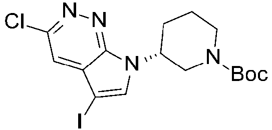 |
| 67B | 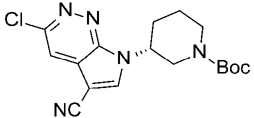 | 67C | 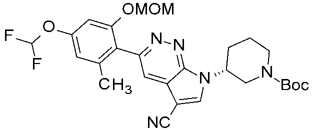 |
| 67D | 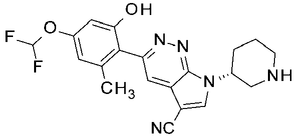 | 67E | 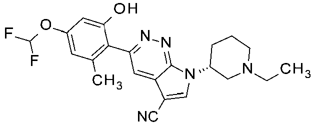 |

**[Table 54]**

| | | | |
|---|---|---|---|
| 68 | 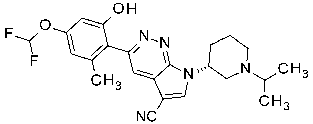 | 69A | 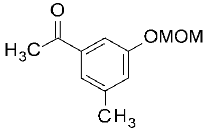 |
| 69B | 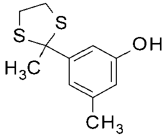 | 69C | 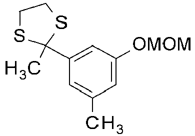 |
| 69D | 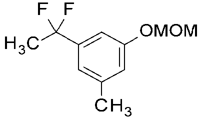 | 69E | 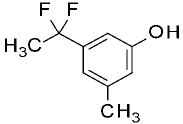 |
| 69F | 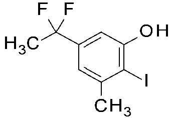 | 69G | 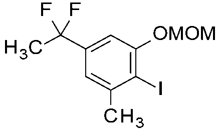 |
| 69H | 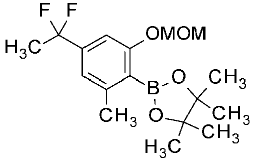 | 69I | 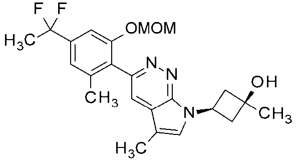 |
| 69J | 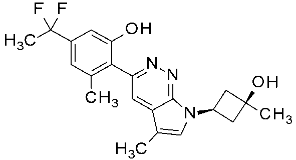 | 70A | 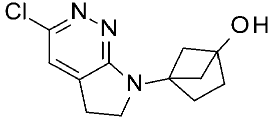 |
| 70B | 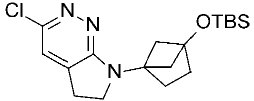 | 70C | 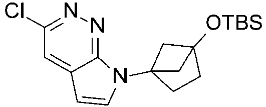 |

**[Table 55]**

| | | | |
|---|---|---|---|
| 70D | 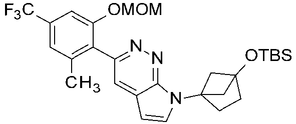 | 70E | 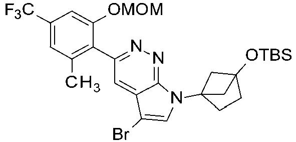 |
| 70F | 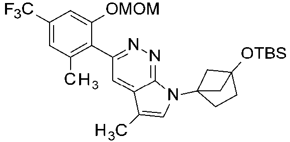 | 70G | 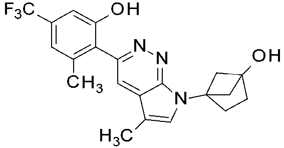 |
| 71A | 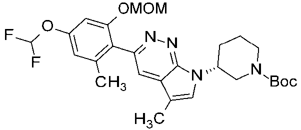 | 71B | 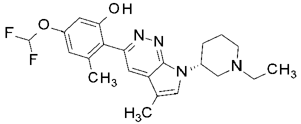 |
| 72A | 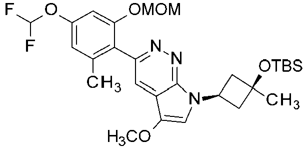 | 72B | 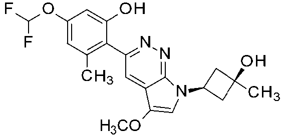 |
| 73A | 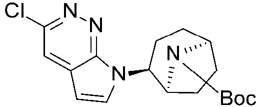 | 73B | 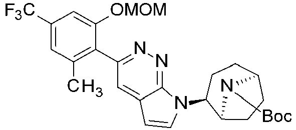 |
| 73C | 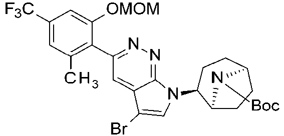 | 73D | 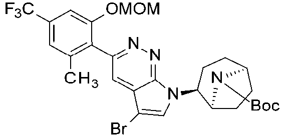 |

**[Table 56]**

| | | | |
|---|---|---|---|
| 73E | 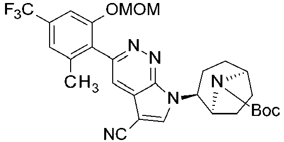 | 73F | 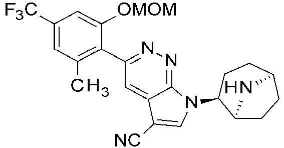 |
| 73G | 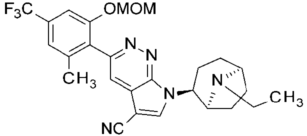 | 74A | 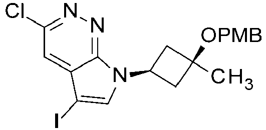 |
| 74B | 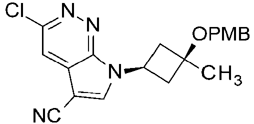 | 74C | 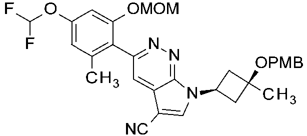 |
| 74D | 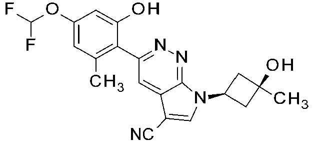 | 75A | 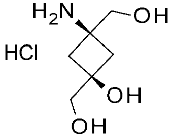 |
| 75B | 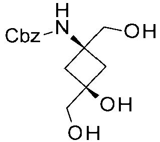 | 75C | 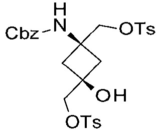 |
| 75D | 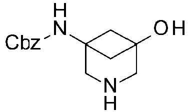 | 75E | 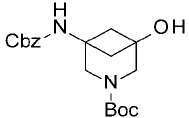 |
| 75F | 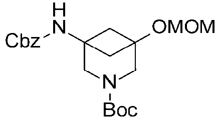 | 75G | 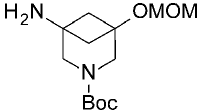 |

**[Table 57]**

| | | | |
|---|---|---|---|
| 75H | 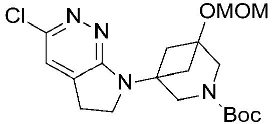 | 75I | 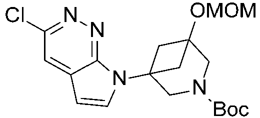 |
| 75J | 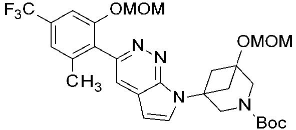 | 75K | 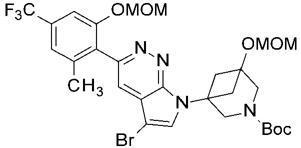 |
| 75L | 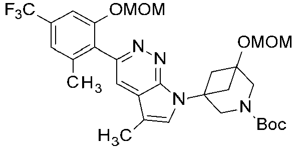 | 75M | 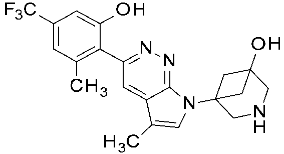 |
| 75N | 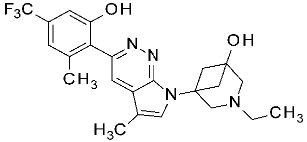 | 76A | 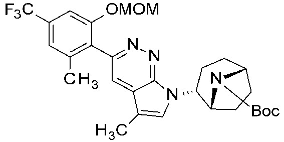 |
| 76B | 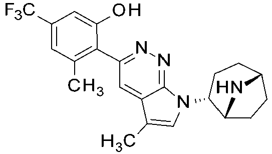 | 76C | 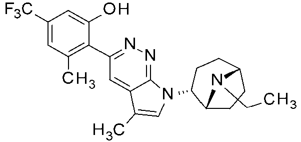 |
| 77A | 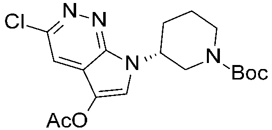 | 77B | 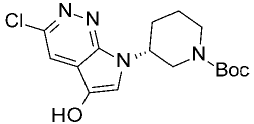 |

**[Table 58]**

| | | | |
|---|---|---|---|
| 77C | 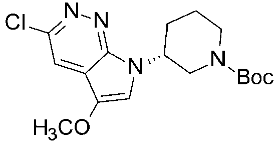 | 77D | 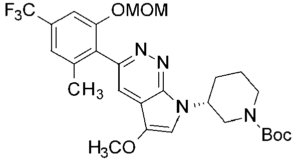 |
| 77E | 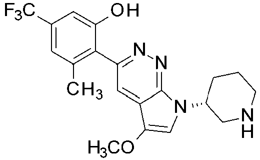 | 77F | 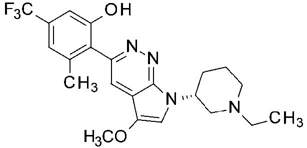 |
| 78A | 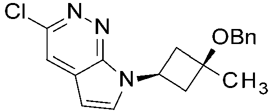 | 78B | 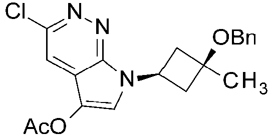 |
| 78C | 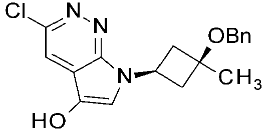 | 78D | 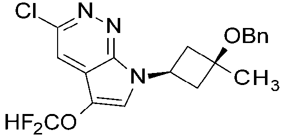 |
| 78E | 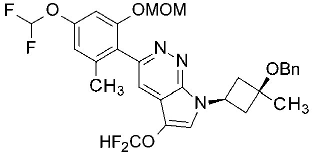 | 78F | 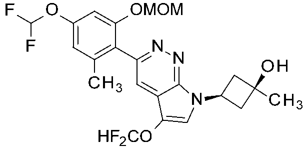 |
| 78G | 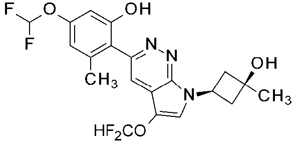 | 79A | 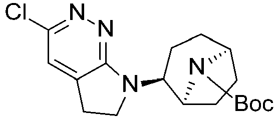 |

**[Table 59]**

| | | | |
|---|---|---|---|
| 79B | 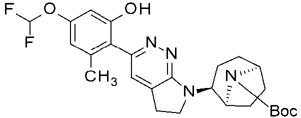 | 79C | 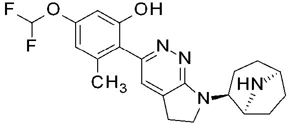 |
| 79D | 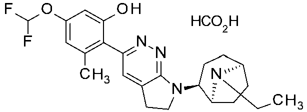 | 80A | 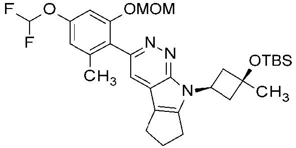 |
| 80B | 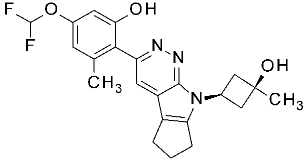 | 81A | 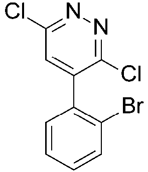 |
| 81B | 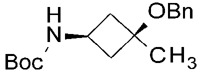 | 81C | 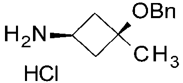 |
| 81D | 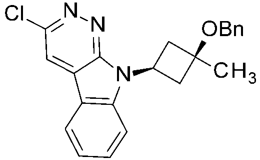 | 81E | 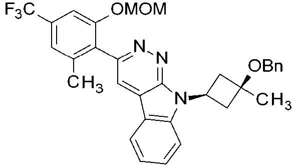 |
| 81F | 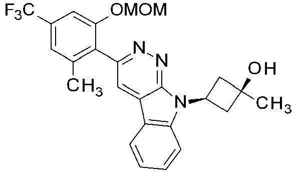 | 81G | 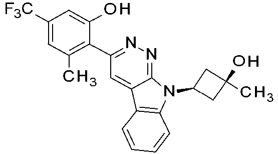 |

**[Table 60]**

| | | | |
|---|---|---|---|
| 82A | 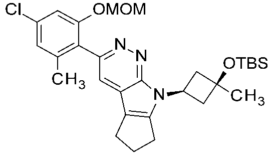 | 82B | 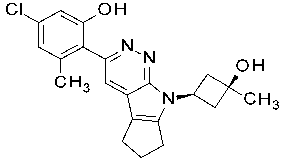 |
| 83A | 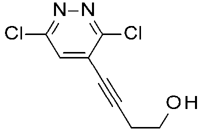 | 83B | 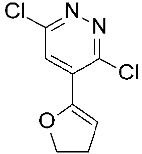 |
| 83C | 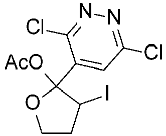 | 83D | 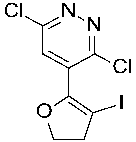 |
| 83E | 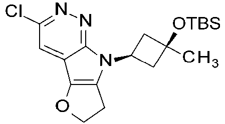 | 83F | 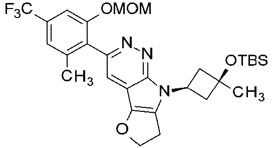 |
| 83G | 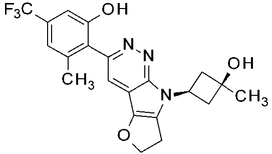 | 84A | 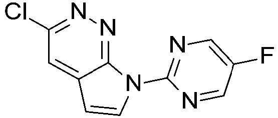 |
| 84B | 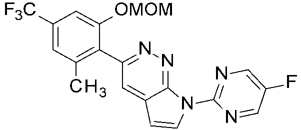 | 84C | 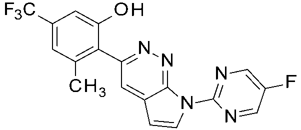 |

**[Table 61]**

| | | | |
|---|---|---|---|
| 85A | 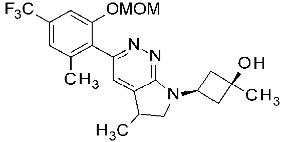 | 85B | 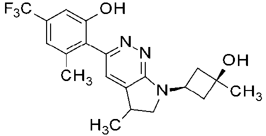 |
| 86A | 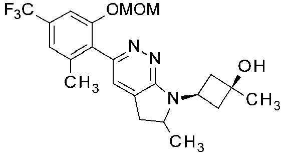 | 86B | 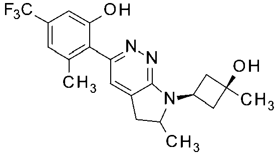 |
| 87A | 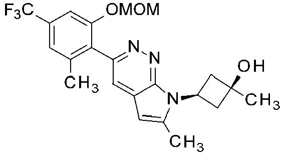 | 87B | 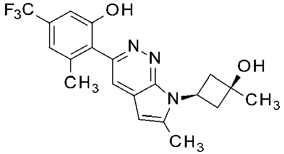 |
| 88 | 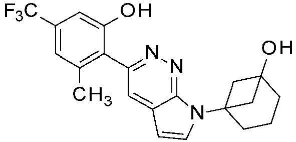 | 89A | 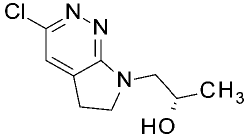 |
| 89B | 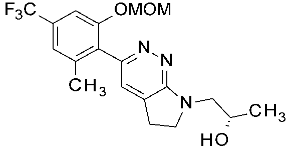 | 89C | 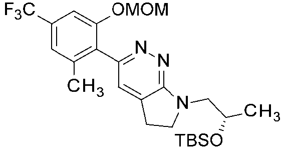 |
| 89D | 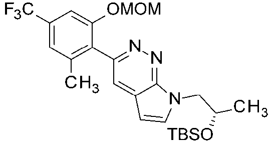 | 89E | 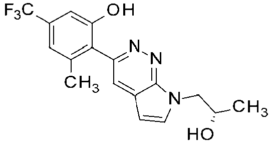 |

**[Table 62]**

| | | | |
|---|---|---|---|
| 90A | 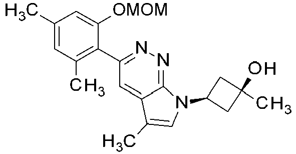 | 90B | 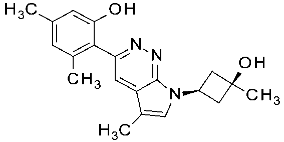 |
| 91A | 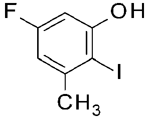 | 91B | 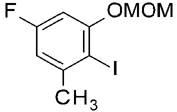 |
| 91C | 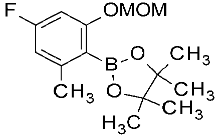 | 91D | 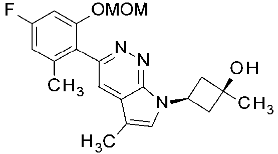 |
| 91E | 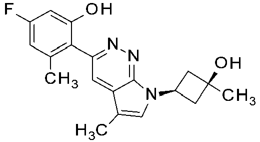 | 92A | 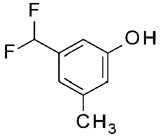 |
| 92B | 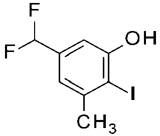 | 92C | 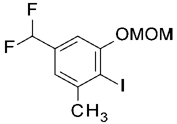 |
| 92D | 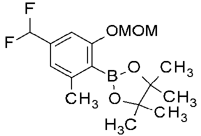 | 92E | 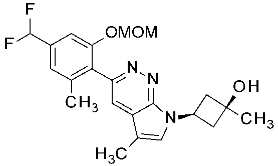 |

**[Table 63]**

| | | | |
|---|---|---|---|
| 92F | 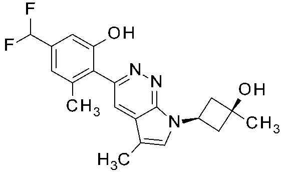 | 93A | 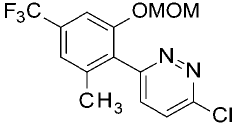 |
| 93B | 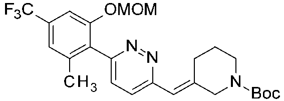 | 93C | 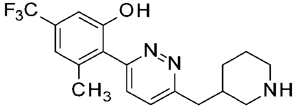 |
| 93D | 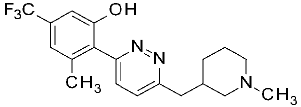 | 94A | 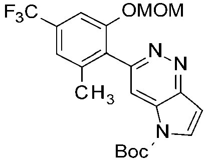 |
| 94B | 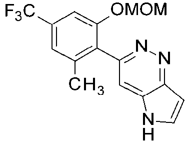 | 94C | 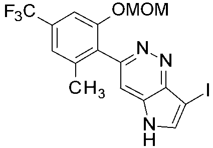 |
| 94D | 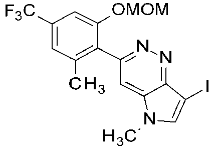 | 94E | 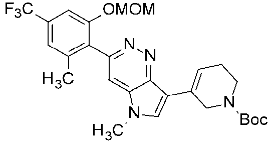 |
| 94F | 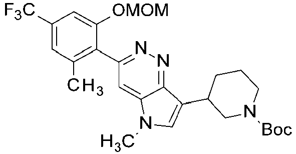 | 94G | 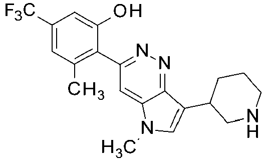 |

**[Table 64]**

| | | | |
|---|---|---|---|
| 94H | 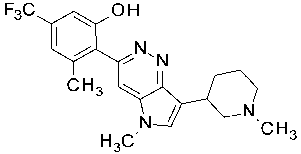 | 95A | 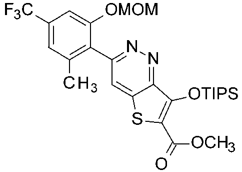 |
| 95B | 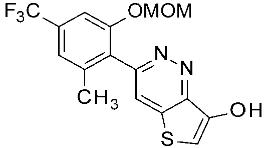 | 95C | 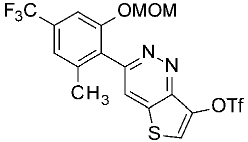 |
| 95D | 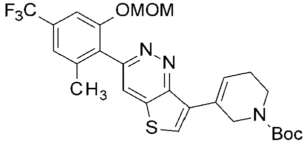 | 95E | 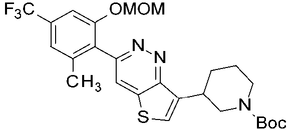 |
| 95F | 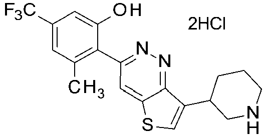 | 95G | 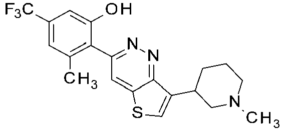 |
| 96A | 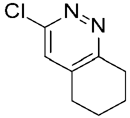 | 96B | 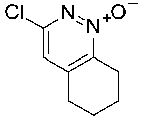 |
| 96C | 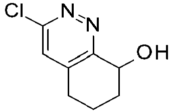 | 96D | 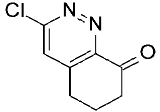 |
| 96E | 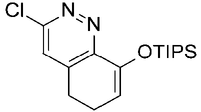 | 96F | 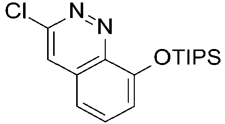 |

**[Table 65]**

| | | | |
|---|---|---|---|
| 96G | 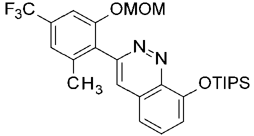 | 96H | 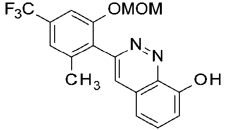 |
| 96I | 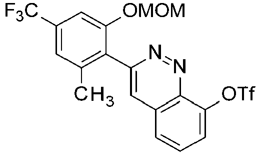 | 96J | 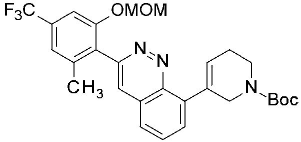 |
| 96K | 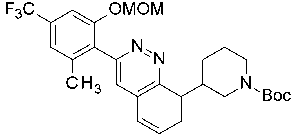 | 96L | 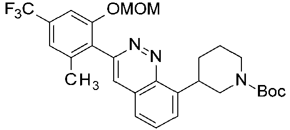 |
| 96M | 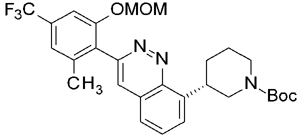 | 96N | 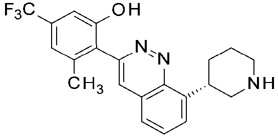 |
| 96O | 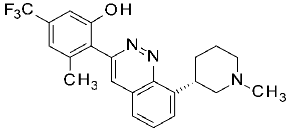 | 97 | 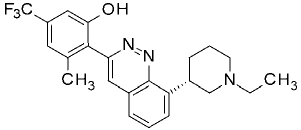 |
| 98A | 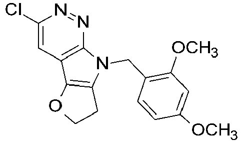 | 98B | 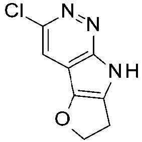 |

**[Table 66]**

| | | | |
|---|---|---|---|
| 98C | 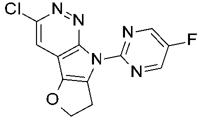 | 98D | 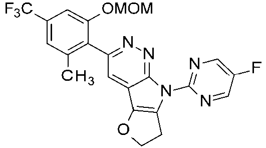 |
| 98E | 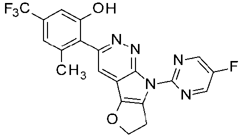 | 98F | 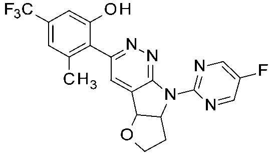 |
| 98G | 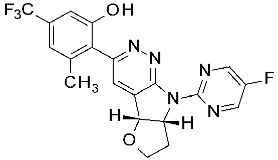 | 99A | 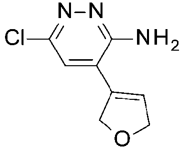 |
| 99B | 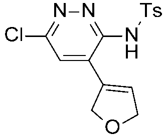 | 99C | 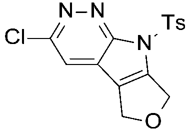 |
| 99D | 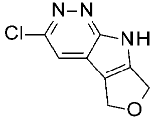 | 99E | 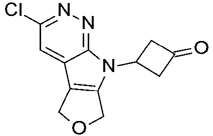 |
| 99F | 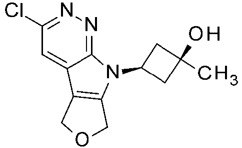 | 99G | 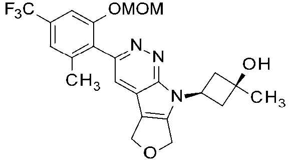 |
| 99H | 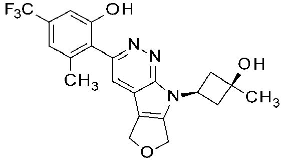 | | |

## Claims

1. A compound of the general formula (1) or a pharmaceutically acceptable salt thereof: 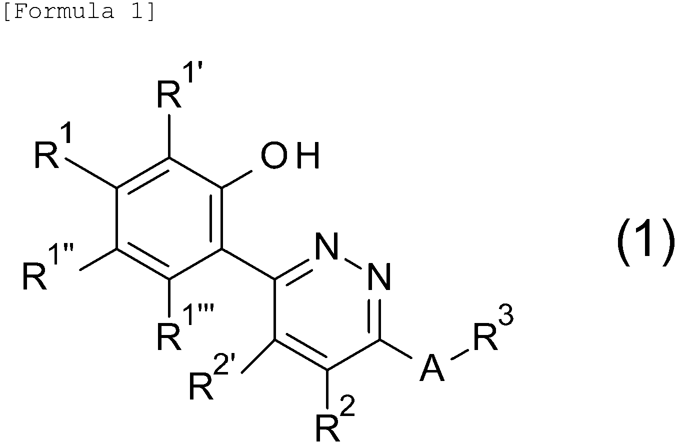 wherein
A is a single bond, a nitrogen atom, or a carbon atom,
R¹ is a halogen atom, a cyano group, a C1-C3 alkyl group optionally substituted with 1 to 3 halogen atoms, or a C1-C3 alkoxy group optionally substituted with 1 to 3 fluorine atoms, R¹' is a hydrogen atom, a halogen atom, or a C1-C3 alkyl group, or
R¹ and R¹' are bonded to each other to form a C1-C4 alkylene group optionally substituted with 1 to 3 substituents selected from substituent group a, or -NH-(CH₂)n- (n is an integer of 1, 2 or 3) which is optionally substituted with 1 to 3 substituents selected from substituent group a, thereby constituting a ring structure,
R¹" is a hydrogen atom, or a halogen atom,
R¹‴ is a hydrogen atom, a halogen atom, a cyano group, or - NH-(CH₂)n- (n is an integer of 1, 2 or 3),
when A is a nitrogen atom,
R² and A are bonded to each other to form any partial structure selected from the group shown below: 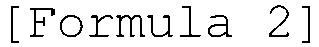 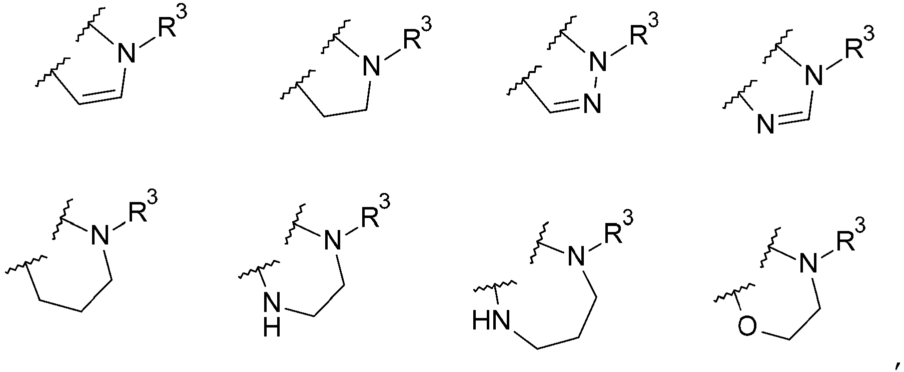
wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group a,
when A is a single bond, or a carbon atom,
R² is a hydrogen atom, or a C1-C3 alkyl group optionally substituted with 1 to 3 substituents selected from substituent group a, or
R² and A are bonded to each other to form any partial structure selected from the group shown below: 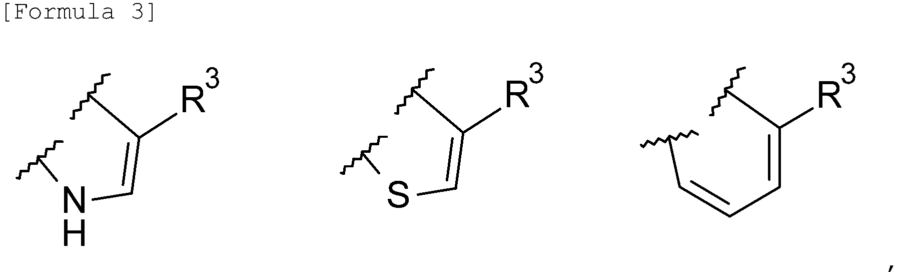 wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group a,
R²' is a hydrogen atom, or a C1-C3 alkyl group, or
R²' and R² are bonded to each other to form -NH-(CH₂)n- (n is an integer of 1, 2 or 3), thereby constituting a ring structure,
R³ is
a C1-C3 alkyl group optionally substituted with 1 to 3 substituents selected from substituent group b,
a C2-C3 alkenyl group optionally substituted with 1 to 3 substituents selected from substituent group b,
a C3-C6 cycloalkyl group optionally substituted with 1 to 3 substituents selected from substituent group b,
a C5-C8 bicycloalkyl group optionally substituted with 1 to 3 substituents selected from substituent group b,
a C5-C8 azabicycloalkyl group optionally substituted with 1 to 3 substituents selected from substituent group b,
a C5-C8 oxabicycloalkyl group optionally substituted with 1 to 3 substituents selected from substituent group b,
a 3-6-membered nitrogen-containing saturated heterocyclic group optionally substituted with 1 to 3 substituents selected from substituent group b, or
a 5-6-membered nitrogen-containing aromatic heterocyclic group optionally substituted with 1 to 3 substituents selected from substituent group b,
the substituent group a consists of
a halogen atom, a cyano group, a C2-C3 alkenyl group, a C3-C6 cycloalkyl group, a C1-C3 alkyl group optionally substituted with 1 to 3 fluorine atoms, a C1-C3 alkoxy group optionally substituted with 1 to 3 fluorine atoms, a C1-C4 alkylene group, a C2-C4 alkanediyl group (including a 1,3-butadiene-1,4-diyl group), a C1-C3 oxyalkylene group, and a cyclopropylacethylene group, and
the substituent group b consists of
a hydroxyl group, a halogen atom, a hydroxy C1-C4 alkyl group,
a C3-C6 cycloalkyl group, a C3-C6 cycloalkylmethyl group, a C1-C3 alkoxy group, a C1-C3 alkyl group optionally substituted with 1 to 3 fluorine atoms, and a 5-membered oxygen-containing saturated heterocyclic group.

2. A compound of the general formula (1a) or a pharmaceutically acceptable salt thereof: 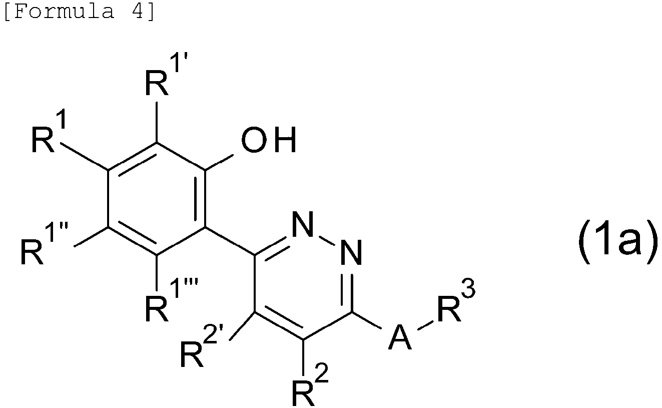 wherein
A is a nitrogen atom,
R¹ is a halogen atom, a cyano group, a C1-C3 alkyl group optionally substituted with 1 to 3 fluorine atoms, or a C1-C3 alkoxy group optionally substituted with 1 to 3 fluorine atoms, R¹' is a hydrogen atom, a halogen atom, or a C1-C3 alkyl group, or
R¹ and R¹' are bonded to each other to form a C1-C4 alkylene group optionally substituted with 1 to 3 substituents selected from substituent group a', thereby constituting a ring structure,
R¹" is a hydrogen atom, or a halogen atom,
R¹‴ is a hydrogen atom, a halogen atom, a cyano group, or a methyl group,
R² and A are bonded to each other to form any partial structure selected from the group shown below: 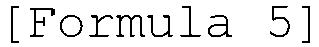 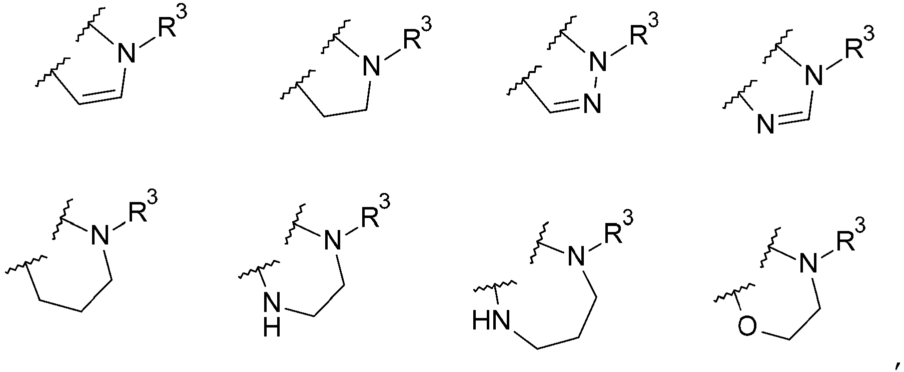
wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group a',
R²' is a hydrogen atom, or a C1-C3 alkyl group, or
R²' and R² are bonded to each other to form -NH-(CH₂)n- (n is an integer of 1, 2 or 3), thereby constituting a ring structure,
R³ is
a C1-C3 alkyl group optionally substituted with 1 to 3 substituents selected from substituent group b',
a C3-C6 cycloalkyl group optionally substituted with 1 to 3 substituents selected from substituent group b',
a C5-C8 bicycloalkyl group optionally substituted with 1 to 3 substituents selected from substituent group b',
a C5-C8 azabicycloalkyl group optionally substituted with 1 to 3 substituents selected from substituent group b',
a C5-C8 oxabicycloalkyl group optionally substituted with 1 to 3 substituents selected from substituent group b',
a 3-6-membered nitrogen-containing saturated heterocyclic group optionally substituted with 1 to 3 substituents selected from substituent group b', or
a 5-6-membered nitrogen-containing aromatic heterocyclic group optionally substituted with 1 to 3 substituents selected from substituent group b',
the substituent group a' consists of
a halogen atom, a cyano group, a C3-C6 cycloalkyl group, a C1-C3 alkyl group optionally substituted with 1 to 3 fluorine atoms, a C1-C3 alkoxy group optionally substituted with 1 to 3 fluorine atoms, a C1-C4 alkylene group, a C2-C4 alkanediyl group (including a 1,3-butadiene-1,4-diyl group), a C1-C3 oxyalkylene group, and a cyclopropylacethylene group, and
the substituent group b' consists of
a hydroxyl group, a halogen atom, a C1-C3 alkyl group, a hydroxy C1-C3 alkyl group, a C3-C6 cycloalkyl group, a C1-C3 alkoxy group, and a 5-membered oxygen-containing saturated heterocyclic group.

3. A compound of the general formula (1b) or a pharmaceutically acceptable salt thereof: 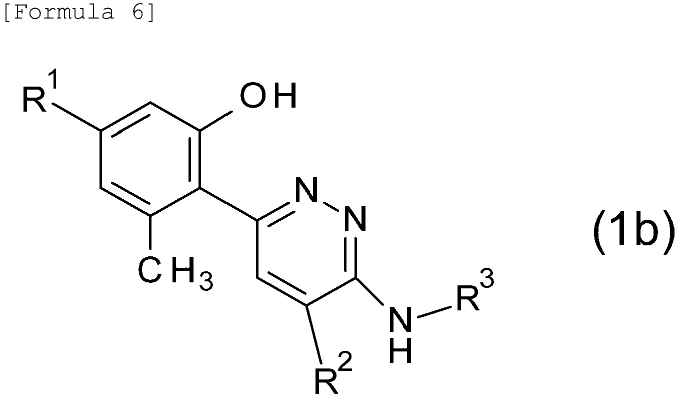 wherein
R¹ is a halogen atom, a C1-C3 alkyl group optionally substituted with 1 to 3 fluorine atoms, or a C1-C3 alkoxy group optionally substituted with 1 to 3 fluorine atoms,
R² and the nitrogen atom are bonded to each other to form any partial structure selected from the group shown below: 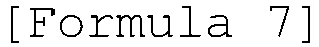 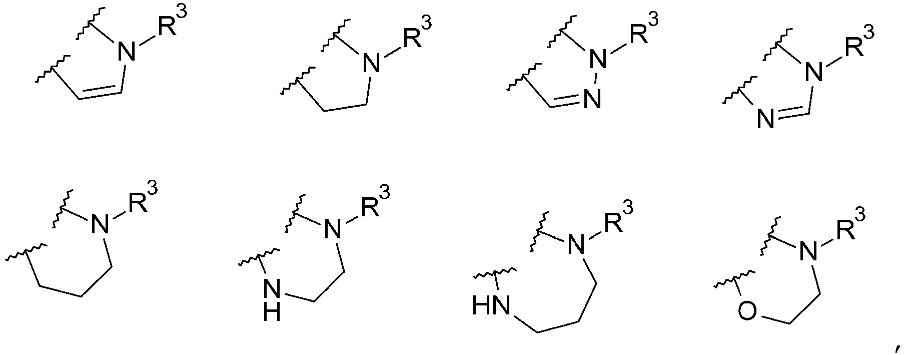
wherein the left end of the partial structure is a bond to R², and the right end of the partial structure is a bond to the nitrogen atom, and for convenience, the partial structure is shown as being substituted with R³, and optionally, the partial structure may be substituted with 1 to 3 substituents selected from substituent group a",
R³ is
a C3-C6 cycloalkyl group optionally substituted with 1 to 3 substituents selected from substituent group b",
a C6-C7 bicycloalkyl group optionally substituted with 1 to 3 substituents selected from substituent group b",
a 3-6-membered nitrogen-containing saturated heterocyclic group optionally substituted with 1 to 3 substituents selected from substituent group b", or
a 5-6-membered nitrogen-containing aromatic heterocyclic group optionally substituted with 1 to 3 substituents selected from substituent group b'',
the substituent group a" consists of
a halogen atom, a cyano group, a C1-C3 alkyl group, a C1-C3 alkoxy group, a C1-C3 alkyl group optionally substituted with 1 to 3 fluorine atoms, a C1-C3 alkoxy group optionally substituted with 1 to 3 fluorine atoms, a C1-C4 alkylene group, and a C1-C3 oxyalkylene group, and
the substituent group b" consists of
a hydroxyl group, a halogen atom, and a C1-C3 alkyl group.

4. The compound according to claim 3, or a pharmaceutically acceptable salt thereof, wherein R¹ is a trifluoromethyl group, a chlorine atom, a difluoromethoxy group, or a trifluoromethoxy group.

5. The compound according to claim 3 or 4, or a pharmaceutically acceptable salt thereof, wherein R² and the nitrogen atom are bonded to each other to form any partial structure selected from the group shown below: 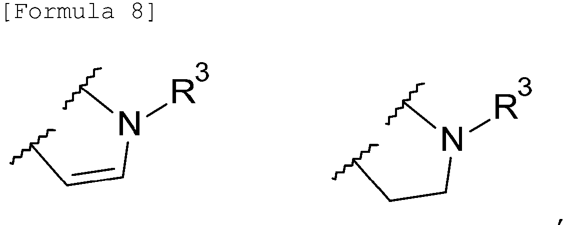 wherein the left end of the partial structure is a bond to R², and the right end of the partial structure is a bond to the nitrogen atom, and for convenience, the partial structure is shown as being substituted with R³, and optionally, the partial structure may be substituted with 1 to 3 substituents selected from substituent group a".

6. The compound according to any one of claims 3 to 5, or a pharmaceutically acceptable salt thereof, wherein the partial structure may be substituted with 1 to 3 substituents selected from the group consisting of a cyano group, a fluorine atom, a methyl group, a methoxy group, a difluoromethoxy group, a propylene group, a 1-oxyethylene group, and a 2-oxyethylene group.

7. The compound according to any one of claims 3 to 6, or a pharmaceutically acceptable salt thereof, wherein R³ is any substituent selected from the following substituent group:

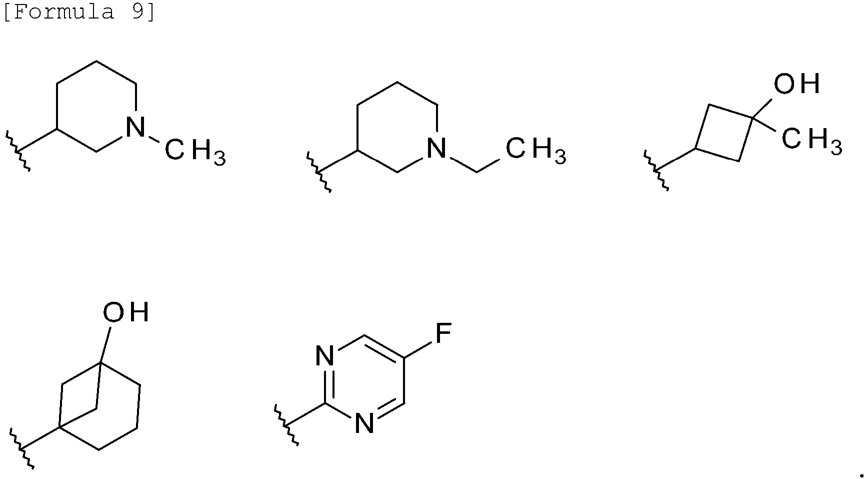

8. Any compound selected from the following group, or a pharmaceutically acceptable salt thereof:
3-methyl-2-{7-[(3R)-1-methylpiperidin-3-yl]-6,7-dihydro-5H-pyrrolo[2,3-c]pyridazin-3-yl}-5-(trifluoromethyl)phenol,
3-methyl-2-{7-[(3R)-1-methylpiperidin-3-yl]-7H-pyrrolo[2,3-c]pyridazin-3-yl}-5-(trifluoromethyl)phenol,
2-{7-[(1s,3s)-3-hydroxy-3-methylcyclobutyl]-7H-pyrrolo[2,3-c]pyridazin-3-yl}-3-methyl-5-(trifluoromethyl)phenol,
2-{7-[(1s,3s)-3-hydroxy-3-methylcyclobutyl]-5-methyl-7H-pyrrolo[2,3-c]pyridazin-3-yl}-3-methyl-5-(trifluoromethyl)phenol,
2-{5-fluoro-7-[(1s,3s)-3-hydroxy-3-methylcyclobutyl]-7H-pyrrolo[2,3-c]pyridazin-3-yl}-3-methyl-5-(trifluoromethyl)phenol,
5-chloro-2-{7-[(1s,3s)-3-hydroxy-3-methylcyclobutyl]-5-methyl-7H-pyrrolo[2,3-c]pyridazin-3-yl}-3-methylphenol,
2-{7-[(1s,3s)-3-hydroxy-3-methylcyclobutyl]-5-methoxy-7H-pyrrolo[2,3-c]pyridazin-3-yl}-3-methyl-5-(trifluoromethyl)phenol,
2-{7-[(3R)-1-ethylpiperidin-3-yl]-6,7-dihydro-5H-pyrrolo[2,3-c]pyridazin-3-yl}-3-methyl-5-(trifluoromethyl)phenol,
2-{8-[(1s,3s)-3-hydroxy-3-methylcyclobutyl]-5,6,7,8-tetrahydrocyclopenta[4,5]pyrrolo[2,3-c]pyridazin-3-yl}-3-methyl-5-(trifluoromethyl)phenol,
2-{5-(difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol,
5-(difluoromethoxy)-2-{7-[(3R)-1-ethylpiperidin-3-yl]-6,7-dihydro-5H-pyrrolo[2,3-c]pyridazin-3-yl}-3-methylphenol,
2-{7-[(3R)-1-ethylpiperidin-3-yl]-6,7-dihydro-5H-pyrrolo[2,3-c]pyridazin-3-yl}-3-methyl-5-(trifluoromethoxy)phenol,
5-{3-[2-hydroxy-6-methyl-4-(trifluoromethyl)phenyl]-5-methoxy-7H-pyrrolo[2,3-c]pyridazin-7-yl}bicyclo[3.1.1]heptan-1-ol,
5-chloro-2-{5-fluoro-7-[(1s,3s)-3-hydroxy-3-methylcyclobutyl]-7H-pyrrolo[2,3-c]pyridazin-3-yl}-3-methylphenol,
5-chloro-2-{7-[(1s,3s)-3-hydroxy-3-methylcyclobutyl]-5-methoxy-7H-pyrrolo[2,3-c]pyridazin-3-yl}-3-methylphenol,
5-chloro-2-{5-(difluoromethoxy)-7-[(1s,3s)-3-hydroxy-3-methylcyclobutyl]-7H-pyrrolo[2,3-c]pyridazin-3-yl}-3-methylphenol,
2-{8-[(1s,3s)-3-hydroxy-3-methylcyclobutyl]-7,8-dihydro-6H-furo[2',3':4,5]pyrrolo[2,3-c]pyridazin-3-yl}-3-methyl-5-(trifluoromethyl)phenol,
7-[(1s,3s)-3-hydroxy-3-methylcyclobutyl]-3-[2-hydroxy-6-methyl-4-(trifluoromethyl)phenyl]-7H-pyrrolo[2,3-c]pyridazine-5-carbonitrile,
2-[7-(5-fluoropyrimidin-2-yl)-7H-pyrrolo[2,3-c]pyridazin-3-yl]-3-methyl-5-(trifluoromethyl)phenol,
2-[(4bR,7aR)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol,
2-[( 4bS,7aS)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, and
2-{8-[(1s,3s)-3-hydroxy-3-methylcyclobutyl]-7,8-dihydro-5H-furo[3',4':4,5]pyrrolo[2,3-c]pyridazin-3-yl}-3-methyl-5-(trifluoromethyl)phenol.

9. 2-{5-Fluoro-7-[(1s,3s)-3-hydroxy-3-methylcyclobutyl]-7H-pyrrolo[2,3-c]pyridazin-3-yl}-3-methyl-5-(trifluoromethyl)phenol,
2-{5-(difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol,
2-{8-[(1s,3s)-3-hydroxy-3-methylcyclobutyl]-7,8-dihydro-6H-furo[2',3':4,5]pyrrolo[2,3-c]pyridazin-3-yl}-3-methyl-5-(trifluoromethyl)phenol,
2-[(4bR,7aR)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol,
2-[(4bS,7aS)-8-(5-fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, or
2-{8-[(1s,3s)-3-hydroxy-3-methylcyclobutyl]-7,8-dihydro-5H-furo[3',4':4,5]pyrrolo[2,3-c]pyridazin-3-yl}-3-methyl-5-(trifluoromethyl)phenol, or a pharmaceutically acceptable salt thereof.

10. 2-{5-Fluoro-7-[(1s,3s)-3-hydroxy-3-methylcyclobutyl]-7H-pyrrolo[2,3-c]pyridazin-3-yl}-3-methyl-5-(trifluoromethyl)phenol, or a pharmaceutically acceptable salt thereof.

11. 2-{5-(Difluoromethoxy)-7-[(1s,3s)-3-hydroxy-3-methylcyclobutyl]-7H-pyrrolo[2,3-c]pyridazin-3-yl}-3-methyl-5-(trifluoromethyl)phenol, or a pharmaceutically acceptable salt thereof.

12. 2-{8-[(1s,3s)-3-Hydroxy-3-methylcyclobutyl]-7,8-dihydro-6H-furo[2',3':4,5]pyrrolo[2,3-c]pyridazin-3-yl}-3-methyl-5-(trifluoromethyl)phenol, or a pharmaceutically acceptable salt thereof.

13. 2-[(4bR,7aR)-8-(5-Fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, or a pharmaceutically acceptable salt thereof.

14. 2-[(4bS,7aS)-8-(5-Fluoropyrimidin-2-yl)-6,7,7a,8-tetrahydro-4bH-furo[2',3':4,5]pyrrolo[2,3-c]pyridazin-3-yl]-3-methyl-5-(trifluoromethyl)phenol, or a pharmaceutically acceptable salt thereof.

15. 2-{8-[(1s,3s)-3-Hydroxy-3-methylcyclobutyl]-7,8-dihydro-5H-furo[3',4':4,5]pyrrolo[2,3-c]pyridazin-3-yl}-3-methyl-5-(trifluoromethyl)phenol, or a pharmaceutically acceptable salt thereof.

16. A compound of the general formula (1c) or a pharmaceutically acceptable salt thereof: 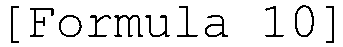 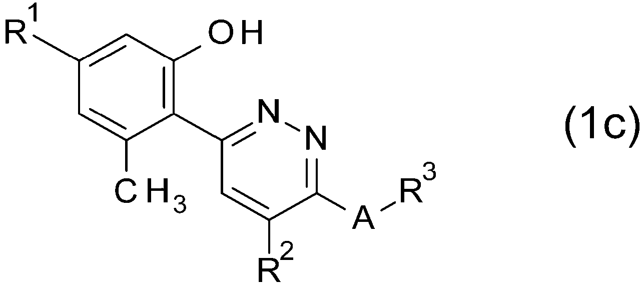 wherein
A is a single bond, or a carbon atom,
R¹ is a halogen atom, a C1-C3 alkyl group optionally substituted with 1 to 3 fluorine atoms, or a C1-C3 alkoxy group optionally substituted with 1 to 3 fluorine atoms,
R² is a hydrogen atom, or a C1-C3 alkyl group optionally substituted with 1 to 3 substituents selected from substituent group c, or
R² and A are bonded to each other to form any partial structure selected from the group shown below: 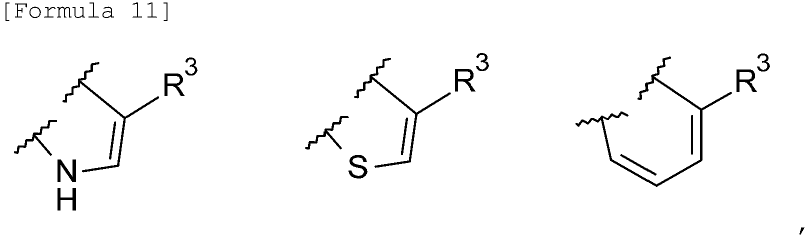
wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group c,
R³ is
a C3-C6 cycloalkyl group optionally substituted with 1 to 3 substituents selected from substituent group d,
a C6-C7 bicycloalkyl group optionally substituted with 1 to 3 substituents selected from substituent group d, or
a 3-6-membered nitrogen-containing saturated heterocyclic group optionally substituted with 1 to 3 substituents selected from substituent group d,
the substituent group c consists of
a halogen atom, a C1-C3 alkyl group optionally substituted with 1 to 3 fluorine atoms, a C1-C3 alkoxy group optionally substituted with 1 to 3 fluorine atoms, a C1-C4 alkylene group, and a C1-C3 oxyalkylene group, and
the substituent group d consists of
a hydroxyl group, and a C1-C3 alkyl group.

17. The compound according to claim 16, or a pharmaceutically acceptable salt thereof, wherein R¹ is a trifluoromethyl group, a chlorine atom, a difluoromethoxy group, or a trifluoromethoxy group.

18. The compound according to claim 16, or a pharmaceutically acceptable salt thereof, wherein R¹ is a trifluoromethyl group.

19. The compound according to any one of claims 16 to 18, or a pharmaceutically acceptable salt thereof, wherein R² is a hydrogen atom.

20. The compound according to any one of claims 16 to 18, or a pharmaceutically acceptable salt thereof, wherein R² and A are bonded to each other to form any partial structure selected from the group shown below: 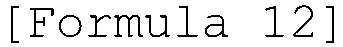 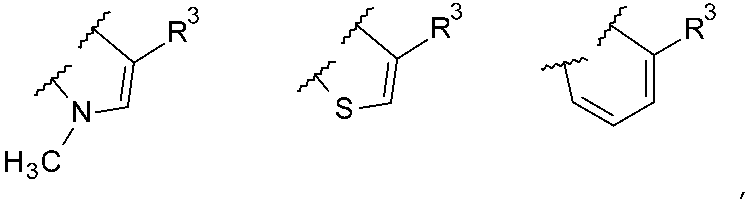 wherein the left end of the partial structure is a bond to R², and the right end of the partial structure is a bond to A, and for convenience, the partial structure is shown as being substituted with R³, and optionally, the partial structure may be substituted with 1 to 3 substituents selected from substituent group c.

21. The compound according to any one of claims 16 to 20, or a pharmaceutically acceptable salt thereof, wherein R³ is any substituent selected from the following substituents:

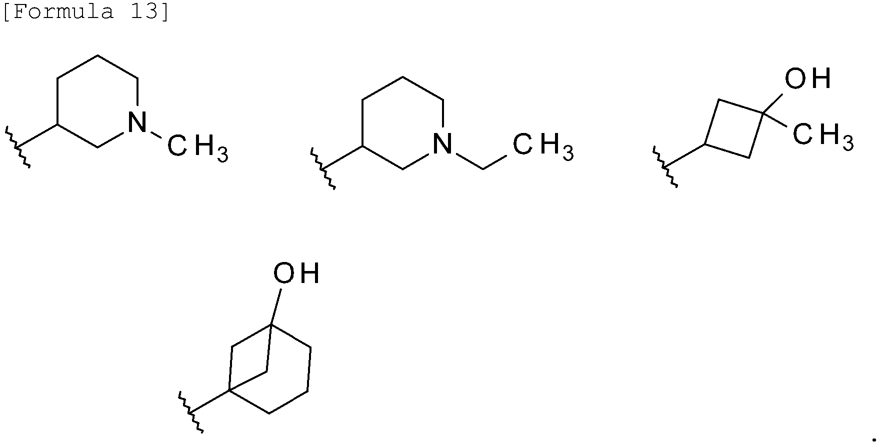

22. Any compound selected from the following group, or a pharmaceutically acceptable salt thereof:
3-methyl-2-{6-[(1-methylpiperidin-3-yl)methyl]pyridazin-3-yl}-5-(trifluoromethyl)phenol,
3-methyl-2-[5-methyl-7-(1-methylpiperidin-3-yl)-5H-pyrrolo[3,2-c]pyridazin-3-yl]-5-(trifluoromethyl)phenol,
3-methyl-2-[7-(1-methylpiperidin-3-yl)thieno[3,2-c]pyridazin-3-yl]-5-(trifluoromethyl)phenol,
3-methyl-2-{8-[(3R)-1-methylpiperidin-3-yl]cinnolin-3-yl}-5-(trifluoromethyl)phenol, and
2-{8-[(3R)-1-ethylpiperidin-3-yl]cinnolin-3-yl}-3-methyl-5-(trifluoromethyl)phenol.

23. A pharmaceutical composition comprising the compound according to any one of claims 1 to 22, or a pharmaceutically acceptable salt thereof, as an active ingredient.

24. The pharmaceutical composition according to claim 23, for inhibiting NLRP3 inflammasome activation.

25. The pharmaceutical composition according to claim 23, for suppressing inflammatory cytokine production mediated by NLRP3 inflammasome activation.

26. The pharmaceutical composition according to claim 23, for suppressing inflammation reaction mediated by NLRP3 inflammasome activation.

27. The pharmaceutical composition according to claim 23, for treating an autoinflammatory disease, an autoimmune disease, and/or an inflammatory disease.

28. The pharmaceutical composition according to claim 27, wherein the autoinflammatory disease, autoimmune disease, and/or inflammatory disease is a disease selected from the diseases listed below:
cryopyrin-associated periodic syndrome (CAPS), systemic lupus erythematosus (SLE), rheumatoid arthritis, psoriasis, systemic scleroderma (SSc), vasculitis syndrome, systemic amyloidosis, alcoholic liver disease (ALD), alcoholic steatohepatitis (ASH), non-alcoholic steatohepatitis (NASH), viral hepatitis, non-alcoholic fatty liver disease (NAFLD), chronic obstructive pulmonary diseases (COPD), asthma, acute lung injury (ALI), interstitial lung disease (ILD), sarcoidosis, diabetic nephropathy, lupus nephritis, primary hyperoxaluria, interstitial cystitis, inflammatory bowel disease (IBD), gout, pseudogout, osteoarthritis, atherosclerosis, diabetes type 1/type 2, heart failure, SARS-CoV-2 infection, EBV infection, viral encephalitis, glaucoma, optic neuritis, age-related macular degeneration, myeloproliferative neoplasms, leukemia, myelodysplastic syndrome, myelofibrosis, lung cancer, colorectal cancer, tumor lysis syndrome, multiple sclerosis, Alzheimer's disease, Huntington's disease, Parkinson's disease, amyotrophic lateral sclerosis, multiple system atrophy, frontotemporal dementia, epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, ischemic stroke, hemorrhagic stroke, and traumatic brain injury.

29. The pharmaceutical composition according to claim 23, for treating multiple sclerosis, Alzheimer's disease, Huntington's disease, Parkinson's disease, amyotrophic lateral sclerosis, multiple system atrophy, frontotemporal dementia, epilepsy, major depressive disorder, schizophrenia, insomnia, autism spectrum disorder, bipolar disorder, panic disorder, anxiety disorder, phobia, obsessive-compulsive disorder, posttraumatic stress disorder (PTSD), postoperative delirium, substance related disorder, eating disorder, ischemic stroke, hemorrhagic stroke, or traumatic brain injury.

30. The pharmaceutical composition according to claim 23, for treating multiple sclerosis, Alzheimer's disease, Parkinson's disease, multiple system atrophy, major depressive disorder, schizophrenia, or autism spectrum disorder.

31. The pharmaceutical composition according to claim 23, for treating multiple sclerosis.

32. The pharmaceutical composition according to claim 23, for treating Alzheimer's disease.

33. The pharmaceutical composition according to claim 23, for treating Parkinson's disease.

34. The pharmaceutical composition according to claim 23, for treating multiple system atrophy.

35. The pharmaceutical composition according to claim 23, for treating major depressive disorder.

36. The pharmaceutical composition according to claim 23, for treating schizophrenia.

37. The pharmaceutical composition according to claim 23, for treating autism spectrum disorder.

38. Use of the compound according to any one of claims 1 to 22, or a pharmaceutically acceptable salt thereof, for the manufacture of a pharmaceutical composition.

39. The use according to claim 23, wherein the pharmaceutical composition is a pharmaceutical composition for treating an autoinflammatory disease, an autoimmune disease, and/or an inflammatory disease.

40. A formulation intended for administration to a mammal, for treating an autoinflammatory disease, an autoimmune disease, and/or an inflammatory disease, comprising an effective amount of the compound according to any one of claims 1 to 22, or a pharmaceutically acceptable salt thereof.

41. A method for treating an autoinflammatory disease, an autoimmune disease, and/or an inflammatory disease with the compound according to any one of claims 1 to 22, or a pharmaceutically acceptable salt thereof.
